(19) Europäisches Patentamt
European Patent Office
Office européen des brevets

(11) Publication number: **0 044 209**
**B1**

(12) # EUROPEAN PATENT SPECIFICATION

(45) Date of publication of patent specification: **31.10.84**

(21) Application number: **81303176.2**

(22) Date of filing: **10.07.81**

(51) Int. Cl.$^3$: **C 07 D 239/42,**
C 07 D 251/12,
C 07 D 491/048,
C 07 D 239/47,
C 07 C 143/828,
C 07 D 239/52,
C 07 D 251/46,
C 07 C 149/30,
C 07 D 239/70,
C 07 D 491/052 //
(C07D491/048, 239/00,
307/00),(C07D491/052, 239/00,
311/00)

(54) **Herbicidal sulfonamides.**

(30) Priority: **11.07.80 US 168892**
**12.05.81 US 259982**
**11.07.80 US 168348**
**29.04.81 US 254256**
**11.07.80 US 168350**
**27.05.81 US 264661**
**11.07.80 US 168349**
**27.04.81 US 253407**
**11.07.80 US 168352**
**12.05.81 US 259981**
**11.07.80 US 168893**
**27.04.81 US 253406**

(43) Date of publication of application:
**20.01.82 Bulletin 82/03**

(45) Publication of the grant of the patent:
**31.10.84 Bulletin 84/44**

(84) Designated Contracting States:
**AT BE CH DE FR GB IT LI LU NL SE**

**The file contains technical information submitted after the application was filed and not included in this specification**

(73) Proprietor: **E.I. DU PONT DE NEMOURS AND COMPANY**
**1007 Market Street**
**Wilmington Delaware 19898 (US)**

(72) Inventor: **Farnham, William Brown**
**2519 West 18th Street**
**Wilmington Delaware 19806 (US)**
Inventor: **Levitt, George**
**3218 Romilly Road**
**Wilmington Delaware 19810 (US)**
Inventor: **Schwing, Gregory Wayne**
**RD 1, Box 191 C**
**Lincoln University Pennsylvania 19352 (US)**
Inventor: **Sauers, Richard Frank**
**504 Revere Court**
**Hockessin Delaware 19707 (US)**
Inventor: **Petersen, Wallace Christian**
**RD 1, Box 104**
**Hockessin Delaware 19707 (US)**

(74) Representative: **Hildyard, Edward Martin et al**
**Frank B. Dehn & Co. European Patent Attorneys**
**Imperial House 15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 044 209 B1

(58) References cited:
EP-A-0 001 485
EP-A-0 015 683
EP-A-0 023 140
US-A-4 120 691
US-A-4 127 405
US-A-4 190 432

**Description**

Background of the Invention

This invention relates to *o*-aminosulfonylphenyl acetic acid derivatives which are useful as plant growth regulants and herbicides.

French Patent No. 1,468,747 discloses the following *para*-substituted phenylsulfonamides, useful as antidiabetic agents:

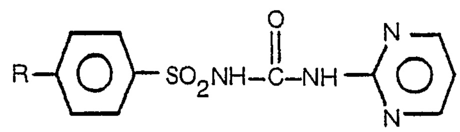

where R = H, halogen, $CF_3$ or alkyl.

Logemann et al., Chem. Ab., *53,* 18052 g (1959), disclose a number of sulfonamides, including uracil derivatives and those having the formula:

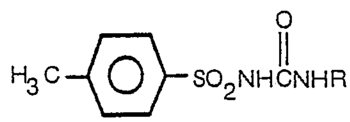

wherein R is butyl, phenyl or 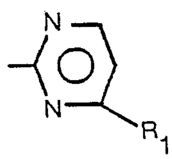

and $R_1$ is hydrogen or methyl. When tested for hypoglycemic effect in rats (oral doses of 25 mg/100 g), the compounds in which R is butyl or phenyl were most potent. The others were of low potency or inactive.

Wojciechowski, J. Acta. Polon. Pharm. *19,* P. 121—5 (1962) [Chem. Ab., *59* 1633 e] describes the synthesis of N-[(2,6-dimethoxypyrimidin-4-yl)aminocarbonyl]-4-methylbenzenesulfonamide:

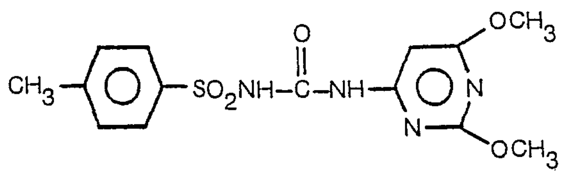

In U.S. Patent 4,127,405, the following compounds are taught to have herbicidal activity:

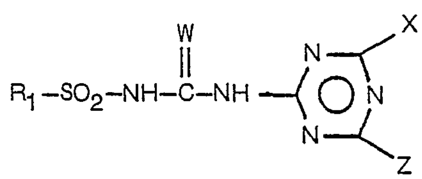 (I)

wherein

$R_1$ is 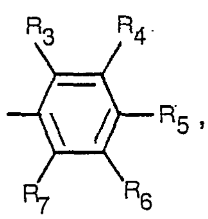 , 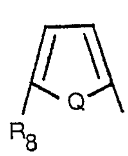 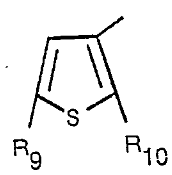 or 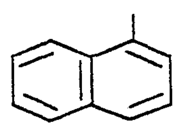 ;

$R_3$ and $R_6$ are independently hydrogen, fluorine, chlorine, bromine, iodine, alkyl of 1—4 carbon atoms, alkoxy of 1—4 carbon atoms, nitro, trifluoromethyl, cyano, $CH_3S(O)_n$— or $CH_3CH_2S(O)_n$—;

$R_4$ is hydrogen, fluorine, chlorine, bromine or methyl;

$R_5$ is hydrogen, fluorine, chlorine, bromine, methyl or methoxy;

$R_7$ is hydrogen, fluorine, chlorine, bromine, alkyl of 1—2 carbon atoms or alkoxy of 1—2 carbon atoms;

$R_8$ is hydrogen, methyl, chlorine or bromine;

$R_9$ and $R_{10}$ are independently hydrogen, methyl, chlorine or bromine;

W and Q are independently oxygen or sulfur;

n is 0, 1 or 2;

X is hydrogen, chlorine, bromine, methyl, ethyl, alkoxy of 1—3 carbon atoms, trifluoromethyl, $CH_3S$— or $CH_3OCH_2$—; and

Z is methyl or methoxy; or their agriculturally suitable salts; provided that:

(a) when $R_5$ is other than hydrogen, at least one of $R_3$, $R_4$, $R_6$ and $R_7$ is other than hydrogen and at least two of $R_3$, $R_4$, $R_6$ and $R_7$ must be hydrogen;

(b) when $R_5$ is hydrogen and all of $R_3$, $R_4$, $R_6$ and $R_7$ are other than hydrogen, then all of $R_3$, $R_4$, $R_6$ and $R_7$ must be either chlorine or methyl; and

(c) when $R_3$ and $R_7$ are both hydrogen, at least one of $R_4$, $R_5$ or $R_6$ must be hydrogen.

In particular, the patent discloses ortho-substituted compounds wherein the substitution is $C_1$—$C_4$ alkyl.

The presence of undesired vegetation causes substantial damage to useful crops, especially agricultural products that satisfy man's basic food needs, such as soybeans, corn, wheat and the like. The current population explosion and concomitant world food shortage demand improvements in the efficiency of producing these crops. Preventing or minimizing the loss of a portion of such valuable crops by killing, or inhibiting the growth of undesired vegetation is one way of improving this efficiency.

A wide variety of materials useful for killing, or inhibiting (controlling) the growth of undesired vegetation is available; such materials are commonly referred to as herbicides. The need exists, however, for effective herbicides that destroy or control weeds while not significantly damaging useful crops.

H. Heterocyclic Chemistry, *8*, 947 (1971) discloses:

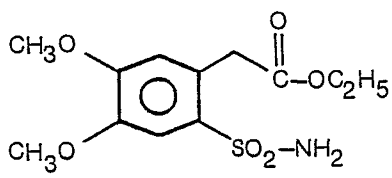

Chemische Berichte, *103*, 1992 (1970) discloses:

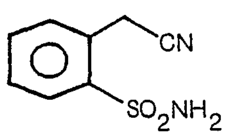

and

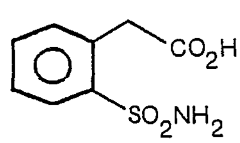

.

## Summary of the Invention

This invention relates to novel compounds of Formula I and their agriculturally suitable salts, suitable agricultural compositions containing them, and their method of use as general and selective pre-emergence and post-emergence herbicides and as plant growth regulants.

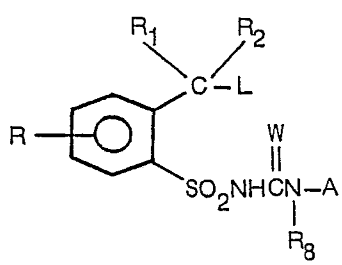

(I)

wherein

L is $CO_2R_{10}$, $CONR_3R_4$, CN, Cl, Br, $NR_3R_4$, $\overset{+}{N}R_3R_4R_4'$, $N(R_4)C(O)R_5$, $N(R_4)C(O)NHR_6$, $N(R_4)C(O)OR_7$, $S(O)_nR_7$, $OR_9$, $SO_2NR_3R_4$, OH, $OC(O)R_{11}$, $OC(O)NHR_{12}$ or $OC(O)OR_{13}$;

R is H, F, Cl, Br, $NO_2$, $CF_3$, $C_1$—$C_3$ alkyl or $C_1$—$C_3$ alkoxy;

$R_1$ is H, Cl or $C_1$—$C_4$ alkyl;

$R_2$ is H, or $CH_3$;

$R_3$ is H, $C_1$—$C_4$ alkyl or $OCH_3$;

$R_4$ is H or $C_1$—$C_4$ alkyl;

$R_3$ and $R_4$ can be taken together to form —$(CH_2)_4$—, —$(CH_2)_5$— or —$(CH_2CH_2)_2O$;

$R_4'$ is H, $CH_3$ or $CH_2CH_3$;

$R_5$ is $C_1$—$C_4$ alkyl optionally substituted with 1—3 atoms of F, Cl or Br, or $C_3$—$C_4$ alkenyl;
$R_6$ is H, $C_1$—$C_4$ alkyl or $C_3$—$C_4$ alkenyl;
$R_7$ is $C_1$—$C_4$ alkyl;
$R_8$ is H, $CH_3$ or $OCH_3$;
$R_9$ is $C_1$—$C_6$ alkyl, $C_3$—$C_4$ alkenyl, $CH_2CF_3$, $CF_2CF_2H$ or $C_5$—$C_6$ cycloalkyl;
$R_{10}$ is H, $C_1$—$C_4$ alkyl, $C_3$—$C_4$ alkenyl, $CH_2CH_2Cl$ or $CH_2CH_2OCH_3$;
$R_{11}$ is H, $C_1$—$C_5$ alkyl, $C_2$—$C_3$ alkenyl, $C_2$—$C_3$ alkynyl, $C_3$—$C_4$ cycloalkyl,

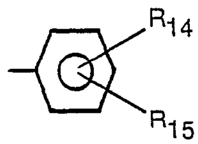 ,

$C_1$—$C_4$ alkyl substituted with 1—4 substituents selected from 0—3 F, 0—3 Cl or 0—3 Br, or $C_2$—$C_3$ alkenyl substituted with 1—3 Cl;
$R_{12}$ is H, $C_1$—$C_6$ alkyl, $C_3$—$C_4$ alkenyl, $C_5$—$C_6$ cycloalkyl,

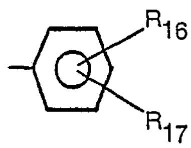 , 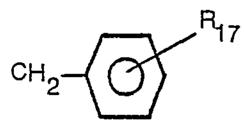

or
$C_5$—$C_6$ cycloalkyl substituted with $CH_3$;

$R_{13}$ is $C_1$—$C_6$ alkyl or 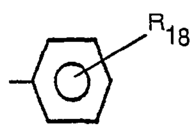 ;

$R_{14}$ and $R_{15}$ are independently H, $NO_2$, $CH_3$, Cl or $OCH_3$;
$R_{16}$ is H, F, Cl, Br, $C_1$—$C_3$ alkyl, $NO_2$, CN, $SO_2CH_3$, $OCH_3$, $SCH_3$, or $CF_3$;
$R_{17}$ is H, Cl or $C_1$—$C_3$ alkyl;
$R_{18}$ is H, $CH_3$ or Cl;
n is 0, 1 or 2;

A is 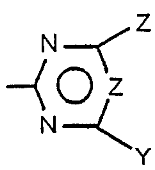 , 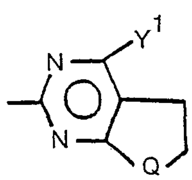 or 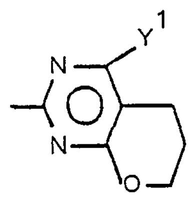

W is O or S;
X is H, Cl, Br, $CH_3$, $CH_2CH_3$, $C_1$—$C_3$ alkoxy, $CF_3$, $SCH_3$ or $CH_2OCH_3$;
Y is $CH_3$ or $OCH_3$;
Z is N, CH, CCl, CBr, CCN, $CCH_3$, $CCH_2CH_3$, $CCH_2CH_2Cl$ or $CCH_2CH=CH_2$;
$Y^1$ is H, $CH_3$, $OCH_3$ or $OCH_2CH_3$;
and
Q is O or $CH_2$;
and their agriculturally suitable salts; provided that:
(1) when $R_1$ is Cl, then L is Cl or Br and $R_2$ is H;
(2) when n is 1, then W is O;
(3) when L is $CONR_3R_4$, then Z is CH or N;
(4) when $R_3$ is $OCH_3$, then $R_4$ is $CH_3$;
(5) the total number of carbon atoms of $R_3$ and $R_4$ is five or less;
(6) when W is S, then $R_8$ is H; and
(7) when L is $SO_2NR_3R_4$, then $R_3$ and $R_4$ are not H.

## Preferred Compounds

Preferred in increasing order for their higher activity and/or more favorable ease of synthesis are:
(1) compounds of the generic scope wherein Z is N, CH, CCl, CBr or $CCH_3$; W is O; and $R_8$ is H or $CH_3$;

(2) compounds of the generic scope wherein L is OH; R is H; $R_1$ and $R_2$ are both $CH_3$; $R_8$ is H or $CH_3$;

A is 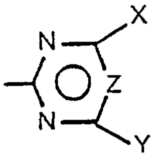 ;

Z is CH or N; X and Y are independently $CH_3$ or $OCH_3$; and W is O;

(3) compounds of *Preferred (1)* wherein L is Cl, Br, $NR_3R_4$, $^+NR_3R_4R_4'$, $CO_2R_{10}$, $S(O)_nR_7$, $OR_9$, $SO_2NR_3R_4$, OH, $OC(O)R_{11}$, $OC(O)NHR_{12}$, $OC(O)OR_{13}$; Z is CH or N; X is $CH_3$ or $OCH_3$; R, $R_1$, $R_2$ are H; and when n is 1 or 2, Z is CH;

(4) compounds of *Preferred (3)* wherein A is 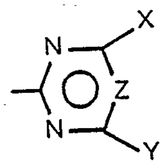 ;

$R_8$ is H; L is Cl, Br, $NR_3R_4$, $CO_2R_{10}$, $S(O)_nR_7$, $OR_9$, $SO_2NR_3R_4$, OH, $OC(O)R_{11}$, $OC(O)NHR_{12}$, $OC(O)OR_{13}$; and $R_{11}$, $R_{12}$ and $R_{13}$ are $C_1$—$C_3$ alkyl;

(5) compounds of *Preferred (4)* wherein L is Cl, Br, $NR_3R_4$, $CO_2R_{10}$, $S(O)_nR_7$, $OR_9$, $SO_2NR_3R_4$ or OH;

(6) compounds of *Preferred (5)* wherein $R_3$ is $C_1$—$C_3$ alkyl or $OCH_3$; $R_4$ is $CH_3$; $R_9$ is $C_1$—$C_4$ alkyl; and $R_{10}$ is $CH_3$ or $CH_2CH_3$;

(7) compounds of *Preferred (6)* wherein $R_7$ and $R_9$ are $CH_3$; and

(8) compounds of *Preferred (7)* wherein L is $CO_2R_{10}$, $S(O)_nR_7$ or $OR_9$.

Our invention includes *inter alia* the groups of compounds defined in claims 16 to 20 hereinafter.

*Specifically preferred* for their highest activity and/or most favorable ease of synthesis are:

2-(Dichloromethyl)-N-[(4-methoxy-6-methylpyrimidin-2-yl)aminocarbonyl]benzenesulfonamide;
2-(Dichloromethyl)-N-[(4,6-dimethylpyrimidin-2-yl)aminocarbonyl]benzenesulfonamide;
2-(Dichloromethyl)-N-[(4-methoxy-6-methyl-1,3,5-triazin-2-yl)aminocarbonyl]benzenesulfonamide;
2-(Chloromethyl)-N-[(4,6-dimethoxy-1,3,5-triazin-2-yl)aminocarbonyl]benzenesulfonamide;
2-(Chloromethyl)-N-[(4,6-dimethoxypyrimidin-2-yl)aminocarbonyl]benzenesulfonamide;
2-(Chloromethyl)-N-[(4,6-dimethylpyrimidin-2-yl)-aminocarbonyl]benzenesulfonamide;
2-(1-Chloroethyl)-N-[(4,6-dimethoxy-1,3-5-triazin-2-yl)aminocarbonyl]benzenesulfonamide;
2-(1-Pyrrolidinylmethyl)-N-[(4,6-dimethoxypyrimidin-2-yl)aminocarbonyl]benzenesulfonamide, hydrochloride;
2-(1-Pyrrolidinylmethyl)-N-[(4,6-dimethoxypyrimidin-2-yl)aminocarbonyl]benzenesulfonamide;
2-[[(4,6-dimethoxypyrimidin-2-yl)aminocarbonyl]aminosulfonyl]benzeneacetic acid, methyl ester;
2-[[(4,-6-dimethylpyrimidin-2-yl)aminocarbonyl]aminosulfonyl]benzeneacetic acid, methyl ester;
2-[[(4-methoxy-6-methylpyrimidin-2-yl)aminocarbonyl]aminosulfonyl]benzeneacetic acid, methyl ester;
2-[[(4,6-dimethoxy-1,3,5-triazin-2-yl)aminocarbonyl]aminosulfonyl]benzeneacetic acid, methyl ester;
2-[[(4-methoxy-6-methyl-1,3,5-triazin-2-yl)aminocarbonyl]aminosulfonyl]benzeneacetic acid, methyl ester;
2-[[(4,6-dimethoxypyrimidin-2-yl)aminocarbonyl]aminosulfonyl]benzeneacetic acid, ethyl ester;
2-[[(4-methoxy-6-methylpyrimidin-2-yl)aminocarbonyl]aminosulfonyl]benzeneacetic acid, ethyl ester;
N-[(4,6-Dimethylpyrimidin-2-yl)aminocarbonyl]-2-methoxymethyl)benzenesulfonamide;
N-[(4-Methoxy-6-methylpyrimidin-2-yl)aminocarbonyl]-2-(methoxymethyl)benzenesulfonamide;
N-[(4,6-Dimethoxypyrimidin-2-yl)aminocarbonyl]-2-(methoxymethyl)benzenesulfonamide;
N-[(4,6-Dimethyl-1,3,5-triazin-2-yl)aminocarbonyl]-2-(methoxymethyl)benzenesulfonamide;
N-[(4-Methoxy-6-methyl-1,3,5-triazin-2-yl)aminocarbonyl]-2-(methoxymethyl)benzenesulfonamide;
N-[(4,6-Dimethoxy-1,3,5-triazin-2-yl)aminocarbonyl]-2-(methoxymethyl)benzenesulfonamide;
N-[(4,6-dimethylpyrimidin-2-yl)aminocarbonyl]-2-(methylthiomethyl)benzenesulfonamide;
N-[(4-Methoxy-6-methylpyrimidin-2-yl)aminocarbonyl]-2-(methylthiomethyl)benzenesulfonamide;
N-[(4,6-Dimethoxypyrimidin-2-yl)aminocarbonyl]-2-(methylthiomethyl)benzenesulfonamide;
N-[(4,6-Dimethyl-1,3,5-triazin-2-yl)aminocarbonyl]-2-(methylthiomethyl)benzenesulfonamide;
N-[(4-Methoxy-6-methyl-1,3,5-triazin-2-yl)aminocarbonyl]-2-(methylthiomethyl)benzenesulfonamide;
N-[(4,6-Dimethoxy-1,3,5-triazin-2-yl)aminocarbonyl]-2-(methylthiomethyl)benzenesulfonamide;
N-[(4,6-Dimethylpyrimidin-2-yl)aminocarbonyl]-2-(methylsulfinylmethyl)benzenesulfonamide;
N-[(4-Methoxy-6-methylpyrimidin-2-yl)aminocarbonyl]-2-(methylsulfonylmethyl)benzenesulfonamide;
N-[(4-Methoxy-6-methylpyrimidin-2-yl)aminocarbonyl]-2-(methylsulfinylmethyl)benzenesulfonamide;
N-[(4,6-Dimethoxypyrimidin-2-yl)aminocarbonyl]-2-(methylsulfonylmethyl)benzenesulfonamide;
N-[(4,6-Dimethoxypyrimidin-2-yl)aminocarbonyl]-2-(methylsulfinylmethyl)benzenesulfonamide;
N-[(4,6-Dimethylpyrimidin-2-yl)aminocarbonyl]-2-(methylsulfonylmethyl)benzenesulfonamide;
N-[(4,6-dimethoxypyrimidin-2-yl)aminocarbonyl]-2-(butoxymethyl)benzenesulfonamide;

N-[(4,6-dimethoxypyrimidin-2-yl)aminocarbonyl]-2-(ethoxymethyl)benzenesulfonamide;
N-[(4,6-dimethoxypyrimidin-2-yl)aminocarbonyl]-2-(1-methylpropyloxymethyl)benzenesulfonamide;
2-[(Dimethylamino)sulfonylmethyl]-N-[(4,6-dimethylpyrimidin-2-yl)aminocarbonyl]benzenesulfonamide;
2-[(Dimethylamino)sulfonylmethyl]-N-[(4,6-dimethoxypyrimidin-2-yl)aminocarbonyl]benzenesulfonamide;
2-[(Dimethylamino)sulfonylmethyl]-N-[(4-methoxy-6-methylpyrimidin-2-yl)aminocarbonyl]benzenesulfonamide;
2-[(Dimethylamino)sulfonylmethyl]-N-[(4,6-dimethyl-1,3,5-triazin-2-yl)aminocarbonyl]benzenesulfonamide;
2-[(Dimethylamino)sulfonylmethyl]-N-[(4,6-dimethoxy-1,3,5-triazin-2-yl)aminocarbonyl]benzenesulfonamide;
2-[(Dimethylamino)sulfonylmethyl]-N-[(4-methoxy-6-methyl-1,3,5-triazin-2-yl)aminocarbonyl]benzenesulfonamide;
N-[(4,6-Dimethoxypyrimidin-2-yl)aminocarbonyl]-2-(hydroxymethyl)benzenesulfonamide;
N-[(4,6-Dimethoxy-1,3,5-triazin-2-yl)aminocarbonyl]-2-(hydroxymethyl)benzenesulfonamide;
N-[(4,6-Dimethylpyrimidin-2-yl)aminocarbonyl]-2-(hydroxymethyl)benzenesulfonamide;
N-[(4-methoxy-6-methylpyrimidin-2-yl)aminocarbonyl]-2-(hydroxymethyl)benzenesulfonamide;
N-[(4,6-Dimethyl-1,3,5-triazin-2-yl)aminocarbonyl]-2-(hydroxymethyl)benzenesulfonamide; and
N-[(4-Methoxy-6-methyl-1,3,5-triazin-2-yl)aminocarbonyl]-2-(hydroxymethyl)benzenesulfonamide.

This invention also relates to novel compounds of Formula II which are useful intermediates for the preparation of the herbicidal compounds of Formula I.

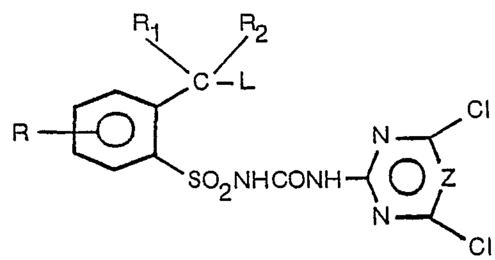

(II)

wherein

$R$, $R_1$, and $R_2$ are as previously defined;
Z is CH or N;
n is 0 or 2; and
L is as previously defined,

provided that:

(1) L is not OH;
(2) when $R_3$ is $OCH_3$, then $R_4$ is $CH_3$ and that $R_{10}$ cannot be H;
(3) when $R_1$ is Cl, then L is Cl or Br and $R_2$ is H;
(4) when L is $NR_3R_4$, $^+NR_3R_4R_4'$, or $SO_2NR_3R_4$, then $R_3$ or $R_4$ cannot be H;
(5) the total number of carbon atoms of $R_3$ and $R_4$ is five or less.

This invention also relates to novel compounds of Formula III which are useful intermediates for the preparation of the compounds of Formula I.

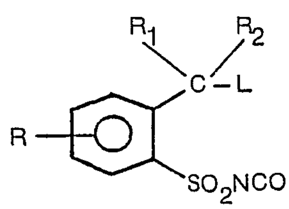

(III)

wherein

$R$, $R_1$, $R_2$, $R_7$ and $R_9$ are as previously defined;
n is 0 or 2;
L is Cl, Br, $CO_2R_{10}$, $SO_2NR_3R_4$, $S(O)_nR_7$ or $OR_9$,

provided that:

(1) when $R_1$ is Cl, then L is Cl or Br and $R_2$ is H;
(2) when L is $SO_2NR_3R_4$, then $R_3$ and $R_4$ cannot be H;
(3) when $R_3$ is $OCH_3$, then $R_4$ is $CH_3$;
(4) $R_{10}$ cannot be H; and
(5) n is 0 or 2.

## Detailed Description of the Invention

As shown in Equation 1, the compounds of Formula I can be prepared by reacting an appropriate 2-aminoheterocycle of Formula IV with an appropriately substituted sulfonyl isocyanate of Formula III; wherein L is Cl, Br, $CO_2R_{10}$, $SO_2NR_3R_4$, $S(O)_nR_7$, $OR_9$;
n is 0 or 2; R, $R_1$, $R_2$, $R_3$, $R_4$, $R_7$, $R_8$, $R_9$, $R_{10}$ and A being as previously defined; $R_3$ $R_4$ and $R_{10}$ cannot be H.

EQUATION 1

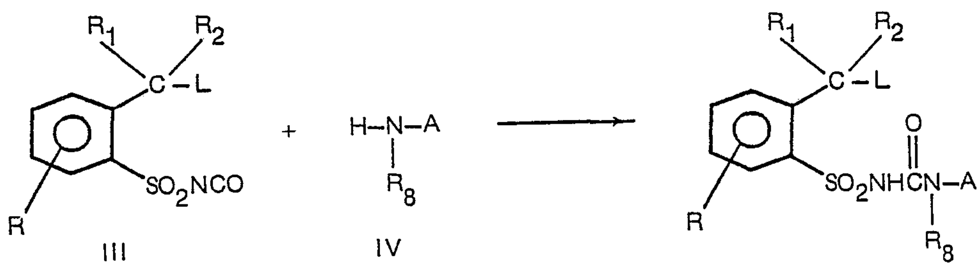

III IV

The reaction is best carried out in inert aprotic organic solvents such as methylene chloride, tetrahydrofuran or acetonitrile, at ambient pressure and temperature. The mode of addition is not critical; however, it is often convenient to add the sulfonyl isocyanate to a stirred suspension of the aminoheterocycle. Since isocyanates usually are liquids, their addition is more easily controlled.

The reaction is generally exothermic. In some cases, the desired product is insoluble in the warm reaction medium and crystallizes from it in pure form. Products soluble in the reaction medium are isolated by evaporation of the solvent, trituration of the solid residue with solvents such as 1-chlorobutane, ethyl ether or pentane and filtration.

The intermediate sulfonyl isocyanate of Formula III can be prepared by reacting corresponding sulfonamides with phosgene in the presence of an alkyl isocyanate such as butyl or cyclohexyl isocyanate at reflux in a solvent such as chlorobenzene, according to the procedure of H. Ulrich and A. A. Y. Sayigh, *Newer Methods of Preparative Organic Chemistry,* Vol. VI, p. 223—241, Academic Press, New York and London. W. Foerst, Ed. In cases where formation of the desired sulfonyl isocyanate is difficult by the above procedure, the preformed sulfonylurea from the reaction of butyl isocyanate with the appropriate sulfonamide is treated with phosgene according to the above reference.

Alternatively, the process of Ulrich and Sayigh can be improved by the addition of a tertiary base to the reaction mixture as shown by Equation 2.

EQUATION 2

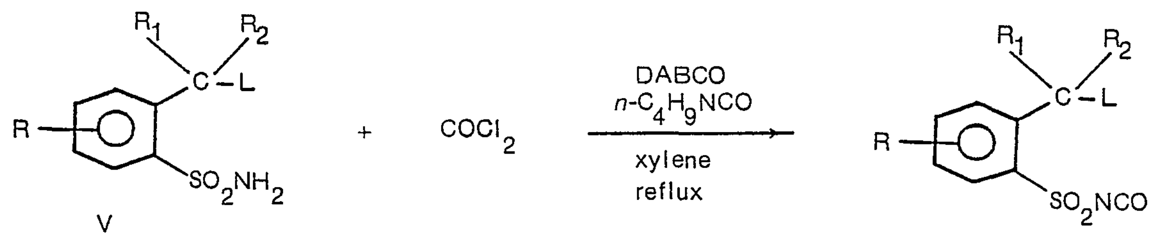


V

wherein L, R, $R_1$, $R_2$ are as described in Equation 1.

III

A mixture of the appropriate benzenesulfonamide V, an alkyl isocyanate and a catalytic amount of 1,4-diaza[2,2,2]bicyclooctane (DABCO) in xylene or other inert solvent of sufficiently high boiling point (e.g. >135°) is heated to approximately 135°. Phosgene is added to the mixture until an excess is present as indicated by a drop in the boiling point. (The mixture is heated further to drive off the excess phosgene). After the mixture is cooled and filtered to remove a small amount of insoluble by-products, the solvent and alkyl isocyanate are distilled *in-vacuo* leaving a residue which is the crude sulfonyl isocyanate III.

Alternatively, the sulfonyl isocyanate of Formula III may be prepared as shown in Equation 3, by the method of Ulrich et al. [J. Org. Chem. *34,* 3200 (1969)].

EQUATION 3

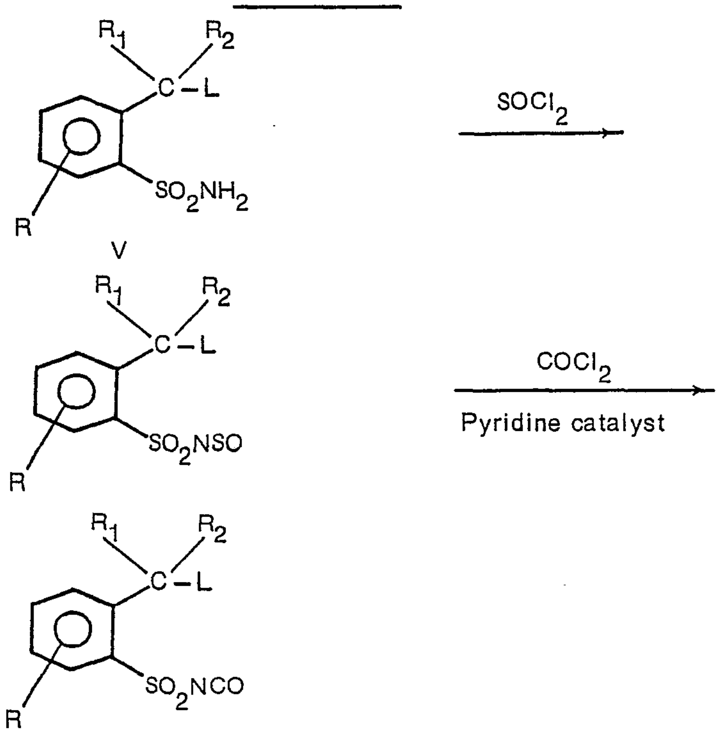


V

III

wherein L, R, $R_1$ and $R_2$ are as described in Equation 1.

The sulfonyl isocyanates of Formula III may also be prepared, as shown in Equation 4, by phosgenation of the butyl ureas of Formula VI.

EQUATION 4

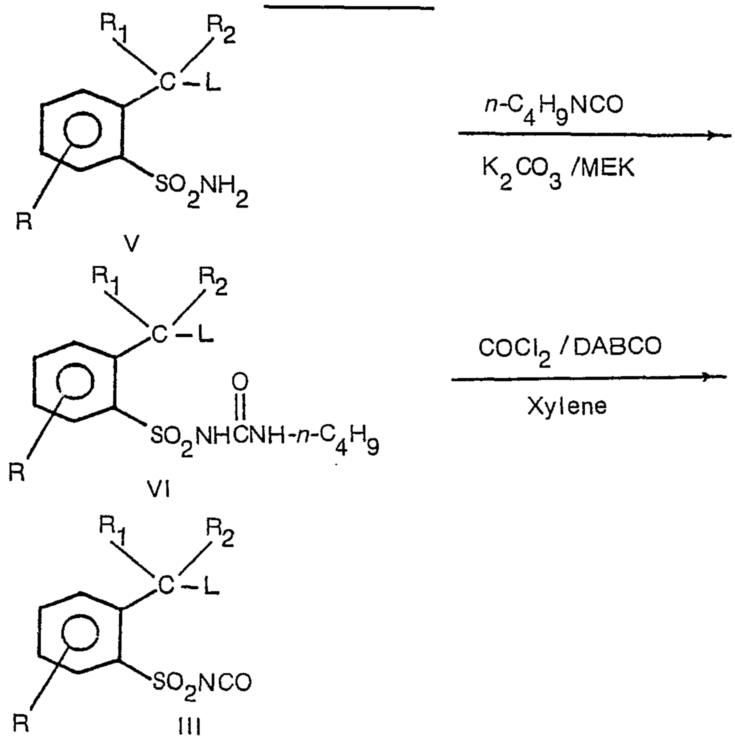


V

VI

III

wherein

L, R, $R_1$, $R_2$, $R_3$ and $R_4$ are as previously described in Equation 1.

The compounds of Formula VI are conveniently prepared by stirring a mixture of the sulfonamides, V, anhydrous potassium carbonate, and *n*-butyl isocyanate in acetone or methyl ethyl ketone at 25—80° until all of the isocyanate has reacted. The products are isolated by quenching in dilute mineral acid and recrystallizing the solid product. The compounds VI are treated with phosgene and a catalytic amount of DABCO in refluxing xylene or chlorobenzene in a manner analogous to that described in Equation 2.

The preparation of these sulfonamides from sulfonyl chlorides with either ammonium hydroxide or anhydrous ammonia is widely reported in the literature, e.g. Crossley et al., *J. Am. Chem. Soc., 60,* 2223 (1938), and P.A. Rossy et al., *J. Org. Chem. 45,* 617 (1980).

For those examples containing a reactive functionality in the position ortho to the sulfonamide moiety, it is convenient to add the sulfonyl chloride to a measured quantity of ammonia (Equation 5) in an inert solvent, e.g. tetrahydrofuran, ethyl acetate, etc., at low temperature (—78° to 0°). Side reactions such as ring formation, elimination, or condensation are thereby substantially avoided.

EQUATION 5

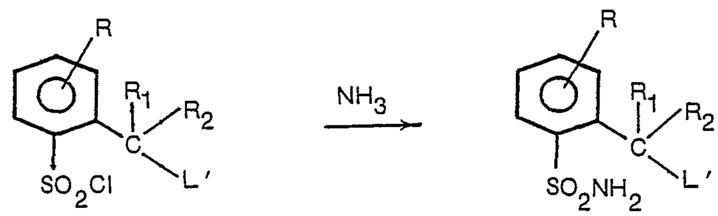


L′ = Cl, Br; and

R, $R_1$ and $R_2$ being as previously defined.

Certain sulfonyl chlorides are best prepared by chlorosulfonation of a substituted benzene according to the teaching of H.T. Clarke et al. *Org. Synth.* Coll. Vol. 1, 2nd Ed. 1941, p. 85. Other benzene-sulfonyl chlorides are best made by diazotization of the appropriate aniline with sodium nitrite in HCl, followed by reaction of the diazonium salt with sulfur dioxide and cuprous chloride in acetic acid according to the teaching of H. L. Yale and F. Sowinski, *J. Org. Chem., 25,* 1824 (1960) or R. A. Abramovitch *J. Org. Chem., 43* 1218 (1978).

Anilines are obtained by reduction of the appropriate nitrobenzenes with iron powder in acetic acid as reported by D. C. Owsley and J. J. Boomfield, *Synthesis,* 118 (1977).

The *o*-alkoxymethyl- or *o*-alkylthiomethylnitrobenzenes VII are prepared via "Williamson Synthesis", according to Equations 6a or 6b.

EQUATION 6

6a)

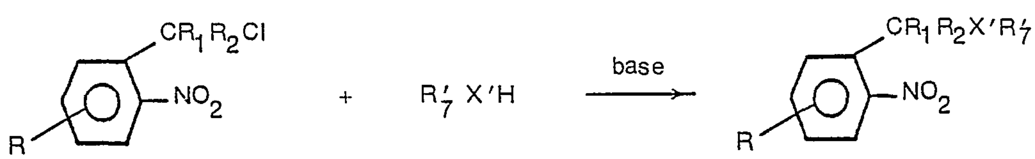


VII VIII

6b)

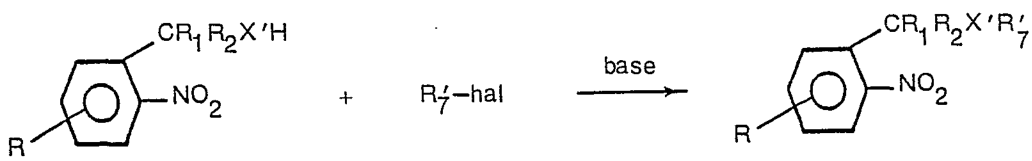


IX VIII

X′ = O or S, $R_7' = R_7$ or $R_9$.

"Williamson Synthesis" has been widely used for the preparation of ethers as reviewed by W. Theilheimer, *Syn. Methods of Org. Chem.,* Vol. VII, p. 112.

Procedure 6a is most useful when $R_7'X'$ is an alkylthio or a primary alkoxy group, and $R^1$ and $R^2$ are both H. The reaction is carried out by mixing *o*-nitrobenzyl chloride and sodium methoxide or sodium alkylmercaptide (for preparation see *Org. Syn.,* Coll. Vol. II, 345) in methanol under reflux or at room temperature, respectively.

Procedure 6a is, however, not applicable in cases where $R_5'X'$ is a branched alkoxy whose corresponding alkoxide anion is a stronger base than it is a nucleophile, and/or either $R_1$ or $R_2$ or both

are alkyl groups. In the presence of a strong base and/or an α-alkyl substituent on *o*-nitrobenzyl chloride the base-catalyzed elimination rather than the desired $SN_2$ displacement occurs, giving rise to 2,2'-dinitrostilbene or other *o*-nitroalkenylbenzenes. In order to avoid these undesired reactions, a more general procedure, 6b, can be employed. The appropriate benzyl alcohol is first treated with base, such as sodium hydride, in an inert organic solvent, typically tetrahydrofuran or glyme, followed by displacement on an alkyl halide, as described by C. A. Brown and D. Barton, *Synthesis,* 434 (1974), and B. A. Stoochnoff and N. L. Benoiton, *Tet. Lett.,* 21 (1973).

Alternatively, *o*-alkoxymethyl or *o*-alkylthio methylbenzenesulfonyl chlorides, XII, can be obtained from an appropriately substituted α-hydroxy-*o*-toluenesulfonic acid-α-sultone, X, via ring-opening reaction with an alkoxide or alkylmercaptide anion as depicted in Equation 7.

EQUATION 7

7a)

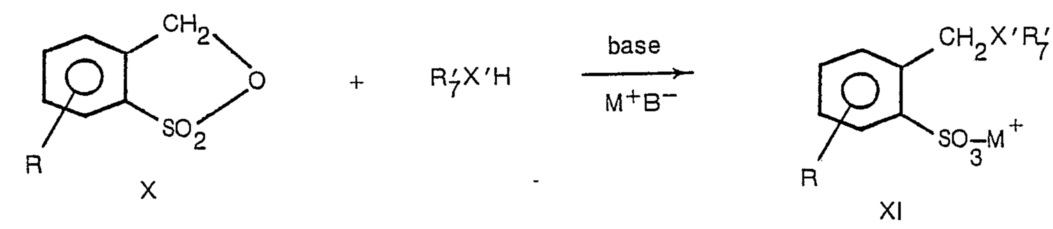

X
XI

7b)

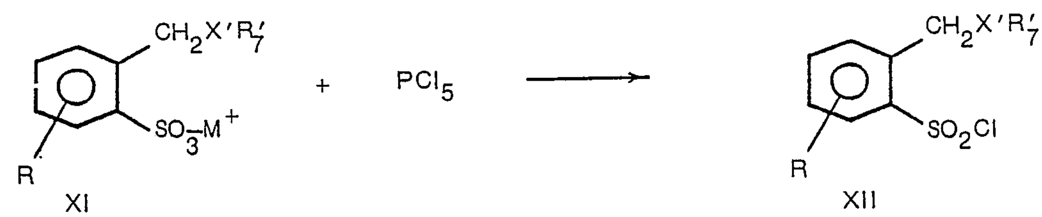

XI
XII

Reaction 7a is closely related to the alkylation of acyloxides and acetamide with sultones as disclosed by J. H. Helberger et al., *Ann., 565,* 22 (1949). Conversion of the sulfonic acid salt to the sulfonyl chloride is then carried out according to the teaching of *Org. Synthesis,* Coll. Vol. IV, 846, 693.

Benzenesulfonamides of Formula XIV can also be derived from compound XIII as illustrated in Equation 8.

EQUATION 8

8a)

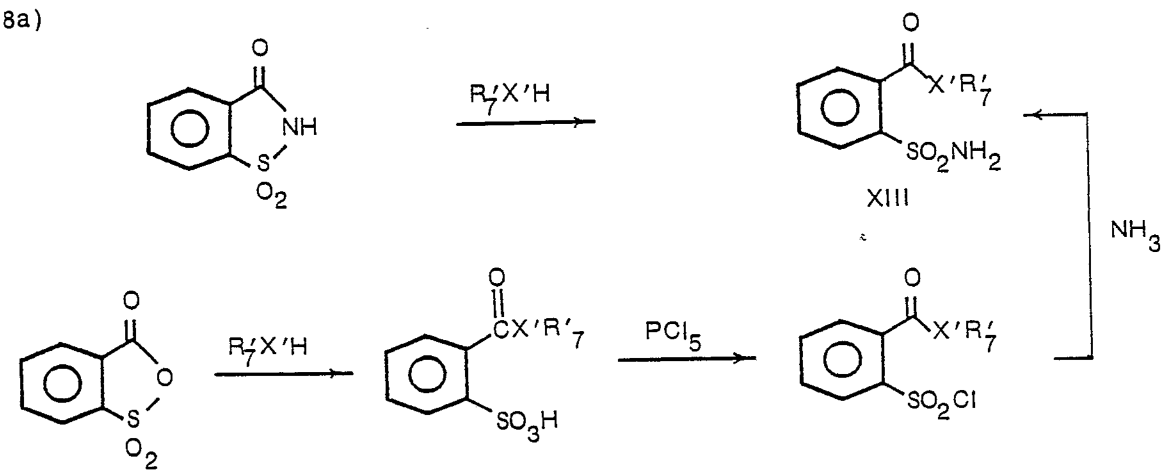

XIII

8b)

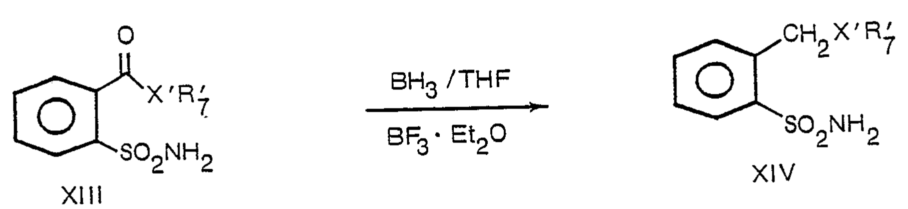

XIII
XIV

Preparation of *o*-sulfamylbenzoic acid esters, XIII, from saccharin or sulfobenzoic acid anhydride is

well known in the art, e.g., B. Loev and M. Kormendy, *J. Org. Chem. 27,* 1703 (1962). The esters XIII, can be readily reduced to the ethers XIV with diborane in a suitable organic solvent, e.g., tetrahydrofuran, in the presence of fifteen folds of boron trifluorideetherate under reflux for 18 hours, as described by R. P. Graber and M. B. Meyers, *J. Org. Chem. 26,* 4773 (1961).

The *o*-alkylsulfinyl- and *o*-alkylsulfonylmethylbenzenesulfonylureas are made from their corresponding *o*-alkylthiomethylbenzenesulfonylureas by means of peroxide oxidation. Reaction of the sulfide-sulfonylurea with aqueous hydrogen peroxide in acetic acid at room temperature for half an hour affords exclusively the sulfoxidesulfonylurea. If the sulfide or sulfoxide is allowed to react for 72 hours under the same conditions, the sulfone is obtained. Oxidation for 20 hours often results in a mixture of both sulfoxide and sulfone, which can be readily separated by column chromatography and eluted with ethyl acetate. Sulfonylureas described above are generally stable under these reaction conditions. They will however, split into heterocyclicamine and *o*-alkylsulfonylbenzenesulfonamide if heated. A general procedure for peroxide oxidation of sulfides to sulfones can be found in the paper by A. M. Van Arendonk and E. C. Kliderer, *J. Am. Chem. Soc., 62,* 3521 (1940).

Substituted benzenesulfonyl chlorides containing an ortho $\alpha$-haloalkyl group can be made by methods known in the art. $\alpha$-Chloroalkyl- and dichloromethylbenzenesulfonyl chlorides can be made by contacting the corresponding alkylbenzenesulfonyl chlorides with dichlorine monoxide in an inert solvent, most conveniently in the temperature range 40—80°. The degree of chlorination is determined by the relative quantity of dichlorine monoxide employed. (F. D. Marsh, U.S. Pat. 4,226,783 issued October 7, 1980).

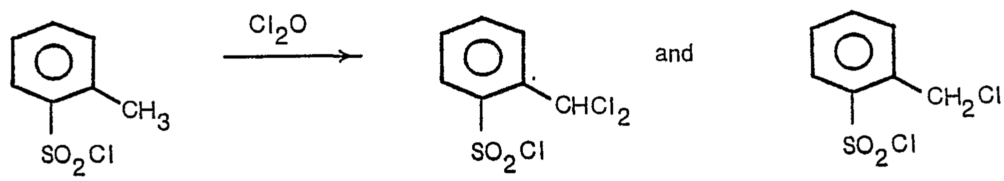


A series of standard functional group transformations may also be used as described (J. F. King, A. Hawson, B. L. Huston, L. J. Danks, and J. Komery, *Can. J. Chem., 49,* 943 (1971) for 2-(chloromethyl)-benzenesulfonyl chloride (Equation 9).

EQUATION 9

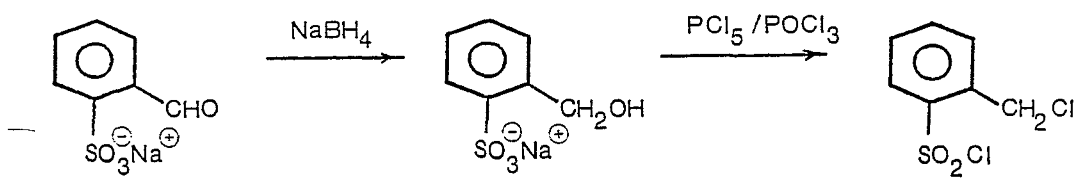


Benzylic bromination using N-bromosuccinimide is useful in the preparation of $\alpha$-bromoalkylbenzenesulfonyl chlorides (Equation 10).

EQUATION 10

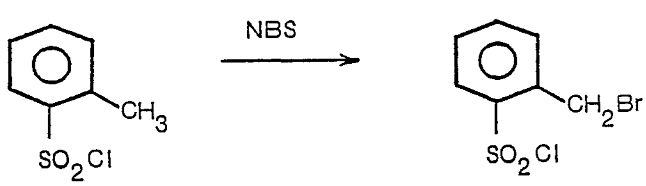


[W. B. Renfrow and M. Devadoss, *J. Org. Chem., 40,* 1525 (1975)]

The sulfonamides of Formula Va can be prepared by the four step reaction sequence shown in Equation 11.

EQUATION 11

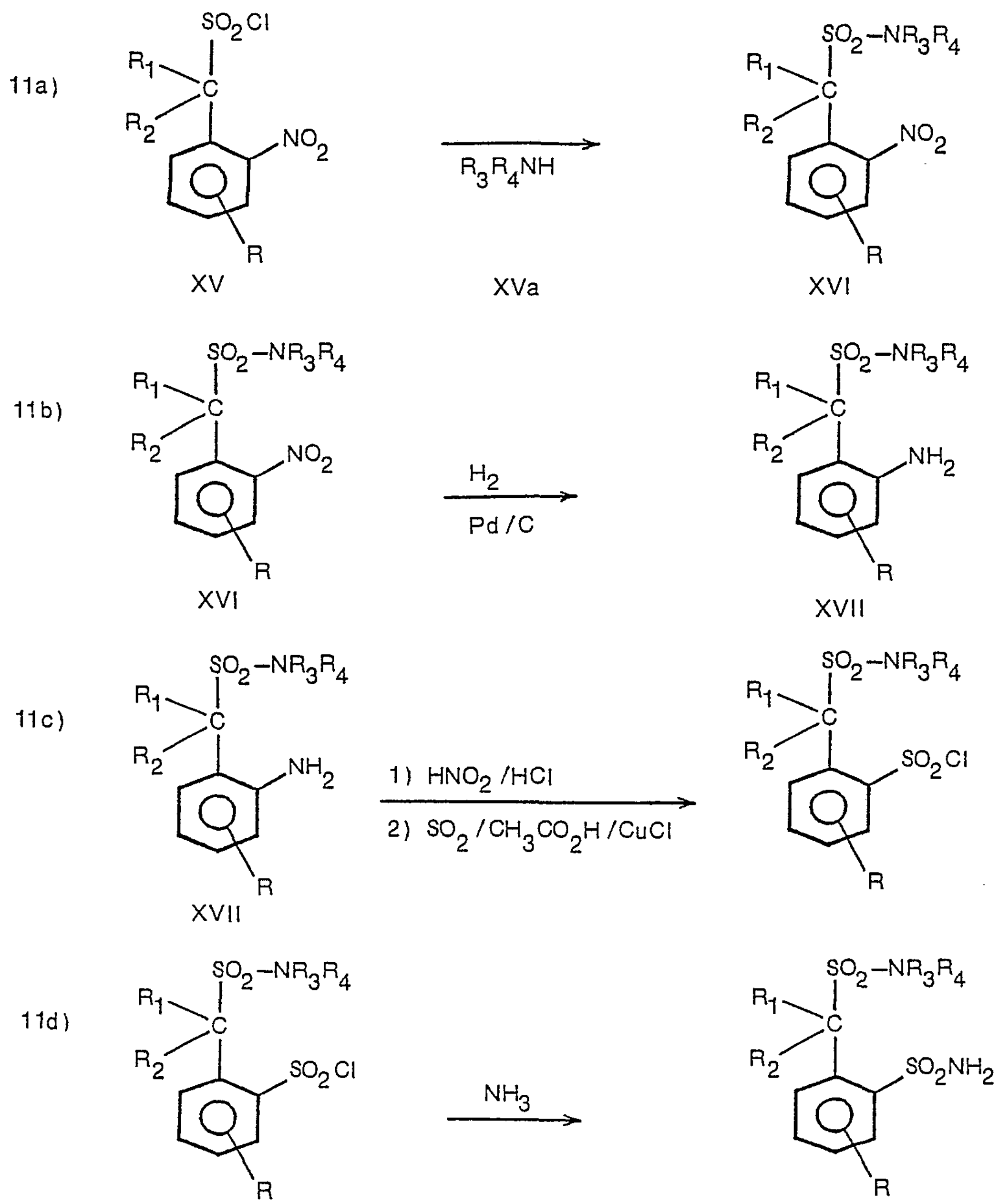


11a) XV XVa XVI

11b) XVI XVII

11c) XVII

11d) Va

wherein R, $R_1$, $R_2$, $R_3$ and $R_4$ are as defined in Equations 1—5, with the exception that R cannot be $NO_2$.

In step 11a, the *o*-nitrobenzylsulfonyl chlorides of Formula XV, which are well-known in the art, are treated with an amine of Formula XVa in an inert organic solvent such as methylene chloride, ethyl ether or tetrahydrofuran at 0—50°. The amine may be taken in excess to act as an acid acceptor; or, alternatively, a tertiary amine such as triethylamine or pyridine may be used as an acid acceptor. The by-product amine hydrochloride is filtered off or washed out of the solvent with water and the product isolated by evaporation of the solvent.

The reduction described in step 11b is accomplished by treating a solution of the compounds of Formula XVI in a solvent such as ethanol, ethyl acetate, or diglyme, in a pressure vessel, with 50—1000 pounds per square inch of hydrogen at 25—150° in the presence of a hydrogenation catalyst such as 5—10% palladium absorbed on carbon. When the theoretical amount of hydrogen has been absorbed, the solution is cooled and the catalyst is removed by filtration. The product is then isolated by evaporation of the solvent.

In the case where $R = NO_2$, the reduction of step *11b* can be accomplished using ammonium

sulfide or sodium hydrosulfide instead of catalytic hydrogenation. This type of procedure is described in *Organic Synthesis* Coll. Vol. III, pgs. 242—3, John Wiley and Sons, Inc., New York and London (1955), the disclosure of which is herein incorporated by reference.

The diazotization and coupling with sulfur dioxide, described in step 11a, is accomplished in the following manner. A solution of the aniline of Formula XVII in a mixture of concentrated hydrochloric acid and glacial acetic acid is treated with a solution of sodium nitrite in water at −5 to 0°. After stirring for 10—15 minutes at 0° to insure complete diazotization, this solution is added to a mixture of an excess of sulfur dioxide, and a catalytic amount of cuprous chloride in glacial acetic acid at 0—5°. The temperature is kept at 0—5° for 1/4 to 1 hour then raised to 20—25° and held at that temperature for 2—4 hours. This solution is then poured into a large excess of ice water. The sulfonyl chloride products, XVII, can be isolated by filtration or by extraction into a solvent such as ethyl ether or methylene chloride followed by evaporation of the solvent.

The amination described in step 11d is conveniently carried out by treating a solution of the sulfonyl chloride of Formula XVII with an excess of anhydrous ammonia in a solvent such as ethyl ether or methylene chloride at 0—25°. If the product sulfonamide, Va, is insoluble it may be isolated by filtration followed by washing out the salts with water. If the product sulfonamide is soluble in the reaction solution, it may be isolated by filtering off the precipitated ammonium chloride and evaporation of the solvent.

Ammonium salts can be prepared by alkylation as described in Equation 12.

EQUATION 12

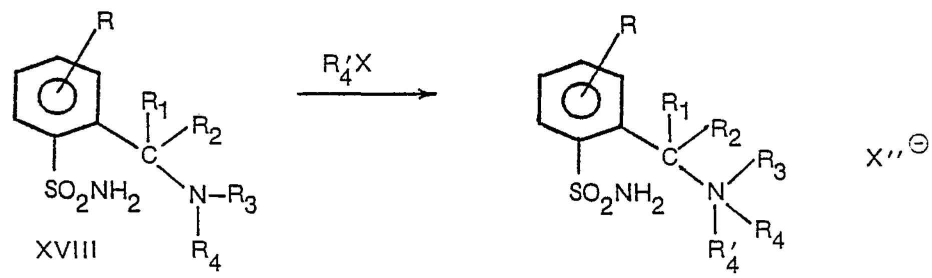

XVIII

wherein X″ is a nucleofugic group, e.g. $Cl^{\ominus}$, $Br^{\ominus}$, $I^{\ominus}$.

Various amides, ureas and carbamates may be produced by well-known reactions of the primary or secondary amine XVIII (at least one of $R_3$ and $R_4$ is H) with the desired acyl chloride, carbamoyl chloride, or chloroformate (Equation 13):

e.g.

EQUATION 13

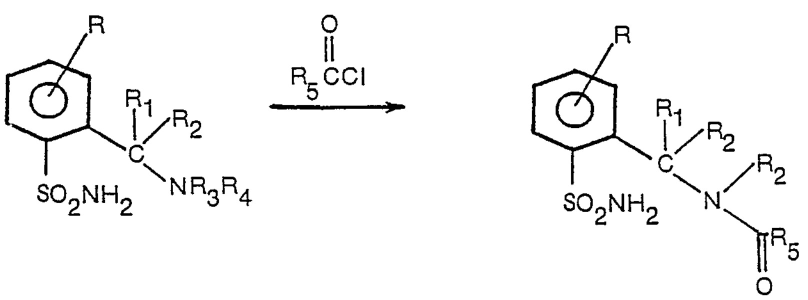

The α-haloalkylbenzenesulfonyl chlorides as prepared in Equations 9 or 10 are converted to the sulfonamides as previously described.

The α-haloalkylbenzenesulfonamides may be converted to other required intermediates for this invention by treatment with appropriate nucleophiles, e.g., acetate ion as described in Equation 15.

EQUATION 15

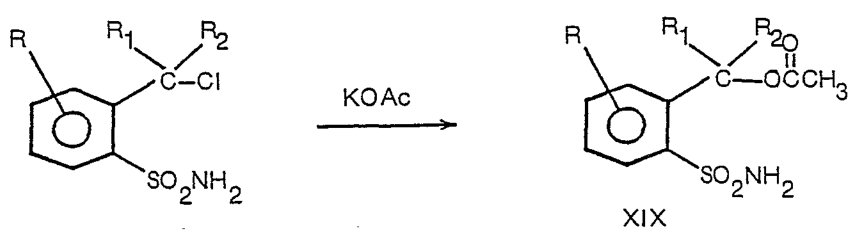

XIX

The compounds of Formula XIX are useful intermediates in the preparation of compounds of this invention.

The synthesis of heterocyclic amines has been reviewed in "The Chemistry of Heterocyclic Compounds" a series published by Interscience Publ., New York and London. 2-Aminopyrimidines are described by D. J. Brown in the Pyrimidines, Vol. XVI of this series. The 2-amino-1,3,5-triazines are reviewed by K. R. Huffman and in The Triazines of this same series. The synthesis of triazines are also described by F. C. Schaefer, U.S. Patent No. 3,154,547 and by K. R. Huffman and F. C. Schaeffer, J. Org. Chem. *28,* 1816—1821 (1963).

The preparation of the aminoheterocycles described by the following formulae are prepared by, for example, methods described in EP—A—15683

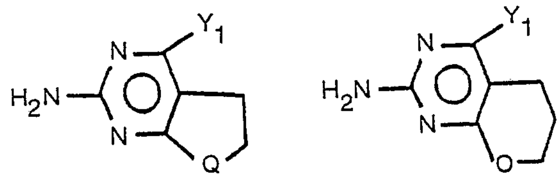

The synthesis of heterocyclic amine derivatives has been reviewed in "The Chemistry of Heterocyclic Compounds", a series published by Interscience Publ., New York and London. 2-Aminopyrimidines are described by D. J. Brown in "The Pyrimidines", Vol. XVI of the above series.

2-Amino-1,3,5-triazines can be synthesized according to the methods described by E. M. Smolin and L. Rapaport in "*s*-Triazines and Derivatives", Vol. XIII of the same series.

The preparation of fused ring pyrimidine amines are disclosed in various publications, such as: Braker et al., *J. Am. Chem. Soc., 69,* 3072 (1947); Mitter and Bhattacharya, *Quart. J. Ind. Chem. Soc. 4,* 152 (1927), Shrage and Hitchings, *J. Org. Chem., 16,* 1153 (1951); Svab et al., Coll. Czech Commun. *32,* 1582 (1967).

Compounds of Formula Ib are prepared by the reaction of about two equivalents of aqueous sodium hydroxide with the compounds of Formula I in which $R_{10}$ is $C_1$—$C_4$, preferably methyl (Ia), followed by acidification of the solution. This process is illustrated in Equation 16, wherein R, $R_1$, $R_2$, $R_8$ and A are as previously defined.

EQUATION 16

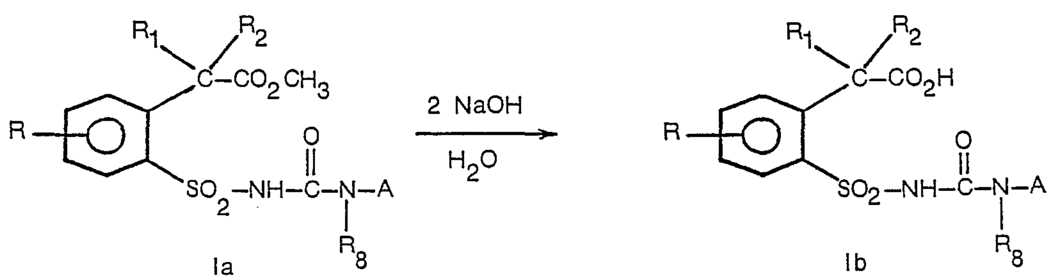


Ia Ib

The reaction is carried out over the course of 2—6 hours in water, by stirring the compound of Formula Ia with two equivalents of sodium hydroxide or potassium hydroxide at ambient temperature. The aqueous solution is then acidified with hydrochloric or sulfuric acid, which causes the compound of Formula Ib to precipitate out. It is then isolated by filtration.

Compounds of Formula Ic are prepared by reacting an appropriately substituted compound of Formula I in which $R_{10}$ is $C_1$—$C_4$ alkyl, preferably methyl (Ia), with a dialkylaluminum-N-alkylamide derivative, as shown in Equation 17, wherein R, $R_1$, $R_2$, $R_3$, $R_4$, $R_8$ and A are as previously defined.

EQUATION 17

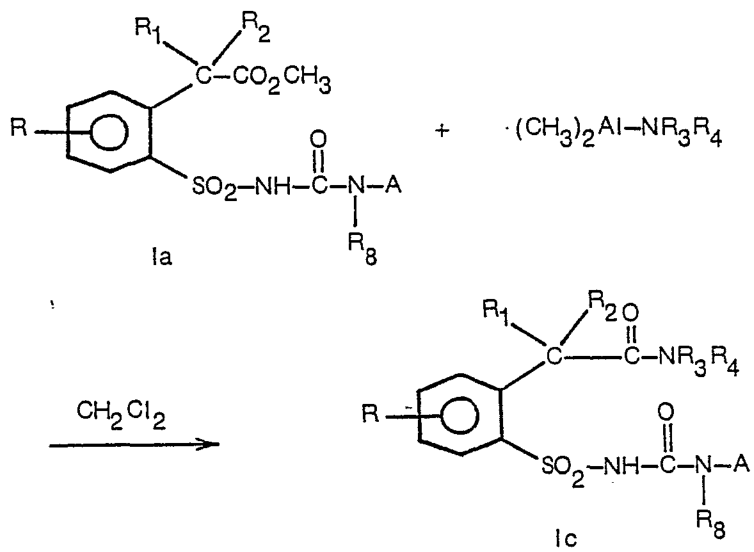


Ia

Ic

The intermediate alkylaminoaluminum compounds are prepared according to A. Basha, M. Lipton and S. W. Weinreb, *Tetrahedron Letters,* 4171 (1977) by reacting the trialkylaluminium, preferably trimethylaluminum, and the corresponding amine. The $(CH_3)_2Al$—$NR_3R_4$ intermediate is co-mingled with a suspension of the compound of Formula VIII in an inert solvent, preferably methylene chloride, and the mixture is refluxed for one to twenty-four hours. The product is isolated by adding aqueous hydrochloric acid to decompose the resulting complex, and then evaporating the methylene chloride phase. The desired product can be further purified by recrystallization or column chromatography.

Compounds of Formula Ic can be prepared by reacting an appropriately substituted sulfonamide of Formula Vb with the methylcarbamate of the appropriate aminoheterocycle XX in the presence of an equivalent amount of trimethylaluminum, as shown in Equation 5, wherein R, $R_1$, $R_2$, $R_8$ and A are as previously defined.

EQUATION 18

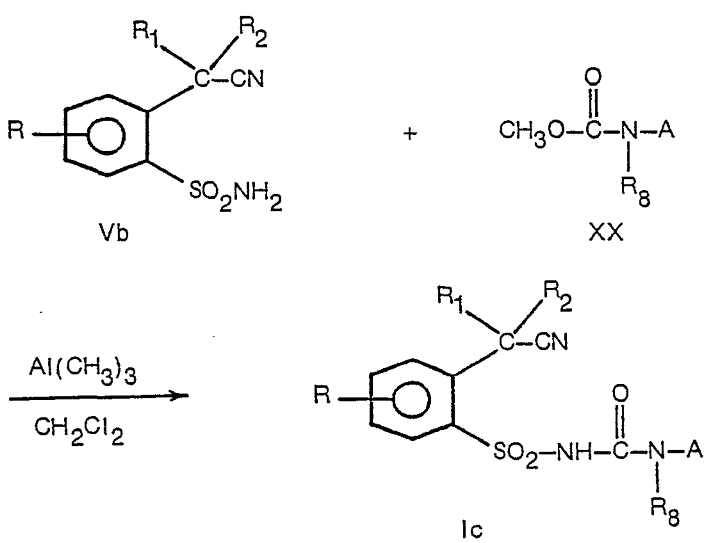


Vb

XX

Ic

The reaction of Equation 18 is best carried out in an inert solvent such as methylene chloride at 10—45° and atmospheric pressure. The preferred mode of addition is to add the trimethylaluminum to a solution or slurry of the sulfonamide Vb; a mildly exothermic reaction occurs accompanied by the evolution of gas. The addition of the heterocyclic carbamate (XX) is then made and the mixture is stirred at ambient to reflux temperature for 6 to 48 hours. The addition of aqueous acid such as dilute hydrochloric or acetic acid removes organic salts from the product contained in the organic phase.

Evaporation of the methylene chloride yields the crude product, which can be purified by recrystallization or column chromatography.

Some of the compounds of Formula I can also be prepared by the method shown in Equation 19, using the novel intermediates of Formula II, wherein L, R, $R_1$, $R_2$, $R_3$, $R_4$ and $R_{10}$ are as previously defined, and wherein Z is CH or N, and $R_{12}$ is $C_1$—$C_3$ alkyl.

EQUATION 19

19a)

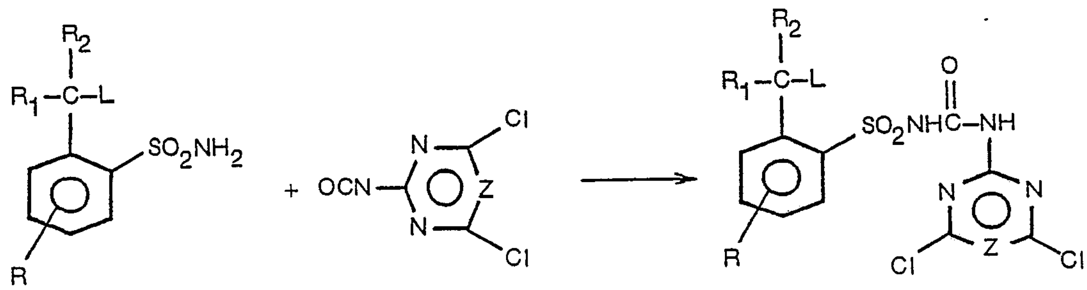

V XX II

19b)

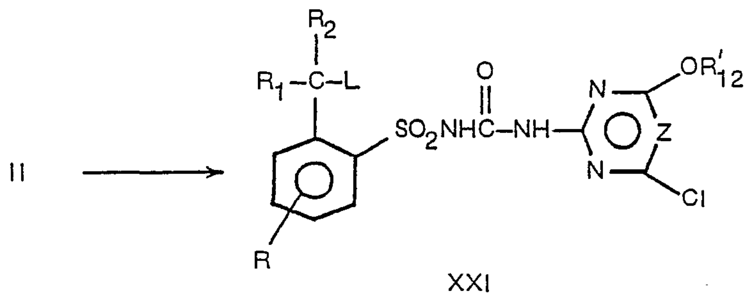

XXI

19c)

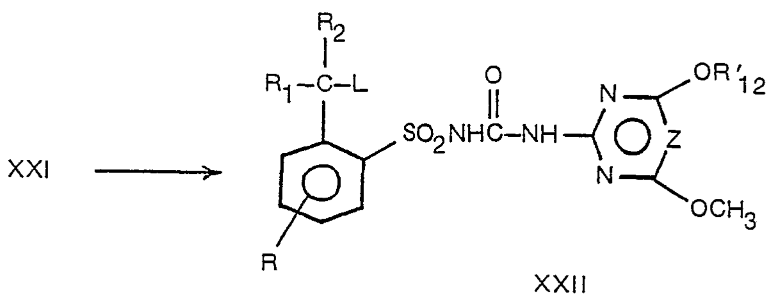

XXII

Reaction Step (19a)

In Reaction Step (19a), an aromatic sulfonamide of Formula V is contacted with a heterocyclic isocyanate of Formula XV to yield a N-(haloheterocyclicaminocarbonyl)aromatic sulfonamide of Formula II.

The heterocyclic isocyanates used in Reaction (19a) may be prepared according to methods described in Swiss Patent 579,062, U.S. Patent 3,919,228, U.S. Patent 3,732,223 and *Angew Chem. Int. Ed. 10,* 402 (1976), the disclosures of which are herein incorporated by reference.

The aromatic sulfonamide and the heterocyclic isocyanate are contacted in the presence of an inert organic solvent, for example, acetonitrile, tetrahydrofuran (THF), toluene, acetone or butanone. Optionally, a catalytic amount of a base, such as 1,4-diazabicyclo[2.2.2]octane (DABCO), potassium carbonate, sodium hydride or potassium *tert*-butoxide, may be added to the reaction mixture. The quantity of base constituting a catalytic amount would be obvious to one skilled in the art. The reaction mixture is preferably maintained at a temperature of about 25 to 110°, and the product can generally be recovered by cooling and filtering the reaction mixture. For reasons of efficiency and economy, the preferred solvents are acetonitrile and THF, and the preferred temperature range is about 60 to 85°C.

Reaction Steps (19b) and (19c)

In Reaction Steps (19b) and (19c), one or two or the halogen atoms on the heterocyclic ring of the

compound of Formula II is displaced by a nucleophilic species. Generally this may be done by contacting the compound of Formula II either with alkanol, $R'_{12}OH$ or with alkoxide, —$OR'_{12}$, where $R'_{12}$ is as defined above.

Thus, in Reaction Step (19b), a compound of Formula II can be contacted with at least one equivalent of alkanol, $R'_{12}OH$. This reaction is sluggish, however, and it is preferred to contact the compound of Formula II with at least two equivalents of alkoxide, $OR'_{12}$. The alkoxide can be provided in a number of ways:

(a) The compound of Formula II can be suspended or dissolved in an alkanol solvent, $R'_{12}OH$, in the presence of at least two equivalents of alkoxide, $OR'_{12}$. The alkoxide can be added directly as alkali metal or alkaline earth metal alkoxide or can be generated by the addition to the alkanol solvent of at least two equivalents of a base capable of generating alkoxide from the solvent. Suitable bases include, but are not limited to, the alkali and alkaline earth metals, their hydrides and *tert*-butoxides. For example, when $R'_{12}$ is methyl, the compound of Formula II could be suspended or dissolved in methanol in the presence of two equivalents of sodium methoxide. Alternatively, two equivalents of sodium hydride could be used in place of the sodium methoxide.

(b) The compound of Formula II can be suspended or dissolved in an inert solvent in the presence of at least two equivalents of alkoxide, $OR'_{12}$. Suitable inert solvents include, but are not limited to, acetonitrile, THF and dimethylformamide. The alkoxide may be added directly as alkali metal or alkaline earth metal alkoxide or may be generated from alkanol and a base as described in (a) above. For example, when $R'_{12}$ is methyl, the compound of Formula II could be suspended or dissolved in THF in the presence of two equivalents of sodium methoxide. Alternatively, two equivalents each of methanol and sodium hydride could be used instead of sodium methoxide.

For reasons of economy and efficiency, procedure (a) is the more preferred method.

It should be noted that two equivalents of alkoxide are required for Reaction Step (19b) whereas only one equivalent of alkanol is needed for the same process. This difference is due to the reaction which is believed to occur between the alkoxide and the sulfonyl nitrogen of the sulfonamide of Formula II. When alkoxide is used, the first equivalent of alkoxide removes a proton from the sulfonyl nitrogen, and it is only the second equivalent which effects displacement of the halogen. As a result, two equivalents of alkoxide are required. The resulting salt must be acidified, e.g., with sulfuric, hydrochloric or acetic acid, to yield a compound of Formula XXI. Applicant, of course, does not intend to be bound by the mechanism described above.

In Reaction Step (19c), a compound of Formula XVI is contacted with either one equivalent of methanol, or with two equivalents of methoxide, —$OCH_3$. When methoxide is used, it may be provided in either of the methods described above in connection with Reaction Step (19b), and the resulting salt can be acidified to yield a compound of Formula XXII.

When $R'_12 = CH_3$, Reaction Steps (19b) and (19c) may be combined. Thus, a compound of Formula II may be contacted either with at least two equivalents of methanol or with at least three equivalents of methoxide.

For a compound of Formula II, certain reaction conditions will favor displacement of only one of the chlorines. These conditions are the use of low temperatures, and when alkoxide is used, the slow addition of the stoichiometric amount of alkoxide or alkoxide-generating base to the medium containing the compound of Formula II.

When alkoxide is used, both Reaction Steps (19b) and (19c) are preferably run at temperatures within the range of about −20° to 80°C, the range of about 0° to 25°C being more preferred. Reaction Steps (19b) and (19c) are more sluggish when alkanol is used instead of alkoxide, and more drastic conditions are required for the reaction to go to completion. Thus, higher temperatures, up to and including the boiling point of the alkanol itself, are required.

As shown in Equation 20, compounds of Formula XXIII, in which R, $R_1$, $R_2$, L and A are as previously defined, are prepared by the reaction of an appropriately substituted sulfonamide, (V), with a heterocyclic isothiocyanate of Formula XXIV.

EQUATION 20

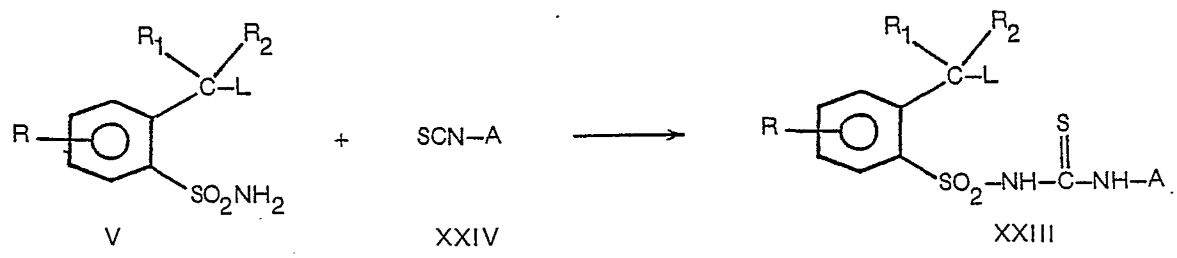

V XXIV XXIII

The Reaction of Equation 20 is best carried out by dissolving or suspending the sulfonamide and isothiocyanate in a polar solvent such as acetone, acetonitrile, ethyl acetate or methyl ethyl ketone, adding an equivalent of a base such as potassium carbonate and stirring the mixture at ambient

temperature up to the reflux temperature for one to twenty-four hours. In some cases, the product precipitates from the reaction mixture and can be removed by filtration. The product is stirred in dilute mineral acid, filtered and washed with cold water. If the product does not precipitate from the reaction mixture it can be isolated by evaporation of the solvent, trituration of the residue with dilute mineral acid and filtering off the insoluble product.

The heterocyclic isothiocyanates which are used in the procedure of Equation 20 are prepared, for example, according ot the method of Japan Patent Application Pub: Kokai 51—143686, June 5, 1976, or that of W. Abraham and G. Barnikow, Tetrahedron 29, 691—7 (1973).

Many of the compounds of Formula I may be prepared as shown in Equation 21 by reaction of an appropriately substituted *o*-hydroxymethylbenzenesulfonylurea, Ia, with an appropriate acid chloride. A, R, $R_1$, $R_2$, $R_8$, and $R_{11}$ being as previously defined.

EQUATION 21

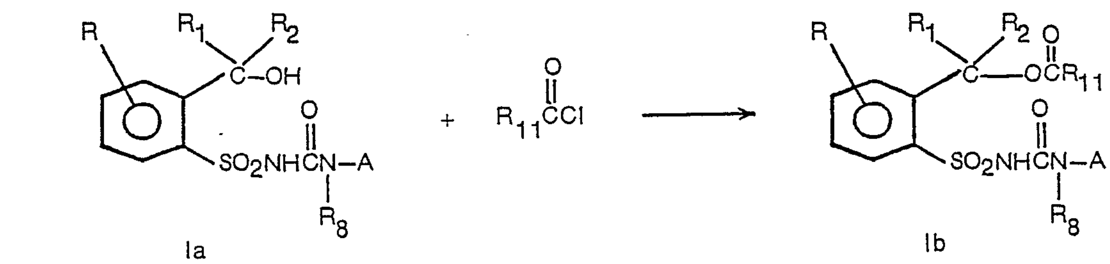

Ia Ib

The reaction of Equation 21 is best carried out in inert aprotic solvents such as methylene chloride, tetrahydrofuran or acetonitrile at 0—80°C. An excess of the acid chloride is used and at least one equivalent of a tertiary amine such as pyridine, triethylamine or 4-dimethylaminopyridine. Isolation is achieved by evaporation of solvent and recrystallization from suitable solvents such as 1-chlorobutane, ethyl acetate or ethyl ether or by column chromatography over silica gel.

Other compounds of Formula I may be prepared as shown in Equation 22 by reaction of an appropriately substituted *o*-hydroxymethylbenzenesulfonylurea, Ia with an appropriate isocyanate. A, R, $R_1$, $R_2$, $R_8$ and $R_{12}$ being as previously defined

EQUATION 22

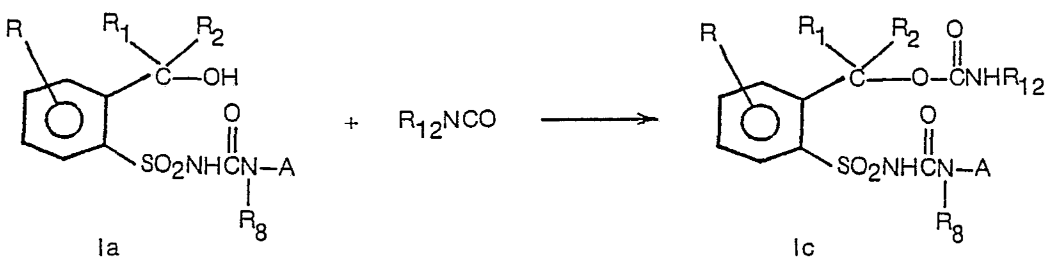

Ia Ic

The reaction of Equation 22 is best carried out in inert aprotic solvents such as methylene chloride, tetrahydrofuran or acetonitrile at 0—80°C. An excess of the isocyanate is used and a catalyst such as dibutyltindilaurate or 1,4-diaza[2,2,2]bicyclooctane (DABCO). Isolation is achieved by evaporation of solvent and recrystallization from suitable solvents such as 1-chlorobutane, ethyl acetate or ethyl ether or by column chromatography over silica gel.

Other compounds of Formula I may be prepared as shown in Equation 23 by reaction of an appropriately substituted *o*-hydroxymethylbenzenesulfonylurea, Ia with an appropriate chloroformate. A, R, $R_1$, $R_2$, $R_8$ and $R_{13}$ being as previously defined.

EQUATION 23

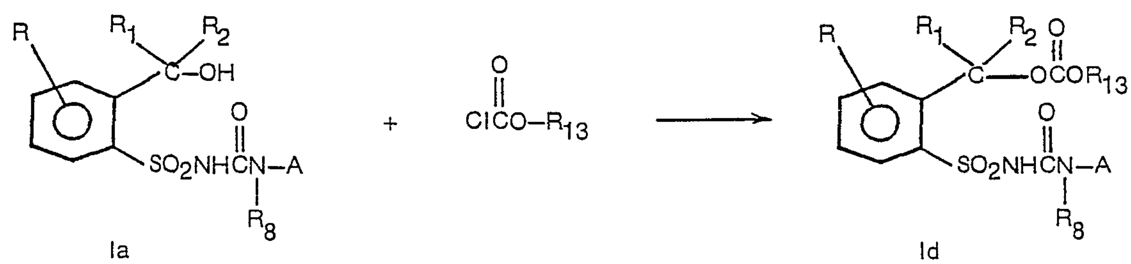

Ia Id

The reaction of Equation 23 is best carried out in inert aprotic solvents such as methylene chloride, tetrahydrofuran or acetonitrile at 0—80°C. An excess of the chlorocarbonate is used and at least one equivalent of a tertiary amine such as pyridine, triethylamine or 4′-dimethylaminopyridine. Isolation is achieved by evaporation of solvent and recrystallization from suitable solvents such as 1-chlorobutane, ethyl acetate or diethyl ether or by column chromatography over silica gel.

The preparation of compounds of Formula If where $R_1 = R_2 = H$; and R, $R_8$ and A are as previously defined, may be carried out as shown in Equation 24.

EQUATION 24

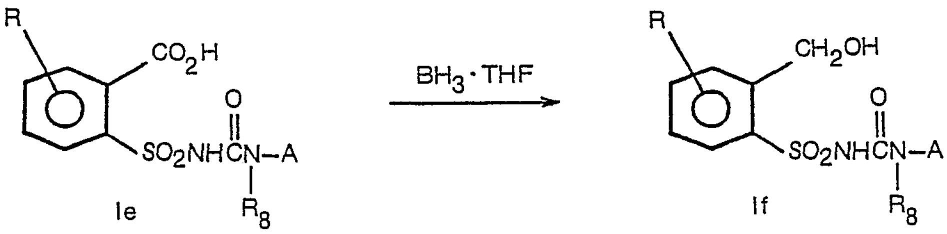


Ie If

The carboxylic acid, Ie, may be converted to the alcohol by reduction with 4—5 equivalents of borane-THF reagent in THF at ambient pressure and temperature for 4 to 18 hours. Isolation is achieved by drowning in dilute acid followed by extraction of the product with a solvent such as methylene chloride, ethyl acetate or ethyl ether. Evaporation of solvent and crystallization or column chromatography on silica gel affords the pure alcohol If.

The carboxylic acids, Ie, may be prepared by hydrolysis of the corresponding methyl esters as shown in Equation 25; R, $R_8$ and A being as previously defined.

EQUATION 25

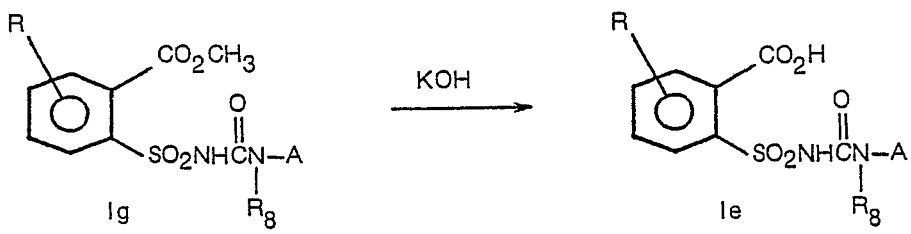


Ig Ie

When A is a pyrimidine type structure, the methyl esters are best hydrolyzed by dissolving in a solution of 80 parts ethanol, 10 parts water and 10 parts potassium hydroxide. The mixture is stirred at ambient temperature for 18 hours followed by pouring into a large excess of water and acidifying to a pH of 2.0. The pure acid, VI, precipitates and is filtered and washed with water.

When A is a triazine and X or Y is alkoxy, the hydrolysis is best performed by dissolving the ester in a solution of potassium t-butoxide in dimethyl sulfoxide at ambient temperature for two hours. Addition of a large volume of water followed by acidification to a pH of 2.0 precipitates the acid, Ie.

The preparation of esters of Formula Ig is described in European Patent Application 7687.

Compounds of Formula If may also be prepared by treatment of the carboxylic acids, Ie or the methyl esters, Ig, with lithium aluminium hydride by the procedures described by R. F. Nystrom and W. G. Brown, J. Am. Chem. Soc. *69*, 2548 (1947) and R. B. Moffett, *Organic Synthesis,* Coll. Vol. 4, 834 (1963). Reduction of the esters with sodium bis-(2-methoxyethoxy)aluminum hydride is described in M. Fieser and L. E. Fieser, *Reagents for Organic Synthesis,* John Wiley & Sons, New York, Vol. 5, p. 596 (1975).

The preparation of compounds of Formula Ij where $R_1 = H$ and $R_2 = CH_3$ may be carried out as shown in Equation 26.

EQUATION 26

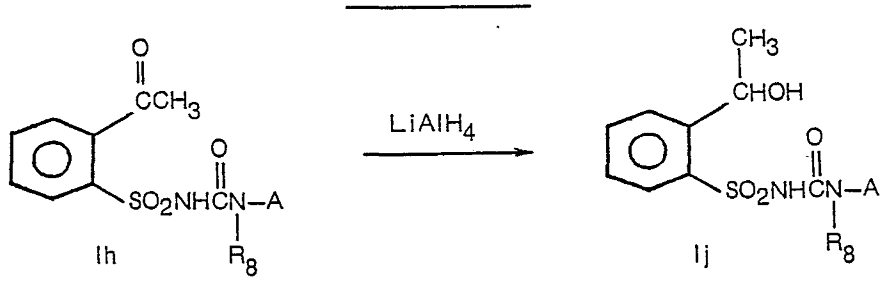


Ih Ij

Compounds of Formula Ih are treated with one equivalent of lithium aluminum hydride (LAH) in a solvent such as ether, tetrahydrofuran or glyme at −20 to 25°C for 1 to 6 hours. Next there is a successive dropwise addition of an equivalent number of ml of water as grams of LAH followed by an equal number of ml of 15% sodium hydroxide followed by 3 times that number of ml of water. This produces a dry granular precipitate of aluminum oxide which is easy to filter. The aqueous phase is then acidified with dilute acid and the product extracted with solvent such as methylene chloride, ethyl acetate or ethyl ether. Evaporation of solvent and crystallization of column chromatography on silica gel affords the pure alcohol, Ij.

Compounds of Formula Ih are prepared by the reaction shown in Equation 27 using an excess of methyl lithium with a carboxylic acid derivative of Formula Ie.

EQUATION 27

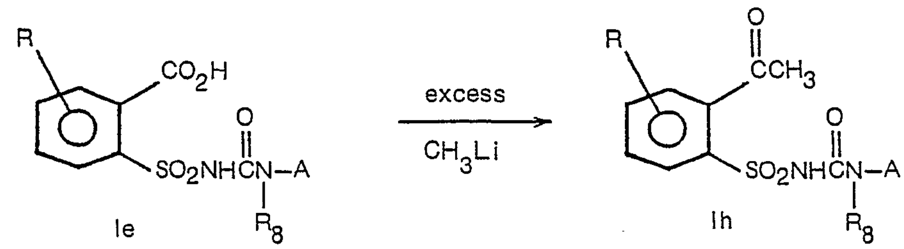


Ie Ih

Compounds of Formula Ie are restricted to structures in which the substituents R, Z, X and Y contain no displaceable halogens, $NO_2$ or CN.

An excess of methyl lithium in a suitable solvent such as diethyl ether, hexane, pentane or benzene is added to a solution or slurry of Ie in a similar solvent at temperatures between −100 and 0°C. The mixture is allowed to warm to room temperature and stir for 30 minutes. Aqueous acid is then added and the compound Ih is extracted into a suitable solvent to free it from salts, followed by evaporation of the solvent. Purification is by chromatography on silica gel.

Another procedure for the preparation of compounds of Formula Ij is the reaction of excess methyl lithium with the corresponding aldehyde Ik as shown in Equation 28. R, $R_8$, A being as previously defined.

EQUATION 28

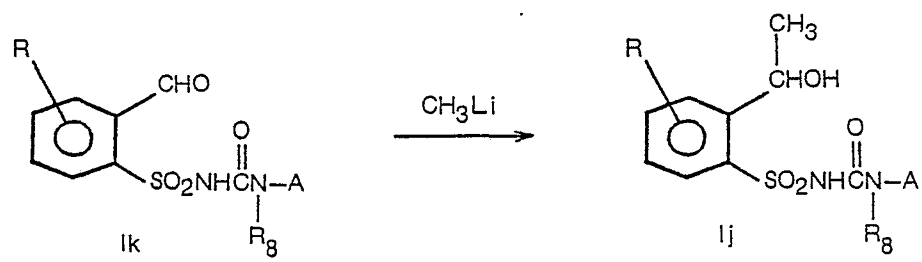


Ik Ij

Compounds of Formula Ik are restricted to structures in which the substituents R, Z, X and Y contain no displaceable halogens; $NO_2$ or CN.

An excess of methyl lithium in a suitable solvent such as diethyl ether, hexane, pentane or benzene is added to a solution or slurry of XI in a similar solvent at temperatures between −100 and 0°C. The mixture is allowed to warm to room temperature and stir for 30 minutes. Aqueous acid is then added and the compound Ij is extracted into a suitable solvent to free it from salts followed by evaporation of the solvent. Purification is by chromatography on silica gel.

Aldehydes of Formula Ik are prepared by the procedure of Equation 29. R, $R_8$ and $R_9$ being as previously defined.

EQUATION 29

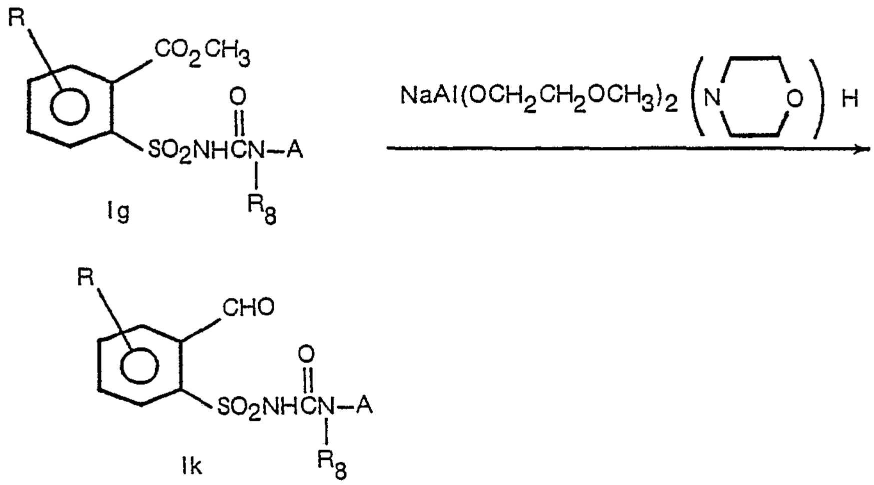


Ig

Ik

Following the procedure of R. Kanayawa and T. Tokoroyama, a solution of sodium bis(2-methoxyethoxy)aluminum hydride in THF is reacted with one equivalent of morpholine. To this solution at −40°C is added methyl ester of Formula Ig and the solution is allowed to warm to 25°C. The product is isolated by addition of aqueous acid and extraction into ether or methylene chloride. Evaporation of solvent and crystallization or column chromatography on silica gel affords the pure aldehyde, Ig.

Aldehydes of Formula Ik may also be prepared from the esters of Formula Ig by treatment with diisobutylaluminum hydride according to procedures of E. Winterfeldt, Synthesis, 617 (1975).

The preparation of compounds of Formula Im where $R_1$=$R_2$=$CH_3$ may be prepared as shown in Equation 30.

EQUATION 30

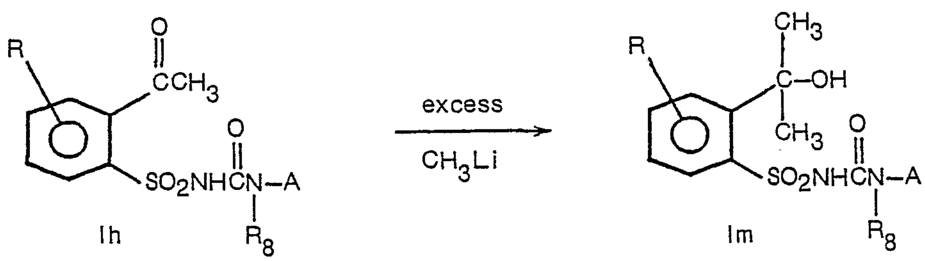


Ih

Im

Compounds of Formula Im are prepared by the reaction of an excess of methyl lithium with acetophenones of Formula Ih.

Compounds of Formula Ih are restricted to structures in which the substituents R, Z, X and Y contain no displaceable halogens, $NO_2$ or CN.

An excess of methyl lithium in a suitable solvent such as diethyl ether, hexane, pentane or benzene is added to a solution or slurry of Ih in a similar solvent at temperatures between −100 and 0°C. The mixture is allowed to warm to room temperature and stir for 30 minutes. Aqueous acid is then added and the compound Im is extracted into a suitable solvent to free it from salts followed by evaporation of the solvent. Purification is by chromatography on silica gel.

Agriculturally suitable salts of compounds of Formula I are also useful herbicides and can be prepared in a number of ways known to the art. For example, metal salts can be made by treating compounds of Formula I with a solution of an alkali or alkaline earth metal salt having a sufficiently basic anion (e.g., hydroxide, alkoxide, carbonate or hydride). Quaternary amine salts can be made by similar techniques.

Salts of compounds of Formula I can also be prepared by exchange of one cation for another. Cationic exchange can be effected by direct treatment of an aqueous solution of a salt of a compound of Formula I (e.g., alkali metal or quaternary amine salt) with a solution containing the cation to be exchanged.

This method is most effective when the desired salt containing the exchanged cation is insoluble in water, e.g., a copper salt, and can be separated by filtration.

Exchange may also be effected by passing an aqueous solution of a salt of a compound of Formula I (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water soluble, e.g., a potassium, sodium or calcium salt.

Acid addition salts, useful in this invention, can be obtained by reacting a compound of Formula I with a suitable acid, e.g., *p*-toluenesulfonic acid, trichloroacetic acid or the like. The reader is referred to all references cited above for further information, which accordingly will not be repeated herein.

In the following examples, all parts are by weight and temperatures in °C unless otherwise indicated. "Nujol", used as a mulling agent for obtaining IR spectroscopic data, is a registered Trade Mark.

Example 1

2-(Dichloromethyl)-N-[(4-methoxy-6-methyl-pyrimidin-2-yl)aminocarbonyl]benzenesulfonamide

*o*-Dichloromethylbenzenesulfonamide (2.40 g, 10 mmol) was treated with thionyl chloride (25 ml) and heated to reflux for 16 hrs. Volatiles were removed under vacuum and the residue was treated with thionyl chloride (20 ml) and refluxed for 22 hrs. Thionyl chloride was removed under vacuum, and two portions of dry toluene were added and evaporated in order to remove last traces of $SOCl_2$. The crude product in toluene (45 ml) was transferred to a three-neck flask, treated with dry pyridine (1.0 ml), and then treated slowly with phosgene such that a slow reflux was maintained while the internal temperature was held at *ca.* 70° for 1.0 hr. Excess phosgene was removed using a stream of dry nitrogen. IR of the crude residue showed only a trace NH stretch and featured a strong band at 2250 $cm^{-1}$.

The crude isocyanate in dry acetonitrile (15 ml) was treated with 2-amino-4-methoxy-6-methyl pyrimidine (1.39 g, 10 mmol). The amine dissolved and a precipitate formed. The mixture was stirred for 16 hrs, filtered, and the solid was washed with *ca.* 3 ml acetonitrile to give 1.51 g of off-white solid, mp 183—185°C. $^1$H nmr: $\delta^{TMS}_{DMSO-d_6}$ 10.3 (brd s, NH), 8.25—7.58 (m, featuring singlet at 8.13), 6.62 (s) 3.98 (s), 2.42 (s). Recrystallization from $CHCl_3$ gave 0.89 g, mp 189—190° (dec.). Mass spec: measured 404.0083; calc'd for $C_{14}H_{14}N_4O_4SCl_2$ 404.0110.

| | | | |
|---|---|---|---|
| *Anal.* Calc'd: | C, 41.49; | H, 3.48; | N, 13.83 |
| Found: | C, 41.22; | H, 3.21; | N, 13.83 |
| | 41.12 | 3.50 | 13.67 |

Example 2

2-(Dichloromethyl)-*N*-[(4,6-dimethylpyrimidin-2-yl)aminocarbonyl]benzenesulfonamide

2-(Dichloromethyl)benzenesulfonyl isocyanato, prepared from 10 mmol of sulfonamide as described in the synthesis of Example 1, was treated with acetonitrile and 2-amino-4,6-dimethyl-pyrimidine (1.23 g). The mixture was stirred at room temperature for 18 hrs, cooled and filtered to provide 1.08 g of white solid, mp 195—197° (Dec). $^1$H nmr: $\delta^{TMS}_{CDCl_3/DMSO-d_6}$ 10.53 (s), 8.20—7.90 (m, featuring singlet at 8.00), 7.87—7.43 (m), 6.83 (s), 2.43 (s). IR (nujol) featured bands at 1710 and 1600 $cm^{-1}$. Mass spectrum featured m/e 388.0174 (calc'd for $C_{14}H_{14}Cl_2N_4O_3S$ 388.0163); 150.0627 (calc'd for $C_7H_7N_3$ 150.0667).

Example 3

2-(Dichloromethyl)-*N*-[(4-methoxy-6-methyl-1,3,5-triazin-2-yl)aminocarbonyl]benzenesulfonamide

2-(Dichloromethyl)benzenesulfonyl isocyanate prepared from 10 mmol of sulfonamide as described in the synthesis of Example 1, was treated with acetonitrile (15 ml) and 2-amino-4-methoxy-6-methyl-1,3,5-triazine (1.40 g). The mixture was stirred for 18 hrs. and filtered. The filtrate was evaporated and recrystallized from acetone to give 0.78 g of white solid, mp 168—169° (dec). $^1$H nmr $\delta^{TMS}_{DMSO-d_6}$ 8.37—7.50 (m, featuring singlet at 8.07), 7.00—6.17 (brds), 4.00 (s), 2.48 (s). IR (nujol) featured bands at 1720 and 1590 $cm^{-1}$. Mass spectrum featured 264.9367 (calc'd for $C_8H_5Cl_2NO_3S$ 264.9367) and 140.0687 (calc'd for $C_5H_8N_4O$ 140.0697).

| | | | |
|---|---|---|---|
| *Anal.* Calc'd: | C, 38.44; | H, 3.23; | N, 17.24 |
| Found: | C, 38.74; | H, 3.13; | N, 16.90 |
| | 38.59; | 3.11 | 17.04 |

Example 4

2-(Chloromethyl)-N-[(4,6-dimethoxy-1,3,5-triazin-2-yl)aminocarbonyl]benzenesulfonamide

A solution of 4,6-dichloro-1,3,5-triazin-2-yl isocyanate (3.10 g, 16.2 mmol) in acetonitrile (15 ml)

was contacted with 2-(chloromethyl)benzene sulfonadice (3.34 g, 16.2 mmol) and stirred for 20 hrs at 25°. The mixture was heated at 50° for 2 hrs, cooled and filtered to provide 3.86 g, mp 162—170°, which was contacted with sodium methoxide (30 mmol) in methanol (60 ml) at 20—25° for 16 hrs. Water (100 ml) was added and the mixture was filtered and neutralized by addition of hydrochloric acid. Filtration and drying gave 2.20 g of white solid, mp 150—152° (dec.). $^1$H nmr $\delta^{TMS}_{DMSO-d_6}$ 11.0 (s), 8.30—7.50 (m), 5.23 (s), 4.03 (s). IR (nujol) featured absorption bands centered at 3200, 1720, 1610 and 1560 $cm^{-1}$.

| | | | |
|---|---|---|---|
| *Anal.* Calc'd for $C_{13}H_{14}N_5ClO_5S$: | C, 40.26; | H, 3.64; | N, 18.06 |
| Found: | C, 38.60; | H, 3.62; | N, 18.40 |

Example 5

2-(Chloromethyl)-*N*-[(4,6-dimethoxypyrimidin-2-yl)aminocarbonyl]benzenesulfonamide

A solution of 2-(chloromethyl)-*N*-(butylaminocarbonyl)benzene sulfonamide (1.33 g, 4.38 mmol) and diazabicyclo[2.2.2]octane (5 mg) in xylene (12 ml) was heated at reflux and contacted in portions with phosgene (1.0 ml. condensed phase). After 2.0 hrs at reflux, the mixture was cooled to room temperature, decanted, and volatiles were removed to give 1.56 g of crude product. The IR spectrum of 2-(chloromethyl)benzenesulfonyl isocyanate featured a strong band at 2250 $cm^{-1}$. The crude sulfonyl isocyanate was contacted with acetonitrile (10 ml) and 2-amino-4,6-dimethoxy-pyrimidine (0.68 g, 4.4 mmol) and stirred for 16 hrs. Filtration gave 1.18 g of white solid, mp 192—193°. The sample was dissolved in 0.5 N NaOH, filtered and pH adjusted to 6.5. The mixture was cooled and filtered and the solid washed with ice water and dried to give 0.90 g of white solid. $^1$H nmr $\delta^{TMS}_{DMSO-d_6}$ 12.7 (brd s,) 10.50 (2, 1H) 8.13—7.97 (m, 1H), 7.73—7.40 (m, 3H), 5.90 (s, 1H), 5.10 (s, 2H), 3.87 (s, 6H).

| | | | | |
|---|---|---|---|---|
| *Anal.* Calc'd: | C, 43.47; | H, 3.91; | N, 14.48; | S, 8.29 |
| Found: | C, 41.76; | H, 3.82; | N, 13.94; | S, 8.23 |

Example 6

2-(Chloromethyl)-*N*-[(4,6-dimethylpyrimidin-2-yl)aminocarbonyl]benzenesulfonamide

A mixture of 2-(chloromethyl)benzene sulfonamide (4.11 g 20 mmol) and thionyl chloride (35 ml) was heated to reflux for 36 hrs. Thionyl chloride was removed under vacuum. The residue was contacted with toluene (100 ml) and pyridine (2 ml), heated to 70°, and treated in portions with phosgene (excess) for 2.0 hrs. The mixture was cooled, and the toluene-soluble portion was decanted and evaporated to give a residue which was treated with acetonitrile (15 ml) and 2-amino-4,6-dimethylpyrimidine (1.70 g, 13.8 mmol). After 16 hrs, the solid was filtered and washed with acetonitrile to give 1.45 g of solid which was taken up in 1.0 N NaOH and filtered. The pH was adjusted to 6.0 with dilute hydrochloric acid and the solid was filtered and dried to provide 1.34 g of cream-coloured solid, mp 176—177° (dec.). IR (KBr) featured absorption bands at 3340—2300, 1700 (strong), 1600, 1500, 1440, 1340 $cm^{-1}$.

Example 7

2-(1-Chloroethyl)-*N*-[(4,6-dimethoxy-1,3,5-triazin-2-yl)aminocarbonyl]benzenesulfonamide

A solution of 4,6-dichloro-1,3,5-triazin-2-yl isocyanate (2.27 g, 11.9 mmol) in acetonitrile (20 ml) was contacted with 2-(1-chloroethyl)benzenesulfonamide (2.61 g, 11.9 mmol) and stirred at 25° for 18 hrs. and at 50° for 2.0 hrs. Solvent was evaporated to give 4.95 g of residue. $^1$H nmr $\delta^{TMS}_{CDCl_3}$ 11.35 (s), 8.87 (s), 8.33—7.30 (m), 6.18 (q, J = 7Hz), 5.5o (s), 1.88 (d, J = 7 Hz). Mass spectrum: observed 244.9915 (calc'd for $C_9H_8O_3NClS$ 244.9913), 163.9643 (calc'd for $C_3H_2N_4Cl_2$ 163.9656).

A solution of the above sulfonyl urea in methanol (20 ml) was contacted with a solution of sodium methoxide (36 mmol) in methanol (65 ml) at 0° for 3.0 hrs. Solvent was evaporated and the residue was taken up in water (120 ml), filtered, and pH was brought to 5.0 g with dilute HCl. The mixture was cooled and filtered and the solid washed with the water and dried to give 3.52 g of white solid, mp 148—150° (dec.). $^1$H nmr $\delta^{TMS}_{(CD_3)_2CO}$ 8.30—7.42 (m), 6.32 (q, J = 7Hz), 4.08 (s), 2.93 (brds), 1.88 (d, J = 7Hz). Mass spectrum showed: 244.9912 (calc'd for $C_9H_8O_3NClS$ 244.9913) 156.0616 (calc'd for $C_5H_8O_2N_4$ 156.0647).

| | | | |
|---|---|---|---|
| *Anal.* Calc'd: | C, 41.85; | H, 4.01; | N, 17.43 |
| Found: | C, 41.92; | H, 4.05; | N, 17.02 |

Example 8

2-(1-Pyrrolidinylmethyl)-*N*-[(4,6-dimethoxypyrimidin-2-yl)aminocarbonyl]benzenesulfonamide, hydrochloride salt

A slurry of 2-(chloromethyl)-*N*-[(4,6-dimethoxypyrimidin-2-yl)aminocarbonyl]benzenesulfonamide (1.00 g) in dimethylformamide (5 ml) was contacted with pyrrolidine (0.45 ml) and stirred for 3.5 hrs. Volatiles were removed under vacuum and the residue was taken up in methanol. Solvent was removed and the water (15 ml) was added and the pH was adjusted to 6.0 by addition of dilute hydrochloric acid. Cooling and scratching produced a white solid which was collected and dried to give 488 mg, m.p. 117—123°. A 400 mg sample was crystallized from acetonitrile to give 269 mg. m.p. 132—134°C. $^1$H nmr $\delta^{TMS}_{CDCl_3}$ 13.0—11.0 (brds 2H), 8.63 (s, 1 NH), 8.37—8.20, 8.05—7.90, and 7.70—7.33 (m, 4H), 5.68 (s, 1H), 5.00 (s, 2H), 3.90 (s, 6H), 3.70—2.90 (m, 4H), 2.30—1.90 (m, 4H). IR (KBr) 3650—2200, 1710, 1610, 1580, 1450, 1360, 1200, 1165 are major absorption bands.

| | | | |
|---|---|---|---|
| *Anal.* Found: | C, 44.99; | H, 5.17; | N, 14.56 |
| Calc'd for $C_{18}H_{23}N_5O_5S{\cdot}HCl$: | C, 47.21; | H, 5.28; | N, 15.30 |

### Example 9

2-(1-Pyrrolidinylmethyl)-*N*-[(4,6-dimethoxypyrimidin-2-yl)aminocarbonyl]benzenesulfonamide

A slurry of 2-(chloromethyl)-*N*-[(4,6-dimethoxypyrimidin-2-yl)aminocarbonyl]benzenesulfonamide (1.00 g) in dimethylformamide (5 ml) was contacted with pyrrolidine (0.45 ml) and stirred for 2.5 hrs. Volatiles were removed under vacuum and the residue was washed several times with ether, taken up in methanol and treated dropwise with a *ca.* 0.5% solution of dry hydrochloric acid in methanol to adjust the apparent pH to 6.5—7.0. Volatiles were again removed to provide 1.34 g. of glassy foam which was chromatographed on silica gel eluting with 2/1 ethyl acetate/methanol. Residues from the major fractions were taken up in warm chloroform and evaporated. Addition of a small volume of acetonitrile yielded, upon cooling and scratching, a total of 280 mg of white solid, up 138—140° (dec). $^1$H nmr $\delta^{TMS}_{CDCl_3}$ 8.10—7.90 (m, 1H), 7.67—7.10 (m, 4H, featuring NH singlet at 7.57), 5.50 (s, 1H), 4.60 (s, 2H), 3.72 (s, 6H), 3.43—3.10 (m, 4H), 2.30—1.90 (m, 4H). IR (KBr) 3680—2300, 1660, 1600, 1450, 1370, 1280, 1150, and 1110 cm$^{-1}$ were major absorption bands.

| | | | |
|---|---|---|---|
| *Anal.* Calc'd for $C_{18}H_{23}N_5O_5S$: | C, 51.29; | H, 5.50; | N, 16.62 |
| Found: | C, 50.40; | H, 5.29; | N, 15.86 |

Contacting a $CDCl_3$ solution of the above compound with dry hydrochloric acid produced the same substance described in Example 8 as judged by $^1$H nmr and IR spectral data.

### Example 10

2-(1-Chloroethyl)benzenesulfonyl chloride

A solution of 2-ethylbenzenesulfonyl chloride (obtained by chlorosulfonation of ethylbenzene) (7.78 g) in carbon tetrachloride (5.0 ml) was contacted with a solution of dichlorine monoxide (82 mmol) in carbon tetrachloride (100 ml) and the mixture was heated in a closed system at 50—60° for 16 hrs. The mixture was dried ($MgSO_4$) and volatiles were removed under vacuum. The residue was kugelrohr distilled to give 7.63 g of colorless oil, bp 75—80° (0.15 mm). $^1$H nmr $\delta^{TMS}_{CDCl_3}$ 8.20—7.35 (m). 6.13 (q, J—7Hz), 1.90 (d, J=7Hz).

### Example 11

2-Dichloromethylbenzene sulfonamide

A solution of *o*-methylbenzenesulfonyl chloride (7.97 g, 42 mmol) in $CCl_4$ (10 ml) was contacted with a solution of $Cl_2O$ in $CCl_4$ (108 ml, 96 mmol) and the mixture was heated at 50° for 23 hrs. The cooled solution was purged with a stream of nitrogen, dried ($MgSO_4$), and evaporated to give 10.1 g of white solid. $^1$H nmr: 8.43—7.47 (m, featuring a singlet at 7.82).

The product sulfonyl chloride was dissolved in tetrahydrofuran (50 ml) and was added to a solution of ammonia (2.7 ml at −78°) in tetrahydrofuran (100 ml) at −78°. The mixture was allowed to warm slowly to 5°. Solvent was removed under vacuum, and the residue was taken up in $CH_2Cl_2$, washed with water, dried, and evaporated to give 8.3 g of solid. Recrystallization from $CHCl_3$ gave 5.34 g of shiny plates, m.p. 131—132°. $^1$H nmr $\delta^{TMS}_{acetone-d_6}$ 8.33—7.47 (m, featuring s at 8.00), 7.30—6.87 (brds). Mass spec: measured 238.9560. Calc'd. for $C_7H_7Cl_2NO_2S$, 238.9574.

### Example 12

2-(Bromomethyl)benzenesulfonyl chloride

A solution of toluenesulfonyl chloride (4.5 g, *ca.* 85/15 mixture of ortho/para isomers) in carbon tetrachloride (40 ml) was contacted with *N*-bromosuccinimide (4.3 g) and azobisisobutyronitrile (50 mg) and heated to reflux for 22 hrs. The cooled mixture was filtered and the solvent was evaporated to give 6.33 g of yellow oil. Kugelrohr distillation provided 4.31 g, b.p. 90—98° (0.2 mm). $^1$H nmr $\delta^{TMS}_{CDCl_3}$ 8.46—7.25 (m), 5.0 (s, area 47), 4.58 (s, area 12), 2.76 and 2.47 (singlets for unchanged toluenesulfonyl chloride contaminant). The ortho/para ratio of bromomethyl compounds was 80/20.

Example 13

2-(Hydroxymethyl)benzenesulfonic acid, sodium salt

A solution of *o*-benzaldehydesulfonic acid, sodium salt (120 g) in water (2 l) was contacted in portions with sodium borohydride (21.6 g) and the solution was stirred for 48 hrs. The pH of the solution was adjusted to 7.0 by addition of hydrochloric acid. Water was removed under reduced pressure, and the residue was added to a large thimble and continuously extracted with ethanol. Concentration and cooling provided 102 g. of white solid, m.p. >300°. Evaporation of the mother liquor gave an additional 13.4 g of product. $^1H$ nmr $\delta^{DSS}_{D2O}$ 8.10—7.85 (m), 7.77—7.26 (m). 5.13 (s), 4.70 (s).

Example 14

2-(Chloromethyl)benzenesulfonyl chloride

2-(Hydroxymethyl)benzenesulfonic acid, sodium salt (76 g) was contacted with phosphorus oxychloride (375 ml) and the stirred mixture was contacted in portions with phosphorus pentachloride (76 g). The mixture was heated at 60—70° for two days. Phosphorus oxychloride was removed under vacuum and the residue was contacted with toluene (700 ml) and decanted into water. The organic layer was washed with water and brine, dried ($MgSO_4$) and evaporated. The product was kugelrohr distilled 90—100°, 0.2 mm) to give 56 g of white solid, m.p. 43—45°. $^1H$ nmr $\delta^{TMS}_{CDCl_3}$ 8.17—8.00 (m), 7.90—7.40 (m), 5.10 (s).

Example 15

2-(Chloromethyl)benzenesulfonamide

A solution of ammonia (5.2 ml) in tetrahydrofuran (250 ml) at −70° was contacted rapidly with a solution of 2-(chloromethyl)benzenesulfonyl chloride (21.8 g) in tetrahydrofuran (125 ml). The stirred mixture was allowed to warm to 0° and volatiles were removed under vacuum. The residue was contacted with ethyl acetate and a small volume of water. The organic layer was dried ($MgSO_4$) and evaporated to give 19.8 g of white solid, m.p. 156—157.5°. Recrystallization from toluene/ethyl acetate gave a sample with m.p. 157—160°. $^1H$ nmr $\delta^{TMS}_{(CD_3)_2CO}$ 8.16—7.35 (m), 6.75 (brds), 5.20 (s), 3.08 (s).

Example 16

2-(1-Chloroethyl)benzenesulfonamide

A solution of ammonia (1.2 ml) in tetrahydrofuran (100 ml) at −70° was contacted rapidly with a solution of 2-(1-chloroethyl)benzenesulfonyl chloride (4.32 g, 18.1 mmol) in tetrahydrofuran (50 ml). The stirred mixture was allowed to warm to 0° and volatiles were removed under reduced pressure. The residue was taken up in ethyl acetate and washed with a small volume of water and brine. The dried ($MgSO_4$) solution was evaporated to give 4.7 g of off white solid which was recrystallized from chloroform to give 3.13 g, m.p. 121—122°. $^1H$ nmr $\delta^{TMS}_{(CD_3)_2CO}$ 8.25—7.30 (m), 6.93 (brds), 6.25 (q, J=7Hz), 3.17 (s), 1.98 (d, J=7Hz).

Example 17

2-(Bromomethyl)benzenesulfonamide

A solution of ammonia (1.0 ml) in tetrahydrofuran (50 ml) at −78° was contacted dropwise with a solution of a 60/40 mixture of 2-(bromomethyl)benzenesulfonyl chloride/4-(bromomethyl)benzenesulfonyl chloride (4.52 g) in tetrahydrofuran (20 ml). The stirred mixture was allowed to warm to 0° and volatiles were removed *in vacuo*. The residue was contacted with water (50 ml) and extracted three times with ethyl acetate. Combined organic portions were washed with brine, dried, and evaporated to give 4.00 g of white solid. $^1H$ nmr $\delta^{TMS}_{(CD_3)_2CO}$ 8.15—7.20 (m), 6.7—6.3 (M, NH), 4.95 (q, area 21), 4.55 (s, area 18), consistent with a *ca.* 60/40 mixture of 2-(bromomethyl)-/4-(bromomethyl)benzenesulfonamides. The sample was fractionally recrystallized from chloroform, but without significant change in isomeric content.

Example 18

A solution of 2-(chloromethyl)benzenesulfonamide (2.00 g, 10 mmol) in acetonitrile (20 ml) was contacted with pyrrolidine (1.6 ml, 20 mmol). After 1.0 hr at ambient temperature, the volatiles were removed under vacuum and the residue was treated with water (40 ml) and chilled. The resulting solid was pulverized, filtered, and dried to give 2.23 g of white solid, m.p. 110—112°.

| | | | |
|---|---|---|---|
| *Anal.* Found: | C, 55.14; | H, 6.71; | N, 11.40 |
| | 54.66 | 6.49 | 11.49 |
| Calc'd for $C_{11}H_{16}N_2O_2S$: | C, 54.97; | H, 6.71; | N, 11.66 |

Example 19

2-(1-Pyrrolidinylmethyl)benzenesulfonamide, methyl iodide salt

A solution of 2-(pyrrolidinomethyl)benzenesulfonamide (0.8 g) in acetonitrile (10 ml) was contacted with methyl iodide (3 ml) and heated to reflux for 2.0 hrs: Solvent was removed and the residue was treated with acetone and ether. The solid was filtered, digested with acetone (20 ml), chilled, and collected. There was obtained 1.00 g of white solid. $^1H$ nmr $\delta^{TMS}_{DMSO-d_6}$ 8.15—7.30 (m), 5.07 (s, 2H), 3.9—3.3 (m, 4H), 2.90 (s, 3H), 2.4—1.8 (m, 4H).

Example 20

2-(Chloromethyl)-*N*-(butylaminocarbonyl)benzenesulfonamide

A mixture of 2-(chloromethyl)benzenesulfonamide (13.2 g, 64 mmol) and butyl isocyanate (7.0 g, 71 mmol) in xylene (80 ml) was contacted with diazabicyclo[2.2.2]octane (0.25 g) and heated to reflux for 5.0 hrs. Volatiles were removed under vacuum and the residue was treated with 0.5 *N* NaOH (200 ml) and extracted with ethyl acetate (75 ml). The aqueous layer was separated, chilled, acidified (pH 4) with hydrochloric acid, and extracted with ethyl acetate. The organic layer was washed with brine, dried ($MgSO_4$), and evaporated to give 14.6 g of white solid. A sample was recrystallized from toluene to give m.p. 104—106°. $^1H$ nmr $\delta^{TMS}_{CDCl_3}$ 9.5—8.1 (brds), 8.05—7.10 (m), 6.28 (t, J=2Hz), 4.91 (s), 3.25—2.95 (m), 1.70—0.70 (m).

Example 21

Methyl o-Isocyanatosulfonylbenzeneacetate

A mixture of 22.9 g methyl *o*-aminosulfonylbenzeneacetate, 13.5 g *n*-butyl isocyanate, 15.2 g potassium carbonate and 250 ml methyl ethyl ketone was stirred and refluxed for three hours. The reaction mixture was cooled, poured into 1250 g of ice-water, and acidified to pH 1.5 with concentrated HCl. The product was filtered, washed and dried. The crude product was recrystallized from 1-chlorobutane to yield 24.3 g of purified N-(*n*-butyl)sulfonylurea derivative of methyl *o*-isocyanatosulfonylbenzeneacetate, m.p. 164—166° (dec.).

To 16.4 g of the above N-(*n*-butyl)sulfonylurea derivative was added 150 ml of xylene and 0.2 g DABCO. The mixture was stirred and heated until 30 ml of xylene had been distilled off. The distillation head was replaced with a dry-ice reflux condenser, and then phosgene was slowly passed into the reaction mixture, which was maintained at 130—138°, over about one hour. When no more phosgene was taken up, the mixture was heated at 132—133° under slow phosgene reflux for one hour longer. The reaction mixture was cooled to room temperature, filtered to remove some solids and concentrated *in vacuo* to yield 25.0 g of the desired sulfonyl isocyanate product as an oil.

Example 22

Methyl 2-[[(4,6-dimethoxypyrimidin-2-yl)aminocarbonyl]aminosulfonyl]benzeneacetate

The 25 g of methyl *o*-isocyanatosulfonylbenzeneacetate from Example 1 was dissolved in 150 ml dry acetonitrile, and 0.2 g DABCO was added. To the stirred solution at 27° was added all at once 7.6 g of 2-amino-4,6-dimethoxypyrimidine. The temperature rose rapidly to 42°. The reaction mixture was then stirred and heated at 48—50° for two hours, and stirred at ambient temperature for a further sixteen hours. The product was recovered by filtration and dried; 16.3 g of the desired sulfonylurea was obtained, m.p. 193—196° (dec.). The product absorbed in the IR at 1730, 1610 and 1570 $cm^{-1}$. It showed NMR absorption at δ3.8 (s, 3H), methyl ester: δ4.1 (s, 6H), pyrimidine methoxyl groups; δ4.3 (s, 2H), benzeneacetic group; δ6.2 (s, 1H), pyrimidine ring hydrogen; δ7.3—8.5 (m, 4H), aromatic hydrogens.

| | | | | |
|---|---|---|---|---|
| Anal. Calc'd for $C_{16}H_{18}N_4O_7S$: | C, 46.82; | H, 4.42; | N, 13.66; | S, 7.81. |
| Found: | C, 46.9, 47.0; | H, 4.4, 4.4; | N, 14.0, 14.0; | S, 8.3, 8.0. |

All data are consistent with the structure of the desired sulfonylurea.

Example 23

2-[[(4,6-Dimethoxypyrimidin-2-yl)aminocarbonyl]aminosulfonyl]benzeneacetic acid

1.03 g of the product of Example 2 was added to a stirred solution of 0.5 g of commercial 50% aqueous sodium hydroxide mixed with 25 ml of water at ambient temperature. A clear solution was obtained after a few minutes. Stirring at ambient temperature was maintained for two hours. 2N hydrochloric acid was added dropwise to pH 2, causing the desired product to precipitate out. The solid product was filtered, washed and dried; 0.95 g was obtained, m.p. 173—175° (dec.). The product absorbed in the IR at 1730, 1690, 1610 and 1580 $cm^{-1}$. It showed NMR absorption at δ4.0 (s, 6H), pyrimidine methoxyl groups; δ4.1 (s, 2H), benzeneacetic group; δ5.9 (s, 1H), pyrimidine ring hydrogen; δ7.3—8.4 (m, 4H), aromatic hydrogens; δ10.5 (s, 1H), carboxylic acid. The spectral data are consistent with the structure of the desired sulfonylurea.

Example 24

Methyl 2-[[(4-methoxy-6-methyl-1,3,5-triazin-2-yl)aminocarbonyl]aminosulfonyl]benzeneacetate

2.3 g Methyl *o*-isocyanatosulfonylbenzeneacetate prepared as described in Example 1, and 0.03 g DABCO were dissolved in 30 ml dry acetonitrile. 1.2 g of 2-Amino-4-methoxy-6-methyl-1,3,5-triazine was added all at once to the stirred solution, which was heated for two hours at 50—55°, then for sixteen hours at ambient temperature. The reaction mixture was filtered and the solids obtained were washed and dried (1.0 g). The solids, which were a mixture of the desired product and unreacted 2-amino-4-methoxy-6-methyl-1,3,5-triazine, were dissolved in 100 ml methylene chloride and washed with 0.3 N hydrochloric acid, which removed unreacted aminotriazine. Evaporation of the methylene chloride yielded 0.7 g of the desired sulfonylurea; m.p. 162—165° (dec.). The product showed NMR absorption at $\delta$2.9 (s, 3H), triazine methyl; $\delta$3.9 (s, 3H), methyl ester; $\delta$4.35 (s, 2H), benzeneacetic group; $\delta$4.4 (s, 3H), triazine methoxyl; $\delta$7.4—8.6 (m, 4H) aromatic hydrogens; consistent with the structure of the desired sulfonylurea.

Example 25

Ethyl 2-[[(4,6-dimethoxypyrimidin-2-yl)aminocarbonyl]aminosulfonyl]benzeneacetate

Following the procedure of Example 1, ethyl *o*-aminosulfonylbenzeneacetate was reacted with *n*-butyl isocyanate to form the N-(*n*-butyl)sulfonylurea derivative; m.p. 140—142°.

Following the procedure of Example 1, the N-(*n*-butyl)sulfonylurea derivative was reacted with phosgene to form ethyl *o*-isocyanatosulfonylbenzeneacetate, an oil. 1.35 g of Ethyl *o*-isocyanatosulfonylbenzeneacetate and 0.015 g of DABCO were dissolved in 15 ml acetonitrile. 0.8 g of 2-Amino-4,6-dimethoxypyrimidine was added all at once, and the reaction mixture was stirred for sixteen hours at ambient temperature. The product was recovered by filtration; 1.2 g of the desired sulfonylurea was obtained, m.p. 168—172° (dec.). The product showed NMR absorption at $\delta$1.1—1.4 (t, 3H), methyl group of ethyl ester; $\delta$4.2 (s, 6H), methoxyl groups of pyrimidine; $\delta$4.1—4.4 (q, 2H), methylene of ethyl ester; $\delta$4.3 (s, 2H), methylene of benzene acetic group; $\delta$6.3 (s, 1H), pyrimidine hydrogen; $\delta$7.4—8.4 (m, 4H), aromatic hydrogens; consistent with the structure of the desired sulfonylurea.

Example 26

Methyl 2-[[(4,6-dichloropyrimidin-2-yl)aminocarbonyl]aminosulfonyl]benzeneacetate

To 2.3 g of 2-(aminosulfonyl)benzeneacetic acid methyl ester in 30 ml of acetonitrile was added 1.5 g of anhydrous potassium carbonate and 1.6 g of 4,6-dichloropyrimidin-2-yl isocyanate. The mixture was stirred at room temperature for 6 hours, diluted with 250 g of water and acidified to a pH of about 4. The resulting solid was filtered and dried to yield 1.6 g of methyl 2-[[(4,6-dichloro-pyrimidin-2-yl)aminocarbonyl]aminosulfonyl]benzeneacetate, a pale yellow solid, m.p. 115—118°C. It showed infrared absorption peaks at 1730, 1600 and 1560 $cm^{-1}$ and nuclear magnetic resonance peaks at 3.7 ppm, methyl ester group; 4.2 ppm, benzeneacetic group; 6.9 ppm, pyrimidin H; 7.4—7.6 ppm, aromatic hydrogens; consistent for the product.

Example 27

Methyl 2-[[(4,6-dimethoxy-1,3,5-triazin-2-yl)aminothioxomethyl]aminosulfonyl]benzeneacetate

To 1.1 g of 2-(aminosulfonyl)benzeneacetic acid methyl ester in 25 ml of ethyl methyl ketone was added 1.9 g of anhydrous potassium carbonate and 1.0 g of 2,4-dimethoxy-1,3,5-triazinyl-6-isothiocyanate. The mixture was stirred at room temperature for 24 hours and then diluted with 200 g of ice-water. The solution was acidified with dilute HCl to a pH of about 4. The resulting product was filtered and dried to give 1.2 g of white solid, m.p. 164—168°C. It showed infrared absorption peaks at 1750, 1630 and 1570 $cm^{-1}$ and nuclear magnetic resonance peaks at 3.9, 4.4, 4.5 and 7.6 ppm, consistent for the product.

Example 28

2-[[(4,6-Dimethoxypyrimidin-2-yl)aminocarbonyl]aminosulfonyl]-N,N-tetramethylene-benzeneacetamide

To 1.42 g of pyrrolidine in 100 ml of dry methylene chloride at room temperature was added 10 ml of commercial two molar trimethylaluminum in toluene. The temperature rose by 5°. After the resulting solution had been stirred for ten minutes, 4.1 g of the product of Example 2 was added portionwise over five minutes. The reaction mixture was stirred and refluxed for sixteen hours. 200 ml of water and 10 ml of concentrated hydrochloric acid was then added to the reaction mixture, the methylene chloride layer was separated, dried by addition of magnesium sulfate, and the solvent removed by distillation at reduced pressure. The residue was a gum. Trituration of the gum with 1-chlorobutane resulted in the formation of crystals. The product was filtered, washed and dried, 3.8 g was obtained; m.p. 133—135° (dec.). The product absorbed in the IR at 1720, 1650, 1600 and 1550 $cm^{-1}$. It showed NMR absorption at $\delta$2.1—2.5 (m, 4H), pyrrolidine hydrogens at positions 3 and 4; $\delta$3.7—4.1 (m, 4H), pyrrolidine hydrogens at positions 2 and 5; $\delta$4.1 (s, 6H), pyrimidine methoxyl groups, $\delta$4.6 (s, 2H), benzeneacetic group; $\delta$6.2 (s, 1H), pyrimidine ring hydrogen; $\delta$7.4—8.4 (m, 4H) aromatic hydrogens. The spectral data are consistent with the structure of the desired sulfonylurea.

Example 29

*o*-Methylthiomethylnitrobenzene

To a stirred solution of 10.8 g (0.2 mol) of sodium methoxide in 70 ml of methanol at room temperature was added dropwise 12 ml (10.8 g, 0.22 mol) of methyl mercaptan through a gas addition funnel connected to a dry-ice condenser. The resulting mixture was stirred at room temperature for 1 hour and then added dropwise to a stirred solution of 30.0 g (0.175 mole) of *o*-nitrobenzyl chloride in 120 ml of methanol over 1 hour, while the temperature rose to 40°C. The reaction mixture was then diluted with 300 ml of water, and extracted 3 times with methylene chloride. The methylene chloride extracts were washed with dilute hydrochloric acid, dried, and concentrated to afford 38.6 g (96%) of yellow oil.

NMR $(CDCl_3)\delta$:
1.97 (3H, s, $CH_3$);
3.97 (2H, s, $CH_2$);
7.3—8.1 (4H, m, 4 aromatic H's).

Example 30

*o*-Methylthiomethylaniline

Iron powder (60 g, 1.0 g-atom) was added slowly through a solid addition funnel to a solution of 53.5 g (0.29 mol) of *o*-methylthiomethylnitrobenzene in 210 ml of glacial acetic acid and 600 ml of absolute ethanol under reflux for a period of 45 minutes. The mixture was then refluxed for 3 hours. The iron powder was filtered off and the filtrate was diluted wtih 800 ml of water. The aqueoũs mixture was extracted twice with 200 ml ethyl acetate and twice with 200 ml ether. The combined organic extracts were washed with water, dried and concentrated to yield 48 g (75% pure) of orange oil.

NMR $(CDCl_3)\delta$:
1.90 (3H, s, $CH_3$);
3.60 (2H, s, $CH_2$);
5.72 (2H, bs, $NH_2$); and
6.5—7.3 (4H, m, 4 aromatic H's).

Example 31

*o*-Methylthiomethylbenzenesulfonyl chloride

To a mixture containing 48 g (75% pure, 0.23 mol) of *o*-methylthiomethylaniline in 280 ml of acetic acid and 90 ml of concentrated hydrochloric acid at 0° was added a solution of 20 g (0.3 mol) of sodium nitrite in 50 ml of water. The resulting diazonium salt solution was then added through a dropping funnel to a mixture of 50 ml of sulfur dioxide and 12 g of cupric chloride in 140 ml of acetic acid and 140 ml of ether at 0°C. The mixture was allowed to stand at 0°C overnight and then poured into 1 l of ice-water. Extraction with ether, washing, drying and evaporation of the solvent gave 26 g (50%) of red oil.

NMR $(CDCl_3)\delta$:
2.46 (3H, s, $CH_3$);
4.67 (2H, s, $CH_2$);
7.2—8.0 (4H, m, 4 aromatic H's);
plus one equivalent of *o*-methylthiomethylchlorobenzene;
2.07 (3H, s, $CH_3$);
4.18 (2H, s, $CH_2$); and
7.0—7.5 (aromatic H's).

The product was used for the next step without further purification.

Example 32

*o*-Methylthiomethylbenzenesulfonamide

To a solution of 26 g (50% pure, 55 mmol) of *o*-methylthiomethylbenzenesulfonyl chloride in 250 ml of methylene chloride was added 4.5 ml of anhydrous ammonia at 0—5° over 1 hour. Stirring was continued at 0—5°C for 2 hours. The solid precipitate was filtered off and the filtrate was evaporated to dryness to afford 24.8 g of orange residue. The crude product was triturated in ether, resulting in light pink crystals: m.p. 119—124°C.

NMR $(CDCl_3)\delta$:
2.05 (3H, s, $CH_3$);
4.18 (2H, s, $CH_2$);
6.72 (2H, bs, $NH_2$);
7.2—7.8 (3H, m, aromatic H's, m- & p-); and
8.0 (1H, dd, aromatic H, *o*-).

Example 33

*o*-Methylthiomethylbenzenesulfonyl isocyanate

Phosgene (2.5 ml, 35 mmol) was added portionwise into a nitrogen-purged mixture containing

6.8 g (31mmol) of *o*-methylthiomethylbenzenesulfonamide, 0.1 g of DABCO, and 3.8 g (38 mmol) of *n*-butyl isocyanate in 60 ml of dry xylene, while the temperature was maintained at 130—135°C between additions. Stirring was continued for 1 hour at 130—135°C. The mixture was cooled to room temperature and the solid was filtered off under nitrogen. The filtrate was concentrated to yield 9.0 g (theo 7.6 g) of oil: IR $cm^{-1}$ 2240 (N=C=O), no NH at ca 3300. The oil was taken up in 90 ml of dry methylene chloride.

Example 34

[N-(4,6-Dimethylpyrimidin-2-yl)aminocarbonyl]-2-methylthiomethylbenzenesulfonamide

A solution of *o*-methylthiomethylbenzenesulfonyl isocyanate in dry methylene chloride (15ml of the above solution from Example 5, 5 mmol) was added with a syringe into a nitrogen-purged flask containing 0.6 g (5 mmol) of 2-amino-4,6-dimethylpyrimidine and a trace of DABCO. The mixture was refluxed for $2\frac{1}{2}$ hours. The solvent was removed *in vacuo* to yield 1.8 g of white solid: m.p. 173—183°C. The unreacted pyrimidine can be removed by washing with dilute hydrochloric acid.

NMR (DMSO-$d_6$)$\delta$:

1.90 (3H, s, $SCH_3$);
2.37 (6H, pyrimidine 4 & 6-$CH_3$'s);
4.08 (2H, s, $CH_2$);
7.03 (1H, s, pyrimidine 5-H);
7.64 (3H, m, benzene H's, m- & p-);
8.16 (1H, m, benzene H, *o*-);
10.6 (1H, bs, $SO_2NHCON\underline{H}$); and
$SO_2N\underline{H}CONH$ not detectable.

Example 35

[N-[(4,6-Dimethylpyrimidin-2-yl)aminocarbonyl]-2-methylsulfinylmethylbenzenesulfonamide

One ml of 30% aqueous hydrogen peroxide was added to a suspension of 0.5 g (1.4 mmol) of [N-[4,6-dimethylpyrimidinyl)aminocarbonyl]-*o*-methylthiomethylbenzenesulfonamide in 15 ml of glacial acetic acid at room temperature. The mixture turned clear in 10 minutes and was stirred for a further 30 minutes. It was then diluted with water and extracted with methylene chloride. The organic extracts were washed, dried and concentrated to give 0.5 g (100%) of white solid: m.p. 176—184°C.

NMR (DMSO-$d_6$)$\delta$:

2.40 (1H, s, pyrimidine 4- & 6-$CH_3$'S);
2.57 (3H, s, $SCH_3$);
4.55 (2H, ABq, J=12 Hz, $ArCH_2\overset{O}{\overset{\|}{S}}$);
7.02 (1H, s, pyrimidine 5-H);
7.6 (3H, m, benzene m- & p- H's);
8.18 (1H, m, benzene O—H);
10.5 (1H, bs, $SO_2NHCON\underline{H}$); and
$SO_2N\underline{H}$ not detectable.

Example 36

[N-(4,6-Dimethylpyrimidin-2-yl)aminocarbonyl]-2-methylsulfonylmethylbenzenesulfonamide

A mixture of 0.1 g (0.2 mmol) of [N-(4,6-dimethylpyrimidinyl)aminocarbonyl-*o*-methylsulfinylmethylbenzenesulfonamide and 0.3 ml of 30% hydrogen peroxide in 5 ml of acetic acid was stirred at room temperature for 3 days. The resulting white solid precipitate was filtered to afford 30 mg of the desired product: m.p. 222—225°C.

NMR (DMSO-$d_6$)$\delta$:

2.42 (6H, s, pyrimidine 4- & 6-$CH_3$'s);
2.94 (3H, s, $SCH_3$);
5.10 (2H, s, $CH_2$);
7.00 (1H, s, pyrimidine 5-H);
7.7 (3H, m, benzene m- & p-H's);
8.1 (1H, m, benzene O—H);
10.5 (1H, bs, $SO_2NHCON\underline{H}$); and $SO_2N\underline{H}$ not detectable.

Example 37

2-(1-Methylethoxymethyl)benzenesulfonamide

To 4.9 g 2-(aminosulfonyl)benzoic acid, 1-methylethyl ester in 37 ml $BF_3 \cdot Et_2O$ at a temperature 0—10° was added over 45 min, 40 ml at 1 molar solution at $BH_3$ in THF. The mixture was refluxed overnight. The resulting cloudy yellow solution was concentrated on a rotary evaporator, acidified with 10% HCl, diluted with 300 ml $H_2O$ and then extracted with three 100 ml portions of ether. The ether

extracts were dried with magnesium sulfate and then concentrated to 4.1 g of wet solid. The product was further purified to an oil by column chromatography on silica with methylene chloride.

NMR $(CDCl_3)\delta$:
1.20 (6H, $(CH_3)_2$);
3.72 (1H, —CH);
4.88 (2H, $CH_2$);
7.2 (2H, $MH_2$); and
7.3—79 (4H, aromatic)

The product was converted to the corresponding sulfonylisocyanate by the procedure of Example 33. The resulting sulfonylisocyanate was used to prepare the corresponding sulfonylureas by the procedure of Example 34.

Example 38

2-Nitrophenylmethyl carbamimidothioate hydrochloride

A solution of 34.3 g of *o*-nitrobenzyl chloride and 15.2 g thiourea in 250 ml of #2B alcohol was refluxed for 1½ hours. The solution was cooled to 60° and 250 ml of 1-chlorobutane added. Further cooling to 20° yielded a precipitate which was filtered, washed with 1-chlorobutane and dried at 65° to give 38.1 g of 2-nitrophenylmethyl carbamimidothioate hydrochloride, m.p. 190—192°.

NMR (DMSO-$d_6$)$\delta$:
4.85 (s, 1.8H, $CH_2$);
7.4—8 (m, 4.2H, 4 aromatics);
9.7 (broad s, 4.0H, 4 NH's).

Example 39

N,N-Dimethyl-2-nitrobenzenemethanesulfonamide

To a slurry of 34.7 g of the compound of Example 1 in 350 ml of water was added 20.5 ml of liquid chlorine at 10—15° over a 45 minute period. After stirring an additional 15 minutes at 10°, the precipitated sulfonyl chloride was filtered off and washed well with water. The wet sulfonyl chloride filter cake was suspended in 200 ml of ether and contacted with 18.0 ml of liquid dimethylamine at 5—15°. After stirring at room temperature for 1½ hours, the precipitate was filtered off and washed well with water, then 1-chlorobutane. Oven drying at 60° overnight gave 15.9 g of N,N-dimethyl-2-nitrobenzenemethanesulfonamide, m.p. 129—132°.

NMR (DMSO-$d_6$)$\delta$:
2.7 (s, 6.2H, $SO_2NMe_2$);
4.8 (s, 1.9H, —$CH_2$—);
7.6—8.3 (m, 3.9H, 4 aromatics).

| | | | | |
|---|---|---|---|---|
| *Anal.* Calcd. for $C_9H_{12}N_2O_4S$: | C, 44.28; | H, 4.96; | N, 11.47; | S, 13.13. |
| Found: | C, 44.6; | H, 4.8 ; | N, 11.4 ; | S, 13.3 . |
| | 44.5 ; | 4.7 ; | 11.4 ; | 13.0 . |

Example 40

N,N-Dimethyl-2-aminobenzenemethanesulfonamide

In a pressure bottle, a mixture of 116 g of the product of Example 2, 1400 ml of 2-methoxyethyl ether and 10 g of 10% palladium on carbon was shaken at 110° under 500 p.s.i. hydrogen until the hydrogen was no longer absorbed. The catalyst was filtered off and the filtrate stripped under reduced pressure to a volume of 200 ml. This residue was poured into 600 ml of ice and the precipitate filtered off and dried to give 84 g of crude product, m.p. 70—78°. Recrystallization from ~600 ml of 1-chlorobutane gave 60.6 g of N,N-dimethyl-2-aminobenzenemethanesulfonamide, m.p. 92—100°.

NMR (DMSO-$d_6$)$\delta$:
2.7 (s, 5.8H, $SO_2NMe_2$);
4.3 (s, 2.1H, $CH_2$);
4.9—5.2 (broad s, 2.0H, $NH_2$);
6.4—7.3 (m, 4.1H, 4 aromatics).

Example 41

2-[(Dimethylamino)sulfonylmethyl]benzenesulfonamide

To a solution of 53.5 g of the product of Example 3 in a mixture of 225 ml of concentrated hydrochloric acid and 75 ml of glacial acetic acid was added a solution of 21.4 g of sodium nitrite in 70 ml of water at −5 to 0°. The solution was stirred at 0° for 15 minutes, then poured into a mixture of 6 g of cuprous chloride, 48 ml of liquid sulfur dioxide in 300 ml of glacial acetic acid at 0—5°. This mixture was stirred at 0° for 1 hour, then at 25° for 2 hours before being poured into 2 liters of ice-water. The precipitate was filtered and washed with water then suspended in 250 ml of ether and treated with

11.0 ml of liquid anhydrous ammonia at 5—15°. After stirring at 25° for 30 minutes the precipitate was filtered off and washed well with ether then water. Oven drying at 60° gave 40.2 g of 2-[(dimethylamino)sulfonylmethyl]benzenesulfonamide, m.p. 145—150°.

NMR (DMSO-$d_6$)$\delta$:

2.7 (s, 6.0H, $SO_2NMe_2$);
4.8 (s, 1.8H, $—CH_2—$);
7.2—8.1 (m, 6.2H, 4 aromatics + $SO_2NH_2$).

Example 42

2-[(Dimethylamino)sulfonylmethyl]benzenesulfonylisocyanate

A solution of 14.0 g of the product of Example 4, 5.0 g of *n*-butyl isocyanate and 0.1 g of DABCO in 90 ml of mixed xylenes was heated to 136°. To this solution was added 3.6 ml of liquid phosgene over a 2 hour period to maintain the temperature between 125° and 136°. The temperature was kept at 130° for ½ hour after the addition. The solution was cooled, and filtered under a nitrogen atmosphere and concentrated at 60—70° *in vacuo* to give 16.0 g of crude 2-[(dimethylamino)sulfonylmethyl]benzenesulfonyl isocyanate as a moisture sensitive oil. An infrared peak at 2200 $cm^{-1}$ confirmed the presence of the $—SO_2NCO$ group.

Example 43

2-[(Dimethylamino)sulfonylmethyl]-N-[(4,6-dimethoxypyrimidin-2-yl)aminocarbonyl]benzenesulfonamide

A mixture of 2.6 g of the product of Example 5, 0.9 g of 2-amino-4,6-dimethoxypyrimidine and a few crystals of DABCO in 15 ml of dry acetonitrile was heated at 50—55° for 1 hour under a nitrogen atmosphere, then stirred overnight at room temperature. The precipitate was filtered off, washed with acetonitrile and dried to give 2.1 g of 2-[(dimethylamino)sulfonylmethyl]-N-[(4,6-dimethoxypyrimidin-2-yl)aminocarbonyl]benzenesulfonamide, m.p. 172—176°.

NMR (DMSO-$d_6$)$\delta$:

2.8 (s, 6.3H, $SO_2NMe_2$);
4.0 (s, 5.6H, Het-$OCH_3$'s);
5.0 (s, 2.0H, $—CH_2—$);
6.1 (s, 0.8H, Het-H);
7.7—8.6 (m, 4.4H, 4 aromatics);
10.8 and 13.2 (broad singlets, NH's).

| | | | | |
|---|---|---|---|---|
| *Anal.* Calcd. for $C_{16}H_{21}N_5O_7S_2$: | C, 41.80; | H, 4.61; | N, 15.24; | S, 13.96. |
| Found: | C, 41.8 ; | H, 4.6 ; | N, 16.1 ; | S, 14.0 . |
| | 42.2 ; | 4.5 ; | 16.1 ; | 14.3 . |

Using the procedures and examples described above and choosing the appropriate aminoheterocycle and sulfonyl isocyanate or sulfonamide, the compounds described in Tables I—VI may be prepared.

Example 44

N-[(4,6-Dimethoxypyrimidin-2-yl)aminocarbonyl]-2-carboxybenzenesulfonamide

A mixture containing 5 g of N-[(4,6-dimethoxypyrimidin-2-yl)aminocarbonyl]-2-methoxycarbonylbenzenesulfonamide, 20 ml of ethanol, 2.5 ml of water and 2.5 g of potassium hydroxide was stirred at ambient temperature and pressure for 18 hours. The mixture was then diluted with 250 ml of water and 20 ml of concentrated hydrochloric acid was added with stirring. The precipitate was filtered and washed with water and dried to yield 4.85 g of the desired product, melting at 161—2°C. The infrared absorption peaks at 3500, 3400 and 1700 $cm^{-1}$ are consistent with the desired structure and the nuclear magnetic resonance absorption peaks at 3.95 ppm, S, 6H, $OCH_3$ of pyrimidine; 5.8 ppm S, 1H, pyrimidine proton at position 5; and 7.6—8.3 ppm, M, 4H, aromatic protons, are consistent with the desired structure.

Example 45

N-[(4,6-Dimethoxypyrimidin-2-yl)aminocarbonyl]-2-(hydroxymethyl)benzenesulfonamide

To a solution of 3.9 g of N-[(4,6-dimethoxypyrimidin-2-yl)aminocarbonyl]-2-carboxybenzenesulfonamide in 100 ml of tetrahydrofuran was added 50 ml of a 1M solution of borane, THF complex at 25°C. The mixture was stirred at 25°C for 18 hours followed by addition of water and HCl. The mixture was extracted with methylene chloride and the desired product crystallized from solution, 1.1 g 29% yield, m.p. 149—150°C. The infrared absorption peaks at 3300 $cm^{-1}$ and 1720 $cm^{-1}$ are consistent with the desired structure and the nuclear magnetic resonance absorption peaks at 3.95 ppm, S, 6H, $OCH_3$ of pyrimidine; 4.9 ppm, S, 2H, benzyl protons; 5.9 ppm, S, 1H, pyrimidine

proton at position 5; and 7.4—8.3 ppm, Multiplet, 4H, aromatic protons are consistent with the desired structure.

Example 46

N-[(4,6-Dimethoxypyrimidin-2-yl)aminocarbonyl]-2-methylcarbonylbenzenesulfonamide

A mixture containing 0.85 g of N-[(4,6-dimethoxypyrimidin-2-yl)aminocarbonyl]-2-carboxybenzenesulfonamide in 50 ml of anhydrous tetrahydrofuran was treated with 40 ml of 1.4 molar solution of methyl lithium (low halide, Aldrich) in ether at 25° under a nitrogen atmosphere. The mixture was stirred for 4 hours at 25° and was then poured into 500 ml of water containing 10 ml of concentrated hydrochloric acid. The precipitated oil was extrated into methylene chloride and the oil on evaporation of solvent was purified by preparative thin layer chromatography on silica gel (Analtec, 2000 micron, 20 x 20 plates) by elution with ethyl acetate/hexane in a one to one ratio. The isolated product was recrystallized from a 1-chlorobutane and hexane mixture to give 0.1 g, m.p. 126—8°. The infrared absorption showed a broadened carbonyl peak at 1710 $cm^{-1}$, and the absence of the 3500 and 3400 $cm^{-1}$ peaks of the starting material. The nuclear magnetic resonance spectrum showed peaks at 2.6 ppm S, 3H,

$$\mathrm{CH_3}\overset{\overset{\displaystyle \mathrm{O}}{\|}}{\mathrm{C}}\mathrm{Ar;}$$

4.0 ppm, S, 6H, $CH_3O$ of pyrimidine; 5.7 ppm S, 1H, pyrimidine proton at position 5; and 7.3—7.7 ppm and 8.0 ppm M, 4H, aromatic, which are consistent with the desired structure.

Example 47

N-[(4,6-Dimethoxypyrimidin-2-yl)aminocarbonyl]-2-($\alpha$-hydroxyethyl)benzenesulfonamide

A mixture containing 1.0 g of N-[(4,6-dimethoxypyrimidin-2-yl)aminocarbonyl]-2-methylcarbonylbenzenesulfonamide in 50 ml of anhydrous THF is treated with 0.1 g of lithium aluminum hydride at 25° under a nitrogen atmosphere. The mixture is stirred for 4 hours at 25° and is then poured into 500 ml of water containing 10 ml of concentrated hydrochloric acid. The precipitated oil is extracted into methylene chloride and the solid on evaporation is purified by column chromatography on silica gel.

Example 48

2-(Acetoxymethyl)benzenesulfonamide

A mixture of 2-(chloromethyl)benzenesulfonamide (2.00 g), potassium acetate (6.0 g) and water (75 ml) was heated to reflux for 1.5 hour. The pH was adjusted to *ca.* 5.0 by addition of hydrochloric acid and the chilled mixture was filtered to give 1.05 of shiny white solid, m.p. 131—133°. $^1H$ NMR $\delta(CD_3)_2CO$ 8.00—7.83 (M), 7.60—7.25 (M), 6.55 (brd s), 5.47 (2), 2.97 (brd s), 2.10 (s), consistent with the assigned structure.

Example 49

2-(Acetoxymethyl)-N-[(4,6-dichloro-1,3,5-triazin-yl)aminocarbonyl]benzenesulfonamide

A solution of 4,6-dichloro-1,3,5-triazin-2-yl isocyanate (0.87 g, 4.5 mmol) in acetonitrile (9 ml) was treated with 2-(acetoxymethyl)benzenesulfonamide (1.04 g, 4.5 mmol) and stirred for 16 hours. Volatiles were removed under vacuum to give a residue whose 'H NMR showed $\delta$ $(CD_3)_2CO$, 8.05—7.80 (m, 1H), 7.65—7.27 (m, 3H), 6.45 (brd s, 1H), 5.50 (s, 2H), 2.10 (s, 3H), consistent with the assigned structure.

Example 50

2-(Hydroxymethyl)-N-[(4,6-dimethoxy-1,3,5-triazin-2-yl)aminocarbonyl]benzenesulfonamide

The crude 2-(acetoxymethyl)-N-[(4,6-dichloro-1,3,5-triazinyl)aminocarbonyl]benzenesulfonamide was treated with methanol (10 ml) and then with a solution of sodium methoxide (14 mmol) in methanol. The mixture was stirred at room temperature for 1.5 hour and evaporated. The residue was taken up in water and filtered. The filtrate was acidified and filtered to give 0.45 g of gummy solid which was recrystallized from a chloroform/acetone mixture to give 115 mg of white solid, m.p. 146—148° (dec.). 'H NMR $\delta$ DMSO-$d_6$ 12.2 (brd s, 1H), 10.73 (s, 1H), 8.00-7.15 (m, 4H), 4.80 (s and ~4.9—4.2 (brd s), 3H) 3.90 (s, 6H), consistent with the assigned structure.

Exemplary compounds within structure I that can be made by one or more of the described methods (Examples 1—50) are listed in Tables I—XXXIV.

TABLE I

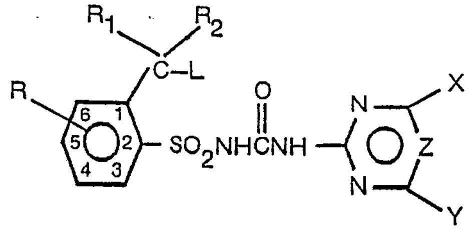

| L | R | $R_1$ | $R_2$ | X | Y | Z | m.p. (°C) |
|---|---|---|---|---|---|---|---|
| Cl | H | H | H | $CH_3$ | $CH_3$ | CH | 176—177° |
| Br | H | H | H | $CH_3$ | $CH_3$ | CH | |
| 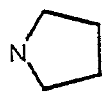 | H | H | H | $CH_3$ | $CH_3$ | CH | |
| 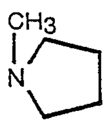 | H | H | H | $CH_3$ | $CH_3$ | CH | |
| $N(CH_3)_3$ | H | H | H | $CH_3$ | $CH_3$ | CH | |
| $NH\overset{O}{C}CH_3$ | H | H | H | $CH_3$ | $CH_3$ | CH | |
| $NH\overset{O}{\overset{\|}{C}}OCH_2CH_3$ | H | H | H | $SCH_3$ | $CH_3$ | CH | |
| $N(C_2H_5)\overset{O}{\overset{\|}{C}}OCH_3$ | H | H | H | Cl | $CH_3$ | CH | |
| $N(CH_3)_2$ | H | H | H | $CH_2OCH_3$ | $CH_3$ | CH | |
| $N(CH_3)(C_2H_5)$ | H | H | H | $CH_3$ | $CH_3$ | CH | |
| $N(C_2H_5)_2$ | H | H | H | $CH_3$ | $CH_3$ | CH | |
| $N(C_3H_7)_2$ | H | H | H | $CH_3$ | $CH_3$ | CH | |
| 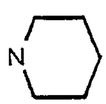 | H | H | H | $CH_3$ | $CH_3$ | CH | |
| 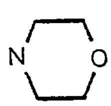 | H | H | H | $CH_3$ | $CH_3$ | CH | |
| $N(OCH_3)CH_3$ | H | H | H | $CH_3$ | $CH_3$ | CH | |
| $NH_2$ | H | H | H | $CH_3$ | $CH_3$ | CH | |
| $NHCH_3$ | H | H | H | $OCH(CH_3)_2$ | $CH_3$ | CH | |
| $NHC_4H_9$ | H | H | H | $CH_3$ | $CH_3$ | CH | |
| $NH\overset{O}{\overset{\|}{C}}CH_2Cl$ | H | H | H | $CH_3$ | $CH_3$ | CH | |

TABLE I (contd.)

| L | R | $R_1$ | $R_2$ | X | Y | Z | m.p.(°C) |
|---|---|---|---|---|---|---|---|
| $NH\overset{O}{\overset{\|}{C}}OCl_3$ | H | H | H | $CH_3$ | $CH_3$ | CH | |
| Cl | H | $CH_3$ | H | $CH_3$ | $CH_3$ | CH | |
| Cl | H | $CH_2CH_2CH_3$ | H | $CH_3$ | $CH_3$ | CH | |
| Cl | H | n-$C_4H_9$ | H | $CH_3$ | $CH_3$ | CH | |
| Cl | H | $CH_3$ | H | $CH_3$ | $CH_3$ | C—Cl | |
| Cl | H | H | H | $CH_3$ | $OCH_3$ | CH | |
| Br | H | H | H | $CH_3$ | $OCH_3$ | CH | |
| Cl | H | H | $CH_3$ | $CH_3$ | $OCH_3$ | CH | |
| Cl | H | H | H | $OCH_3$ | $OCH_3$ | CH | 192—193° |
| Br | H | H | H | $OCH_3$ | $OCH_3$ | CH | |
| 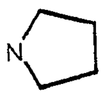 | H | H | H | $OCH_3$ | $OCH_3$ | CH | 138—140° |
| $N(CH_3)_3$ | H | H | H | $OCH_3$ | $OCH_3$ | CH | |
| Cl | H | H | $CH_3$ | $OCH_3$ | $OCH_3$ | CH | |
| 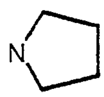 | H | H | $CH_3$ | $OCH_3$ | $OCH_3$ | CH | |
| Cl | 5-Cl | H | H | $CH_3$ | $CH_3$ | CH | |
| Cl | 4-$NO_2$ | H | H | $CH_3$ | $OCH_3$ | CH | |
| Br | 4-$NO_2$ | H | H | $CH_3$ | $OCH_3$ | CH | |
| Cl | 4-Cl | H | H | $OCH_3$ | $OCH_3$ | CH | |
| Cl | H | Cl | H | $CH_3$ | $CH_3$ | CH | 195—197° |
| Br | H | Cl | H | $CH_3$ | $CH_3$ | CH | |
| Cl | H | Cl | H | $OCH_3$ | $CH_3$ | CH | 189—190° |
| Cl | H | Cl | H | $OCH_3$ | $OCH_3$ | CH | |
| Cl | H | H | H | $CH_3$ | $CH_3$ | N | |
| Cl | H | H | H | $CH_3$ | $OCH_3$ | N | |
| Cl | H | H | H | H | $CH_3$ | N | |
| Cl | H | H | $CH_3$ | $CH_3$ | $CH_3$ | N | |
| Cl | H | H | $CH_3$ | $CH_3$ | $OCH_3$ | N | |
| Cl | H | H | H | $OCH_3$ | $OCH_3$ | N | 150—152° |
| Cl | H | H | $CH_3$ | $OCH_3$ | $OCH_3$ | N | 148—150° |
| Br | H | H | $CH_3$ | $OCH_3$ | $OCH_3$ | N | |

TABLE I (contd.)

| L | R | $R_1$ | $R_2$ | X | Y | Z | m.p.(°C) |
|---|---|---|---|---|---|---|---|
| 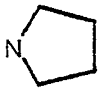 | H | H | $CH_3$ | $OCH_3$ | $OCH_3$ | N | |
| Cl | 4-$NO_2$ | H | H | $CH_3$ | $CH_3$ | N | |
| Cl | 4-$NO_2$ | H | H | $CH_3$ | $OCH_3$ | N | |
| Cl | 4-Cl | H | H | $CH_3$ | $OCH_3$ | N | |
| 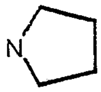 | 5-Cl | H | H | $CH_3$ | $OCH_3$ | N | |
| 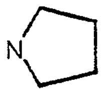 | 5-F | H | H | $OCH_3$ | $OCH_3$ | N | |
| Cl | H | Cl | H | $CH_3$ | $CH_3$ | N | |
| Cl | H | Cl | H | $OCH_3$ | $CH_3$ | N | 168—169° |
| Cl | H | Cl | H | $OCH_3$ | $OCH_3$ | N | |
| $OCH_3$ | H | H | H | $CH_3$ | $CH_3$ | CH | 164—167° |
| $OCH_3$ | H | H | H | $OCH_3$ | $CH_3$ | CH | 163—166° |
| $OCH_3$ | H | H | H | $OCH_3$ | $OCH_3$ | CH | 171—176° |
| $SCH_3$ | H | H | H | $CH_3$ | $CH_3$ | CH | 173—183° |
| $SCH_3$ | H | H | H | $OCH_3$ | $CH_3$ | CH | 173—184° |
| $SCH_3$ | H | H | H | $OCH_3$ | $OCH_3$ | CH | 165—181° |
| $S(O)CH_3$ | H | H | H | $CH_3$ | $CH_3$ | CH | 176—184° |
| $S(O)CH_3$ | H | H | H | $OCH_3$ | $CH_3$ | CH | 142—150° |
| $S(O)CH_3$ | H | H | H | $OCH_3$ | $OCH_3$ | CH | 178—184° |
| $S(O)_2CH_3$ | H | H | H | $CH_3$ | $CH_3$ | CH | 222—225° |
| $S(O)_2CH_3$ | H | H | H | $OCH_3$ | $CH_3$ | CH | 195—198° |
| $S(O)_2CH_3$ | H | H | H | $OCH_3$ | $OCH_3$ | CH | 193—199° |
| $S(O)CH_3$ | H | $CH_3$ | H | $CH_3$ | $CH_3$ | CH | |
| $OCH_3$ | H | $CH_3$ | $CH_3$ | $CH_3$ | $CH_3$ | CH | |
| $S(O)_2CH_3$ | H | $CH_3$ | $CH_3$ | $OCH_3$ | $OCH_3$ | CH | |
| $OCH_2CH_3$ | H | H | H | $CH_3$ | $CH_3$ | CH | 133—140° |
| $OCH_2CH_3$ | H | H | H | $OCH_3$ | $CH_3$ | CH | 124—130° |
| $OCH_2CH_3$ | H | H | H | $OCH_3$ | $OCH_3$ | CH | 122—127° |
| $SCH_2CH_3$ | H | H | H | $CH_3$ | $CH_3$ | CH | |
| $SCH_2CH_3$ | H | H | H | $OCH_3$ | $OCH_3$ | CH | |

TABLE I (contd.)

| L | R | $R_1$ | $R_2$ | X | Y | Z | m.p.(°C) |
|---|---|---|---|---|---|---|---|
| $S(O)C_2H_5$ | H | H | H | $OCH_3$ | $OCH_3$ | CH | |
| $S(O)_2C_2H_5$ | H | H | H | $OCH_3$ | $CH_3$ | CH | |
| $OCH(CH_3)_2$ | H | H | H | $CH_3$ | $CH_3$ | CH | 165—171° |
| $OCH(CH_3)_2$ | H | H | H | $OCH_3$ | $CH_3$ | CH | 147—150° |
| $OCH(CH_3)_2$ | H | H | H | $OCH_3$ | $OCH_3$ | CH | 120—130° |
| $SCH(CH_3)_2$ | H | H | H | $CH_3$ | $CH_3$ | CH | |
| $S(O)CH(CH_3)_2$ | H | H | H | $CH_3$ | $CH_3$ | CH | |
| $O(CH_2)_3CH_3$ | H | H | H | $CH_3$ | $CH_3$ | CH | 136—142° |
| $O(CH_2)_3CH_3$ | H | H | H | $OCH_3$ | $CH_3$ | CH | 134—139° |
| $O(CH_2)_3CH_3$ | H | H | H | $OCH_3$ | $CH_3$ | CH | 116—121° |
| $OCH(CH_3)CH_2CH_3$ | H | H | H | $CH_3$ | $CH_3$ | CH | 112—121° |
| $OCH(CH_3)CH_2CH_3$ | H | H | H | $OCH_3$ | $CH_3$ | CH | 125—128° |
| $OCH(CH_3)CH_2CH_3$ | H | H | H | $OCH_3$ | $OCH_3$ | CH | 114—116° |
| $OCH_3$ | H | H | H | $CH_3$ | $CH_3$ | N | 123—135° |
| $OCH_3$ | H | H | H | $OCH_3$ | $CH_3$ | N | 137—140° |
| $OCH_3$ | H | H | H | $OCH_3$ | $OCH_3$ | N | 138—143° |
| $SCH_3$ | H | H | H | $CH_3$ | $CH_3$ | N | 162—180° |
| $SCH_3$ | H | H | H | $OCH_3$ | $CH_3$ | N | 141—150° |
| $SCH_3$ | H | H | H | $OCH_3$ | $OCH_3$ | N | 113—133° |
| $S(O)CH_3$ | H | H | H | $CH_3$ | $CH_3$ | N | 169—172° |
| $S(O)CH_3$ | H | H | H | $OCH_3$ | $CH_3$ | N | 166—171° |
| $S(O)CH_3$ | H | H | H | $OCH_3$ | $OCH_3$ | N | 161—167° |
| $S(O)_2CH_3$ | H | H | H | $CH_3$ | $CH_3$ | N | 212—216° |
| $S(O)_2CH_3$ | H | H | H | $OCH_3$ | $CH_3$ | N | 185—189° |
| $S(O)_2CH_3$ | H | H | H | $OCH_3$ | $OCH_3$ | N | 189—195° |
| $OCH_3$ | H | $CH_3$ | H | $OCH_3$ | $OCH_3$ | N | |
| $SCH_3$ | H | $CH_3$ | H | $CH_3$ | $CH_3$ | N | |
| $S(O)CH_3$ | H | $CH_3$ | H | $OCH_3$ | $CH_3$ | N | |
| $S(O)_2CH_3$ | H | $CH_3$ | H | $OCH_3$ | $CH_3$ | N | |
| $OCH_2CH_3$ | H | H | H | $OCH_3$ | $CH_3$ | N | 118—124° |
| $OCH_2CH_3$ | H | H | H | $OCH_3$ | $OCH_3$ | N | 131—140° |
| $SCH_2CH_3$ | H | H | H | $OCH_3$ | $CH_3$ | N | |

TABLE I (contd.)

| L | R | $R_1$ | $R_2$ | X | Y | Z | m.p. (°C) |
|---|---|---|---|---|---|---|---|
| $S(O)_2CH_2CH_3$ | H | H | H | $OCH_3$ | $CH_3$ | N | |
| $OCH(CH_3)_2$ | H | H | H | $CH_3$ | $CH_3$ | N | 134—137° |
| $OCH(CH_3)_2$ | H | H | H | $OCH_3$ | $CH_3$ | N | |
| $OCH(CH_3)_2$ | H | H | H | $OCH_3$ | $OCH_3$ | N | |
| $S(O)CH(CH_3)_2$ | H | H | H | $OCH_3$ | $CH_3$ | N | |
| $S(O)_2CH(CH_3)_2$ | H | H | H | $OCH_3$ | $CH_3$ | N | |
| $O(CH_2)_3CH_3$ | H | H | H | $CH_3$ | $CH_3$ | N | 98—105° |
| $O(CH_2)_3CH_3$ | H | H | H | $OCH_3$ | $CH_3$ | N | 84—98° |
| $O(CH_2)_3CH_3$ | H | H | H | $OCH_3$ | $OCH_3$ | N | 125—128° |
| $OCHCH_2CH_3$<br>\|<br>$CH_3$ | H | H | H | $CH_3$ | $CH_3$ | N | 105—110° |
| $OCHCH_2CH_3$<br>\|<br>CH | H | H | H | $OCH_3$ | $OCH_3$ | N | 112—121° |
| $OC_2H_5$ | H | $CH_3$ | H | $CH_3$ | $CH_3$ | N | |
| $OCH_2CF_3$ | 6-Cl | H | H | $CH_3$ | $OCH_3$ | CH | |
| $OCF_2CF_2H$ | 4-Br | H | H | $CH_3$ | $OCH_3$ | N | |
| $OCH_2CH{=}CH_2$ | 6-$NO_2$ | H | H | $OCH_2CH_3$ | $CH_3$ | CH | |
| $OCH_2C(CH_3){=}CH_2$ | 4-$CF_3$ | H | H | $OCH_2CH_2CH_3$ | $OCH_3$ | N | |
| 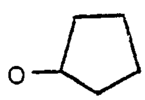 | H | H | H | $CH_3$ | $CH_3$ | CH | |
| 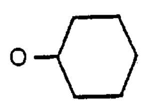 | H | H | H | $CH_3$ | $OCH_3$ | N | |
| $OCH_2CH(CH_3)_2$ | 4-$(CH_3)_2CHO$ | H | H | $OCH_3$ | $CH_3$ | N | |
| $OCH_3$ | H | H | H | $OCH_3$ | $CH_3$ | C—C | |
| $CO_2CH_3$ | H | H | H | $CH_3$ | $CH_3$ | N | |
| $CO_2CH_3$ | H | H | H | $CH_3$ | $CH_3O$ | CH | 194—196° (dec) |
| $CO_2CH_3$ | H | H | H | $CH_3$ | $CH_3$ | CH | 195—198° (dec) |
| $CO_2CH_3$ | H | H | H | $CH_3O$ | $CH_3O$ | N | 182—184° (dec) |
| $CO_2C_2H_5$ | H | H | H | $CH_3$ | $CH_3$ | CH | |
| $CO_2C_2H_5$ | H | H | H | $CH_3$ | $CH_3O$ | CH | 168—170° (dec) |
| $CO_2C_2H_5$ | H | H | H | $CH_3O$ | $CH_3O$ | N | |
| $CO_2CH(CH_3)_2$ | H | H | H | $CH_2CH_3$ | $OCH_3$ | N | |

TABLE I (contd.)

| L | R | $R_1$ | $R_2$ | X | Y | Z | m.p.(°C) |
|---|---|---|---|---|---|---|---|
| $CO\text{-}n\text{-}C_4H_9$ | H | H | H | $CH_2OCH_3$ | $OCH_3$ | N | |
| $CO_2C_2H_5$ | H | H | H | $CH_3$ | $CH_3O$ | N | |
| $CO_2—CH_2—CH{=}CH_2$ | H | H | H | $CH_3O$ | $CH_3O$ | CH | 174—177° (dec) |
| $CO_2—CH_2—CH{=}CH_2$ | H | H | H | $CH_3$ | $CH_3O$ | N | |
| $CO_2CH(CH_3)_2$ | H | H | H | $CH_3$ | $CH_3O$ | CH | 135—150° |
| $CO_2CH(CH_3)_2$ | H | H | H | $CH_3O$ | $CH_3O$ | CH | 165—168° (dec) |
| $CO_2H$ | H | H | H | $CH_3$ | $CH_3$ | CH | 162—164° (dec) |
| $CO_2H$ | H | H | H | $CH_3O$ | $CH_3$ | CH | 207—209° (dec) |
| $CO_2H$ | H | H | H | $CH_3O$ | $CH_3O$ | CH | 173—175° (dec) |
| $CO_2H$ | H | H | H | $CH_3O$ | $CH_3$ | N | 142—144° (dec) |
| $CO_2H$ | H | H | H | $CH_3O$ | $CH_3O$ | N | 155—157° (dec) |
| $CO_2CH_3$ | H | $CH_3$ | H | $CH_3$ | $CH_3O$ | CH | |
| $CO_2CH_3$ | H | $CH_3$ | H | $CH_3O$ | $CH_3O$ | CH | |
| $CO_2CH_3$ | H | $CH_3$ | H | $CH_3$ | $CH_3O$ | N | |
| $CO_2CH_3$ | H | $CH_3$ | H | $CH_3O$ | $CH_3O$ | N | |
| $CO_2CH_3$ | H | $CH_3CH_2$ | H | $CH_3O$ | $CH_3O$ | CH | |
| $CO_2CH_3$ | H | $n\text{-}C_4H_9$ | H | $CH_3O$ | $CH_3O$ | CH | |
| $CO_2CH_3$ | H | $CH_3$ | $CH_3$ | $CH_3O$ | $CH_3O$ | CH | |
| $CO_2CH_3$ | 5-F | H | H | $CH_3O$ | $CH_3O$ | CH | |
| $CO_2CH_3$ | 4-Cl | H | H | $CH_3O$ | $CH_3$ | N | |
| $CO_2CH_3$ | 4-Br | H | H | $CH_3O$ | $CH_3O$ | CH | |
| $CO_2CH_3$ | $4\text{-}CF_3$ | H | H | $CH_3O$ | $CH_3$ | N | |
| $CO_2CH_3$ | $4\text{-}CH_3$ | H | H | $CH_3O$ | $CH_3$ | N | |
| $CO_2CH_3$ | $5\text{-}OCH_3$ | H | H | $CH_3O$ | $CH_3O$ | CH | |
| $CO_2CH_3$ | $4\text{-}n\text{-}C_3H_7$ | H | H | $CH_3O$ | $CH_3$ | N | |
| $CO_2CH_3$ | $5\text{-}CH_3CH_2CH_2O$ | H | H | $CH_3O$ | $CH_3O$ | CH | |
| $CO_2CH_3$ | 3-Cl | H | H | $CH_3O$ | $CH_3$ | N | |
| $CO_2CH_3$ | H | H | H | $CH_3O$ | $CH_3O$ | CH | |
| $CO_2CH_3$ | 5-Cl | H | H | $CH_3O$ | $CH_3O$ | N | |
| $\overset{O}{\overset{\Vert}{C}}—N(CH_3)_2$ | H | H | H | $OCH_3$ | $CH_3$ | N | |

TABLE I (contd.)

| L | R | $R_1$ | $R_2$ | X | Y | Z | m.p.(°C) |
|---|---|---|---|---|---|---|---|
| 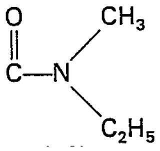 | H | H | H | O-$n$-$C_3H_7$ | $CH_3$ | N | |
| 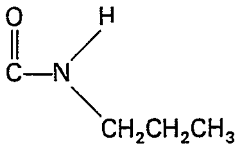 | H | H | H | H | $CH_3$ | N | |
| 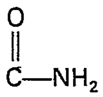 | H | H | H | $OCH_3$ | $OCH_3$ | CH | |
| 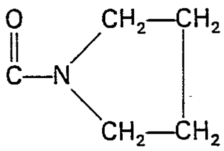 | H | H | H | $OCH_3$ | $OCH_3$ | CH | 133—315° (dec.) |
| 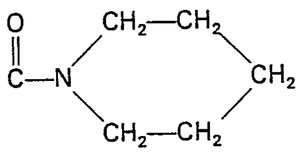 | H | H | H | $OCH_3$ | $OCH_3$ | CH | |
| 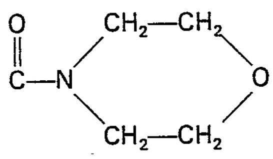 | H | H | H | $OCH_3$ | $OCH_3$ | CH | |
| 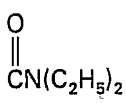 | H | H | H | H | $CH_3$ | CH | |
| CN | H | H | H | $CH_3$ | $OCH_3$ | N | |
| CN | H | H | H | $CH_3$ | $OCH_3$ | CH | |
| 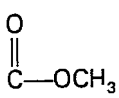 | H | H | H | H | $CH_3$ | $OCH_3$ | |
| 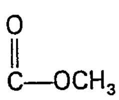 | H | H | H | H | $CH_3$ | $OCH_2CH_2Cl$ | |
| 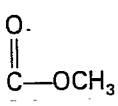 | H | H | H | H | $CH_3$ | $OCH_2CH{=}CH_2$ | |
| 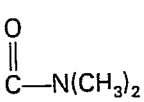 | H | H | H | $CH_3O$ | $CH_3$ | CH | |
| 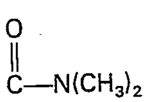 | H | H | H | $CH_3O$ | $CH_3O$ | N | |

TABLE I (contd.)

| L | R | $R_1$ | $R_2$ | X | Y | Z | m.p.(°C) |
|---|---|---|---|---|---|---|---|
| $\overset{O}{\overset{\|}{C}}—N(CH_3)_2$ | H | H | H | $CH_3O$ | $CH_3O$ | CH | 155—157° |
| $\overset{O}{\overset{\|}{C}}—NHCH_3$ | H | H | H | $CH_3O$ | $CH_3O$ | CH | 178—180° |

TABLE II

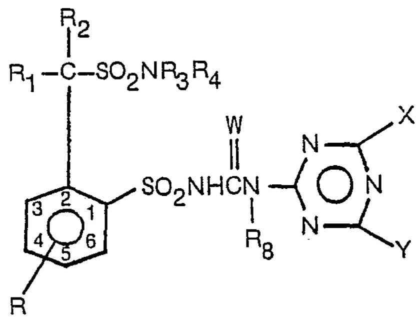

| R | $R_1$ | $R_2$ | $R_4$ | W | $R_3$ | $R_8$ | X | Y | m.p. (°C) |
|---|---|---|---|---|---|---|---|---|---|
| H | H | H | $CH_3$ | O | $CH_3$ | H | $CH_3O$ | $CH_3O$ | 200—205° (d) |
| H | H | H | $CH_3$ | O | $CH_3$ | H | $CH_3O$ | $CH_3$ | 200—205° (d) |
| H | H | H | $CH_3$ | O | $CH_3$ | H | $CH_3$ | $CH_3$ | 209—210° (d) |
| H | H | H | $CH_3$ | O | $CH_3O$ | H | $CH_3O$ | $CH_3O$ | |
| H | H | H | $CH_3$ | O | $CH_3CH_2$ | H | $CH_3O$ | $CH_3O$ | |
| H | H | H | $CH_3$ | O | $CH_3CH_2CH_2$ | H | $CH_3O$ | $CH_3O$ | |
| H | H | H | $CH_3$ | O | $(CH_3)_2CH$ | H | $CH_3O$ | $CH_3O$ | |
| H | H | H | $CH_3$ | O | $CH_3(CH_2)_3$ | H | $CH_3O$ | $CH_3O$ | |
| H | H | H | $CH_3CH_2$ | O | $CH_3CH_2$ | H | $CH_3O$ | $CH_3O$ | |
| H | H | H | $CH_3$ | O | $CH_3$ | H | Cl | $CH_3O$ | |
| H | H | H | $CH_3$ | O | $CH_3$ | H | Br | $CH_3O$ | |
| H | H | H | $CH_3$ | O | $CH_3$ | H | $CH_3CH_2$— | $CH_3O$ | |
| H | H | H | $CH_3$ | O | $CH_3$ | H | $CH_3CH_2O$— | $CH_3$ | |
| H | H | H | $CH_3$ | O | $CH_3$ | H | >—O— | $CH_3$ | |
| H | H | H | $CH_3$ | S | $CH_3$ | H | $CF_3$ | $CH_3O$ | |
| H | H | H | $CH_3$ | S | $CH_3$ | H | $CH_3S$ | $CH_3O$ | |
| H | H | H | $CH_3$ | O | $CH_3$ | H | $CH_3OCH_2$— | $CH_3$ | |
| H | H | H | $CH_3$ | O | $CH_3$ | H | $CH_3OCH_2$— | $CH_3O$ | |

TABLE III

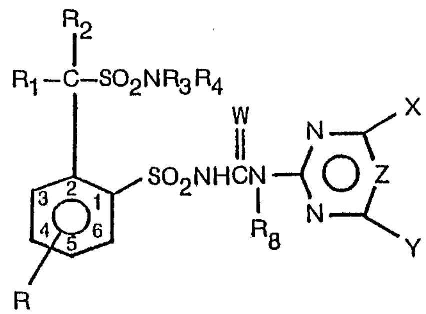

| R | $R_1$ | $R_2$ | $R_4$ | W | $R_3$ | $R_8$ | X | Y | Z | m.p. (°C) |
|---|---|---|---|---|---|---|---|---|---|---|
| H | H | H | $CH_3$ | O | $CH_3$ | H | $CH_3O$ | $CH_3O$ | CH | 172—176° (d) |
| H | H | H | $CH_3$ | O | $CH_3$ | H | $CH_3O$ | $CH_3$ | CH | 181—183° (d) |
| H | H | H | $CH_3$ | O | $CH_3$ | H | $CH_3$ | $CH_3$ | CH | 203—204° (d) |
| H | $CH_3$ | $CH_3$ | $CH_3$ | O | $CH_3$ | H | $CH_3O$ | $CH_3O$ | CH | |
| 3-Cl | H | H | $CH_3$ | O | $CH_3$ | H | $CH_3O$ | $CH_3O$ | CH | |
| 4-Cl | H | H | $CH_3$ | O | $CH_3$ | H | $CH_3O$ | $CH_3O$ | CH | |
| 6-Cl | H | H | $CH_3$ | S | $CH_3$ | H | $CH_3O$ | $CH_3O$ | CH | |
| H | H | H | $CH_3$ | O | $CH_3$ | H | H | $CH_3$ | C-Cl | |
| H | H | H | $CH_3$ | O | $CH_3$ | H | $CH_3$ | $CH_3$ | C—CN | |
| H | H | H | $CH_3$ | O | $CH_3$ | H | $CH_3$ | $CH_3$ | C—$CH_3$ | |
| H | H | H | $CH_3$ | O | $CH_3$ | H | H | $CH_3$ | C—$CH_3$ | |
| H | H | H | $CH_3$ | O | $CH_3$ | H | $CH_3$ | $CH_3$ | C—$CH_2CH_3$ | |
| H | H | H | $CH_3$ | O | $CH_3$ | $CH_3$ | $CH_3$ | $CH_3$ | CH | |

TABLE IV

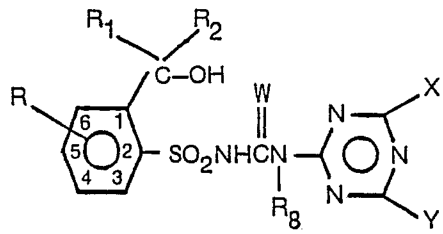

| R | $R_1$ | $R_2$ | W | $R_8$ | X | Y | m.p. (°C) |
|---|---|---|---|---|---|---|---|
| H | H | H | O | H | $CH_3$ | $OCH_3$ | |
| H | H | $CH_3$ | O | H | $CH_3$ | $OCH_3$ | |
| H | $CH_3$ | $CH_3$ | O | H | $OCH_3$ | $OCH_3$ | |
| 5-$CH_3$ | H | H | O | H | Cl | Br | |
| H | $CH_3$ | H | O | H | Cl | Cl | |
| H | H | H | O | H | $SCH_3$ | $OCH_3$ | |
| H | H | H | O | H | Br | $CH_3O$ | |
| H | H | $CH_3$ | O | H | 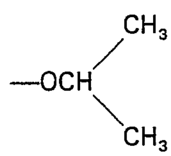 | $CH_3O$ | |
| H | H | H | O | H | $CF_3$ | Cl | |
| H | H | H | O | H | $OC_2H_5$ | Cl | |
| H | H | H | S | H | $CH_3$ | $CH_3$ | |
| H | H | H | S | H | $CH_3$ | $OCH_3$ | |
| H | H | H | S | H | $OCH_3$ | $OCH_3$ | |
| H | H | H | O | $CH_3$ | $CH_3$ | $CH_3$ | |
| H | H | H | O | $CH_3$ | $CH_3$ | $OCH_3$ | |
| H | H | H | O | $CH_3$ | $OCH_3$ | $OCH_3$ | |
| H | H | H | O | $OCH_3$ | $CH_3$ | $CH_3$ | |
| H | H | H | O | $OCH_3$ | $CH_3$ | $OCH_3$ | |
| H | H | H | O | $OCH_3$ | $OCH_3$ | $OCH_3$ | |

TABLE V

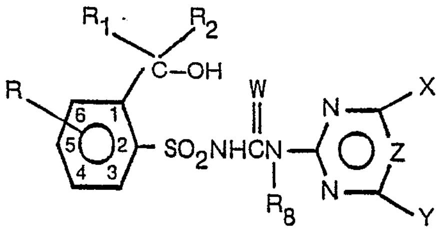

| R | $R_1$ | $R_2$ | W | $R_8$ | X | Y | Z | m.p. (°C) |
|---|---|---|---|---|---|---|---|---|
| H | H | H | O | H | $OCH_3$ | $OCH_3$ | CH | |
| H | H | $CH_3$ | O | H | $CH_3$ | $OCH_3$ | CH | |
| H | H | $CH_3$ | O | H | $OCH_3$ | $OCH_3$ | CH | |
| H | $CH_3$ | $CH_3$ | O | H | $OCH_3$ | $OCH_3$ | CH | |
| 4-Cl | H | H | O | H | $CH_3$ | $CH_3$ | CH | |
| 6-Cl | H | H | O | H | $CH_3$ | $OCH_3$ | CH | |
| 4-Cl | H | H | O | H | $OCH_3$ | $OCH_3$ | CH | |
| 4-$NO_2$ | H | H | O | H | $CH_3$ | $CH_3$ | CH | |
| 4-$OCH_3$ | H | H | O | H | $CH_3$ | $OCH_3$ | CH | |
| 4-$CF_3$ | H | H | O | H | $CH_3$ | $OCH_3$ | CH | |
| H | H | H | O | H | H | $CH_3$ | CCl | |
| H | H | H | O | H | $CH_3$ | $CH_3$ | C—CN | |
| H | H | H | O | H | $CH_3$ | $CH_3$ | C—$CH_3$ | |
| H | H | H | O | H | $CH_3$ | $CH_3$ | C—$CH_2CH_3$ | |
| H | H | H | O | H | H | $CH_3$ | C—$CH_2CH_2Cl$ | |
| H | $CH_3$ | $CH_3$ | O | H | H | $OCH_3$ | C—Cl | |
| H | H | H | S | H | $CH_3$ | $OCH_3$ | CH | |
| H | H | H | S | H | $OCH_3$ | $OCH_3$ | CH | |
| H | H | H | O | $CH_3$ | $CH_3$ | $OCH_3$ | CH | |
| H | H | H | O | $CH_3$ | $OCH_3$ | $OCH_3$ | CH | |
| H | H | H | O | $OCH_3$ | $OCH_3$ | $OCH_3$ | CH | |
| 4-$CH_3$ | H | H | O | H | $OCH_3$ | $OCH_3$ | CH | |
| H | H | H | O | H | $CH_2OCH_3$ | $CH_3$ | CH | |
| H | H | H | O | H | $SCH_3$ | $OCH_3$ | CH | |

TABLE VI

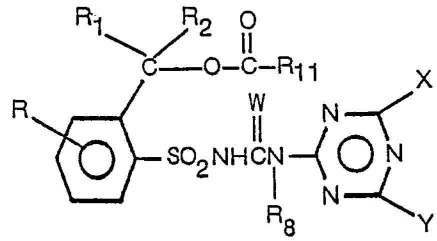

| R | $R_1$ | $R_2$ | $R_{11}$ | W | $R_8$ | X | Y | m.p. (°C) |
|---|---|---|---|---|---|---|---|---|
| H | H | H | H | O | H | $OCH_3$ | $OCH_3$ | |
| H | H | H | $—CH_3$ | O | H | $CH_3$ | $OCH_3$ | |
| H | H | H | $—CH_2CH_3$ | O | H | $OCH_3$ | $OCH_3$ | |
| H | H | H | $CF_3$ | O | H | $OCH_3$ | $OCH_3$ | |
| H | H | H | $CH_2Cl$ | O | H | $CH_3$ | $OCH_3$ | |
| H | H | H | $CH_2CH{=}CH_2$ | O | H | $CH_3$ | $OCH_3$ | |
| H | H | H | 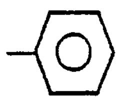 | O | H | $CH_3$ | $OCH_3$ | |
| H | H | H | 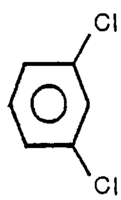 | O | H | $CH_3$ | $OCH_3$ | |
| H | H | H | 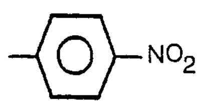 | O | H | $OCH_3$ | $OCH_3$ | |
| H | H | H | $CH_3$ | O | H | $CH_2OCH_3$ | $OCH_3$ | |
| H | H | H | $CH_3$ | O | H | H | $CH_3$ | |
| H | H | H | $CH_3$ | O | H | H | $OCH_3$ | |
| H | H | H | $CH_3$ | O | H | Br | $OCH_3$ | |
| H | H | H | $—CH_2OCH_3$ | O | H | $SCH_3$ | $CH_3$ | |
| H | H | H | 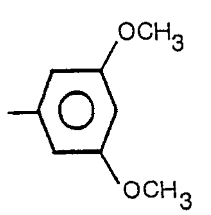 | O | H | $OCH_3$ | $OCH_3$ | |

TABLE VII

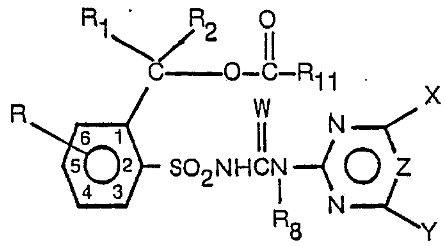

| R | $R_1$ | $R_2$ | $R_{11}$ | W | $R_8$ | X | Y | Z | m.p. (°C) |
|---|---|---|---|---|---|---|---|---|---|
| H | H | H | H | O | H | $CH_3$ | $CH_3$ | CH | |
| H | H | H | $CH_3$ | O | H | $CH_3$ | $OCH_3$ | CH | |
| H | H | H | $CF_3$ | O | H | $CH_3$ | $OCH_3$ | CH | |
| 6-Cl | H | H | $CH_3$ | O | H | $OCH_3$ | $OCH_3$ | CH | |
| H | H | H | $CH_3$ | O | H | Cl | $OCH_3$ | CH | |
| H | H | H | $CH_3$ | O | H | $CF_3$ | $OCH_3$ | CH | |
| H | H | H | $CH_3$ | O | H | $SCH_3$ | $OCH_3$ | CH | |
| H | H | H | $CH_3$ | O | H | $CH_2OCH_3$ | $CH_3$ | CH | |
| H | H | H | $CH_3$ | O | H | $CH_2OCH_3$ | $OCH_3$ | CH | |
| H | H | H | $-CH{=}CH_2$ | S | H | $CH_3$ | $OCH_3$ | CH | |
| H | H | H | $-C{\equiv}CCH_3$ | S | H | $CH_3$ | $OCH_3$ | CH | |
| H | H | H | 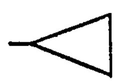 | S | H | $OCH_3$ | $OCH_3$ | CH | |
| H | H | H | 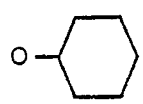 | S | H | $OCH_3$ | $CH_3$ | CH | |
| 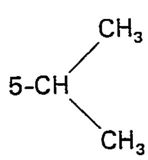 | H | H | $CH_3$ | S | H | $OCH_3$ | $CH_3$ | CH | |
| H | H | H | $CH_3$ | S | H | $OCH_3$ | $OCH_3$ | CH | |
| H | H | H | $CH_3$ | O | $CH_3$ | $OCH_3$ | $OCH_3$ | CH | |

TABLE VIII

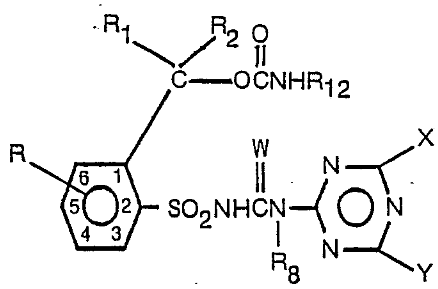

| R | $R_1$ | $R_2$ | $R_{12}$ | W | $R_8$ | X | Y | m.p. (°C) |
|---|---|---|---|---|---|---|---|---|
| H | H | H | H | O | H | $CH_3$ | $CH_3$ | |
| H | H | H | $—CH_3$ | O | H | $OCH_3$ | $OCH_3$ | |
| H | H | H | $—CH_2CH_3$ | O | H | $CH_3$ | $OCH_3$ | |
| H | H | $CH_3$ | $CH_3$ | O | H | $OCH_3$ | $OCH_3$ | |
| 4-$CF_3$ | H | H | $CH_3$ | O | H | OCH | $OCH_3$ | |
| 4-Cl | H | H | $CH_3$ | O | H | $CH_3$ | $OCH_3$ | |
| H | H | H | $CH_3$ | O | H | Cl | $OCH_3$ | |
| H | H | H | $CH_3$ | O | H | $SCH_3$ | $OCH_3$ | |
| H | H | H | $CH_3$ | O | H | $CH_2OCH_3$ | $OCH_3$ | |
| H | H | H | $CH_3$ | O | H | H | $CH_3$ | |
| H | H | H | 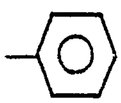 | O | H | $OCH_3$ | $OCH_3$ | |
| H | H | H | 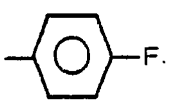 | O | H | $OCH_3$ | $CH_3$ | |
| H | H | H | 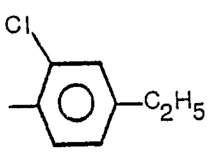 | O | H | $OCH_3$ | $OCH_3$ | |
| H | H | H | 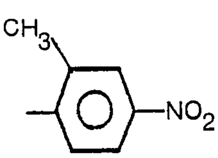 | O | H | $OCH_3$ | $CH_3$ | |
| H | H | H | 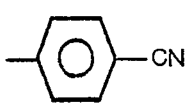 | O | H | $OCH_3$ | $CH_3$ | |
| H | H | H | 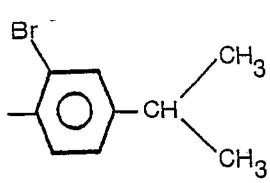 | O | H | $CH_3$ | $OCH_3$ | |

TABLE VIII (contd.)

| R | $R_1$ | $R_2$ | $R_{12}$ | W | $R_5$ | X | Y | m.p.(°C) |
|---|---|---|---|---|---|---|---|---|
| H | H | H | 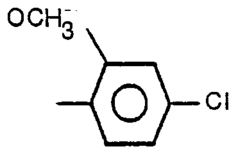 | O | H | $CH_3$ | $OCH_3$ | |
| H | H | H | 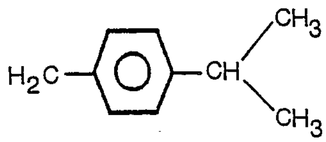 | O | H | $CH_3$ | $OCH_3$ | |
| H | H | H | $CH_3$ | S | H | $OCH_3$ | $OCH_3$ | |
| H | H | H | $CH_3$ | O | $OCH_3$ | $OCH_3$ | $OCH_3$ | |

TABLE IX

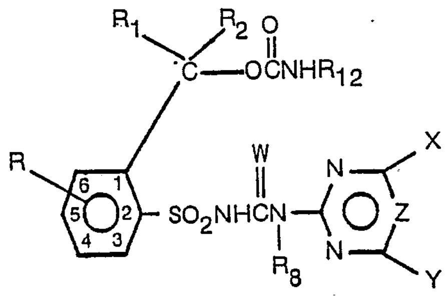

| R | $R_1$ | $R_2$ | $R_{12}$ | W | $R_8$ | X | Y | Z | m.p. (°C) |
|---|---|---|---|---|---|---|---|---|---|
| H | H | H | H | O | H | $CH_3$ | $OCH_3$ | CH | |
| H | H | H | $—CH_3$ | O | H | $CH_3$ | $OCH_3$ | CH | |
| H | H | H | $CH_2CH{=}CH_2$ | O | H | $CH_3$ | $OCH_3$ | CH | |
| H | H | H | 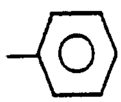 | O | H | $OCH_3$ | $OCH_3$ | CH | |
| 4-F | H | H | $CH_3$ | O | H | $OCH_3$ | $OCH_3$ | CH | |
| 4-Cl | H | H | $CH_3$ | O | H | $OCH_3$ | $OCH_3$ | CH | |
| H | H | H | $CH_2$— 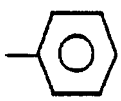 | O | H | $OCH_3$ | $OCH_3$ | CH | |
| 4-$NO_2$ | H | H | $CH_3$ | O | H | $OCH_3$ | $OCH_3$ | CH | |
| H | H | H | $CH_3$ | O | H | Br | $OCH_3$ | CH | |
| H | H | H | $CH_3$ | O | H | $OCH_2CH_3$ | $CH_3$ | CH | |
| H | H | H | $CH_3$ | O | H | $OCH(CH_3)_2$ | $CH_3$ | CH | |
| H | H | H | $CH_3$ | O | H | $CF_3$ | $OCH_3$ | CH | |
| H | H | H | 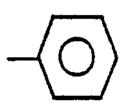 | O | H | $OCH_3$ | $OCH_3$ | CH | |
| H | H | H | $CH_2—(CH_2)_4CH_3$ | O | H | $CH_3$ | $OCH_3$ | CH | |
| H | H | H | $—CH_2—CH{=}CH—CH_3$ | O | H | $CH_3$ | $OCH_3$ | CH | |
| H | H | H | 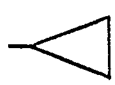 | O | H | $OCH_3$ | $OCH_3$ | CH | |
| H | H | H | 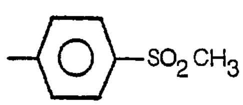 | O | H | $CH_3$ | $OCH_3$ | CH | |
| H | H | H | 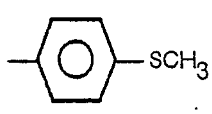 | O | H | $OCH_3$ | $OCH_3$ | CH | |

TABLE X

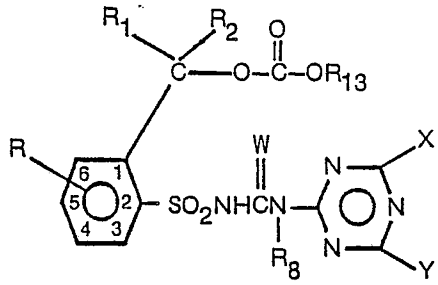

| R | $R_1$ | $R_2$ | $R_{13}$ | W | $R_8$ | X | Y | m.p. (°C) |
|---|---|---|---|---|---|---|---|---|
| H | H | H | $—CH_3$ | O | H | $CH_3$ | $CH_3$ | |
| H | H | H | $—CH_3$ | O | H | $CH_3$ | $OCH_3$ | |
| H | H | H | $—CH_2CH_3$ | O | H | $OCH_3$ | $OCH_3$ | |
| H | H | H | 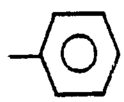 | O | H | $OCH_3$ | $OCH_3$ | |
| H | H | H | 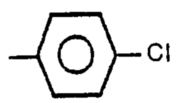 | O | H | $CH_3$ | $OCH_3$ | |
| H | H | $CH_3$ | $CH_3$ | O | H | $OCH_3$ | $OCH_3$ | |
| 4-F | H | H | $CH_3$ | O | H | $OCH_3$ | $OCH_3$ | |
| 4-Cl | H | H | $CH_3$ | O | H | $OCH_3$ | $OCH_3$ | |
| 4-$CH_3$ | H | H | $CH_3$ | O | H | $OCH_3$ | $OCH_3$ | |
| H | H | H | $CH_2(CH_2)_4CH_3$ | O | H | $OCH_3$ | $OCH_3$ | |
| H | H | H | 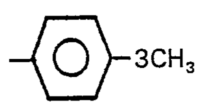 | O | H | $OCH_3$ | $OCH_3$ | |
| H | H | H | $CH_3$ | S | H | $OCH_3$ | $OCH_3$ | |
| H | H | H | $CH_3$ | S | $CH_3$ | $CH_3$ | $OCH_3$ | |
| H | H | H | $CH_3$ | S | $OCH_3$ | $CH_3$ | $CH_3$ | |
| 4-$NO_2$ | H | H | $CH_3$ | O | H | $OCH_3$ | $OCH_3$ | |
| 4-$OCH_3$ | H | H | $CH_3$ | O | H | $OCH_3$ | $OCH_3$ | |
| 4-$CF_3$ | H | H | $CH_3$ | O | H | $OCH_3$ | $OCH_3$ | |
| H | H | H | $CH_3$ | O | H | $OCH(CH_3)_2$ | $CH_3$ | |
| H | H | H | $CH_3$ | O | H | $CF_3$ | $OCH_3$ | |

TABLE XI

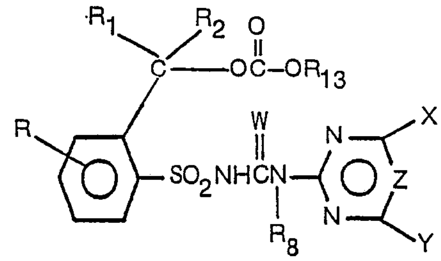

| R | $R_1$ | $R_2$ | $R_{13}$ | W | $R_8$ | X | Y | Z | m.p. (°C) |
|---|---|---|---|---|---|---|---|---|---|
| H | H | H | $CH_3$ | O | H | $OCH_3$ | $OCH_3$ | CH | |
| H | H | H | $CH_2CH_3$ | O | H | $CH_3$ | $OCH_3$ | CH | |
| H | $CH_3$ | $CH_3$ | $CH_3$ | O | H | $OCH_3$ | $OCH_3$ | CH | |
| H | H | H | $CH_3$ | O | H | H | $CH_3$ | CCl | |
| H | H | H | $CH_3$ | O | H | $CH_3$ | $CH_3$ | C—CN | |
| H | H | H | $CH_3$ | O | H | H | $CH_3$ | $CCH_3$ | |
| H | H | H | $CH_3$ | O | H | $CH_3$ | $CH_3$ | $CCH_2CH_3$ | |
| H | H | H | $CH_3$ | O | H | H | $CH_3$ | $CCH_2CH_3$ | |
| H | H | H | $CH_3$ | O | H | $CH_3$ | $CH_3$ | $C—CH_2CH_2Cl$ | |
| H | H | H | $CH_3$ | O | H | H | $CH_3$ | $C—CH_2CH{=}CH_2$ | |
| H | H | H | $CH_2CH_2CH_3$ | O | H | $OCH_3$ | $CH_3$ | CH | |
| H | H | H | $CH_3$ | S | H | $CH_3$ | $OCH_3$ | $CH_2$ | |
| H | H | H | $CH_3$ | O | $CH_3$ | $OCH_3$ | $OCH_3$ | $CH_2$ | |
| H | H | H | $CH_3$ | O | $OCH_3$ | $CH_3$ | $OCH_3$ | $CH_2$ | |

TABLE XII

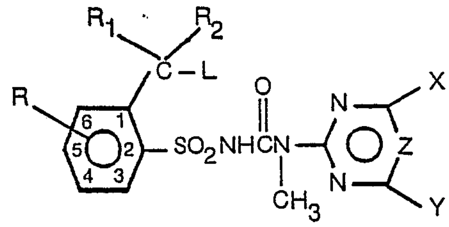

| L | R | $R_1$ | $R_2$ | X | Y | Z |
|---|---|---|---|---|---|---|
| $OCH_3$ | H | H | H | $CH_3$ | $CH_3$ | CH |
| $OCH_3$ | H | H | H | $OCH_3$ | $OCH_3$ | CH |
| $SCH_3$ | H | H | H | $CH_3$ | $CH_3$ | CH |
| $SCH_3$ | H | H | H | $OCH_3$ | $OCH_3$ | CH |
| $S(O)CH_3$ | H | H | H | $OCH_3$ | $OCH_3$ | CH |
| $S(O)_2CH_3$ | H | H | H | $OCH_3$ | $OCH_3$ | CH |
| $SCH_3$ | H | $CH_3$ | H | $OCH_3$ | $OCH_3$ | CH |
| $S(O)_2C_2H_5$ | H | $CH_3$ | H | $OCH_3$ | $CH_3$ | CH |
| $(S)CH_2CH_2CH_3$ | H | H | H | $OCH_3$ | $CH_3$ | N |
| $S(O)_2CH_3$ | H | H | H | $OCH_3$ | $CH_3$ | N |
| $OCH_3$ | H | $CH_3$ | H | $OCH_3$ | $CH_3$ | N |
| $SCH_3$ | H | $CH_3$ | H | $OCH_3$ | $CH_3$ | N |
| $S(O)CH_3$ | H | $CH_3$ | H | $OCH_3$ | $CH_3$ | N |
| $S(O)_2CH_3$ | H | $CH_3$ | H | $OCH_3$ | $CH_3$ | N |

TABLE XIII

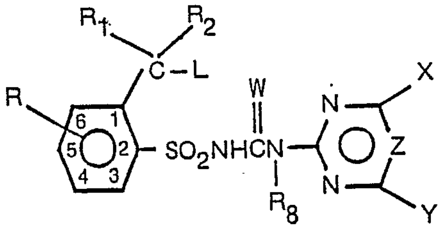

| L | R | $R_1$ | $R_2$ | $R_8$ | W | X | Y | Z | m.p. (°C) |
|---|---|---|---|---|---|---|---|---|---|
| Cl | H | H | H | $CH_3$ | O | $CH_3$ | $CH_3$ | CH | |
| Cl | H | H | H | $CH_3$ | O | $OCH_3$ | $CH_3$ | CH | |
| Cl | H | H | H | $CH_3$ | O | $OCH_3$ | $OCH_3$ | CH | |
| Cl | H | H | H | $CH_3$ | O | $CH_3$ | $CH_3$ | N | |
| Cl | H | H | H | $CH_3$ | O | $CH_3$ | $OCH_3$ | N | |
| Cl | H | H | H | $CH_3$ | O | $OCH_3$ | $OCH_3$ | N | |
| 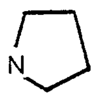 | H | H | H | $CH_3$ | O | $OCH_3$ | $OCH_3$ | CH | |
| 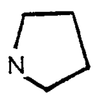 | H | H | H | $CH_3$ | O | $OCH_3$ | $CH_3$ | N | |
| $NH\overset{O}{\overset{\|}{C}}OCH_3$ | H | H | H | $CH_3$ | O | $OCH_3$ | $CH_3$ | N | |
| $—NH\overset{O}{\overset{\|}{C}}NHCH_3$ | H | H | H | $CH_3$ | O | $OCH_3$ | $OCH_3$ | CH | |
| Cl | H | H | H | H | S | $CH_3$ | $CH_3$ | CH | |
| Cl | H | H | H | $OCH_3$ | O | $CH_3$ | $CH_3$ | CH | |
| $OCH_3$ | H | H | H | $OCH_3$ | O | $CH_3$ | $CH_3$ | CH | |
| $OCH_3$ | H | $CH_3$ | H | $OCH_3$ | O | $OCH_3$ | $OCH_3$ | CH | |
| $SCH_3$ | H | $CH_3$ | H | $OCH_3$ | O | $CH_3$ | $CH_3$ | CH | |
| $S(O)_2CH_3$ | H | H | H | $OCH_3$ | O | $OCH_3$ | $CH_3$ | CH | |
| $OC_2H_5$ | H | H | H | $OCH_3$ | O | $OCH_3$ | $CH_3$ | CH | |
| $SCH_3$ | H | H | H | $OCH_3$ | O | $OCH_3$ | $CH_3$ | CH | |

TABLE XIV

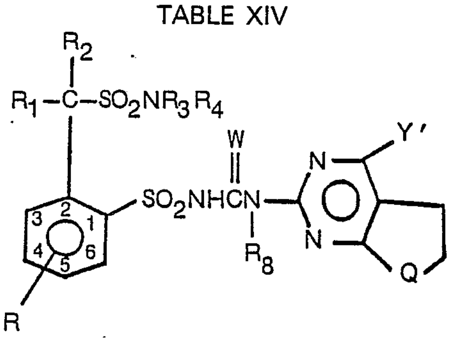

| R | $R_1$ | $R_2$ | $R_4$ | W | $R_3$ | $R_8$ | Y′ | Q | m.p. (°C) |
|---|---|---|---|---|---|---|---|---|---|
| H | H | H | $CH_3$ | O | $CH_3$ | H | $C_2H_5O$ | O | |
| H | H | H | $CH_3$ | O | $CH_3$ | H | $CH_3O$ | O | |
| H | H | H | $CH_3$ | O | $CH_3$ | H | $CH_3$ | O | |
| H | H | H | $CH_3$ | O | $CH_3O$ | H | $CH_3O$ | O | |

TABLE XV

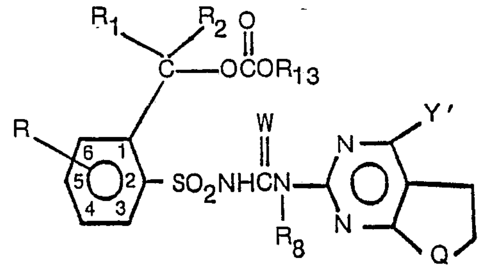

| R | $R_1$ | $R_2$ | $R_{13}$ | W | $R_8$ | Y′ | Q | m.p. (°C) |
|---|---|---|---|---|---|---|---|---|
| H | H | H | $CH_3$ | O | H | $CH_3$ | O | |
| H | H | H | $CH_3$ | O | H | $OCH_3$ | O | |
| H | H | H | $CH_2CH_3$ | O | H | $CH_3$ | O | |
| H | H | $CH_3$ | $CH_3$ | O | H | $OCH_3$ | O | |
| H | $CH_3$ | $CH_3$ | $CH_3$ | O | H | $OCH_3$ | O | |
| 4-F | H | H | $CH_3$ | O | H | $OCH_3$ | O | |
| 4-Cl | H | H | $CH_3$ | O | H | $OCH_3$ | O | |
| 4-$CH_3$ | H | H | $CH_3$ | O | H | $OCH_3$ | O | |
| 4-$CF_3$ | H | H | $CH_3$ | O | H | $OCH_3$ | O | |
| 6-Cl | H | H | $CH_3$ | O | H | $CH_3$ | O | |
| H | H | H | $CH_3$ | O | H | H | O | |
| H | H | H | $CH_3$ | O | H | H | $CH_2$ | |
| H | H | H | $CH_3$ | O | H | Cl | $CH_2$ | |
| H | H | H | $CH_3$ | O | H | $CH_3$ | $CH_2$ | |
| H | H | H | $CH_3$ | O | H | $OCH_3$ | $CH_2$ | |
| H | H | H | $-CH_2CH_2CH_3$ | O | H | $OCH_3$ | O | |
| H | H | H | 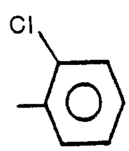 | O | H | $CH_3$ | O | |
| H | H | H | $CH_3$ | S | H | $OCH_3$ | $CH_2$ | |
| H | H | H | $CH_3$ | O | $OCH_3$ | $OCH_3$ | O | |
| H | H | H | $CH_3$ | O | $OCH_3$ | $OCH_3$ | $CH_2$ | |
| H | H | H | $CH_3$ | O | H | $OC_2H_5$ | O | |
| H | H | H | $CH_3$ | O | H | $OC_2H_5$ | $CH_2$ | |

TABLE XVI

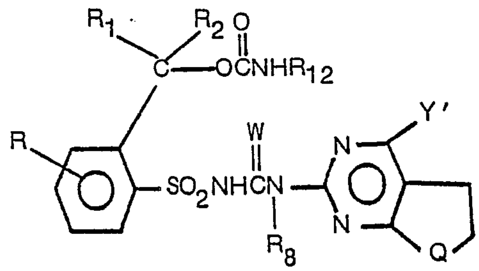

| R | $R_1$ | $R_2$ | $R_{12}$ | W | $R_8$ | Y' | Q | m.p. (°C) |
|---|---|---|---|---|---|---|---|---|
| H | H | H | H | O | H | $OCH_3$ | O | |
| H | H | H | $CH_3$ | O | H | $CH_3$ | O | |
| H | H | H | $CH_3$ | O | H | $OCH_3$ | O | |
| H | H | H | $CH_2CH{=}CH_2$ | O | H | $OCH_3$ | O | |
| H | H | H | 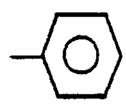 | O | H | $CH_3$ | O | |
| H | H | $CH_3$ | $CH_3$ | O | H | $OCH_3$ | O | |
| H | $CH_3$ | $CH_3$ | $CH_3$ | O | H | $OCH_3$ | O | |
| H | H | H | $CH_3$ | O | H | H | O | |
| H | H | H | $CH_3$ | O | H | H | $CH_2$ | |
| H | H | H | $CH_3$ | O | H | Cl | $CH_2$ | |
| H | H | H | $CH_3$ | O | H | $CH_3$ | $CH_2$ | |
| H | H | H | $CH_3$ | S | H | $OCH_3$ | O | |
| H | H | H | $CH_3$ | S | H | $CH_3$ | $CH_2$ | |
| H | H | H | $CH_3$ | O | $CH_3$ | $OCH_3$ | $CH_2$ | |
| H | H | H | $CH_3$ | O | $OCH_3$ | $CH_3$ | O | |
| H | H | H | $CH_3$ | O | H | $OC_2H_5$ | O | |
| H | H | H | $—CH_2—(CH_2)_4CH_3$ | O | H | $OCH_3$ | O | |
| H | H | H | 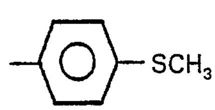 | O | H | $CH_3$ | O | |
| 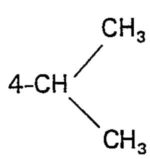 | H | H | $CH_3$ | O | H | $OCH_3$ | O | |

TABLE XVII

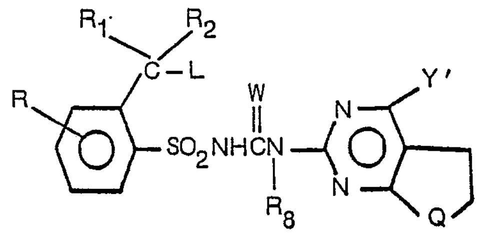

| L | R | $R_1$ | $R_2$ | Y' | Q | $R_8$ | W |
|---|---|---|---|---|---|---|---|
| $CO_2CH_3$ | H | H | H | $CH_3$ | O | H | O |
| $CO_2C_2H_5$ | H | $CH_3$ | $CH_3$ | $CH_3O$ | O | H | O |
| $CO_2CH_3$ | H | H | H | $CH_3$ | $CH_2$ | $CH_3$ | O |
| $CO_2CH_3$ | H | H | H | $CH_3O$ | O | H | S |
| $S(O)CH_3$ | H | H | H | $CH_3O$ | O | H | O |
| $OCH(CH_3)_2$ | H | H | H | $CH_3O$ | O | H | O |
| $OC_2H_5$ | H | H | H | $CH_3$ | O | H | O |
| $SCH_3$ | H | H | H | $CH_3O$ | O | H | $CH_2$ |

TABLE XVIII

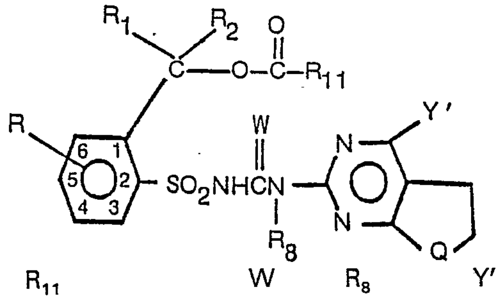

| R | $R_1$ | $R_2$ | $R_{11}$ | W | $R_8$ | Y' | Q | m.p. (°C) |
|---|---|---|---|---|---|---|---|---|
| H | H | H | H | O | H | $CH_3$ | O | |
| H | H | H | H | O | H | $OCH_3$ | O | |
| H | H | H | $CH_3$ | O | H | $CH_3$ | O | |
| H | H | H | $CH_3$ | O | H | $OCH_3$ | O | |
| H | H | H | $CH_2CH_3$ | O | H | $CH_3$ | O | |
| H | H | H | $CF_3$ | O | H | $CH_3$ | O | |
| H | H | H | $CF_3$ | O | H | $OCH_3$ | O | |
| H | H | H | $CH_2Cl$ | O | H | $OCH_3$ | O | |
| H | H | H | $CH_2CH{=}CH_2$ | O | H | $OCH_3$ | O | |
| H | H | H | 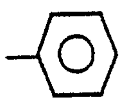 | 960 | H | $CH_3$ | O | |
| H | H | $CH_3$ | $CH_3$ | O | H | $OCH_3$ | O | |
| H | $CH_3$ | $CH_3$ | $CH_3$ | O | H | $OCH_3$ | O | |
| H | H | H | $CH_3$ | O | H | Cl | $CH_2$ | |
| H | H | H | $CH_3$ | O | H | $OCH_3$ | $CH_2$ | |
| H | H | H | $CH_3$ | S | H | $CH_3$ | O | |
| H | H | H | $CH_3$ | S | H | $OCH_3$ | $CH_2$ | |
| H | H | H | $CH_3$ | O | $CH_3$ | $CH_3$ | O | |
| H | H | H | $CH_3$ | O | $OCH_3$ | $CH_3$ | $CH_2$ | |
| H | H | H | $CH_3$ | O | $CH_3$ | $OCH_3$ | O | |
| H | H | H | CF | O | H | $OC_2H_5$ | O | |
| H | H | H | Cl | O | H | $OC_2H_5$ | $CH_2$ | |
| H | H | H | 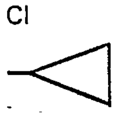 | 970 | H | $CH_3$ | O | |
| H | H | H | $—CH{=}CH_2$ | O | H | $CH_3$ | O | |
| $4\text{-}OC_2H_5$ | H | H | $—CH{=}CH_2$ | O | H | $CH_3$ | O | |
| $4\text{-}CH_3$ | H | H | $CH_3$ | O | H | $CH_3$ | $CH_2$ | |
| H | H | H | 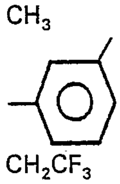 | 980 | H | $CH_3$ | O | |
| H | H | H | $CH_2CF_3$ | O | H | $OCH_3$ | O | |

TABLE XIX

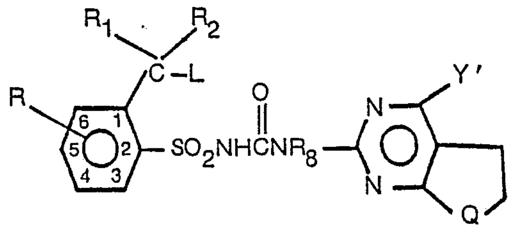

| R | $R_1$ | $R_2$ | $R_8$ | Y' | Q | L |
|---|---|---|---|---|---|---|
| H | H | H | H | $CH_3$ | O | Cl |
| H | H | H | H | $CH_3O$ | O | Cl |
| H | H | H | H | Cl | O | Cl |
| H | H | H | H | H | O | Cl |
| H | H | H | H | $CH_3$ | $CH_2$ | Cl |
| H | H | H | H | $CH_3O$ | $CH_2$ | Cl |
| H | Cl | H | H | $CH_3O$ | $CH_2$ | Cl |
| H | H | H | H | $CH_3$ | O | Br |
| H | H | H | H | H | O | 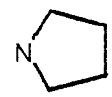 |
| H | H | H | H | $CH_3$ | L | $+NMe_3$ |
| H | H | H | H | $CH_3$ | O | $NHCOCH_3$ |
| H | H | H | H | $CH_3$ | O | $NH\overset{O}{\overset{\|}{C}}NHCH_3$ |
| H | H | H | H | $CH_3O$ | O | $NHCH_3$ |
| H | H | H | H | $CH_3$ | O | $NCH_3CONHCH_3$ |
| H | H | H | H | $CH_3$ | O | $N(C_2H_5)CO_2CH_3$ |
| H | H | H | H | $CH_3$ | O | $NHCO_2Et$ |
| H | H | H | H | $CH_3$ | O | Cl |
| 4-F | H | H | H | $CH_3O$ | $CH_2$ | Cl |
| 4-Cl | H | H | H | $CH_3O$ | $CH_2$ | Cl |
| 4-Br | H | H | H | $CH_3O$ | $CH_2$ | Cl |
| 4-$NO_2$ | H | H | H | $CH_3O$ | $CH_2$ | Cl |
| 4-$CF_3$ | H | H | H | $CH_3O$ | $CH_2$ | Cl |
| 5-$CH_3$ | H | H | H | $CH_3O$ | $CH_2$ | Cl |
| 4-$CH(CH_3)_2$ | H | H | H | $CH_3O$ | $CH_2$ | Cl |
| 4-$OCH_3$ | H | H | H | $CH_3O$ | $CH_2$ | Cl |

TABLE XIX (continued)

| R | $R_1$ | $R_2$ | $R_8$ | Y′ | Q | L |
|---|---|---|---|---|---|---|
| 5-$OCH(CH_3)_2$ | H | H | H | $CH_3O$ | $CH_2$ | Cl |
| H | H | H | H | $OCH_2CH_3$ | $CH_2$ | Cl |
| H | H | H | H | $OCH_2CH_3$ | O | Cl |
| H | H | H | $CH_3$ | $CH_3$ | $CH_2$ | Cl |
| H | H | H | $CH_3$ | $OCH_3$ | $CH_2$ | Cl |
| H | H | H | H | $CH_3$ | O | OH |
| H | H | $CH_3$ | H | $CH_3$ | O | OH |
| H | H | $CH_3$ | H | $OCH_3$ | O | OH |
| H | $CH_3$ | $CH_3$ | H | $CH_3$ | O | OH |
| H | H | H | H | H | $CH_2$ | OH |
| H | H | H | H | Cl | $CH_2$ | OH |
| H | H | H | H | $CH_3$ | $CH_2$ | OH |
| H | H | H | H | H | O | OH |
| 4-$CH_3$ | H | H | H | $OCH_3$ | O | OH |
| 5-$OC_2H_5$ | H | H | H | $CH_3$ | O | OH |
| 4-$OCH_2CH_2CH_3$ | H | H | H | $OCH_3$ | O | OH |
| H | H | H | $CH_3$ | $CH_3$ | O | OH |
| H | H | H | $CH_3$ | $OCH_3$ | O | OH |
| H | H | H | H | $OC_2H_5$ | O | OH |
| H | H | H | H | $OC_2H_5$ | $CH_2$ | OH |

TABLE XX

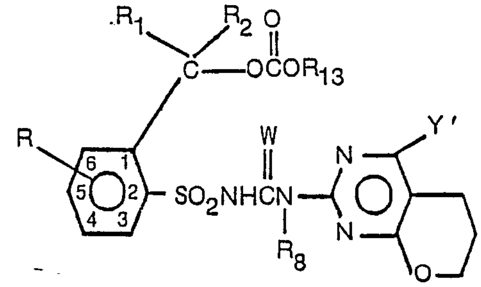

| R | $R_1$ | $R_2$ | $R_{13}$ | W | $R_8$ | Y' | m.p. (°C) |
|---|---|---|---|---|---|---|---|
| H | H | H | $—CH_3$ | O | H | $CH_3$ | |
| H | H | H | $—CH_3$ | O | H | $OCH_3$ | |
| H | H | H | $—CH_2CH_3$ | O | H | $CH_3$ | |
| H | H | H | 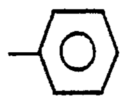 | O | H | $CH_3$ | |
| H | H | $CH_3$ | $CH_3$ | O | H | $OCH_3$ | |
| H | H | H | $CH_3$ | O | H | H | |
| H | H | H | $CH_3$ | O | H | Cl | |
| $4\text{-}OC_2H_5$ | H | H | $CH_3$ | O | H | $OCH_3$ | |
| 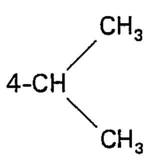 | H | H | $CH_3$ | O | H | $OCH_3$ | |
| $4\text{-}CH_3$ | H | H | $CH_3$ | O | H | $OCH_3$ | |
| $4\text{-}CF_3$ | H | H | $CH_3$ | | | $OCH_3$ | |
| H | H | H | $CH_3$ | S | H | $OCH_3$ | |
| H | H | H | $CH_3$ | O | $CH_3$ | $CH_3$ | |
| H | H | H | $CH_3$ | O | $CH_3$ | $OCH_3$ | |
| H | H | H | $CH_3$ | O | $OCH_3$ | $OCH_3$ | |

TABLE XXI

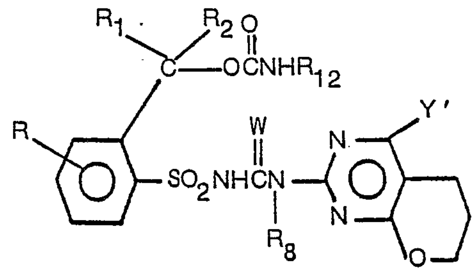

| R | R$_1$ | R$_2$ | R$_{12}$ | W | R$_8$ | Y' | m.p. (°C) |
|---|---|---|---|---|---|---|---|
| H | H | H | H | O | H | $CH_3$ | |
| H | H | H | $-CH_3$ | O | H | $OCH_3$ | |
| H | H | H | $-CH_2CH_3$ | O | H | $CH_3$ | |
| H | H | H | $-CH(CH_3)_2$ | O | H | $CH_3$ | |
| H | H | H | $CH_2CH{=}CH_2$ | O | H | $OCH_3$ | |
| H | H | H | 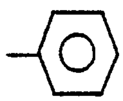 | O | H | $CH_3$ | |
| H | H | $CH_3$ | $CH_3$ | O | H | $OCH_3$ | |
| 4-$CH_3$ | H | H | $CH_3$ | O | H | $OCH_3$ | |
| H | H | H | $CH_3$ | O | H | H | |
| H | H | H | $CH_3$ | O | H | Cl | |
| H | H | H | $CH_3$ | O | H | $CH_3$ | |
| H | H | H | $CH_3$ | O | H | $OCH_3$ | |
| H | H | H | $CH_3$ | S | H | $CH_3$ | |
| H | H | H | $CH_3$ | S | H | $OCH_3$ | |
| H | H | H | $CH_3$ | O | $CH_3$ | $CH_3$ | |
| H | H | H | $CH_3$ | O | $CH_3$ | $OCH_3$ | |
| H | H | H | $CH_3$ | O | $OCH_3$ | $CH_3$ | |
| H | H | H | $CH_3$ | O | $OCH_3$ | $OCH_3$ | |
| H | H | H | $CH_3$ | O | H | $OC_2H_5$ | |
| H | H | H | $-CH_2-(CH_2)_4CH_3$ | O | H | $OCH_3$ | |
| H | H | H | $CH_2-CH{=}CH-CH_3$ | O | H | $CH_3$ | |
| H | H | H | 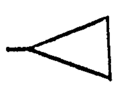 | 1050 | H | $OCH_3$ | |

TABLE XXII

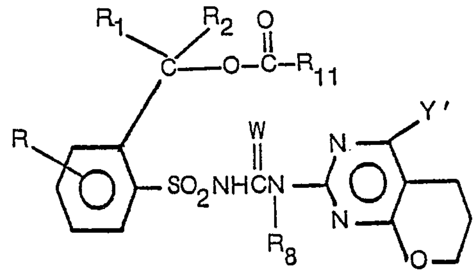

| R | $R_1$ | $R_2$ | $R_{11}$ | W | $R_8$ | Y′ | m.p. (°C) |
|---|---|---|---|---|---|---|---|
| H | H | H | H | O | H | $CH_3$ | |
| H | H | H | H | O | H | $OCH_3$ | |
| H | H | H | $CH_3$ | O | H | $CH_3$ | |
| H | H | H | $CH_3$ | O | H | $OCH_3$ | |
| 4-$OC_2H_5$ | H | H | $—CH{=}CH_2$ | O | H | $CH_3$ | |
| 4-$CH_3$ | H | H | $CH_3$ | O | H | $CH_3$ | |
| 5-$CH_3$ | H | H | $CH_3$ | O | H | $OCH_3$ | |
| 5-$NO_2$ | H | H | $CH_3$ | O | H | $OCH_3$ | |
| 5-$OCH_3$ | H | H | $CH_3$ | O | H | $OCH_3$ | |
| 3-$CF_3$ | H | H | $CH_3$ | O | H | $OCH_3$ | |
| 5-Cl | H | H | $CH_3$ | O | H | $CH_3$ | |
| 6-Cl | H | H | $CH_3$ | O | H | $CH_3$ | |

TABLE XXIII

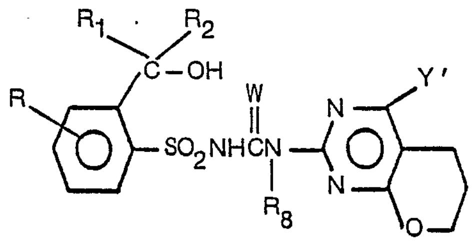

| R | $R_1$ | $R_2$ | W | $R_8$ | Y' | m.p. (°C) |
|---|---|---|---|---|---|---|
| H | H | H | O | H | $CH_3$ | |
| H | H | H | O | H | $OCH_3$ | |
| H | H | $CH_3$ | O | H | $CH_3$ | |
| H | H | $CH_3$ | O | H | $OCH_3$ | |
| H | $CH_3$ | $CH_3$ | O | H | $CH_3$ | |
| H | H | H | O | H | $OCH_3$ | |
| H | H | H | O | H | H | |
| 5-$CH_3$ | H | H | O | H | $OCH_3$ | |
| 4-$CH_3$ | H | H | O | H | Cl | |
| 4-$OCH_2CH_2CH_3$ | H | H | O | H | $OCH_3$ | |
| H | H | H | S | H | $CH_3$ | |
| H | H | H | S | H | $OCH_3$ | |
| H | H | H | O | $CH_3$ | $CH_3$ | |

TABLE XXIV

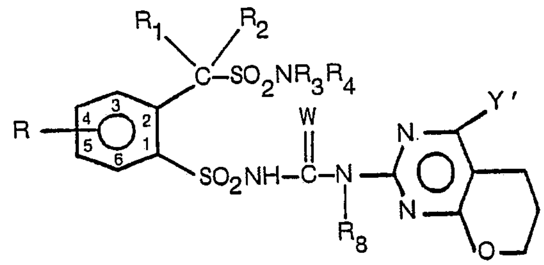

| R | $R_1$ | $R_2$ | $R_3$ | $R_4$ | W | $R_8$ | Y' |
|---|---|---|---|---|---|---|---|
| H | H | H | $CH_3$ | $CH_3$ | O | H | $CH_3$ |
| H | H | H | $CH_3$ | $CH_3$ | O | H | $C_2H_5O$ |
| H | H | H | $CH_3$ | $CH_3$ | O | H | H |
| H | H | H | $CH_3$ | $CH_3$ | S | H | $CH_3O$ |
| H | $CH_3$ | $CH_3$ | $CH_3$ | $CH_3$ | O | H | $CH_3O$ |
| 3-Cl | H | H | $CH_3$ | $CH_3$ | O | H | $CH_3O$ |
| 4-Cl | H | H | $CH_3$ | $CH_3$ | O | H | $CH_3O$ |
| 5-Cl | H | H | $CH_3$ | $CH_3$ | O | H | $CH_3O$ |
| 6-CL | H | H | $CH_3$ | $CH_3$ | O | H | $CH_3O$ |

TABLE XXV

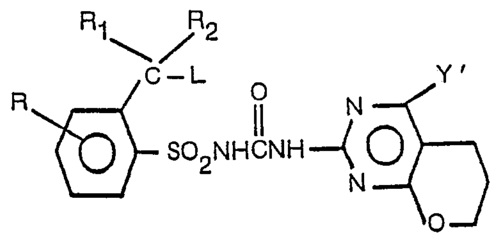

| L | R | $R_1$ | $R_2$ | $Y_1$ | Q |
|---|---|---|---|---|---|
| $OCH_3$ | H | H | H | $CH_3O$ | O |
| $SCH_3$ | H | H | H | $CH_3$ | O |
| $S(O)CH_3$ | H | H | H | $CH_3O$ | O |
| $S(O)CH_2CH_2CH_2CH_3$ | 4-$CH_3CH_2CH_2$ | H | H | $CH_3$ | $CH_2$ |
| $S(O)_2C(CH_3)_2$ | 5-$(CH_3)_2CH$ | H | H | $CH_3O$ | $CH_2$ |

TABLE XXVI

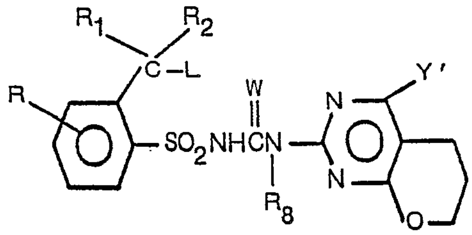

| L | R | $R_1$ | $R_2$ | $R_8$ | Y' | W |
|---|---|---|---|---|---|---|
| $CO_2CH_3$ | H | H | H | H | H | O |
| $CO_2CH_3$ | H | H | H | H | $CH_3$ | O |
| $CO_2CH_3$ | H | H | H | H | $CH_3O$ | O |
| $CO_2CH_3$ | H | $CH_3$ | H | H | $CH_3CH_2O$ | O |
| $CO_2CH_3$ | H | H | H | H | $CH_3$ | S |
| H | H | H | $OCH_2CH_3$ | H | $NHCONHCH_3$ | O |
| 5-Cl | H | Cl | $CH_3$ | H | Cl | O |
| H | H | H | $CH_3$ | H | Cl | O |
| H | H | H | $OCH_3$ | H | Cl | O |
| H | Cl | H | $CH_3$ | H | Cl | O |
| H | Cl | H | $OCH_3$ | H | Cl | O |
| H | H | H | $OCH_2CH_3$ | H | Cl | O |
| H | Cl | H | $OCH_2CH_3$ | H | Cl | O |
| H | H | H | $CH_3$ | H | 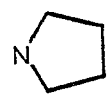 | O |
| H | H | H | $CH_3$ | H | $N^{\oplus}(CH_3)_3$ | O |
| H | H | H | $CH_3$ | H | $NH\overset{O}{\overset{\|}{C}}CH_3$ | O |

TABLE XXVII

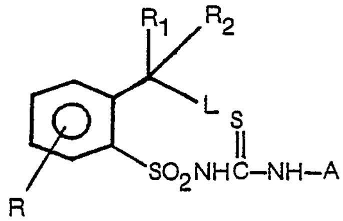

| L | R | $R_1$ | $R_2$ | A |
|---|---|---|---|---|
| $OCH_3$ | H | H | H | 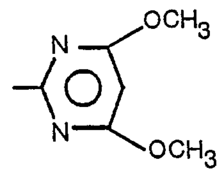 |
| $OCH_3$ | H | H | H | 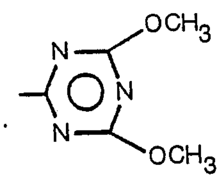 |
| $OCH_3$ | H | H | H | 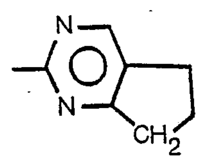 |
| $OCH_3$ | H | H | H | 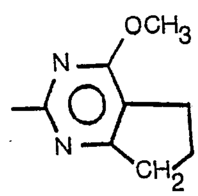 |
| $OCH_3$ | H | H | H | 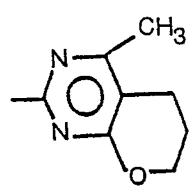 |
| $OCH_3$ | H | $CH_3$ | H | 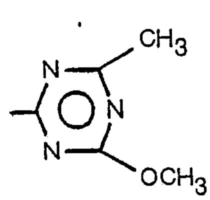 |
| $OCH_3$ | H | $CH_3$ | H | 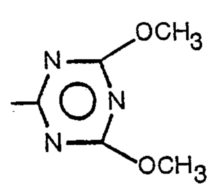 |

TABLE XXVII (continued)

| L | R | $R_1$ | $R_2$ | A |
|---|---|---|---|---|
| $SCH_3$ | H | H | H | 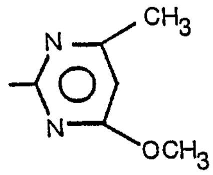 |
| $SCH_3$ | H | H | H | 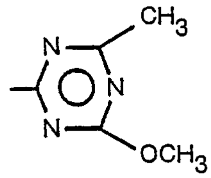 |
| $SCH_3$ | H | H | H | 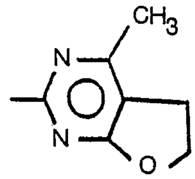 |
| $SCH_3$ | H | H | H | 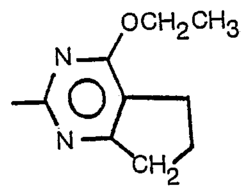 |
| $SCH_3$ | H | H | H | 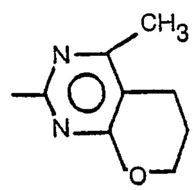 |
| 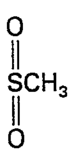 | H | H | H | 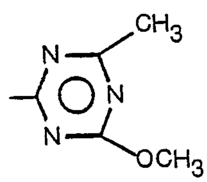 |
| 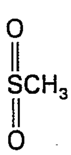 | H | H | H | 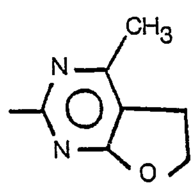 |
| $S(O)_2CH_3$ | H | $CH_3$ | $CH_3$ | 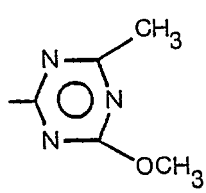 |

TABLE XXVIII

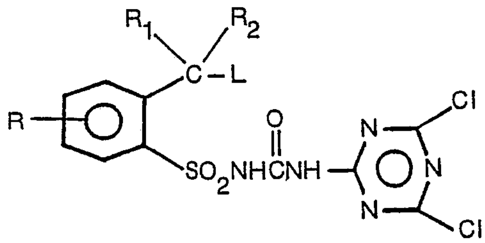

| R | $R_1$ | $R_2$ | L |
|---|---|---|---|
| H | H | H | OH |
| H | H | $CH_3$ | OH |
| H | $CH_3$ | $CH_3$ | OH |
| 5-Cl | $CH_3CH_2$ | H | OH |
| 6-F | $CH_3CH_2CH_2$ | H | OH |
| 3-Br | $(CH_3)_2CH$ | H | OH |
| 5-$NO_2$ | $CH_3CH_2CH_2CH_2$ | H | OH |
| 5-$CF_3$ | $CH_3CH_2CHCH_3$ | H | OH |
| 4-$CH_3$ | H | H | OH |
| 5-$CH_3CH_2CH_2$ | H | H | OH |
| 5-$CH_3O$ | H | H | OH |
| 5-$CH_3CH_2O$— | H | H | OH |
| 5-$CH_3CH_2CH_2O$— | H | H | OH |
| H | H | H | $O\overset{O}{\overset{\|}{C}}H$ |
| H | H | H | $O—\overset{O}{\overset{\|}{C}}CH_3$ |
| H | H | H | $O—\overset{O}{\overset{\|}{C}}CH_2CH_3$ |
| H | H | H | $O—\overset{O}{\overset{\|}{C}}—(CH_2)_4CH_3$ |
| H | H | H | $O—\overset{O}{\overset{\|}{C}}—CH{=}CH_2$ |
| H | H | H | 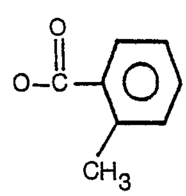 |

TABLE XXVIII (continued)

| R | $R_1$ | $R_2$ | L |
|---|---|---|---|
| H | H | H | 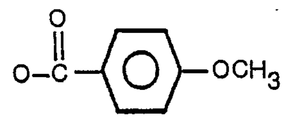 |
| H | H | H | 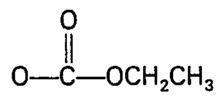 |
| H | H | H | 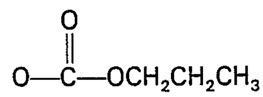 |
| H | H | H | 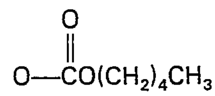 |

TABLE XXIX

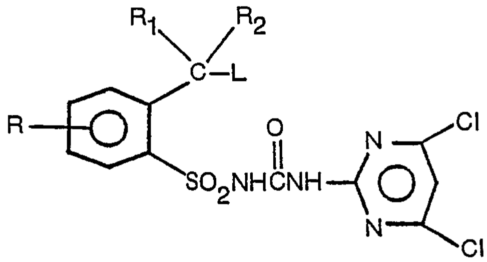

| R | $R_1$ | $R_2$ | L |
|---|---|---|---|
| H | H | H | 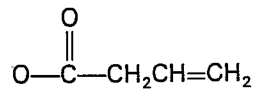 |
| H | H | H | 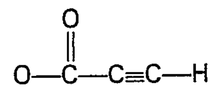 |
| H | H | H | 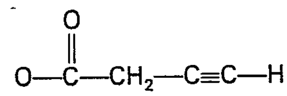 |
| H | H | H | 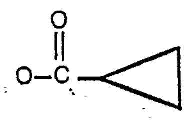 |
| H | H | H | 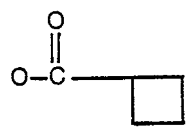 |
| H | H | H | 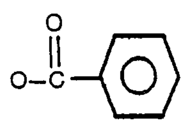 |
| H | H | H | 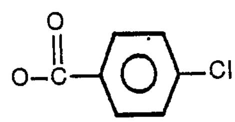 |
| H | H | H | 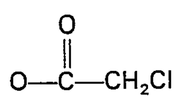 |
| H | H | H | 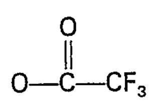 |
| H | H | H | 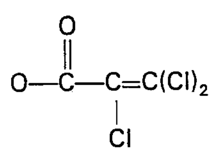 |
| H | H | H | 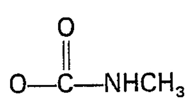 |

TABLE XXIX (continued)

| R | $R_1$ | $R_2$ | L |
|---|---|---|---|
| H | H | H | 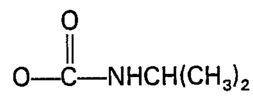 |
| H | H | H | 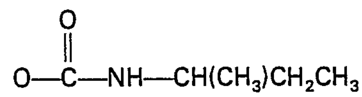 |
| H | H | H | 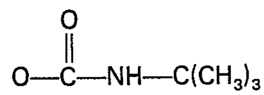 |
| H | H | H | 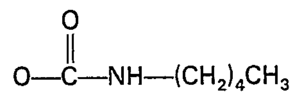 |
| H | H | H | 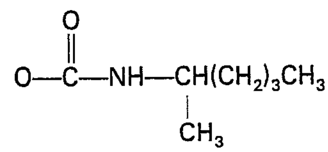 |
| H | H | H | 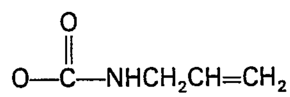 |
| H | H | H | 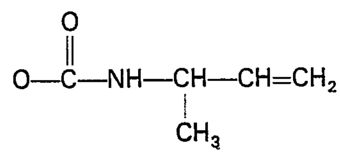 |
| H | H | H | 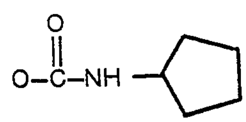 |
| H | H | H | 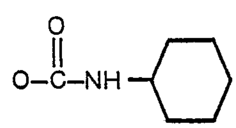 |
| H | H | H | 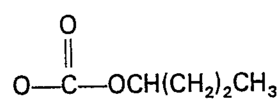 |
| H | H | H | 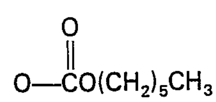 |
| H | H | H | 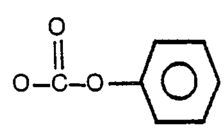 |

TABLE XXX

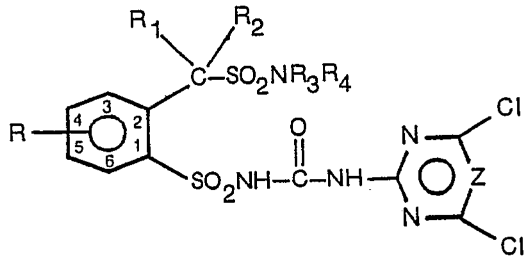

| R | $R_1$ | $R_2$ | $R_3$ | $R_4$ | Z |
|---|---|---|---|---|---|
| H | H | H | $CH_3O$ | $CH_3$ | CH |
| H | H | H | $C_2H_5$ | $CH_3$ | CH |
| H | H | H | $CH_3$ | $CH_3$ | N |
| H | H | H | $CH_3O$ | $CH_3$ | N |
| H | H | H | $C_2H_5$ | $CH_3$ | N |
| 3-Cl | H | H | $CH_3$ | $CH_3$ | N |
| 4-Cl | H | H | $CH_3$ | $CH_3$ | N |
| 5-Cl | H | H | $CH_3$ | $CH_3$ | N |
| 6-Cl | H | H | $CH_3$ | $CH_3$ | N |

TABLE XXXI

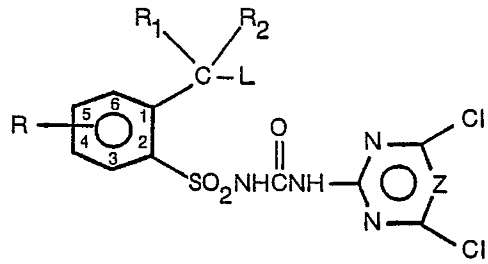

| L | R | $R_1$ | $R_2$ | Z |
|---|---|---|---|---|
| $CO_2CH_3$ | H | H | H | N |
| $CO_2CH_3$ | H | H | H | CH |
| $CO_2C_2H_5$ | H | $CH_3$ | H | N |
| $OCH_3$ | H | H | H | CH |
| $SO_2CH_3$ | H | H | H | CH |
| $OCH_3$ | H | H | H | N |
| $SCH_3$ | H | H | H | N |
| $S(O)_2CH_3$ | H | H | H | N |
| $SCH_2CH_2CH_2CH_3$ | 4-$CH_3CH_2CH_2$ | H | H | CH |
| $OCH_3$ | H | H | H | N |
| $SCH_3$ | H | $CH_3CH_2$ | H | CH |
| Cl | H | H | H | CH |
| Cl | H | H | Cl | CH |
| Cl | 5-$OCH_3$ | H | H | N |
| Cl | 6-$CH_3$ | H | H | CH |
| 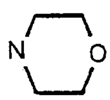 | H | H | H | CH |

TABLE XXXII

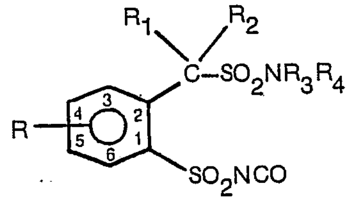

| R | $R_1$ | $R_2$ | $R_3$ | $R_4$ |
|---|---|---|---|---|
| H | H | H | $CH_3$ | $CH_3$ |
| H | H | H | $C_2H_5$ | $CH_3$ |

TABLE XXXIII

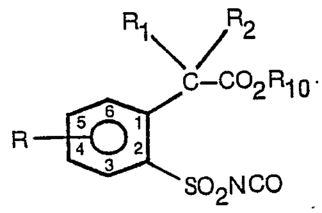

| R | $R_1$ | $R_2$ | $R_{10}$ |
|---|---|---|---|
| H | H | H | $CH_3$ |
| H | H | H | $CH_3CH_2$ |
| H | H | H | $(CH_3)_2CH$ |
| H | H | H | $CH_3CH_2CH_2CH_2$ |
| H | H | H | $CH_2{=}CH{-}CH_2$ |
| H | $CH_3$ | H | $CH_3$ |
| 3-Cl | H | H | $CH_3$ |
| 4-$NO_2$ | H | H | $CH_3$ |
| 4-$CF_3$ | H | H | $CH_3$ |
| 3-$CH_3$ | H | H | $CH_3$ |
| 4-$CH_3O$ | H | H | $CH_3$ |
| 5-F | H | H | $CH_3CH_2$ |
| 5-Br | H | H | $CH_3CH_2$ |

TABLE XXXIV

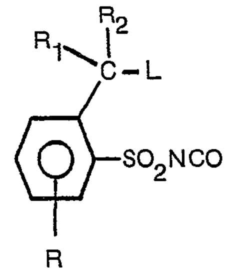

| L | $R_1$ | $R_2$ | R |
|---|---|---|---|
| Cl | H | H | H |
| Cl | Cl | H | H |
| Br | H | H | H |
| Br | Cl | H | H |
| Cl | H | $CH_3$ | H |
| Cl | H | H | 5-$NO_2$ |
| Cl | H | H | 6-Cl |
| $OCH_3$ | H | H | $CH_3$ |
| $OCH_3$ | H | $CH_3$ | $CH_3$ |
| $SCH_3$ | H | H | H |
| $SCH_3$ | H | H | $CH_3$ |
| $SCH_3$ | H | $CH_3$ | $CH_3$ |
| $OCH_2CF_3$ | 5-Cl | H | H |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid diluent(s). More specifically, they will contain these ingredients in the following approximate proportions:

TABLE XXXV

| | Active Ingredient | Weight Percent* Diluent(s) | Surfactant(s) |
|---|---|---|---|
| Wettable powders | 20—90 | 0—74 | 1—10 |
| Oil Suspensions, Emulsions, Solutions, (including Emulsifiable Concentrates) | 3—50 | 40—95 | 0—15 |
| Aqueous Suspension | 10—50 | 40—84 | 1—20 |
| Dusts | 1—25 | 70—99 | 0—5 |
| Granules and Pellets | 0.1—95 | 5—99.9 | 0—15 |
| High Strength Compositions | 90—99 | 0—10 | 0—2 |

*Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering,* December 4, 1967, pp. 147ff. and "Perry's Chemical Engineer's Handbook", 5th Ed., McGraw-Hill, New York, 1973, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182;

H. Gysin and E. Knusli, U.S. Patent 2,891,855, June 23, 1959, Col. 3, line 66 through Col. 5, line 17 and Examples 1—4;

G. C. Klingman, "Weed Control as a Science", John Wiley & Sons, Inc., New York, 1961, pp. 81—96; and

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

Example 51

Wettable Powder

| | |
|---|---|
| 2-[[(4,6-dimethoxypyrimidin-2-yl)aminocarbonyl]aminosulfonyl]benzeneacetic acid, methyl ester | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 52

Wettable Powder

| | |
|---|---|
| 2-[[(4,6-dimethylpyrimidin-2-yl)aminocarbonyl]aminosulfonyl]benzeneacetic acid, methyl ester | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 53

Extruded Pellet

| | |
|---|---|
| 2-[[(4-methoxy-6-methylpyrimidin-2-yl)aminocarbonyl]-aminosulfonyl]benzeneacetic acid, methyl ester | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 54

Wettable Powder

| | |
|---|---|
| 2-[[(4-methoxy-6-methyl-1,3,5-triazin-2-yl)aminocarbonyl]-aminosulfonyl]benzeneacetic acid, methyl ester | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 55

Low Strength Granule

| | |
|---|---|
| 2-[[(4,6-dimethoxypyrimidin-2-yl)aminocarbonyl]aminosulfonyl]benzeneacetic acid, ethyl ester | 1% |
| N,N-dimethylformamide | 9% |
| attapulgite granules | 90% |
| (U.S.S. 20—40 sieve) | |

The active ingredient is dissolved in the solvent and the solution is sprayed upon dedusted granules in a double cone blender. After spraying of the solution has been completed, the blender is allowed to run for a short period and then the granules are packaged.

Example 56

Solution

| | |
|---|---|
| 2-[[(4,6-dimethoxypyrimidin-2-yl)aminocarbonyl]aminosulfonyl]benzeneacetic acid, methyl ester, sodium salt | 5% |
| water | 95% |

The salt is added directly to the water with stirring to produce the solution, which may then be packaged for use.

Example 57

Wettable Powder

| | |
|---|---|
| 2-[[(4-methoxy-6-methyl-1,3,5-triazin-2-yl)aminocarbonyl]-aminosulfonyl]benzenacetic acid, methyl ester | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 58

Wettable Powder

| | |
|---|---|
| 2-[[(4,6-dimethoxypyrimidin-2-yl)aminocarbonyl]aminosulfonyl]benzeneacetic acid, ethyl ester | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 59

Wettable Powder

| | |
|---|---|
| 2-(dichloromethyl)-N-[(4-methoxy-6-methylpyrimidin-2-yl)aminocarbonyl]benzenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles of active essentially all below 10 microns in diameter. The product is reblended before packaging.

Example 60

Granule

| | |
|---|---|
| wettable powder of Example 59 | 5% |
| attapulgite granules | 95% |
| (U.S.S. 20—40 mesh; 0.84—0.42 mm) | |

A slurry of wettable powder containing ~25% solids is sprayed on the surface of attapulgite granules in a double-cone blended. The granules are dried and packaged.

Example 61

Aqueous Suspension

| | |
|---|---|
| 2-(dichloromethyl)-N-[(4,6-dimethylpyrimidin-2-yl)-aminocarbonyl]benzenesulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1% |
| water | 56.7% |

The ingredients are blended and ground together in a sand mill to produce particles essentially of under 5 microns in size.

Example 62

Granule

| | |
|---|---|
| 2-(dichloromethyl)-N-[(4-methoxy-6-methylpyrimidin-2-yl)-aminocarbonyl]benzenesulfonamide | 80% |
| wetting agent | 1% |
| crude ligninsulfonate salt (containing 5—20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules

of the desired size range are made. The spraying is stopped, but fluidization is continued, optionally with heat, until the water constant is reduced to the desired level, generally less than 1%. The material is then discharged, screened to the desired size range, generally 14—100 mesh (1410—149 microns), and packaged for use.

Example 63

High-Strength Concentrate

| | |
|---|---|
| 2-(dichloromethyl)-N-[(4,6-dimethylpyrimidin-2-yl)-aminocarbonyl]benzenesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 64

Wettable Powder

| | |
|---|---|
| N-[(4-6-dimethylpyrimidin-2-yl)aminocarbonyl]-2-(methoxymethyl)benzenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns and then reblended.

Example 65

Granule

| | |
|---|---|
| wettable powder of Example 64 | 5% |
| attapulgite granules | 95% |
| (U.S.S. 20—40 mesh; 0.84—0.42 mm) | |

A slurry of wettable powder containing ≈25% solids is sprayed on the surface of attapulgite granules in a double-cone blender. The granules are dried and packaged.

Example 66

Extruded Pellet

| | |
|---|---|
| N-[(4,6-dimethoxypyrimidin-2-yl)amino-carbonyl]-2-(methoxymethyl)benzenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm

long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 67

Oil Suspension

| | |
|---|---|
| N-[(4,6-dimethyl-1,3,5-triazin-2-yl)-aminocarbonyl]-2-(methoxymethyl)benzenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 68

Solution

| | |
|---|---|
| N-[(4-methoxy-6-methylpyrimidin-2-yl)-aminocarbonyl]-2-(methylthiomethyl)-benzenesulfonamide, sodium salt | 5% |
| water | 95% |

The salt is added directly to the water with stirring to produce the solution, which may then be packaged for use.

Example 69

Low Strength Granule

| | |
|---|---|
| N-[(4,6-dimethoxypyrimidin-2-yl)-aminocarbonyl]-2-(methylthiomethyl)benzenesulfonamide | 0.1% |
| attapulgite granules | 99.9% |
| (U.S.S. 20—40 mesh) | |

The active ingredient is dissolved in a solvent and the solution is sprayed upon dedusted granules in a double cone blender. After spraying of the solution has been completed, the material is warmed to evaporate the solvent. The material is allowed to cool and then packaged.

Example 70

Dust

| | |
|---|---|
| N-[(4,6-dimethoxypyrimidin-2-yl)aminocarbonyl]-2-(methylthiomethyl)benzenesulfonamide | 10% |
| attapulgite | 10% |
| Pyrophyllite | 80% |

The active ingredient is blended with attapulgite and then passed through a hammer mill to produce particles substantially all below 200 microns. The ground concentrate is then blended with powdered pyrophyllite until homogeneous.

Example 71

Wettable Powder

| | |
|---|---|
| 2-[(Dimethylamino)sulfonylmethyl]-N-[(4-methoxy-6-methylpyrimidin-2-yl)aminocarbonyl]-benzenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles of active essentially all below 10 microns in diameter. The product is reblended before packaging.

Example 72

Wettable Powder

| | |
|---|---|
| 2-[(Dimethylamino)sulfonylmethyl]-N-[(4-methoxy-6-methyl-1,3,5-triazin-2-yl)aminocarbonyl]-benzenesulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 73

Low strength Granule

| | |
|---|---|
| 2-[(Dimethylamino)sulfonylmethyl]-N-[(4,6-dimethoxy-1,3,5-triazin-2-yl)aminocarbonyl]benzenesulfonamide | 1% |
| N,N-dimethylformamide | 9% |
| attapulgite granules | 90% |
| (U.S.S. 20—40 sieve) | |

The active ingredient is dissolved in the solvent and the solution is sprayed upon dedusted granules in a double cone blender. After spraying of the solution has been completed, the blender is allowed to run for a short period and then the granules are packaged.

Example 74

High Strength Concentrate

| | |
|---|---|
| 2-[(Dimethylamino)sulfonylmethyl]-N-[(4,6-dimethoxy-1,3,5-triazin-2-yl)aminocarbonyl]benzenesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 75

Wettable Powder

| | |
|---|---|
| 2-[(Dimethylamino)sulfonylmethyl]-N-[(4-methoxy-6-methyl-1,3,5-triazin-2-yl)aminocarbonyl]benzenesulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 76

Oil Suspension

| | |
|---|---|
| 2-[(Dimethylamino)sulfonylmethyl]-N-[(4,6-dimethoxypyrimidin-2-yl)aminocarbonyl]benzenesulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 77

Oil Suspension

| | |
|---|---|
| N-[(4,6-Dimethoxy-1,3,5-triazin-2-yl)aminocarbonyl)-2-(hydroxymethyl)-benzenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 78

Aqueous Suspension

| | |
|---|---|
| N-[(4,6-Dimethoxypyrimidin-2-yl)-aminocarbonyl]-2-(hydroxymethyl)-benzenesulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| Water | 56.7% |

The ingredients are blended and ground together in a sand mill to produce particles essentially all under 5 microns in size.

Example 79

Granule

| | |
|---|---|
| N-[(4,6-Dimethoxy-1,3,5-triazin-2-yl)-aminocarbonyl]-2-(hydroxymethyl)-benzenesulfonamide | 80% |
| wetting agent | 1% |
| crude ligninsulfonate salt (containing 5—20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water constant is reduced to the desired level, generally less than 1%. The material is then discharged, screened to the desired size range, generally 14—100 mesh (1410—149 microns), and packaged for use.

Example 80

Wettable Powder

| | |
|---|---|
| N-[(4,6-Dimethoxy-1,3,5-triazin-2-yl)-aminocarbonyl]-2-(hydroxymethyl)-benzenesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Utility

The compounds of the present invention are active herbicides. They have utility for broadspectrum pre- and/or post-emergence weed control in areas where complete control of all vegetation is desired, such as around fuel storage tanks, ammunition depots, industrial storage areas, oil well sites, drive-in theaters, around billboards, highway and railroad structures. By properly selecting rate and time of application, compounds of this invention may be used to modify plant growth beneficially, and also selectively control weeds in crops such as wheat and barley.

The precise amount of the compound of Formula I to be used in any given situation will vary according to the particular end result desired, the amount of foliage present, the weeds to be controlled, the soil type, the formulation and mode of application, weather conditions, etc. Since so many variables play a role, it is not possible to state a rate of application suitable for all situations. Broadly speaking, the compounds of this invention are used at levels of about 0.001 to 20 kg/ha with a preferred range of 0.01 to 10 kg/ha. In general, the higher rates of application from within this range will be selected for adverse conditions or where extended persistence in soil is desired.

The compounds of Formula I may be combined with other herbicides and are particularly useful in combination with ureas such as 3-(3,4-dichlorophenyl)-1,1-dimethylurea (diuron); the triazines such as 2-chloro-4-(ethylamino)-$\varepsilon$-(isopropylamino)-*s*-triazine (atrazine); the uracils such as 5-bromo-3-*sec*-butyl-6-methyluracil (bromacil); N-(phosphonomethyl)glycine (glyphosate); 3-cyclohexyl-1-methyl-6-dimethylamino-*s*-triazine-2,4(1H,3H)-dione (hexazinone); N,N-dimethyl-2,2-diphenylacetamide (diphenamide); 2,4-dichlorophenoxyacetic acid (2,4-d) (and closely related compounds); 4-chloro-2-butynyl-3-chlorophenylcarbamate (barban); 5-(2,3-dichloroallyl)diisopropylthiocarbamate (diallate); 5-(2,3,3-trichloroallyl)diisopropylthiocarbamate (triallate); 1,2-dimethyl-3,5-diphenyl-1H-pyrazolium methyl sulfate (difenzoquat methyl sulfate); methyl 2-[4-(2,4-dichlorophenoxy)-phenoxy]propanoate (diclofop methyl); 4-amino-6-*tert*-butyl-3-(methylthio)-1,2,4-triazin-5(4H)one (metribuzin); 3-(3,4-dichlorophenyl)-1-methoxy-1-methylurea (linuron); 3-isopropyl-1H-2,1,3-benzothiodiazin-4(3H)-one-2,2-dioxide (bentazon); $\alpha,\alpha,\alpha$-trifluoro-2,6-dinitro-N,N-dipropyl-*p*-toluidine (trifluralin); 1,1′-dimethyl-4,4′-bipyridinium ion (paraquat); monosodium methanearsonate (MSMA); 2-chloro-2′,6′-diethyl(methoxymethyl)acetanilide (alachlor); and 1,1-dimethyl-3-($\alpha,\alpha,\alpha$-trifluoro-*m*-tolyl)urea (fluometuron), and 5-[2-chloro-4-(trifluoromethyl) phenoxy-2-nitrobenzoic acid, methyl ester (acifluorfen, methyl).

The activity of these compounds was discovered in greenhouse tests. The test procedures are described and the data shown below.

Test A

Seeds of crabgrass (*Digitaria* spp.), barnyardgrass (*Echinochloa crusgalli*), wild oats (*Avena fatua*), cassia (*Cassia tora*), morningglory (*Ipomoea* sp.), cocklebur (*Xanthium* spp.), sorghum, corn, soybean, rice, wheat and nutsedge tubers (*Cyperus rotundus*) were planted in a growth medium and treated pre-emergence with the chemicals dissolved in a nonphytotoxic solvent. At the same time, cotton having five leaves (including cotyledonary ones), bush beans with the second trifoliate leaf expanding, crabgrass and barnyardgrass with two leaves, wild oats with one leaf, cassia with three leaves (including cotyledonary ones), morningglory and cocklebur with four leaves (including the cotyledonary ones), sorghum and corn with three leaves, soybean with two cotyledonary leaves, rice with two leaves, wheat with two leaves, and nutsedge with three to five leaves were sprayed. Treated plants and controls were maintained in a greenhouse for sixteen days, then all species were compared to controls and visually rated for response to treatment.

The ratings, shown in Table A, are based on a numerical scale extending from 0 = no injury, to 10 = complete kill. The accompanying descriptive symbols have the following meanings:

C = chlorosis/necrosis;

D = defoliation;

E = emergence inhibition;

G = growth retardation;

H = formative effects;

S = albinism;

U = unusual pigmentation;

6F = delayed flowering; and

6Y = flowerbuds abscised.

COMPOUND 1

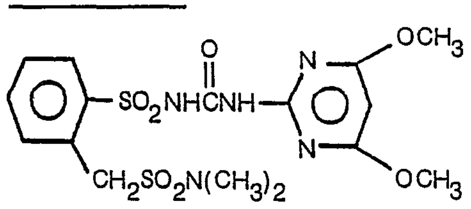

COMPOUND 2

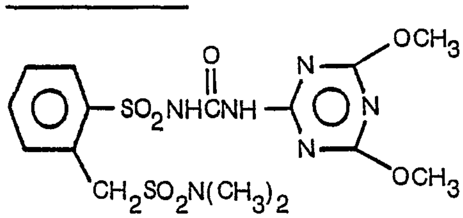

COMPOUND 3

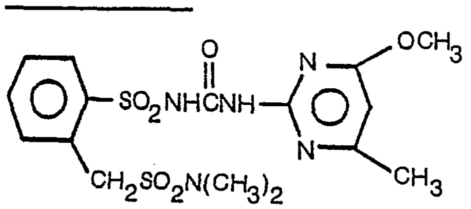

COMPOUND 4

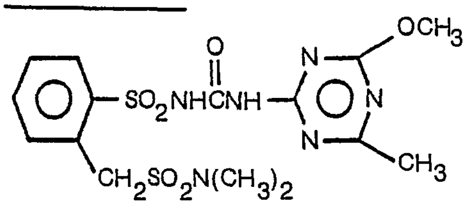

COMPOUND 5

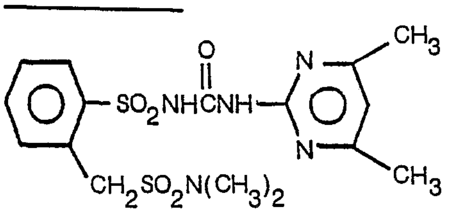

COMPOUND 6

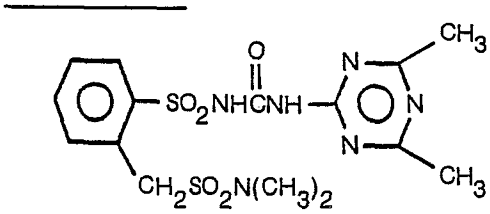

COMPOUND 7

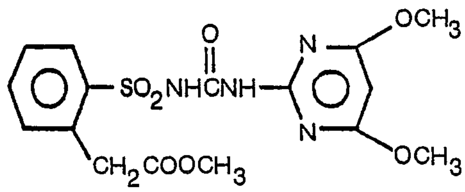

COMPOUND 8

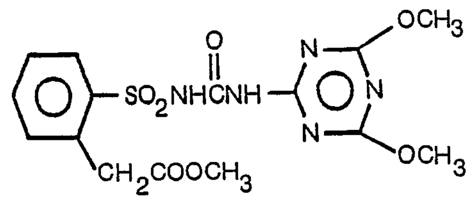

COMPOUND 9

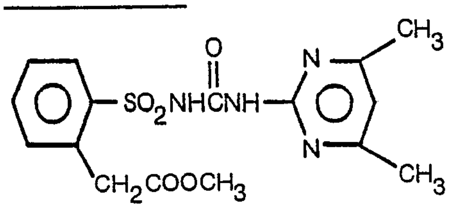

COMPOUND 10

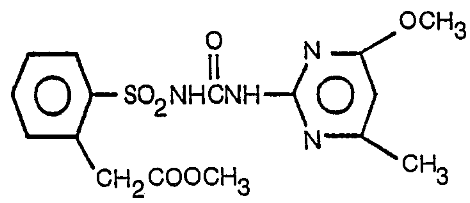

COMPOUND 11

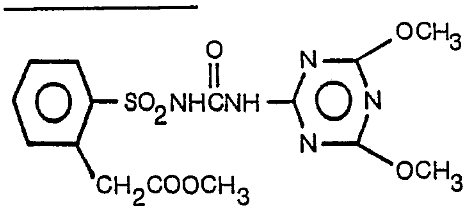

COMPOUND 12

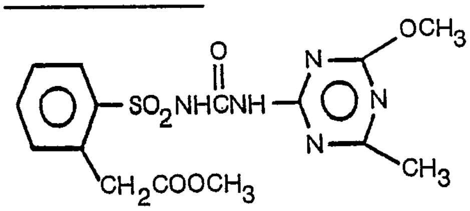

COMPOUND 13

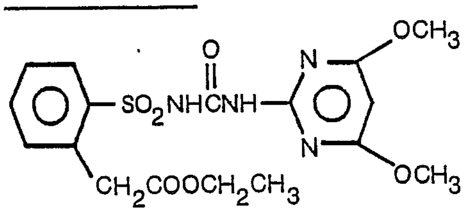

COMPOUND 14

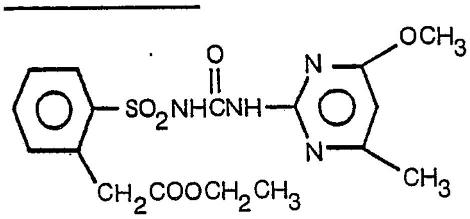

COMPOUND 15

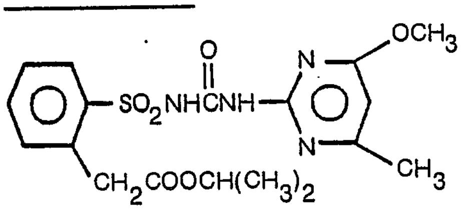

COMPOUND 16

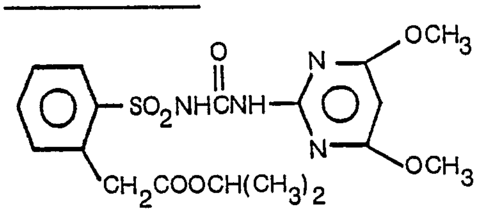

COMPOUND 17

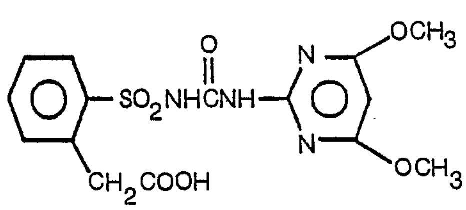

COMPOUND 18

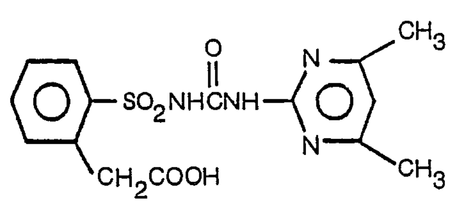

COMPOUND 19

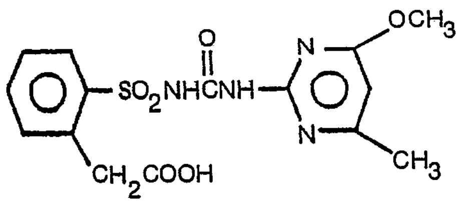

COMPOUND 20

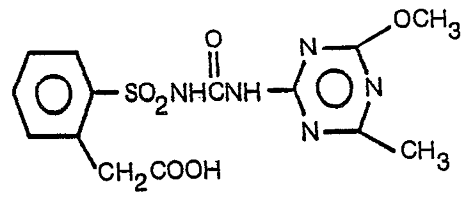

COMPOUND 21

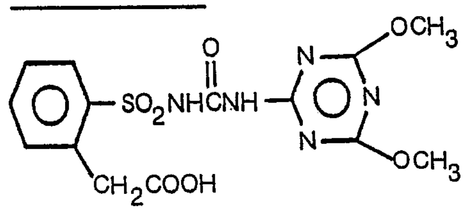

TABLE A

| | COMPOUND 1 | | COMPOUND 2 | |
|---|---|---|---|---|
| Rate, kg/ha | 0.1 | 2 | 0.1 | 2 |
| POST-EMERGENCE | | | | |
| Bushbean | 5C, 9G, 6Y | 4C, 9G, 6Y | 1C, 4G, 6Y | 4S, 9G, 6Y |
| Cotton | 5C, 9G | 5C, 8G | 1C | 5C, 8G |
| Morningglory | 4C, 9H | 5C, 9G | 2C, 5G | 3C, 6G |
| Cocklebur | 3C, 9G | 4C, 9G | 0 | 1C |
| Cassia | 3C, 7H | 3C, 6G | 1C | 1C |
| Nutsedge | 1C, 9G | 1C, 9G | 0 | 1C, 5G |
| Crabgrass | 1C, 3G | 8G | 0 | 0 |
| Barnyardgrass | 3C, 9H | 9C | 0 | 0 |
| Wild Oats | 0 | 0 | 0 | 0 |
| Wheat | 0 | 0 | 0 | 0 |
| Corn | 2C, 7H | 5U, 9C | 0 | 0 |
| Soybean | 3C, 9G | 4C, 9G | 1C, 1H | 2C, 4G |
| Rice | 1C, 5G | 8G | 0 | 2G |
| Sorghum | 5C, 9G | 9C | 0 | 1C, 2G |
| PRE-EMERGENCE | | | | |
| Morningglory | 2C, 5H | 9G | 0 | 3C, 6G |
| Cocklebur | 1C, 3H | 9H | 0 | 6G |
| Cassia | 6C | 3C, 8G | 0 | 2C |
| Nutsedge | 10E | 10E | 0 | 3G |
| Crabgrass | 1C | 2C, 8G | 0 | 0 |
| Barnyardgrass | 3C, 8H | 5C, 9H | 0 | 1C, 2G |
| Wild Oats | 1C | 3C, 7G | 0 | 3G |
| Wheat | 1C | 4G | 0 | 4G |
| Corn | 2C, 9H | 9H | 0 | 3C, 9H |
| Soybean | 3C, 5H | 9H | 0 | 2C, 3G |
| Rice | 2C, 6G | 10E | 0 | 3G |
| Sorghum | 2C, 9G | 5C, 9H | 0 | 3C, 8G |

TABLE A (Continued)

| | COMPOUND 3 | | COMPOUND 4 | |
|---|---|---|---|---|
| Rate, kg/ha | 0.1 | 2 | 0.1 | 2 |
| POST-EMERGENCE | | | | |
| Bushbean | 5C, 9G, 6Y | 5C, 9G, 6Y | 2C, 5G, 6Y | 3C, 7G, 6Y |
| Cotton | 3C, 3H, 7G | 5C, 7G | 3C, 3H | 3C, 4H |
| Morningglory | 3C, 8H | 6C, 9G | 2C, 4H | 3C, 4G |
| Cocklebur | 3C, 9H | 4C, 9G | 1C | 1C |
| Cassia | 3C | 3C, 5G | 1C | 1C |
| Nutsedge | 2C, 6G | 3C, 8G | 2G | 0 |
| Crabgrass | 0 | 2C, 5G | 0 | 0 |
| Barnyardgrass | 1C, 6H | 4C, 9H | 0 | 0 |
| Wild Oats | 0 | 1C, 6G | 0 | 0 |
| Wheat | 0 | 3G | 0 | 0 |
| Corn | 2C, 5H | 2C, 9H | 0 | 1C, 4G |
| Soybean | 2C, 8G, 5X | 2C, 8G | 1C, 3G | 1C, 4G |
| Rice | 1C, 2G | 3C, 7G | 0 | 2G |
| Sorghum | 3C, 9H | 3C, 9G | 0 | 2C, 4G |
| PRE-EMERGENCE | | | | |
| Morningglory | 3C, 5H | 9G | 1C | 4C, 9G |
| Cocklebur | 9H | 9H | 0 | 2C |
| Cassia | 3C, 3H | 2C, 8G | 2C | 2C |
| Nutsedge | 2C, 6G | 10E | 1C | 8G |
| Crabgrass | 2C | 2C, 8G | 2G | 4G |
| Barnyardgrass | 2C, 6H | 5C, 9H | 1C | 6G |
| Wild Oats | 2C, 6G | 4C, 8G | 0 | 4G |
| Wheat | 2C | 1C, 7G | 2G | 0 |
| Corn | 3C, 8H | 9G | 1C, 6G | 9G, 2C |
| Soybean | 3C, 6H | 9H | 1C | 1H, 1C |
| Rice | 3C, 7G | 9H | 1C, 4G | 2C |
| Sorghum | 5C, 8H | 4C, 9H | 1C, 3G | 9G, 3C |

TABLE A (Continued)

| | COMPOUND 5 | | COMPOUND 6 |
|---|---|---|---|
| Rate, kg/ha | 0.1 | 2 | 2 |
| POST-EMERGENCE | | | |
| Bushbean | 3G | 2C, 9G | 0 |
| Cotton | 1C | 2C, 1H | 0 |
| Morningglory | 1C, 9H | 2C | 0 |
| Cocklebur | 1C | 1C | 0 |
| Cassia | 0 | 2G | 0 |
| Nutsedge | 0 | 1C, 5G | 0 |
| Crabgrass | 0 | 0 | 0 |
| Barnyardgrass | 0 | 0 | 0 |
| Wild Oats | 0 | 0 | 0 |
| Wheat | 0 | 0 | 0 |
| Corn | 0 | 0 | 0 |
| Soybean | 1C | 0 | 0 |
| Rice | 0 | 0 | 0 |
| Sorghum | 1C | 1C, 3G | 0 |
| PRE-EMERGENCE | | | |
| Morningglory | 1C | 3C, 8G | 0 |
| Cocklebur | 0 | 9H | 0 |
| Cassia | 0 | 2C | 0 |
| Nutsedge | 0 | 5G | 4G |
| Crabgrass | 0 | 2C | 5G |
| Barnyardgrass | 0 | 2C, 6G | 1C, 3G |
| Wild Oats | 0 | 4G | 0 |
| Wheat | 2G | 2G | 0 |
| Corn | 1C | 2C, 8H | 2C, 4G |
| Soybean | 1C | 1H | 1H |
| Rice | 2C | 5C | 1C |
| Sorghum | 2C | 3C, 7G | 2C, 5G |

TABLE A (Continued)

| | COMPOUND 7 | COMPOUND 8 | COMPOUND 9 | COMPOUND 10 |
|---|---|---|---|---|
| Rate, kg/ha | .05 | 0.4 | 0.4 | 0.4 |
| POST-EMERGENCE | | | | |
| Bushbean | 9C | 9C | 9D, 9G, 6Y | 9D, 9G, 6Y |
| Cotton | 5C, 9G | 3C, 9G | 2U, 6C, 9G | 10C |
| Morningglory | 3C, 8H | 9C | 5C, 9G | 9C |
| Cocklebur | 5C, 9H | 9C | 5C, 9G | 5C, 9H |
| Cassia | 6C, 9G | 3C, 7G | 2C, 6G | 5C, 9G |
| Nutsedge | 3C, 9G | 1C | 9G | 3C, 9G |
| Crabgrass | 4G | 0 | 10C | 9C |
| Barnyardgrass | 4G | 1C, 2H | 3C, 9H | 9C |
| Wild Oats | 0 | 0 | 6C, 9G | 5C, 9G |
| Wheat | 0 | 0 | 9C | 6C, 9G |
| Corn | 2C, 8H | 0 | 1C, 9G | 5U, 9G |
| Soybean | 2C, 9G | 10C | 2C, 8G | 9C |
| Rice | 5C, 9G | 2G | 4C, 9G | 9C |
| Sorghum | 9G | 3G | 9C | 5U, 9C |
| PRE-EMERGENCE | | | | |
| Morningglory | 9G | 9G | 5C, 9G | 9C, 9G |
| Cocklebur | 9H | 9H | 9H | 9H |
| Cassia | 8G | 8G | 9G | 1C, 9G |
| Nutsedge | 10E | 2G | 10E | 10E |
| Crabgrass | 5G | 1C | 1C, 7G | 2C, 9G |
| Barnyardgrass | 3C, 5G | 3C | 2C, 9H | 3C, 9H |
| Wild Oats | 6G | 0 | 5C, 9H | 3C, 9H |
| Wheat | 5G | 0 | 9H | 9H |
| Corn | 8G | 7G | 9H | 10H |
| Soybean | 1H | 7H | 8H | 8H |
| Rice | 10E | 9H | 10E | 10E |
| Sorghum | 9G | 6G | 10H | 5C, 9H |

TABLE A (Continued)

| | COMPOUND 11 | COMPOUND 12 | COMPOUND 13 | COMPOUND 14 |
|---|---|---|---|---|
| Rate, kg/ha | .05 | .05 | 0.4 | 0.4 |
| POST-EMERGENCE | | | | |
| Bushbean | 9D, 9G, 6Y | 9C | 7C, 9G | 6C, 9G |
| Cotton | 4C, 9G | 5C, 9G | 5C, 9G | 5C, 9G |
| Morningglory | 9C | 10C | 3C, 8H | 9C |
| Cocklebur | 9C | 10C | 9C | 9C |
| Cassia | 3C, 5G | 5C, 9G | 9C | 5C, 8G |
| Nutsedge | 0 | 0 | 2C, 8G | 7G |
| Crabgrass | 1C | 0 | 2C | 2C, 5G |
| Barnyardgrass | 1C | 1C | 2C, 5H | 3C, 9H |
| Wild Oats | 0 | 0 | 1C | 2C, 5G |
| Wheat | 0 | 0 | 0 | 1C, 5G |
| Corn | 1C | 2C, 5H | 2C, 6H | 2U, 9G |
| Soybean | 5C, 8G | 5C, 9G | 6C, 9G | 6C, 9G |
| Rice | 1C, 4G | 1C, 6G | 3C, 8G | 5C, 9G |
| Sorghum | 0 | 1C, 6G | 2C, 8H | 2C, 8G |
| PRE-EMERGENCE | | | | |
| Morningglory | 9G | 5C, 9G | 7G | 9G |
| Cocklebur | 9H | 9H | 9H | 9H |
| Cassia | 8G | 3C, 8G | 2C, 8G | 8G |
| Nutsedge | 8G | 10E | 10E | 10E |
| Crabgrass | 3G | 2G | 1C | 3G |
| Barnyardgrass | 1C | 1C | 2C, 6H | 2C, 8H |
| Wild Oats | 0 | 1C | 3G | 8G |
| Wheat | 0 | 2G | 0 | 8G |
| Corn | 1C, 6G | 1C, 8G | 2C, 8G | 2C, 9G |
| Soybean | 2C, 3H | 2C, 5H | 0 | 1C, 3H |
| Rice | 5G | 1C, 7G | 10E | 10E |
| Sorghum | 1C, 4G | 2C, 9H | 2C, 9G | 5C, 9H |

TABLE A (Continued)

| | COMPOUND 15 | COMPOUND 16 | COMPOUND 17 | COMPOUND 18 |
|---|---|---|---|---|
| Rate, kg/ha | .05 | 0.4 | 0.4 | 0.4 |
| POST-EMERGENCE | | | | |
| Bushbean | 1C, 6Y | 4C, 7G, 6Y | 9C | 2H, 6F |
| Cotton | 2C | 2C, 2H | 4C, 8G | 1H |
| Morningglory | 3B, 8G | 0 | 1C | 0 |
| Cocklebur | 3C, 6G | 2C, 6G | 3C, 9G | 0 |
| Cassia | 2C | 2C | 3C, 5G | 0 |
| Nutsedge | 1C | 0 | 4G | 0 |
| Crabgrass | 0 | 0 | 2C | 0 |
| Barnyardgrass | 0 | 0 | 1C, 5G | 1C |
| Wild Oats | 0 | 0 | 0 | 0 |
| Wheat | 0 | 0 | 0 | 0 |
| Corn | 0 | 0 | 1C | 0 |
| Soybean | 1C | 0 | 1C, 3H | 0 |
| Rice | 0 | 2G | 5G | 2C |
| Sorghum | 1C | 1C | 1C, 5H | 1C |
| PRE-EMERGENCE | | | | |
| Morningglory | 2C, 5G | 0 | 6G | 0 |
| Cocklebur | 2C, 5G | 5H | 8H | 0 |
| Cassia | 1C | 4G | 2C, 5G | 0 |
| Nutsedge | 10E | 0 | 9G | 0 |
| Crabgrass | 2G | 0 | 2C | 0 |
| Barnyardgrass | 2G | 2G | 3C | 1C |
| Wild Oats | 0 | 0 | 1C | 0 |
| Wheat | 0 | 0 | 1C | 0 |
| Corn | 2C, 2G | 2C, 4G | 3C, 6H | 0 |
| Soybean | 0 | 1C | 2C | 0 |
| Rice | 2C | 1C, 5G | 9H | 0 |
| Sorghum | 2G | 4G | 2C, 8H | 0 |

TABLE A(Continued)

| | COMPOUND 19 | COMPOUND 20 | COMPOUND 21 |
|---|---|---|---|
| Rate, kg/ha | 0.4 | 0.4 | 0.4 |
| POST-EMERGENCE | | | |
| Bushbean | 4C,7G,6Y | 9C | 4S,5G,6Y |
| Cotton | 2C,3H | 5C,9G | 4C,7G |
| Morningglory | 1C | 9C | 2C,5G |
| Cocklebur | 1C | 10C | 2C |
| Cassia | 3C | 4C,8G | 2C |
| Nutsedge | 0 | 3G | 0 |
| Crabgrass | 2C | 1C | 0 |
| Barnyardgrass | 3C,7H | 2C,6H | 0 |
| Wild Oats | 3G | 0 | 0 |
| Wheat | 2G | 0 | 0 |
| Corn | 2C,6H | 2C,9H | 0 |
| Soybean | 3C,5G | 9C | 2C |
| Rice | 2C,6G | 1C,3G | 0 |
| Sorghum | 2C,7H | 2C,5G | 0 |
| PRE-EMERGENCE | | | |
| Morningglory | 8G | 9G | 5G |
| Cocklebur | 5H | 9H | 0 |
| Cassia | 5G | 5C,8G | 0 |
| Nutsedge | 3G | 8G | 10E |
| Crabgrass | 1C | 0 | 1C |
| Barnyardgrass | 2C,7H | 2G | 1C |
| Wild Oats | 8G | 0 | 0 |
| Wheat | 5G | 0 | 0 |
| Corn | 2C,7G | 2C,6G | 1C |
| Soybean | 1C,1H | 2C,6H | 0 |
| Rice | 2C,8G | 1C,3G | 0 |
| Sorghum | 2C,8H | 2C,8H | 0 |

TABLE A (Continued)

| | 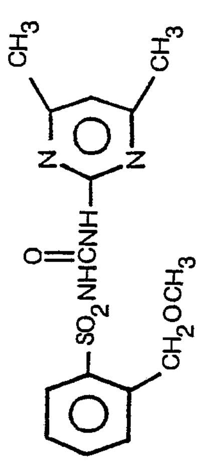 | 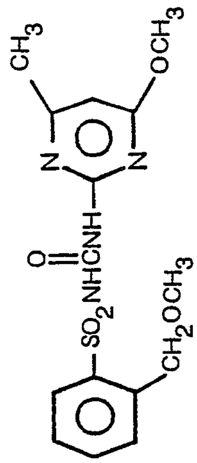 | 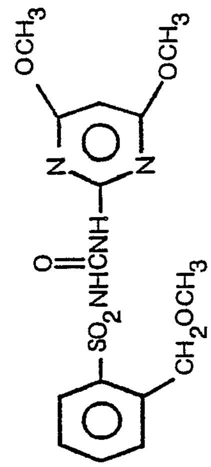 |
|---|---|---|---|
| Rate, kg/ha | 0.4 | 0.4 | 0.4 |
| POST-EMERGENCE | | | |
| Bushbean | 9C | 9C | 9C |
| Cotton | 9C | 9C | 9C |
| Morningglory | 10C | 10C | 10C |
| Cocklebur | 9C | 10C | 10C |
| Cassia | 9C | 9C | 9C |
| Nutsedge | 9C | 9C | 9C |
| Crabgrass | 9C | 9C | 6C, 9G |
| Barnyardgrass | 10C | 10C | 9C |
| Wild Oats | 9C | 9C | 6C, 9G |
| Wheat | 9C | 9C | 9C |
| Corn | 9C | 9C | 9C |
| Soybean | 9C | 9C | 9C |
| Rice | 9C | 9C | 9C |
| Sorghum | 9C | 10C | 9C |
| PRE-EMERGENCE | | | |
| Morningglory | 9G | 9C | 9G |
| Cocklebur | 9G | 9G | 9H |
| Cassia | 9G | 3C, 9G | 2C, 9G |
| Nutsedge | 10E | 10E | 10E |
| Crabgrass | 2C, 9G | 3C, 9G | 2C, 9G |
| Barnyardgrass | 5C, 9H | 6C, 9H | 7C, 9H |
| Wild Oats | 4C, 9H | 5C, 9H | 1C, 9G |
| Wheat | 9H | 10H | 9G |
| Corn | 10E | 10E | 9H |
| Soybean | 9H | 9H | 9H |
| Rice | 10E | 10E | 10E |
| Sorghum | 6C, 9H | 10H | 6C, 9H |

TABLE A (Continued)

| | 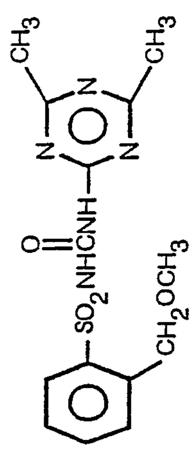 | 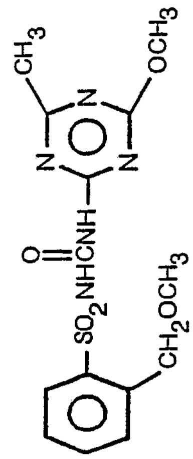 | 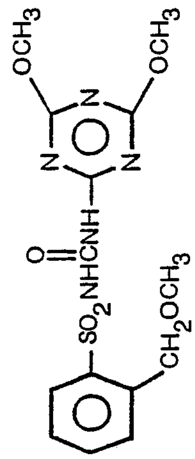 |
|---|---|---|---|
| Rate, kg/ha | 0.4 | 0.4 | 0.4 |
| POST-EMERGENCE | | | |
| Bushbean | 9C | 9C | 9C |
| Cotton | 7C,9G | 10C | 9C |
| Morningglory | 10C | 10C | 10C |
| Cocklebur | 10C | 9C | 10C |
| Cassia | 9C | 9C | 9C |
| Nutsedge | 10C | 8C | 9C |
| Crabgrass | 9C | 9C | 2C,6G |
| Barnyardgrass | 10C | 10C | 9C |
| Wild Oats | 6C,9G | 5C,9G | 2C |
| Wheat | 9C | 10C | 1C |
| Corn | 10C | 9C | 7C,9C |
| Soybean | 9C | 9C | 9C |
| Rice | 10C | 10C | 9C |
| Sorghum | 10C | 10C | 1C,9H |
| PRE-EMERGENCE | | | |
| Morningglory | 9C | 9C | 9C |
| Cocklebur | 9H | 9G | 9H |
| Cassia | 5C,9G | 2C,9G | 2C,9G |
| Nutsedge | 10E | 1C,9G | 9G |
| Crabgrass | 2C,8G | 3C,8G | 3C,5G |
| Barnyardgrass | 6C,9H | 5C,9H | 3C,9H |
| Wild Oats | 6C,9H | 5C,9H | 1C,8G |
| Wheat | 9G | 1C,9G | 5G |
| Corn | 9H | 9H | 9H |
| Soybean | 9H | 9H | 9H |
| Rice | 10E | 10E | 10E |
| Sorghum | 10H | 3C,9H | 9H |

0 044 209

TABLE A (Continued)

| | 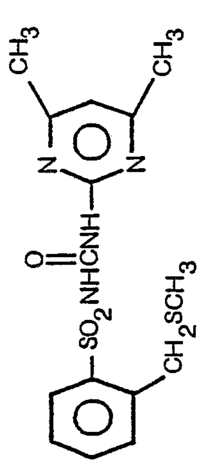 | 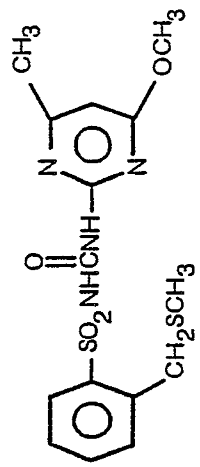 | 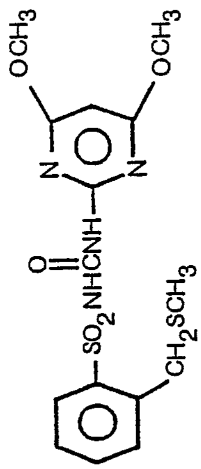 |
|---|---|---|---|
| Rate, kg/ha | 0.4 | 0.4 | 0.4 |
| POST-EMERGENCE | | | |
| Bushbean | 3C,6Y,7G | 9C | 9C |
| Cotton | 3C,3H,8G | 9C | 9C |
| Morningglory | 3C,6H | 5C,9G | 10C |
| Cocklebur | 3C,8G | 5C,9G | 10C |
| Cassia | 3C | 2C,6G | 9C |
| Nutsedge | 5G | 2C,8G | 6C,8G |
| Crabgrass | 1C,5G | 9C | 10C |
| Barnyardgrass | 10C | 9C | 9C |
| Wild Oats | 9C | 9C | 9C |
| Wheat | 2C,6C | 9C | 5C,9G |
| Corn | 1C,5H | 7U,9C | 5U,9C |
| Soybean | 1C,2G | 6C,9G | 9C |
| Rice | 2C,8G | 6C,9G | 5C,9G |
| Sorghum | 2C,8G | 3C,9G | 9C |
| PRE-EMERGENCE | | | |
| Morningglory | 9G | 9C | 9C |
| Cocklebur | 9H | 9H | 9H |
| Cassia | 3C,7H | 4C,9G | 9C |
| Nutsedge | 9G | 10E | 10E |
| Crabgrass | 2C,6G | 5C,9G | 6C,9H |
| Barnyardgrass | 2C,9H | 6C,9H | 6C,9H |
| Wild Oats | 3C,9G | 4C,9G | 4C,9G |
| Wheat | 9G | 3C,9G | 9G |
| Corn | 2C,9H | 10H | 10H |
| Soybean | 2C,2H | 8H | 9H |
| Rice | 10E | 10E | 10E |
| Sorghum | 2C,9G | 7C,9H | 6C,9H |

TABLE A (Continued)

| | 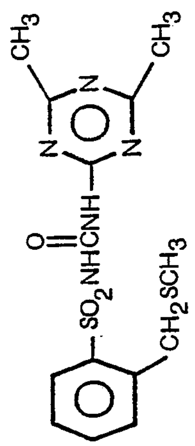 | 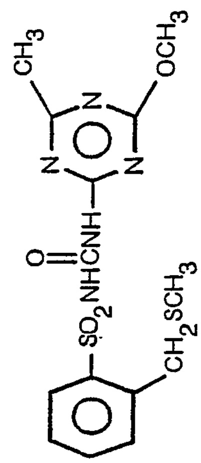 | 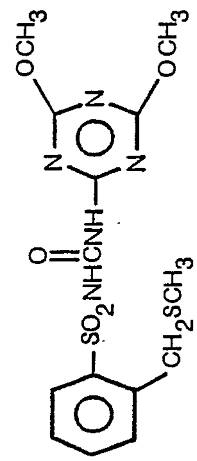 |
|---|---|---|---|
| Rate, kg/ha | 0.4 | 0.4 | 0.4 |
| POST-EMERGENCE | | | |
| Bushbean | 3H, 7G | 6C, 6Y, 9G | 2C, 6Y, 9G |
| Cotton | 1H | 3H, 4C, 9G | 3C, 5H |
| Morningglory | 1H | 1C, 2G | 2C, 7G |
| Cocklebur | 5C, 9G | 4C, 9G | 2C, 6G |
| Cassia | 0 | 2C | 2C |
| Nutsedge | 0 | 0 | 0 |
| Crabgrass | 0 | 0 | 5G |
| Barnyardgrass | 0 | 5H | 3H |
| Wild Oats | 0 | 0 | 0 |
| Wheat | 0 | 0 | 0 |
| Corn | 0 | 8H | 4C, 9G |
| Soybean | 1H | 2C, 7X, 8G | 2C, 5X, 8G |
| Rice | 0 | 2C, 7G | 9C |
| Sorghum | 0 | 2C, 8G | 1C, 6H |
| PRE-EMERGENCE | | | |
| Morningglory | 5G | 9G | 7G |
| Cocklebur | 5H | 9H | 9H |
| Cassia | 6G | 8G | 5G |
| Nutsedge | 0 | 0 | 0 |
| Crabgrass | 0 | 0 | 0 |
| Barnyardgrass | 0 | 2C, 7H | 6H |
| Wild Oats | 0 | 7G | 3G |
| Wheat | 0 | 2G | 0 |
| Corn | 0 | 9H | 1C, 8H |
| Soybean | 0 | 6H | 1C, 3H |
| Rice | 0 | 5H | 1C, 9H |
| Sorghum | 0 | 2C, 9G | 3C |

TABLE A (Continued)

| | 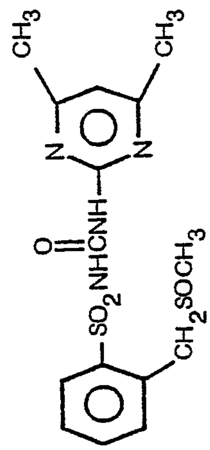 | 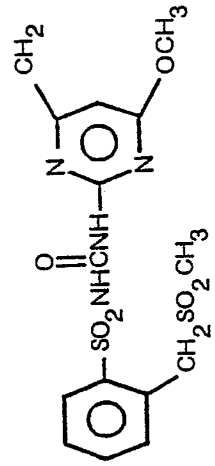 | 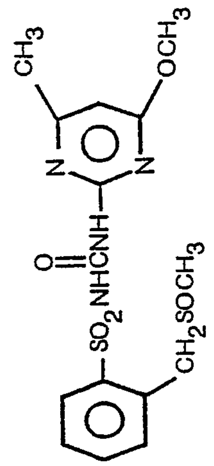 |
|---|---|---|---|
| Rate, kg/ha | 0.4 | 0.4 | 0.4 |
| POST-EMERGENCE | | | |
| Bushbean | 4S, 5G, 6F | 6C, 6Y, 9G | 6C, 6Y, 9G |
| Cotton | 3C, 5H | 7C, 9G | 6C, 9G |
| Morningglory | 3C, 8H | 10C | 9C |
| Cocklebur | 3C, 5G | 6C, 9G | 5C, 9G |
| Cassia | 1C | 4C, 7G | 6C, 8G |
| Nutsedge | 2C, 6G | 2C, 9G | 8G |
| Crabgrass | 1C | 3C, 7G | 5C, 9G |
| Barnyardgrass | 4C, 8H | 6C, 9H | 9C |
| Wild Oats | 4C, 9H | 2C | 4C, 8G |
| Wheat | 2C, 7G | 0 | 3C, 6G |
| Corn | 2C, 5G | 3U, 9G | 5C, 9G |
| Soybean | 3H | 2C, 9G | 3C, 9G |
| Rice | 4C, 9G | 3C, 9G | 5C, 9G |
| Sorghum | 1C, 9G | 2C, 8G | 4U, 9G |
| PRE-EMERGENCE | | | |
| Morningglory | 8H | 9G | 9C |
| Cocklebur | 10H | 9H | 9H |
| Cassia | 2C, 5G | 6C, 9G | 5C, 8G |
| Nutsedge | 2C, 8H | 10E | 10E |
| Crabgrass | 0 | 5C, 9G | 5C, 9G |
| Barnyardgrass | 2C | 6C, 9H | 5C, 9H |
| Wild Oats | 6G | 4C, 7G | 5C, 8G |
| Wheat | 4G | 2C, 7G | 1C, 9G |
| Corn | 3C, 8H | 6C, 9G | 3C, 9G |
| Soybean | 2C | 9H | 8H |
| Rice | 9H | 9H | 10E |
| Sorghum | 5C, 8G | 6C, 9H | 6C, 9H |

TABLE A (Continued)

| | 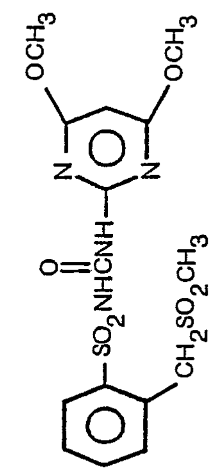 | 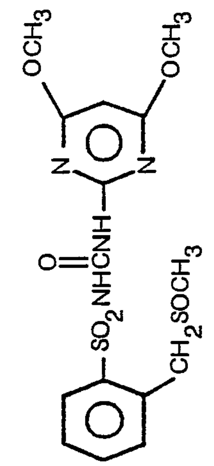 | 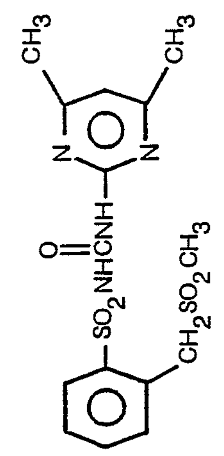 |
|---|---|---|---|
| Rate, kg/ha | 0.4 | 0.4 | 0.4 |
| POST-EMERGENCE | | | |
| Bushbean | 5C,6Y,8G | 9C | 1C,2H |
| Cotton | 9C | 9C | 3C,3H,9G |
| Morningglory | 10C | 10C | 4C,9G |
| Cocklebur | 10C | 9C | 4C,9G |
| Cassia | 6C,9G | 5C,9G | 2C,4H |
| Nutsedge | 6C,9G | 2C,9G | 2C |
| Crabgrass | 2C,7G | 3C,9H | 1C |
| Barnyardgrass | 9C | 9C | 4C,9H |
| Wild Oats | 0 | 3C,9H | 5C,9H |
| Wheat | 0 | 2C,4G | 1C |
| Corn | 3U,9H | 10C | 0 |
| Soybean | 4C,9G | 6C,9G | 2H |
| Rice | 3C,9G | 6C,9G | 6C,9G |
| Sorghum | 2U,9G | 9C | 4C,9H |
| PRE-EMERGENCE | | | |
| Morningglory | 10C | 4C,9G | 8H |
| Cocklebur | 9H | 9H | 9H |
| Cassia | 6C,9G | 5C,9G | 2C |
| Nutsedge | 10E | 10E | 0 |
| Crabgrass | 2C,7G | 5C,9G | 0 |
| Barnyardgrass | 5C,9H | 6C,9H | 1C,6H |
| Wild Oats | 2C,8G | 5C,8G | 9G |
| Wheat | 0 | 9G | 0 |
| Corn | 5C,9H | 10H | 2C,6H |
| Soybean | 9H | 9H | 2C |
| Rice | 10E | 10E | 2C,9H |
| Sorghum | 10H | 10H | 4C,9H |

TABLE A (Continued)

| | 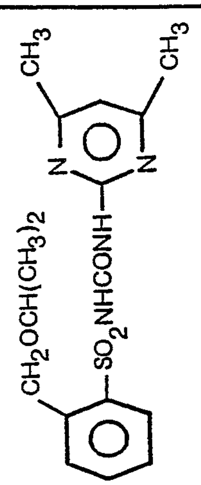 | 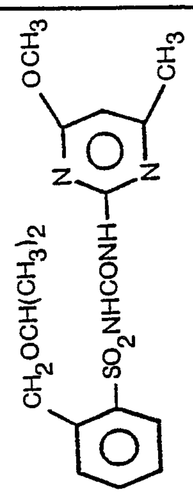 | 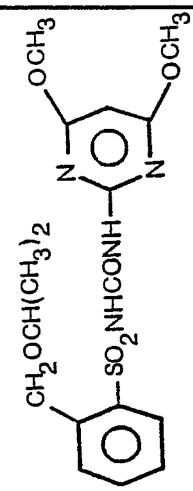 |
|---|---|---|---|
| Rate, kg/ha | .05 | .05 | .05 |
| POST-EMERGENCE | | | |
| Bushbean | 1C, 4G, 6Y | 9D, 9G, 6Y | 9C |
| Cotton | 2C, 3H, 7G | 2C, 3H, 8G | 3C, 9G |
| Morningglory | 2H | 2C, 2G | 3C, 9G |
| Cocklebur | 2C, 7G | 4C, 9G | 6C, 9G |
| Cassia | 2C | 3C, 8G | 2C, 8H |
| Nutsedge | 2C, 9G | 5C, 9G | 6C, 9G |
| Crabgrass | 0 | 5G | 3C, 5H |
| Barnyardgrass | 0 | 3C, 9H | 3C, 9H |
| Wild Oats | 0 | 2C, 5G | 2C, 7G |
| Wheat | 0 | 1C, 3G | 0 |
| Corn | 2C, 7H | 2U, 9G | 2C, 8H |
| Soybean | 5H | 5C, 9G | 6C,9G |
| Rice | 5G | 1C, 8G | 3C, 8G |
| Sorghum | 2C, 9H | 2U, 9G | 2C, 9H |
| PRE-EMERGENCE | | | |
| Morningglory | 6G | 9G | 9G |
| Cocklebur | 9H | 9H | 9H |
| Cassia | 2C, 5H | 1C, 9G | 9G |
| Nutsedge | 2G | 9G | 10E |
| Crabgrass | 0 | 3G | 2C, 5G |
| Barnyardgrass | 1H | 4C, 9H | 3C, 9H |
| Wild Oats | 2C, 5G | 4C, 9G | 1C, 7G |
| Wheat | 1C, 4G | 1C, 9G | 3G |
| Corn | 2C, 8H | 10H | 2C, 9H |
| Soybean | 2C | 9H | 9H |
| Rice | 2C, 6G | 2C, 8G | 4C, 8H |
| Sorghum | 1C, 7H | 2C, 9H | 2C, 8H |

TABLE A (Continued)

| | 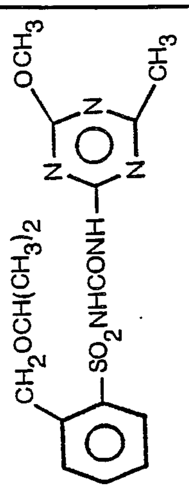 | 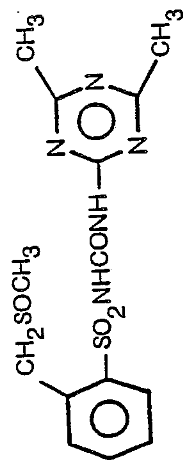 | 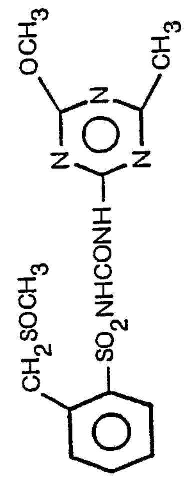 |
|---|---|---|---|
| Rate, kg/ha | .05 | .05 | 0.4 |
| POST-EMERGENCE | | | |
| Bushbean | 9C | 0 | 3C, 9G, 6Y |
| Cotton | 5C, 9G | 0 | 2C, 2H, 7G |
| Morningglory | 6C, 9G | 0 | 2C |
| Cocklebur | 6C, 9G | 0 | 1C |
| Cassia | 2C, 5H | 0 | 1C |
| Nutsedge | 1C | 0 | 0 |
| Crabgrass | 2G | 0 | 0 |
| Barnyardgrass | 1C, 4H | 0 | 1C |
| Wild Oats | 0 | 0 | 0 |
| Wheat | 0 | 0 | 0 |
| Corn | 2C, 9G | 0 | 2C, 9H |
| Soybean | 2C, 9G | 0 | 1C, 6H |
| Rice | 0 | 0 | 2C, 8G |
| Sorghum | 1C, 9G | 0 | 2C, 8H |
| PRE-EMERGENCE | | | |
| Morningglory | 2C, 9G | 10E | 2C, 6H |
| Cocklebur | 2C, 9H | 0 | 9H |
| Cassia | 5C, 9G | 0 | 5C |
| Nutsedge | 5G | 0 | 0 |
| Crabgrass | 0 | 0 | 0 |
| Barnyardgrass | 7H | 0 | 1C |
| Wild Oats | 0 | 0 | 0 |
| Wheat | 0 | 0 | 0 |
| Corn | 3C, 9G | 0 | 2C, 8H |
| Soybean | 2C, 8H | 0 | 1C, 3G |
| Rice | 2C, 5G | 0 | 2C, 5G |
| Sorghum | 2C, 9H | 0 | 2C, 9H |

TABLE A (Continued)

| | 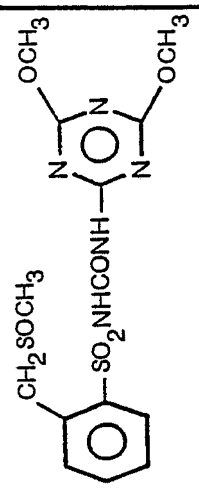 | 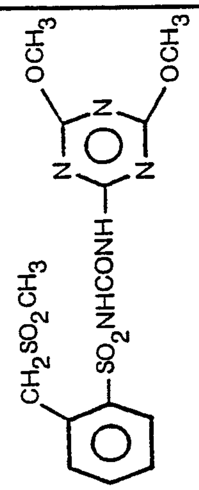 | 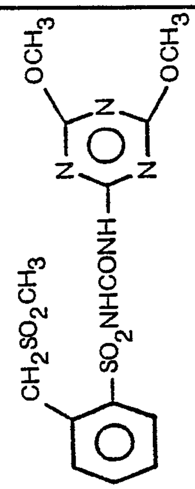 |
|---|---|---|---|
| Rate, kg/ha | 0.4 | 0.05 | 0.05 |
| POST-EMERGENCE | | | |
| Bushbean | 4S, 9G, 6Y | 3C, 7G, 6Y | 2C, 6G, 6Y |
| Cotton | 1C, 2G | 0 | 2G |
| Morningglory | 1C | 4G | 1H, 3G |
| Cocklebur | 0 | 4G | 0 |
| Cassia | 0 | 0 | 0 |
| Nutsedge | 0 | 0 | 0 |
| Crabgrass | 0 | 3G | 0 |
| Barnyardgrass | 1C, 2H | 1H | 0 |
| Wild Oats | 0 | 0 | 0 |
| Wheat | 0 | 0 | 0 |
| Corn | 2C, 9H | 2C, 7H | 1C, 5H |
| Soybean | 1C, 5H | 1H | 4H |
| Rice | 2C, 8G | 2G | 2G |
| Sorghum | 2C, 6H | 2C, 9H | 1C, 6H |
| PRE-EMERGENCE | | | |
| Morningglory | 1C | 0 | 0 |
| Cocklebur | 0 | 0 | 0 |
| Cassia | 0 | 0 | 0 |
| Nutsedge | 0 | 0 | 0 |
| Crabgrass | 0 | 0 | 0 |
| Barnyardgrass | 1C, 2H | 1C | 0 |
| Wild Oats | 0 | 0 | 0 |
| Wheat | 0 | 0 | 0 |
| Corn | 2C, 8H | 9H | 3G |
| Soybean | 2C | 1C | 1C |
| Rice | 1C, 5G | 0 | 3G |
| Sorghum | 1C, 7G | 2C, 4G | 2C |

TABLE A (Continued)

| | 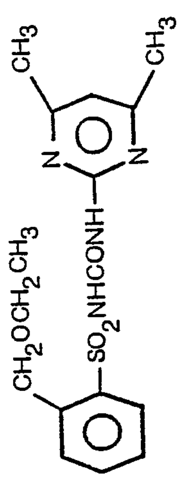 | 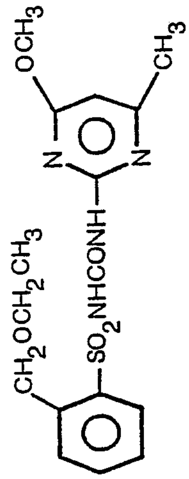 | 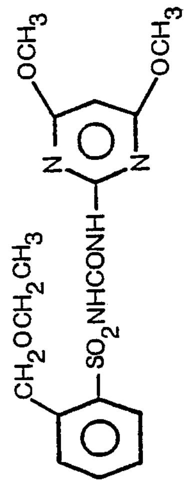 |
|---|---|---|---|
| Rate, kg/ha | .05 | .05 | .05 |
| POST-EMERGENCE | | | |
| Bushbean | 9C | 9C | 9C |
| Cotton | 6C,9G | 9C | 6C,9G |
| Morningglory | 3C,7G | 5C,9G | 9C |
| Cocklebur | 9C | 9C | 9C |
| Cassia | 3C,9G | 6C,9G | 2C,9G |
| Nutsedge | 9C | 9C | 9C |
| Crabgrass | 2C,5G | 2C,7G | 1C,3G |
| Barnyardgrass | 6C,9H | 9C | 4C,9H |
| Wild Oats | 9H | 2C,9G | 0 |
| Wheat | 2C,9G | 2C,9G | 0 |
| Corn | 3C,9G | 5U,9G | 2U,9G |
| Soybean | 2C,9G,5X | 4C,9G | 4C,9G |
| Rice | 3C,9G | 3C,9G | 2C,9G |
| Sorghum | 4U,9G | 3C,9G | 1U,9G |
| PRE-EMERGENCE | | | |
| Morningglory | 9G | 9G | 9G |
| Cocklebur | 9H | 9H | 9H |
| Cassia | 1C,9G | 2C,9G | 2C,9G |
| Nutsedge | 10E | 10E | 10E |
| Crabgrass | 1C,4G | 2C,6G | 2C,6G |
| Barnyardgrass | 2C,9H | 6C,9H | 3C,9H |
| Wild Oats | 1C,9G | 3C,9G | 2C,9H |
| Wheat | 9G | 2C,9G | 2C,7G |
| Corn | 1C,9G | 9H | 2U,9G |
| Soybean | 2C,3G | 9H | 9H |
| Rice | 10E | 9H | 4C,9H |
| Sorghum | 5C,9G | 3C,9H | 2C,9G |

TABLE A (Continued)

| | 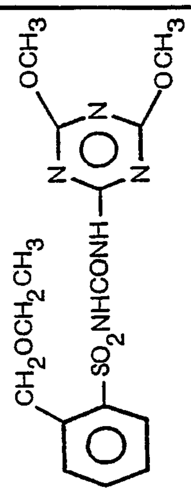 | 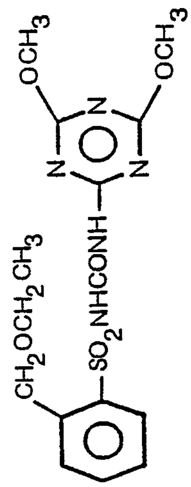 | 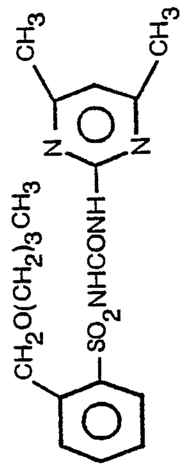 |
|---|---|---|---|
| Rate, kg/ha | .05 | .05 | .05 |
| POST-EMERGENCE | | | |
| Bushbean | 9C | 9C | 0 |
| Cotton | 9C | 9C | 1C, 3H |
| Morningglory | 10C | 9C | 1C, 6G |
| Cocklebur | 9C | 9C | 1C, 6F |
| Cassia | 5C, 9G | 5C, 9G | 1C |
| Nutsedge | 2C, 8G | 2C, 8G, 5X | 1C, 8G |
| Crabgrass | 1C, 2G | 1C | 1C, 7G |
| Barnyardgrass | 3C, 9H | 1C, 3G | 1C, 6H |
| Wild Oats | 4G | 0 | 6G |
| Wheat | 0 | 0 | 0 |
| Corn | 5U, 9G | 2C, 9H | 2C, 7H |
| Soybean | 4C, 9G | 3H, 9G, 7X | 2H, 8G |
| Rice | 6G | 6G | 5G |
| Sorghum | 2U, 9G | 1C, 7G | 2C, 9G |
| PRE-EMERGENCE | | | |
| Morningglory | 9G | 9G | 0 |
| Cocklebur | 9H | 9H | 0 |
| Cassia | 3C, 9G | 2C, 9G | 1C |
| Nutsedge | 6G | 8G | 0 |
| Crabgrass | 1C | 1C, 2G | 1C |
| Barnyardgrass | 2C, 8H | 2C, 8H | 1C, 5G |
| Wild Oats | 5G | 1C, 3G | 2C, 7G |
| Wheat | 2G | 0 | 3G |
| Corn | 9G | 2C, 9H | 2C, 7G |
| Soybean | 8H | 7H | 1C |
| Rice | 8H | 6G | 2C |
| Sorghum | 2C, 9G | 2C, 8G | 2C, 6G |

TABLE A (Continued)

| | 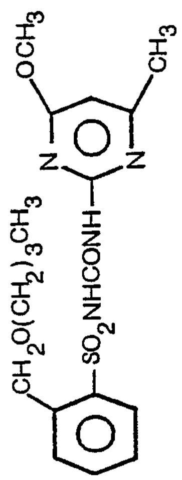 | 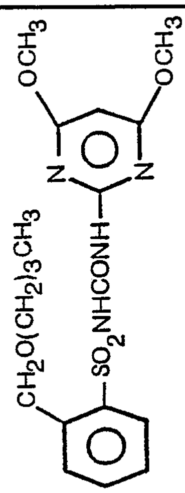 | 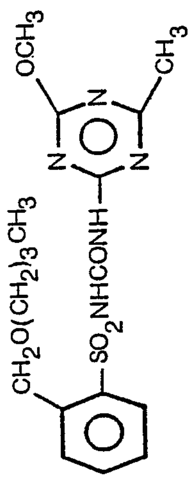 |
|---|---|---|---|
| Rate, kg/ha | .05 | .05 | .05 |
| POST-EMERGENCE | | | |
| Bushbean | 6S, 9G, 6Y | 2C, 9G, 6Y | 2C, 5G, 6Y |
| Cotton | 3C, 7G | 1C, 5G | 1C, 1H |
| Morningglory | 2C, 6G | 1C, 5G | 2C, 9G |
| Cocklebur | 3C, 9H | 1C, 9G | 2C, 9G |
| Cassia | 2C, 5G | 1C, 3G | 1C |
| Nutsedge | 4G | 1C, 7G | 0 |
| Crabgrass | 2C, 8G | 1C, 5G | 0 |
| Barnyardgrass | 2C, 8H | 2C, 9H | 0 |
| Wild Oats | 1C, 6G | 2G | 0 |
| Wheat | 1G | 0 | 0 |
| Corn | 1U, 9G | 2C, 7G | 2C, 8H |
| Soybean | 3C, 9G | 2C, 8G | 2C, 9H |
| Rice | 8G | 2C, 8G | 0 |
| Sorghum | 3C, 9H | 3C, 9G | 2C, 9G |
| PRE-EMERGENCE | | | |
| Morningglory | 1H, 7G | 1C | 9G |
| Cocklebur | 9H | 5H | 9H |
| Cassia | 2C, 6G | 2C, 3G | 0 |
| Nutsedge | 1C, 7G | 9G | 0 |
| Crabgrass | 1C, 5G | 2C | 0 |
| Barnyardgrass | 2C, 9H | 3C, 9H | 1C, 5G |
| Wild Oats | 2C, 8G | 2C, 7G | 0 |
| Wheat | 1C, 8G | 5G | 0 |
| Corn | 2C, 9G | 2C, 8G | 2C, 7G |
| Soybean | 9H | 5G | 1C |
| Rice | 4C, 9H | 2C, 9H | 2C |
| Sorghum | 2C, 9H | 2C, 9G | 3C, 9H |

TABLE A (Continued)

| | 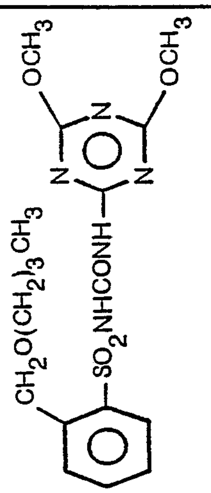 | 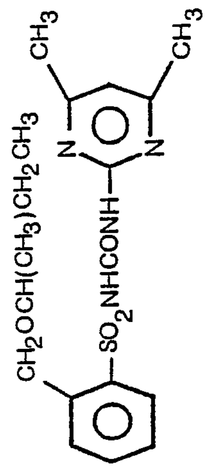 | 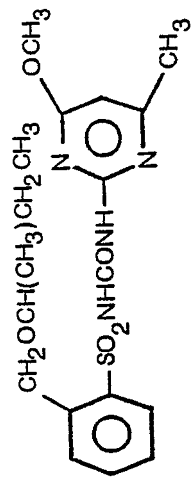 |
|---|---|---|---|
| Rate, kg/ha | 0.05 | 0.05 | 0.05 |
| POST-EMERGENCE | | | |
| Bushbean | 5S, 9G, 6Y | 2C | 4S, 9G, 6Y |
| Cotton | 2C, 5G | 4G | 3C, 3H |
| Morningglory | 1C, 7G | 2C, 7G | 4C, 9G |
| Cocklebur | 3G, 6F | 1C, 3G | 2C, 9G |
| Cassia | 3C | 2C | 2C, 7G |
| Nutsedge | 0 | 2C, 8G | 2C, 8G |
| Crabgrass | 0 | 2G | 7G |
| Barnyardgrass | 0 | 2H | 9H |
| Wild Oats | 0 | 0 | 1C, 4G |
| Wheat | 0 | 0 | 2G |
| Corn | 2G | 2C, 7H | 8G |
| Soybean | 1C, 9G | 1C, 8G | 9C |
| Rice | 0 | 1C, 3G | 1C, 8G |
| Sorghum | 2C, 8G | 2C, 9G | 1C, 9G |
| PRE-EMERGENCE | | | |
| Morningglory | 8G | 2G | 9G |
| Cocklebur | 9H | 9H | 9H |
| Cassia | 1C | 0 | 1C, 7G |
| Nutsedge | 0 | 5G | 9G |
| Crabgrass | 0 | 4G | 2C, 6G |
| Barnyardgrass | 1C | 3C, 6G | 1C, 9H |
| Wild Oats | 0 | 1C, 6G | 8G |
| Wheat | 0 | 3G | 8G |
| Corn | 1C, 3G | 2C, 6G | 9G |
| Soybean | 3G | 1C, 1H | 9H |
| Rice | 2C | 3C, 7G | 4C, 9H |
| Sorghum | 2C, 8G | 1C, 8G | 2C, 9H |

TABLE A (Continued)

| | 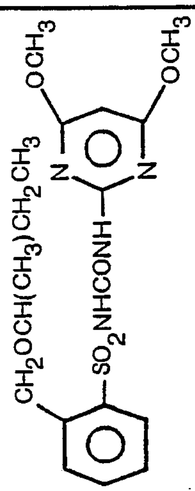 | 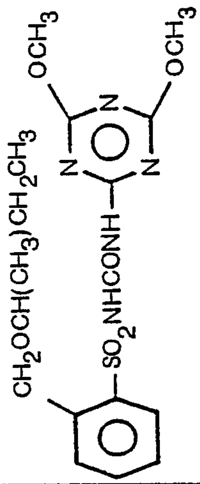 |
|---|---|---|
| Rate kg/ha | 0.05 | 0.05 |
| POST-EMERGENCE | | |
| Bushbean | 9D, 9G, 6Y | 3C, 9G, 6Y |
| Cotton | 2C, 3H | 0 |
| Morningglory | 6B, 8G | 10C |
| Cocklebur | 3C, 9G | 1C, 3G |
| Cassia | 2C | 0 |
| Nutsedge | 1C, 9G | 0 |
| Crabgrass | 2C,5G | 0 |
| Barnyardgrass | 8H | 0 |
| Wild Oats | 1H | 0 |
| Wheat | 1G | 0 |
| Corn | 2C, 8H | 1C, 6G |
| Soybean | 9C | 2C, 8G |
| Rice | 7G | 0 |
| Sorghum | 9G | 2G |
| PRE-EMERGENCE | | |
| Morningglory | 8G | 9G |
| Cocklebur | 8H | 9G |
| Cassia | 2C, 8G | 1C |
| Nutsedge | 8G | 0 |
| Crabgrass | 2C, 5G | 0 |
| Barnyardgrass | 2C, 8H | 1C |
| Wild Oats | 2C, 6G | 0 |
| Wheat | 1C, 3G | 0 |
| Corn | 2C, 9H | 1C, 5G |
| Soybean | 9H | 2H |
| Rice | 5C, 9H | 1C, 4G |
| Sorghum | 9G | 3C, 8H |

TABLE A (Continued)

| | |
|---|---|
| | 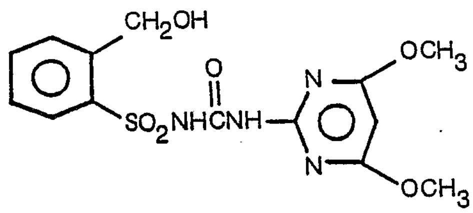 |
| Rate kg/ha | 0.05 |
| POST-EMERGENCE | |
| Bushbean | 5C, 10D, 6Y |
| Cotton | 3C, 4H, 8G |
| Morningglory | 2G |
| Cocklebur | 5C, 9G |
| Cassia | 5C, 9G |
| Nutsedge | 1C, 9G |
| Crabgrass | 3G |
| Barnyardgrass | 3C, 9H |
| Wild Oats | 1C, 3G |
| Wheat | 0 |
| Corn | 2C, 9H |
| Soybean | 9C |
| Rice | 2C, 9G |
| Sorghum | 2C, 9H |
| PRE-EMERGENCE | |
| Morningglory | 8G |
| Cocklebur | 9H |
| Cassia | 1C, 8G |
| Nutsedge | 10E |
| Crabgrass | 2C |
| Barnyardgrass | 2C, 9H |
| Wild Oats | 8G, O |
| Wheat | 6G, O |
| Corn | 1C, 9H |
| Soybean | 2C, 8H |
| Rice | 10E |
| Sorghum | 2C, 9G |

TABLE A (Continued)

| | 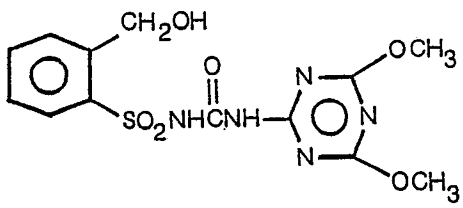 |
|---|---|
| Rate kg/ha | 0.05 |
| POST-EMERGENCE | |
| Bushbean | 2C, 7G, 6Y |
| Cotton | 0 |
| Morningglory | 1C |
| Cocklebur | 0 |
| Cassia | 1C |
| Nutsedge | 0 |
| Crabgrass | 2G |
| Barnyardgrass | 0 |
| Wild Oats | 0 |
| Wheat | 0 |
| Corn | 2C, 9H |
| Soybean | 1C, 6H |
| Rice | 1C, 4G |
| Sorghum | 2C, 9H |
| PRE-EMERGENCE | |
| Morningglory | 2C |
| Cocklebur | 0 |
| Cassia | 1C |
| Nutsedge | 0 |
| Crabgrass | 0 |
| Barnyardgrass | 2C, 7H |
| Wild Oats | 0 |
| Wheat | 0 |
| Corn | 9H |
| Soybean | 2C, 3H |
| Rice | 1C |
| Sorghum | 2C, 9H |

TABLE A (Continued)

| | 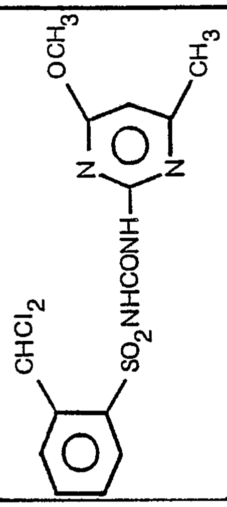 | 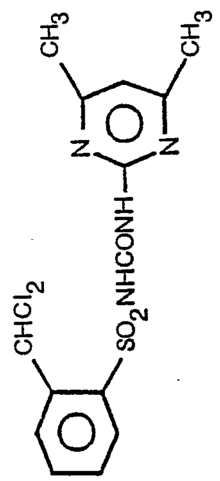 | 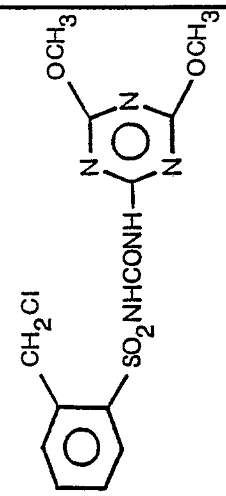 |
|---|---|---|---|
| Rate kg/ha | 0.4 | 0.4 | 0.4 |
| POST-EMERGENCE | | | |
| Bushbean | 9D, 8G, 6Y | 9C | 7C |
| Cotton | 5U, 9D, 9G | 9C | 3C |
| Morningglory | 10C | 10C | 1C |
| Cocklebur | 9C | 9C | 10C |
| Cassia | 9C | 9C | 1C |
| Nutsedge | 2C, 9G | 9C | 0 |
| Crabgrass | 2C, 8G | 2C | 0 |
| Barnyardgrass | 10C | 9C | 0 |
| Wild Oats | 9C | 3C, 8H | 0 |
| Wheat | 2C, 9G | 9C | 0 |
| Corn | 3U, 9C | 9C | 2C, 3H |
| Soybean | 5C, 9G | 9C | 7C |
| Rice | | 6C, 9G | 0 |
| Sorghum | 9C | 10C | 2C |
| PRE-EMERGENCE | | | |
| Morningglory | 9G | 9H | 3G |
| Cocklebur | 9G | 9H | 6G, 5C |
| Cassia | 9G | 4C, 9G | 5G |
| Nutsedge | 9G | 10H | 0 |
| Crabgrass | 8G | 3C, 7G | 0 |
| Barnyardgrass | 5C, 9H | 3C, 9H | 3G |
| Wild Oats | 3C, 9G | 2C, 9G | 0 |
| Wheat | 9G | 2C, 9G | 0 |
| Corn | 9G | 9H | 3C |
| Soybean | 9H | 9H | 2C |
| Rice | 10E | 10E | 0 |
| Sorghum | 5C, 9H | 10E | 4G |

TABLE A (Continued)

| | 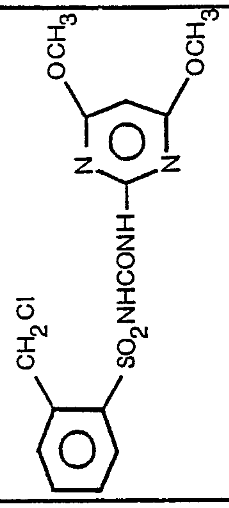 | 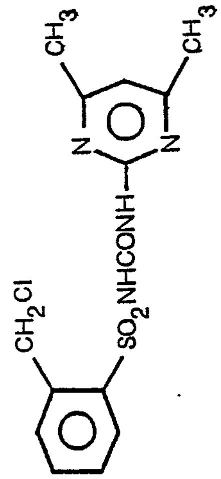 | 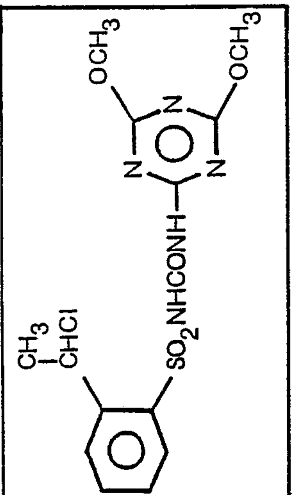 |
|---|---|---|---|
| Rate kg/ha | 0.1 | 0.1 | 0.4 |
| POST-EMERGENCE | | | |
| Bushbean | 5C, 9G, 6Y | 10G, 5H | 8C |
| Cotton | 3C, 3H, 9G | 0 | 8C |
| Morningglory | 1C, 2H | 10G, 7C | 10C |
| Cocklebur | 2C, 9G | 10G | 9C |
| Cassia | 3C, 5H | 6G, 3C | 8C |
| Nutsedge | 2C, 7G | 3G | 3G |
| Crabgrass | 1C, 3G | 4G | 3C |
| Barnyardgrass | 3C, 9H | 0 | 7C |
| Wild Oats | 1C | 0 | 3G |
| Wheat | 1C | 0 | 0 |
| Corn | 2C, 9H | 7G, 5H | 10C |
| Soybean | 9C | 10G, 9C | 8C |
| Rice | 1C, 8G | 8G, 3C | 7C |
| Sorghum | 1C, 9H | 10C | 7C |
| PRE-EMERGENCE | | | |
| Morningglory | 8G | 9C | 8C |
| Cocklebur | 8H | 6G | 8C |
| Cassia | 8G | 5G | 5G |
| Nutsedge | 10E | 8G | 3G |
| Crabgrass | 5G | 4G | 3C |
| Barnyardgrass | 2C, 8H | 5C | 8C |
| Wild Oats | 7G | 5G | 6C |
| Wheat | 4G | 5G | 6G |
| Corn | 8H | 5G, 2C | 9C |
| Soybean | 5H | 6G, 3C | 9C |
| Rice | 10E | 7G, 4C | 10C |
| Sorghum | 1C, 9G | 5G, 3H | 9C |

TABLE A (Continued)

| | 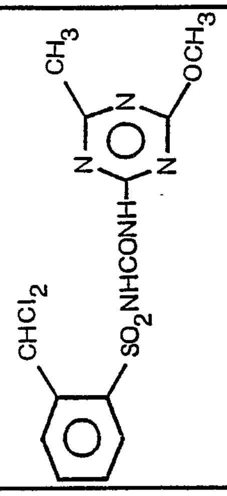 | 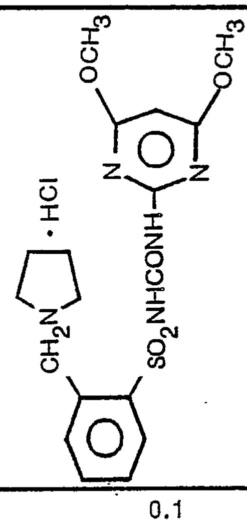 |
|---|---|---|
| Rate-kg/ha | 0.4 | 0.1 |
| POST-EMERGENCE | | |
| Bushbean | 7C, 9G | 3C, 8G, 6Y |
| Cotton | 9C | 9C, 5G |
| Morningglory | 9C | 6C, 9G |
| Cocklebur | 9C | 4C, 9G |
| Cassia | 2C, 5G | 2C, 3G |
| Nutsedge | 7C, 9G | 4G |
| Crabgrass | 1C, 4G | 3G |
| Barnyardgrass | 2C | 9H |
| Wild Oats | 0 | 1C |
| Wheat | 0 | 0 |
| Corn | 5U, 9G | 3C, 9H |
| Soybean | 9G | 2C, 9G |
| Rice | 0 | 9C |
| Sorghum | 1C, 7H | 3C, 9G |
| PRE-EMERGENCE | | |
| Morningglory | 9G | 9G |
| Cocklebur | 9H | 2C |
| Cassia | 9G | 3C |
| Nutsedge | 9G | 3G |
| Crabgrass | 2C, 7G | 1C |
| Barnyardgrass | 2C, 9H | 1C |
| Wild Oats | 2C, 6G | 0 |
| Wheat | 3G | 0 |
| Corn | 9H | 2C, 6G |
| Soybean | 9H | 2C, 2H |
| Rice | 9H | 2C, 8G |
| Sorghum | 1C, 9H | 3C, 8H |

TABLE A (Continued)

| | 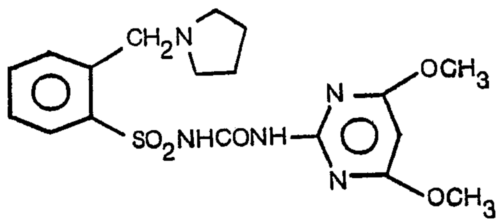 |
|---|---|
| Rate kg/ha | 0.1 |
| POST-EMERGENCE | |
| Bushbean | 8D, 9G, 6Y |
| Cotton | 5C, 9G |
| Morningglory | 6C, 9G |
| Cocklebur | 4C, 9G |
| Cassia | 2C, 7G |
| Nutsedge | 5G |
| Crabgrass | 1C |
| Barnyardgrass | 2C, 9H |
| Wild Oats | 2C |
| Wheat | 1C |
| Corn | 3C, 9H |
| Soybean | 2C, 9G |
| Rice | 3C, 9G |
| Sorghum | 2C, 9G |
| PRE-EMERGENCE | |
| Morningglory | 2C, 9G |
| Cocklebur | 2C, 5H |
| Cassia | 2C, 3H |
| Nutsedge | 4G |
| Crabgrass | 1C |
| Barnyardgrass | 1C, 3G |
| Wild Oats | 0 |
| Wheat | 0 |
| Corn | 3C, 8G |
| Soybean | 2C, 7H |
| Rice | 3C, 7G |
| Sorghum | 3C, 9G |

Test B

Two plastic bulb pans were filled with fertilized and limed Fallsington silt loam soil. One pan was planted with corn, sorghum, Kentucky bluegrass and several grassy weeds. The other pan was planted with cotton, soybeans, purple nutsedge (*Cyperus rotundus*), and several broadleaf weeds. The following grassy and broadleaf weeds were planted: crabgrass (*Digitaria sanguinalis*), barnyardgrass (*Echinochloa crusgalli*), wild oats (*Avena fatua*), johnsongrass (*Sorghum halepense*), dallisgrass (*Paspalum dilatatum*), giant foxtail (*Setaria faberii*), cheatgrass (*Bromus secalinus*), mustard (*Brassica arvensis*), cocklebur (*Xanthium pensylvanicum*), pigweed (*Amaranthus retroflexus*), morningglory (*Ipomoea hederacea*), cassia (*Cassia tora*), teaweed (*Sida spinosa*), velvetleaf (*Abutilon theophrasti*), and jimsonweed (*Datura stramonium*). A 12.5 cm diameter plastic pot was also filled with prepared soil and planted with rice and wheat. Another 12.5 cm pot was planted with sugarbeets. The above four containers were treated pre-emergence with several test compounds within the scope of the invention.

Twenty-eight days after treatment, the plants were evaluated and visually rated for response to the chemical treatments utilizing the rating system described previously for Test A. The data are summarized in Table B.

TABLE B

| PRE-EMERGENCE ON FALLSINGTON SILT LOAM SOIL | | |
|---|---|---|
| | 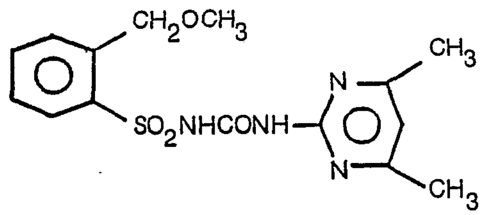 | |
| Rate, kg/ha | 0.03 | 0.12 |
| | | |
| Crabgrass | 4G | 7G, 3C |
| Barnyardgrass | 8G, 5H | 10C |
| Sorghum | 9G, 9C | 10E |
| Wild Oats | 7G, 5H | 9G, 9C |
| Johnsongrass | 8G, 4C | 9G, 9C |
| Dallisgrass | 3G | 7G, 5C |
| Giant Foxtail | 4G | 5G |
| Ky. bluegrass | 7G, 3C | 10C |
| Cheatgrass | 8G | 10E |
| Sugarbeets | 9G, 9C | 10C |
| Corn | 5G, 2H | 9G, 9C |
| Mustard | 10C | 10C |
| Cocklebur | 5G | 7G |
| Pigweed | 9G, 9C | 10E |
| Nutsedge | 5C | 5E, 9G |
| Cotton | 4G, 3H | 8G |
| Morningglory | 3G | 7G |
| Cassia | 7G | 9G, 8C |
| Teaweed | 5G | 7G, 3C |
| Velvetleaf | 8G, 5H | 9G, 8C |
| Jimsonweed | 6G | 7G |
| Soybean | 3G | 6G, 5H |
| Rice | 10F | 10E |
| Wheat | 5G, 3C | 7G, 7C |
| | | |
| | | |
| | | |

TABLE B (Continued)

| PRE-EMERGENCE ON FALLSINGTON SILT LOAM SOIL | | | | |
|---|---|---|---|---|
| | 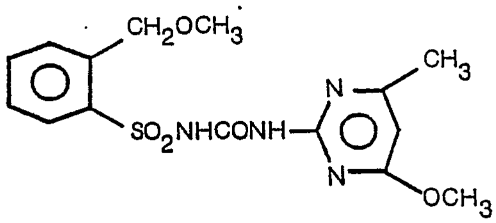 | | | |
| Rate, kg/ha | 0.01 | 0.015 | 0.03 | 0.12 |
| | | | | |
| Crabgrass | 0 | 3G | 5G | 7G, 3C |
| Barnyardgrass | 5G | 7G, 3H | 9G, 8C | 9G, 9C |
| Sorghum | 2G | 6G, 3H | 10C | 10C |
| Wild Oats | 0 | 5G | 5G, 3C | 9G, 9C |
| Johnsongrass | 3G | 6G, 3H | 8G, 5H | 9G, 5C |
| Dallisgrass | 2G | 2G | 3G | 8G, 3C |
| Giant Foxtail | 3G | 9E | 6G | 9G, 9C |
| Ky. bluegrass | 4G | 7G, 5E | 7G, 5C | 10C |
| Cheatgrass | – | 5G | 7G | 10E |
| Sugarbeets | 5G | 10C | 10C | 10C |
| Corn | 0 | 5G | 7G, 5H | 10C |
| Mustard | 8G, 7C | 9G, 8C | 10C | 10C |
| Cocklebur | 0 | 0 | 3G | 8G |
| Pigweed | 0 | 3G | 10E | 10E |
| Nutsedge | 0 | 0 | 5G | 8G |
| Cotton | 0 | 0 | 7G, 3H | 8G, 4C |
| Morningglory | 3G | 3G | 6G | 9G |
| Cassia | 0 | 6G | 9G, 6C | 9G, 8C |
| Teaweed | 0 | 3G | 5G | 7G, 5C |
| Velvetleaf | 0 | 2G | 9G | 9G, 8C |
| Jimsonweed | 0 | 4G | 8G, 8C | 9G, 8C |
| Soybean | 0 | 3G | 7G, 5H | 9G, 8C |
| Rice | 5G | 7G, 3H | 8G, 7C | 10E |
| Wheat | 0 | 0 | 5G, 3C | 6G, 3C |
| | | | | |
| | | | | |
| | | | | |

TABLE B (Continued)

| PRE-EMERGENCE ON FALLSINGTON SILT LOAM SOIL | | |
|---|---|---|
| | 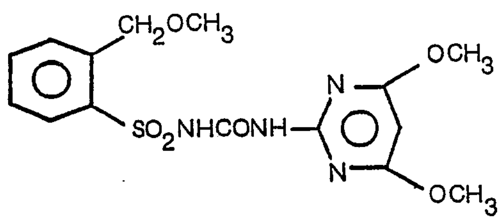 | |
| Rate, kg/ha | 0.03 | 0.12 |
| | | |
| Crabgrass | 2G | 5G |
| Barnyardgrass | 7G, 5H | 10C |
| Sorghum | 8G, 5H | 10C |
| Wild Oats | 5G, 3H | 6G, 4C |
| Johnsongrass | 6G | 7G, 5H |
| Dallisgrass | 0 | 5G |
| Giant Foxtail | 0 | 7G, 7C |
| Ky. bluegrass | 8G, 8C | 10C |
| Cheatgrass | 5G | 7G, 3C |
| Sugarbeets | 9G, 9C | 10C |
| Corn | 3H | 9G, 9C |
| Mustard | 10C | 10C |
| Cocklebur | 4G | 7G |
| Pigweed | 10E | 10E |
| Nutsedge | 7G | 8G, 4C |
| Cotton | 6G | 7G, 5H |
| Morningglory | 3G | 9G, 3C |
| Cassia | 8G | 9G, 8C |
| Teaweed | 4G | 7G, 3C |
| Velvetleaf | 8C, 3H | 9G, 9C |
| Jimsonweed | 8G, 8C | 9G, 8C |
| Soybean | 4C, 3H | 7G, 5H |
| Rice | 8G, 5C | 10C |
| Wheat | 3G | 3G |
| | | |
| | | |
| | | |

TABLE B (Continued)

| PRE-EMERGENCE ON FALLSINGTON SILT LOAM SOIL | | |
|---|---|---|
| | 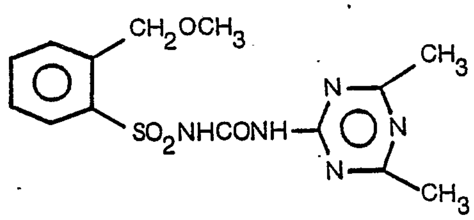 | |
| Rate, kg/ha | 0.03 | 0.12 |
| | | |
| Crabgrass | 0 | 5G |
| Barnyardgrass | 3G | 5G, 3H |
| Sorghum | 8G, 3H | 9G, 8C |
| Wild Oats | 3G | 7G, 4C |
| Johnsongrass | 6G | 8G, 5H |
| Dallisgrass | 0 | 0 |
| Giant Foxtail | 0 | 2G |
| Ky. bluegrass | 4G, 3C | 8G, 8C |
| Cheatgrass | 0 | 5G |
| Sugarbeets | 9G, 9C | 10C |
| Corn | 3H | 7G, 3H |
| Mustard | 10C | 10C |
| Cocklebur | 4G | 6G, 3H |
| Pigweed | 9G, 8C | 9G, 8C |
| Nutsedge | 0 | 5G |
| Cotton | 3C | 7G, 5H |
| Morningglory | 3G | 6G |
| Cassia | 3G | 8G |
| Teaweed | 3G | 7G, 5H |
| Velvetleaf | 7G | 9G, 5C |
| Jimsonweed | 6G | 9G, 9C |
| Soybean | 4G | 7G, 5H |
| Rice | 7G, 3C | 10E |
| Wheat | 0 | 3G |
| | | |
| | | |
| | | |

TABLE B (Continued)

| PRE-EMERGENCE ON FALLSINGTON SILT LOAM SOIL | | |
|---|---|---|
| | 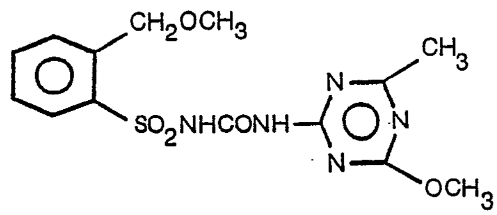 | |
| Rate, kg/ha | 0.03 | 0.12 |
| | | |
| Crabgrass | 5G | 6G |
| Barnyardgrass | 4G | 7G, 5H |
| Sorghum | 9G, 9C | 10C |
| Wild Oats | 6G, 2C | 6G, 4C |
| Johnsongrass | 6G | 7G, 3H |
| Dallisgrass | 0 | 5G |
| Giant Foxtail | 0 | 6G |
| Ky. bluegrass | 7G, 6C | 8G, 9C |
| Cheatgrass | 0 | 6G, 3C |
| Sugarbeets | 10C | 10C |
| Corn | 7G, 5H | 8G, 9C |
| Mustard | 10C | 10C |
| Cocklebur | 6G | 7G, 5H |
| Pigweed | 10E | 10E |
| Nutsedge | 5G | 7G |
| Cotton | 8G, 5H | 9G, 3C |
| Morningglory | 8G | 9G, 8C |
| Cassia | 9G, 3C | 9G, 8C |
| Teaweed | 7G, 3C | 8G, 5C |
| Velvetleaf | 9G, 5C | 9G, 5C |
| Jimsonweed | 9G, 9C | 10C |
| Soybean | 8G, 5H | 9G, 8C |
| Rice | 6G, 3C | 7G, 6C |
| Wheat | 0 | 3G |
| | | |
| | | |
| | | |

TABLE B (Continued)

| PRE-EMERGENCE ON FALLSINGTON SILT LOAM SOIL | | |
|---|---|---|
| | 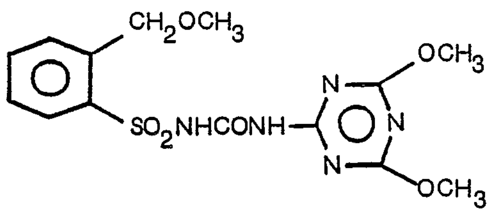 | |
| Rate, kg/ha | 0.03 | 0.12 |
| | | |
| Crabgrass | 0 | 0 |
| Barnyardgrass | 2G | 5G |
| Sorghum | 6G, 3H | 8G, 5H |
| Wild Oats | 0 | 4G |
| Johnsongrass | 0 | 6G, 3H |
| Dallisgrass | 0 | 3G |
| Giant Foxtail | 0 | 0 |
| Ky. bluegrass | 3G | 6G |
| Cheatgrass | 0 | 0 |
| Sugarbeets | 8G, 9C | 10C |
| Corn | 5G | 6G, 3H |
| Mustard | 9G, 9C | 10C |
| Cocklebur | 7G | 6G, 3H |
| Pigweed | 8G | 10C |
| Nutsedge | 0 | 3G |
| Cotton | 7G, 3H | 8G |
| Morningglory | 8G, 5H | 9G, 3C |
| Cassia | 9G, 4C | 9G, 6C |
| Teaweed | 6G, 3C | 7G, 3C |
| Velvetleaf | 8G, 5H | 9G, 7C |
| Jimsonweed | 7G, 4C | 9G, 7C |
| Soybean | 7G, 5H | 8G, 5H |
| Rice | 6G | 7G |
| Wheat | 0 | 0 |
| | | |
| | | |
| | | |

TABLE B (Continued)

| PRE-EMERGENCE ON FALLSINGTON SILT LOAM SOIL | | |
|---|---|---|
| | 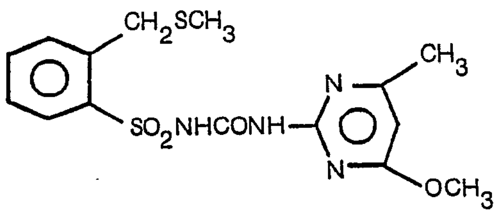 | |
| Rate, kg/ha | 0.03 | 0.12 |
| | | |
| Crabgrass | 0 | 3G |
| Barnyardgrass | 4G | 7G |
| Sorghum | 6G, 3H | 10C |
| Wild Oats | 2G | 5G, 5C |
| Johnsongrass | 5G | 8G, 5H |
| Dallisgrass | 0 | 4G |
| Giant Foxtail | 5G | 7G |
| Ky. bluegrass | 5G | 7G, 5C |
| Cheatgrass | 10E | 8G, 9C |
| Sugarbeets | 6G, 5C | 9G, 9C |
| Corn | 0 | 4G |
| Mustard | 9G, 8C | 9G, 8C |
| Cocklebur | 3G | 5G |
| Pigweed | 6G | 8G |
| Nutsedge | 5G | 7G |
| Cotton | 3G | 4G |
| Morningglory | 2C | 8G, 5H |
| Cassia | 3G | 5G, 3H |
| Teaweed | 3G | 7G |
| Velvetleaf | – | 5G, 3H |
| Jimsonweed | 3G | 8G, 5H |
| Soybean | 4G, 3H | 5G, 5H |
| Rice | 6G | 7G, 3H |
| Wheat | 2G | 4G |
| | | |
| | | |
| | | |

TABLE B (Continued)

| PRE-EMERGENCE ON FALLSINGTON SILT LOAM SOIL | | |
|---|---|---|
| | 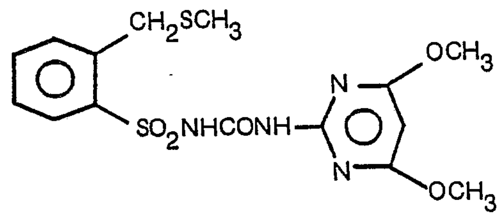 | |
| Rate, kg/ha | 0.03 | 0.12 |
| | | |
| Crabgrass | 2G | 6G, 3H |
| Barnyardgrass | 8G, 5H | 9G, 9C |
| Sorghum | 10C | 10E |
| Wild Oats | 5G | 5G, 5C |
| Johnsongrass | 9G, 9C | 10C |
| Dallisgrass | 3G | 8G, 8C |
| Giant Foxtail | 8G, 5H | 10C |
| Ky. bluegrass | 6G, 3C | 10C |
| Cheatgrass | 7C | 10E |
| Sugarbeets | 8G, 8C | 10C |
| Corn | 6G, 3H | 8G, 8C |
| Mustard | 10C | 10C |
| Cocklebur | 4G | 7G, 7C |
| Pigweed | 8G, 5C | 10C |
| Nutsedge | 7G | 10C |
| Cotton | 6G, 3H | 8G, 5H |
| Morningglory | 6G, 3H | 8G, 5H |
| Cassia | 7G, 5H | 8G, 7C |
| Teaweed | 4G | 8G, 5H |
| Velvetleaf | 8G, 5H | 8G, 5H |
| Jimsonweed | 8G, 5H | 9G, 6C |
| Soybean | 5G, 5H | 7G, 5H |
| Rice | 10E | 10E |
| Wheat | 3G | 5G |
| | | |
| | | |
| | | |

TABLE B (Continued)

PRE-EMERGENCE ON FALLSINGTON SILT LOAM SOIL

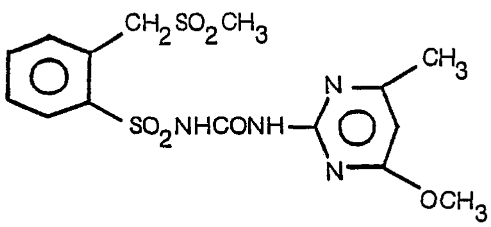

| Rate, kg/ha | 0.03 | 0.12 |
|---|---|---|
| | | |
| Crabgrass | 0 | 0 |
| Barnyardgrass | 4G | 7G, 8C |
| Sorghum | 10C | 9G, 9C |
| Wild Oats | 3G | 5G |
| Johnsongrass | 6G, 3H | 7G, 3H |
| Dallisgrass | – | – |
| Giant Foxtail | 5G | 7G, 3H |
| Ky. bluegrass | 6G | 7G, 5C |
| Cheatgrass | – | – |
| Sugarbeets | 9G, 9C | 10C |
| Corn | 3G | 9G, 8C |
| Mustard | 9G, 9C | 10C |
| Cocklebur | 6G, 2C | 6G, 5H |
| Pigweed | 0 | 6G |
| Nutsedge | 4G | 6G |
| Cotton | 4G, 5H | 6G, 5H |
| Morningglory | 4G, 3H | 8G, 5H |
| Cassia | 2C | 5G, 3C |
| Teaweed | 3G, 2C | 5G, 3H |
| Velvetleaf | 2C | 8G, 5H |
| Jimsonweed | 5G, 5H | 7G, 5C |
| Soybean | 4G, 5H | 6G, 5H |
| Rice | 6G | 8G, 5H |
| Wheat | 2G | 3G |

TABLE B (Continued)

| PRE-EMERGENCE ON FALLSINGTON SILT LOAM SOIL | | |
|---|---|---|
| | 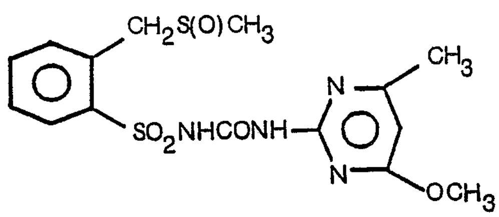 | |
| Rate, kg/ha | 0.03 | 0.12 |
| | | |
| Crabgrass | 0 | 3G |
| Barnyardgrass | 4G | 7G, 8C |
| Sorghum | 9G, 9C | 10C |
| Wild Oats | 3G | 7G, 3H |
| Johnsongrass | 4G, 2H | 9G, 8C |
| Dallisgrass | – | – |
| Giant Foxtail | 3G | 6G, 3H |
| Ky. bluegrass | 5G | 7G, 5C |
| Cheatgrass | – | – |
| Sugarbeets | 7G, 8C | 10C |
| Corn | 0 | 4G, 3H |
| Mustard | 9G, 9C | 10C |
| Cocklebur | 4G | 7G, 3H |
| Pigweed | 3G | 8G, 8C |
| Nutsedge | 4G | 9G, 8E |
| Cotton | 3G | 5G, 3H |
| Morningglory | 4G, 3H | 7G, 5H |
| Cassia | 2G | 6G, 3C |
| Teaweed | 2G | 3G, 2C |
| Velvetleaf | 4G | 6G, 5H |
| Jimsonweed | 3G | 8G, 5H |
| Soybean | 3G | 5G, 3H |
| Rice | 6G | 8G, 5C |
| Wheat | 2G | 4G |
| | | |
| | | |
| | | |

TABLE B (Continued)

| PRE-EMERGENCE ON FALLSINGTON SILT LOAM SOIL | | |
|---|---|---|
| | 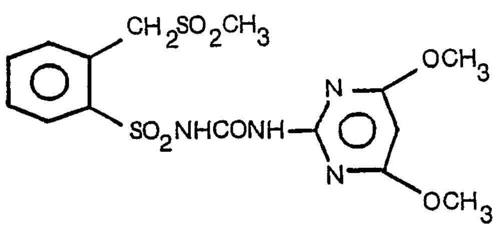 | |
| Rate, kg/ha | 0.03 | 0.12 |
| | | |
| Crabgrass | 3G | 3G |
| Barnyardgrass | 8G, 8C | 10C |
| Sorghum | 10C | 10C |
| Wild Oats | 2G | 4G |
| Johnsongrass | 7G, 3H | 9G, 9C |
| Dallisgrass | – | – |
| Giant Foxtail | 5G | 8G, 3H |
| Ky. bluegrass | 6G | 7G, 3C |
| Cheatgrass | – | – |
| Sugarbeets | 10C | 10C |
| Corn | 7G, 5H | 9G, 9C |
| Mustard | 10C | 10C |
| Cocklebur | 3G | 7G, 5H |
| Pigweed | 8G, 8C | 9G, 8C |
| Nutsedge | 10E | 10E |
| Cotton | 6G, 5H | 6G, 5H |
| Morningglory | 4G, 3H | 8G, 5H |
| Cassia | 7G, 5C | 8G, 6C |
| Teaweed | 3G, 2C | 5G, 3H |
| Velvetleaf | 7G, 5H | 8G, 5H |
| Jimsonweed | 8G, 7C | 8G, 5C |
| Soybean | 6G, 5H | 7G, 5H |
| Rice | 7G | 8G, 5C |
| Wheat | 0 | 2G |
| | | |
| | | |
| | | |

TABLE B (Continued)

| PRE-EMERGENCE ON FALLSINGTON SILT LOAM SOIL | | |
|---|---|---|
| | 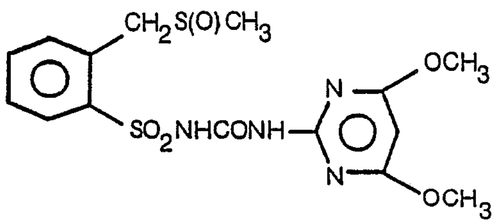 | |
| Rate, kg/ha | 0.03 | 0.12 |
| | | |
| Crabgrass | 3G | 9G, 8C |
| Barnyardgrass | 7G, 8C | 10C |
| Sorghum | 10C | 10C |
| Wild Oats | 5G | 8G, 8C |
| Johnsongrass | 9G, 8C | 10C |
| Dallisgrass | – | – |
| Giant Foxtail | 7G, 3H | 10C |
| Ky. bluegrass | 7G, 5C | 10C |
| Cheatgrass | – | – |
| Sugarbeets | 10C | 10C |
| Corn | 6G, 5H | 10C |
| Mustard | 9G, 9C | 10C |
| Cocklebur | 4G | 8G, 5H |
| Pigweed | 8G, 7C | 10C |
| Nutsedge | 6G | 10E |
| Cotton | 4G | 8G, 5H |
| Morningglory | 6G, 3H | 8G, 5H |
| Cassia | 7G, 5C | 9G, 8C |
| Teaweed | 0 | 6G, 5H |
| Velvetleaf | 6G, 5H | 8G, 3H |
| Jimsonweed | 5G | 8G, 7C |
| Soybean | 7G, 5H | 8G, 5H |
| Rice | 8G, 7C | 10C |
| Wheat | 3G | 5G |
| | | |
| | | |
| | | |

TABLE B (Continued)

| PRE-EMERGENCE ON FALLSINGTON SILT LOAM SOIL | |
|---|---|
| | 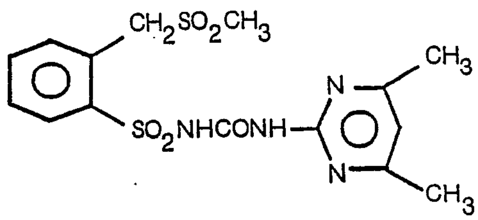 |
| Rate, kg/ha | 0.25 |
| | |
| Crabgrass | 0 |
| Barnyardgrass | 0 |
| Sorghum | 7G, 5H |
| Wild Oats | 4G |
| Johnsongrass | 3G |
| Dallisgrass | 0 |
| Giant Foxtail | 0 |
| Ky. bluegrass | 0 |
| Cheatgrass | 4G |
| Sugarbeets | 10C |
| Corn | 0 |
| Mustard | 9G, 9C |
| Cocklebur | 0 |
| Pigweed | 0 |
| Nutsedge | 0 |
| Cotton | 4G, 3H |
| Morningglory | 4G, 2H |
| Cassia | 3G |
| Teaweed | 0 |
| Velvetleaf | 6G |
| Jimsonweed | 0 |
| Soybean | 0 |
| Rice | 6G |
| Wheat | 0 |
| | |
| | |
| | |

TABLE B (Continued)

| PRE-EMERGENCE ON FALLSINGTON SILT LOAM | | | | |
|---|---|---|---|---|
| | COMPOUND 7 | | COMPOUND 8 | COMPOUND 9 |
| Rate, kg/ha | 0.06 | 0.25 | 0.25 | 0.12 |
| | | | | |
| Crabgrass | 0 | 0 | 0 | 0 |
| Barnyardgrass | 2G | 3G | 0 | 3G |
| Sorghum | 7G, 5H | 7G, 5H | 2G | 0 |
| Wild Oats | 0 | 0 | 0 | 0 |
| Johnsongrass | 0 | 0 | 0 | 0 |
| Dallisgrass | 0 | 0 | 0 | 0 |
| Giant Foxtail | 0 | 3G | 0 | 0 |
| Ky. bluegrass | 0 | 4G | 0 | 0 |
| Cheatgrass | 0 | 3G | 0 | 0 |
| Sugarbeets | 0 | 7G | 0 | 3G |
| Corn | 0 | 0 | 0 | 0 |
| Mustard | 7G, 3C | 8G, 6C | 5G | 4G |
| Cocklebur | 5G | 7G, 3H | 0 | 0 |
| Pigweed | 0 | 8G, 8C | 3G | 3G |
| Nutsedge | 4G | 8G | 0 | 0 |
| Cotton | 4G | 5G, 3H | 0 | 0 |
| Morningglory | 3G | 6G | 0 | 0 |
| Cassia | 0 | 5G | 3G | 4G |
| Teaweed | 0 | 0 | 3G | 0 |
| Velvetleaf | 3G | 6G, 5H | 3G | 0 |
| Jimsonweed | 0 | 4G | 0 | 0 |
| Soybean | 0 | 0 | 2G | 0 |
| Rice | 4G | 5G | 0 | 0 |
| Wheat | 0 | 0 | 0 | 0 |
| | | | | |
| | | | | |
| | | | | |

TABLE B (Continued)

| PRE-EMERGENCE ON FALLSINGTON SILT LOAM | | | | |
|---|---|---|---|---|
| | COMPOUND 10 | | COMPOUND 14 | |
| Rate, kg/ha | 0.03 | 0.12 | 0.06 | 0.25 |
| | | | | |
| Crabgrass | 0 | 2G | 0 | 3G |
| Barnyardgrass | 2G | 5G | 3G | 5G, 3C |
| Sorghum | 3G, 2H | 9G, 5H | 8G, 5H | 9G, 5H |
| Wild Oats | 2G | 4G | 4G | 7G |
| Johnsongrass | 0 | 5G, 3H | 5G, 3H | 5G, 3H |
| Dallisgrass | 0 | 3G | 3G | 0 |
| Giant Foxtail | 0 | 3G | 3G, 3H | 7G, 3H |
| Ky. bluegrass | 0 | 2G | 4G | 7G, 3H |
| Cheatgrass | 3G | 4G | 5G, 3C | 7G, 3C |
| Sugarbeets | 5G | 7G, 3H | 8G, 9C | 10C |
| Corn | 0 | 2G | 3G, 2C | 6G, 2H |
| Mustard | 8G, 8C | 8G, 8C | 9G, 9C | 10C |
| Cocklebur | 0 | 3G | 6G, 3H | 9G, 9C |
| Pigweed | 7G | 8G | 5G, 3C | 10C |
| Nutsedge | 0 | 0 | 7G | 8G |
| Cotton | 3G | 5G, 3H | 3H | 5H |
| Morningglory | 3G | 6G | 6G, 5H | 6G, 5H |
| Cassia | 4G | 4G | 4G | 7G |
| Teaweed | 0 | 3G | 2G | 5G |
| Velvetleaf | 4G, 3H | 6G, 5H | 5G, 3H | 7G, 8C |
| Jimsonweed | 0 | 5G, 5C | 6G, 4C | 7G, 7C |
| Soybean | 2G | 0 | 2G | 5G, 3H |
| Rice | 4G | 6G | 7G, 3H | 8G, 5H |
| Wheat | 0 | 0 | 3G | 5G |
| | | | | |
| | | | | |
| | | | | |

TABLE B (Continued)

| PRE-EMERGENCE ON FALLSINGTON SILT LOAM | | | | |
|---|---|---|---|---|
| | 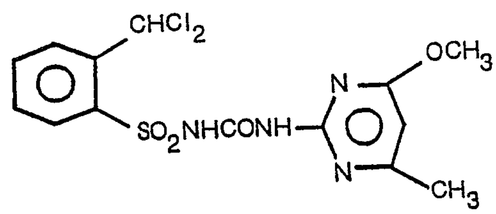 | | | |
| Rate, kg/ha | 0.015 | 0.03 | 0.06 | 0.25 |
| | | | | |
| Crabgrass | 0 | 0 | 6G | 8G, 6C |
| Barnyardgrass | 0 | 0 | 9G, 9C | 10C |
| Sorghum | 5G | 7G, 3H | 10C | 10E |
| Wild Oats | 0 | 0 | 7G, 5C | 10C |
| Johnsongrass | 0 | 4G, 3H | 9G, 9C | 10C |
| Dallisgrass | 0 | 2G | 8G, 5C | 10C |
| Giant Foxtail | 0 | 4G | 10C | 10C |
| Ky. bluegrass | 5G | 6G, 3C | 10C | 10C |
| Cheatgrass | 4G | 4G | 10E | 10E |
| Sugarbeets | 0 | 5G | 10C | 10C |
| Corn | 0 | 0 | 10C | 10C |
| Mustard | 9G, 8C | 9G, 8C | 10C | 10C |
| Cocklebur | 6G, 3H | 6G, 3H | 8G, 5H | 8G, 8C |
| Pigweed | – | – | – | – |
| Nutsedge | 0 | 0 | 9G | 10E |
| Cotton | 0 | 2G | 9G | 10C |
| Morningglory | 2G | 5G | 10C | 10C |
| Cassia | 0 | 5G | 10C | 10C |
| Teaweed | 0 | 0 | 10C | 10C |
| Velvetleaf | 0 | 0 | 10C | 10C |
| Jimsonweed | 0 | 0 | 6G | 10C |
| Soybean | 0 | 0 | 9G, 9C | 9G, 8C |
| Rice | 5G | 6G | 10E | 10E |
| Wheat | 2G | 4G | 8G, 8C | 10C |
| | | | | |
| | | | | |
| | | | | |

TABLE B (Continued)

| PRE-EMERGENCE ON FALLSINGTON SILT LOAM | | | | |
|---|---|---|---|---|
| | 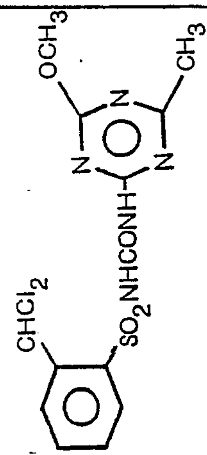 | | 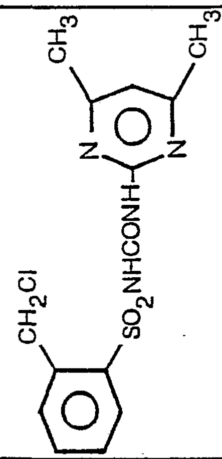 | |
| Rate, kg/ha | 0.125 | 0.5 | 0.03 | 0.25 |
| | | | | |
| Crabgrass | 0 | 3G | 0 | 0 |
| Barnyardgrass | 5G, 3C | 7G, 3C | 0 | 3G |
| Sorghum | 8G, 5H | 10C | 4G | 7G, 5H |
| Wild Oats | 0 | 0 | 0 | 0 |
| Johnsongrass | 5G | 7G | 0 | 3G |
| Dallisgrass | 0 | 3G | 0 | 0 |
| Giant Foxtail | 5G | 10E | 0 | 0 |
| Ky. bluegrass | 6G, 3C | 8G, 8C | 0 | 0 |
| Cheatgrass | 3G | 7G, 5C | 0 | 5G |
| Sugarbeets | 7G, 5H | 8G, 8C | 0 | 2G |
| Corn | 8G, 5H | 9G, 9C | 0 | 2C |
| Mustard | 10C | 10C | 0 | 8G, 3C |
| Cocklebur | 7G | 9G, 8C | 0 | 2G |
| Pigweed | – | – | 5G | 5G |
| Nutsedge | 3G | 6G | 0 | 7G |
| Cotton | 8G | 9G, 8C | 0 | 0 |
| Morningglory | 8G | 9G, 8C | 0 | 0 |
| Cassia | 8G, 8C | 8G, 8C | 3G | 4G |
| Teaweed | 10C | 10C | 0 | 2G |
| Velvetleaf | 8G, 9C | 10C | 0 | 6G |
| Jimsonweed | 0 | 3G | 0 | 0 |
| Soybean | 8G, 5H | 9G, 5H | 0 | 0 |
| Rice | 0 | 6G, 4C | 4G | 5G |
| Wheat | 0 | 0 | 0 | 3G |
| | | | | |
| | | | | |
| | | | | |

TABLE B (Continued)

| PRE-EMERGENCE ON FALLSINGTON SILT LOAM | | |
|---|---|---|
| | 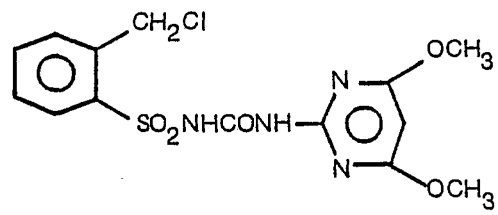 | |
| Rate, kg/ha | 0.03 | 0.125 |
| | | |
| Crabgrass | 0 | 0 |
| Barnyardgrass | 0 | 4G |
| Sorghum | 4G | 7G, 3H |
| Wild Oats | 0 | 0 |
| Johnsongrass | 0 | 7G, 3H |
| Dallisgrass | 6G | 10E |
| Giant Foxtail | 0 | 0 |
| Ky. bluegrass | 0 | 5G |
| Cheatgrass | 0 | 0 |
| Sugarbeets | 0 | 0 |
| Corn | 0 | 3G |
| Mustard | 4G | 10C |
| Cocklebur | 0 | 0 |
| Pigweed | 0 | 5G |
| Nutsedge | 0 | 0 |
| Cotton | 0 | 0 |
| Morningglory | 0 | 0 |
| Cassia | 0 | 3G |
| Teaweed | 0 | 2G |
| Velvetleaf | 0 | 7G, 3H |
| Jimsonweed | 0 | 0 |
| Soybean | 0 | 6G, 3H |
| Rice | 0 | 6G, 3H |
| Wheat | 0 | 0 |
| | | |
| | | |
| | | |

Test C

Twenty-five cm diameter plastic pots filled with Fallsington silt loam were planted with soybeans, cotton, alfalfa, corn, rice, wheat, sorghum, velvetleaf (*Abutilon theophrasti*), sesbania (*Sesbania exaltats*), Cassia (*Cassia tora*), morningglory (*Ipomoea hederacea*), jimsonweed (*Datura stramonium*), cocklebur (*Xanthium pennsylvanicum*), crabgrass (*Digitaria* spp.), nutsedge (*Cyperus rotundus*), barnyardgrass (*Echinochloa crusgalli*), giant foxtail (*Setaria faberii*) and wild oats (*Avena fatua*). Approximately two weeks after planting, the young plants and the soil around them were sprayed overall with the test chemicals dissolved in a non-phytotoxic solvent. Two weeks after treatment, all species were compared to untreated controls and visually rated for response to treatment. The rating system was as described previously for Test A. The data are presented in Table C. Several of the compounds tested by this procedure are useful for the post-emergence control of weeds in wheat.

TABLE C

| | 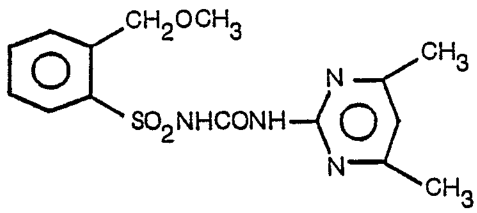 | |
|---|---|---|
| Rate, kg/ha | 0.06 | 0.015 |
| | | |
| Soybeans | 10G,7C | 10G,7C |
| Velvetleaf | 9C | 9C |
| Sesbania | 10C | 10G,9C |
| Cassia | 10G,7C | 10G,4C |
| Cotton | 9G,4C | 7G,3C |
| Morningglory | 10G,5C | 3G |
| Alfalfa | 6G,2C | 2G |
| Jimsonweed | – | 3G,2C |
| Cocklebur | 7C,10G | 4G |
| Corn | 8G,4U | 5G,3H |
| Crabgrass | 3G,1C | 1C |
| Rice | 8G,3C | 8G,2C |
| Nutsedge | 8G,3C | 3G,2C |
| Barnyardgrass | 8G,4C | 8G |
| Wheat | 6G | 1G |
| Giant Foxtail | 3G,1C | 0 |
| Wild Oats | 7G,2C | 5G |
| Sorghum | 8G,4C | 8G,2C |
| | | |
| | | |
| | | |
| | | |
| | | |
| | | |
| | | |
| | | |
| | | |

TABLE C (Continued)

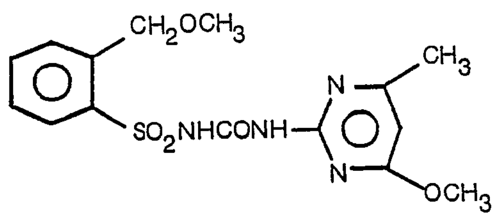

| Rate, kg/ha | 0.06 | 0.015 |
|---|---|---|
| | | |
| Soybeans | 10G, 9C | 10G, 8C |
| Velvetleaf | 10C | 9C |
| Sesbania | 10C | 10C |
| Cassia | 10G, 8C | 10G, 7C |
| Cotton | 9C | 9C |
| Morningglory | 10C | 10C |
| Alfalfa | 9G, 6C | 9G, 6C |
| Jimsonweed | – | – |
| Cocklebur | 8G, 3C | 5G, 3C |
| Corn | 9G, 7C | 8G, 4U |
| Crabgrass | 6G, 1C | 3G |
| Rice | 10G, 5C | 10G, 5C |
| Nutsedge | 9G, 6C | 7G |
| Barnyardgrass | 8G, 4C | 8G, 2C |
| Wheat | 8G, 2C | 6G, 2C |
| Giant Foxtail | 8G | 6G, 1C |
| Wild Oats | 9G, 3C | 8G, 2C |
| Sorghum | 9G, 3C | 9G, 2C |
| | | |
| | | |
| | | |
| | | |
| | | |
| | | |
| | | |
| | | |
| | | |

TABLE C (Continued)

| | 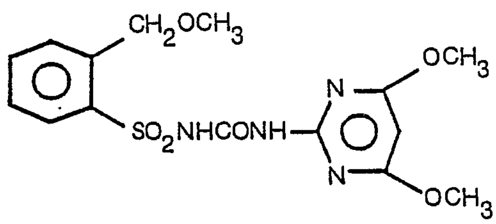 | |
|---|---|---|
| Rate, kg/ha | 0.06 | 0.015 |
| | | |
| Soybeans | 10G, 8C | 10G, 8C |
| Velvetleaf | 10C | 10C |
| Sesbania | 10C | 10C |
| Cassia | 10G, 8C | 10G, 7C |
| Cotton | 10G, 6C | 9G, 6C |
| Morningglory | 10C | 10C |
| Alfalfa | 8C | 10G, 7C |
| Jimsonweed | – | – |
| Cocklebur | 10C | 1G |
| Corn | 7G, 5H | 6G, 5H |
| Crabgrass | 3G | 0 |
| Rice | 9G, 5C | 8G, 1C |
| Nutsedge | 9G, 5C | 8G |
| Barnyardgrass | 3C, 8G | 8G |
| Wheat | 2G, 1C | 1C |
| Giant Foxtail | 7G, 2C | 7G |
| Wild Oats | 0 | 0 |
| Sorghum | 8G, 2C | 4G |

TABLE C (Continued)

| | 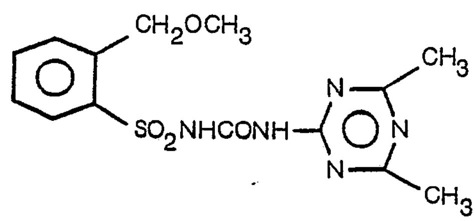 | |
|---|---|---|
| Rate, kg/ha | 0.06 | 0.015 |
| | | |
| Soybeans | 10G, 7C | 10G, 4C |
| Velvetleaf | 10G, 8C | 5G, 1C |
| Sesbania | 10G, 9C | 10G, 6C |
| Cassia | 10G, 7C | 10G, 5C |
| Cotton | 6G, 3C | 5G, 3C |
| Morningglory | 10C | 6G, 2C |
| Alfalfa | 8C | 6G, 3C |
| Jimsonweed | – | – |
| Cocklebur | 10G, 6C | 5G, 3C |
| Corn | 8G, 5C | 8G, 4U |
| Crabgrass | 0 | 0 |
| Rice | 10G, 7C | 9G |
| Nutsedge | 3G, 1C | 2G |
| Barnyardgrass | 3C, 8G | 3C, 5G |
| Wheat | 0 | 0 |
| Giant Foxtail | 5G | 5G |
| Wild Oats | 1C | 0 |
| Sorghum | 8G, 3U | 7G, 3C |

TABLE C (Continued)

| | | |
|---|---|---|
| | 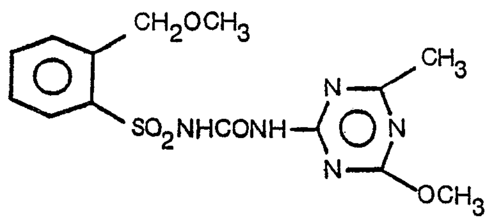 | |
| Rate, kg/ha | 0.06 | 0.015 |
| | | |
| Soybeans | 10G, 8C | 10G, 7C |
| Velvetleaf | 10C | 10C |
| Sesbania | 10C | 10C |
| Cassia | 10C, 8C | 10G, 8C |
| Cotton | 10G, 7C | 10G, 7C |
| Morningglory | 10C | 10C |
| Alfalfa | 10C | 9C |
| Jimsonweed | – | 5G, 2C |
| Cocklebur | 10G, 7C | 10G, 7C |
| Corn | 8G, 5C | 8G, 4U |
| Crabgrass | 1G | 1G |
| Rice | 8G, 3C | 6G |
| Nutsedge | 1G | 2G |
| Barnyardgrass | 7G, 3C | 3G |
| Wheat | 0 | 0 |
| Giant Foxtail | 4G | 4G, 1C |
| Wild Oats | 0 | 0 |
| Sorghum | 8G, 3C | 7G, 3C |

TABLE C (Continued)

| | 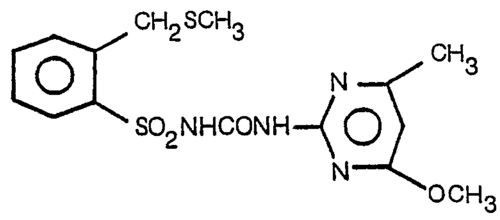 | |
|---|---|---|
| Rate, kg/ha | 0.25 | 0.06 |
| | | |
| Soybeans | 9G, 6C | 7G, 4C |
| Velvetleaf | 9G, 5C | 7G, 3C |
| Sesbania | 4C, 7G | – |
| Cassia | 8G, 4C | 5G, 3C |
| Cotton | 8G, 5C | 7G, 3C |
| Morningglory | 9G, 5C | 7G, 4C |
| Alfalfa | 4C, 9G | 4C, 9G |
| Jimsonweed | – | – |
| Cocklebur | 10G, 5C | 10G, 5C |
| Corn | 8G, 8U | 7G, 7U |
| Crabgrass | 6G, 3C | 5G |
| Rice | 9G, 6C | 8G, 5C |
| Nutsedge | 7G, 1C | 3G |
| Barnyardgrass | 10C | 8G, 5C |
| Wheat | 5G, 2C | 4G |
| Giant Foxtail | – | – |
| Wild Oats | 7G, 4C | 7G, 4C |
| Sorghum | 8G, 5C | 8G, 4C |

TABLE C (Continued)

| | | |
|---|---|---|
| | 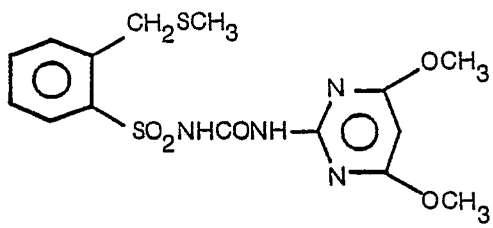 | |
| Rate, kg/ha | 0.25 | 0.06 |
| | | |
| Soybeans | 10G, 7C | 10G, 7C |
| Velvetleaf | 10G, 5C | 7G, 3C |
| Sesbania | – | – |
| Cassia | 5C, 8G | 4C, 7G |
| Cotton | 9G, 5C | 4G, 2C |
| Morningglory | 9G, 6C | 9G, 6C |
| Alfalfa | 9C | 8C |
| Jimsonweed | – | – |
| Cocklebur | 10G, 7C | 10G, 5C |
| Corn | 9G, 9U | 7G, 9U |
| Crabgrass | 8G, 6C | 6G, 3C |
| Rice | 8G, 7C | 9C |
| Nutsedge | 7G, 2C | 5G, 2C |
| Barnyardgrass | 10C | 10C |
| Wheat | 7G, 2C | 5G |
| Giant Foxtail | – | 10C |
| Wild Oats | 8G, 6C | 8G, 4C |
| Sorghum | 9G, 6C | 9G, 4C |

TABLE C (Continued)

| | 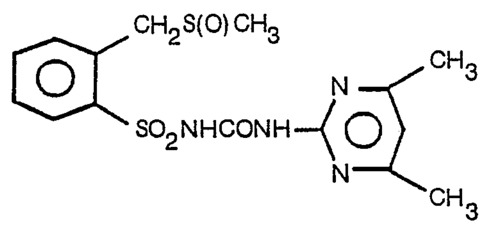 | |
|---|---|---|
| Rate, kg/ha | 0.12 | 0.03 |
| | | |
| Soybeans | 1H | 1H |
| Velvetleaf | 3G, 1C | 5G, 1C |
| Sesbania | 1C | 0 |
| Cassia | 3C | 0 |
| Cotton | 2C | 0 |
| Morningglory | 7G, 3C | 6G, 3C |
| Alfalfa | 3C, 5G | 3C, 5G |
| Jimsonweed | 3G | – |
| Cocklebur | – | 0 |
| Corn | 5G, 3C | 1G,2C |
| Crabgrass | 5G, 1C | 0 |
| Rice | 9G, 3C | 9G, 3C |
| Nutsedge | 4G, 1C | 0 |
| Barnyardgrass | 9G, 3C | 3G |
| Wheat | 5G | 0 |
| Giant Foxtail | 8G, 3C | 5G, 2C |
| Wild Oats | 8C | – |
| Sorghum | 9G, 4C | 9G, 3C |

TABLE C (Continued)

| | 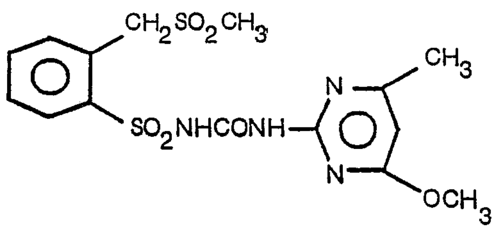 | |
|---|---|---|
| Rate, kg/ha | 0.12 | 0.03 |
| | | |
| Soybeans | 10G, 7C | 10G, 6C |
| Velvetleaf | 7G, 3C | 4G, 2C |
| Sesbania | 10G, 8C | 9G, 3C |
| Cassia | 9G, 5C | 6G, 4C |
| Cotton | 8G, 4C | 7G, 4C |
| Morningglory | 10G, 7C | 9G, 3C |
| Alfalfa | 9C | 8C |
| Jimsonweed | – | 10G, 6C |
| Cocklebur | 10G, 7C | 10G, 6C |
| Corn | 8G, 3C | 5G, 3C |
| Crabgrass | 6G, 1C | 5G, 2C |
| Rice | 8G, 3C | 5G |
| Nutsedge | 8G, 3C | 0 |
| Barnyardgrass | 9G, 2C | 8G |
| Wheat | 2G | 0 |
| Giant Foxtail | 8G, 4C | 8G, 4C |
| Wild Oats | – | 5G, 3C |
| Sorghum | 9G, 4C | 9G, 4C |
| | | |
| | | |
| | | |
| | | |
| | | |
| | | |
| | | |
| | | |
| | | |

TABLE C (Continued)

| | 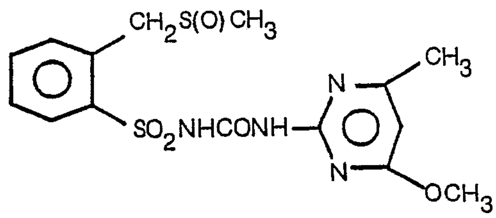 | |
|---|---|---|
| Rate, kg/ha | 0.12 | 0.03 |
| | | |
| Soybeans | 10G, 6C | 9G, 5C |
| Velvetleaf | 8G, 3C | 5G, 3C |
| Sesbania | 8G, 3C | 5G, 3C |
| Cassia | 8G, 6C | 8G, 6C |
| Cotton | 8G, 4C | 6G, 3C |
| Morningglory | 9G, 4C | 9G, 3C |
| Alfalfa | 8C | 7G, 3C |
| Jimsonweed | 8G, 4C | – |
| Cocklebur | 10G, 8C | 9G, 3C |
| Corn | 10C | 8G, 5U |
| Crabgrass | 3C, 7G | 0 |
| Rice | 8C | 8G, 3C |
| Nutsedge | 9G | 3C |
| Barnyardgrass | 10C | 10C |
| Wheat | 7C | 2G |
| Giant Foxtail | 10C | 10C |
| Wild Oats | 10C | 8G, 4C |
| Sorghum | 10C | 9G, 4C |
| | | |
| | | |
| | | |
| | | |
| | | |
| | | |
| | | |
| | | |
| | | |

TABLE C (Continued)

| | |
|---|---|
| | 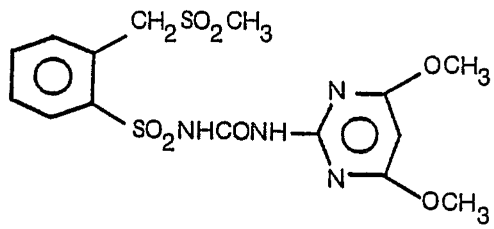 |
| Rate, kg/ha. | 0.03 |
| | |
| Soybeans | 10G, 7C |
| Velvetleaf | 7G, 5C |
| Sesbania | 9G, 6C |
| Cassia | 10G, 7C |
| Cotton | 8G, 3C |
| Morningglory | 9G, 4C |
| Alfalfa | 9C |
| Jimsonweed | 7G, 3C |
| Cocklebur | 10G, 6C |
| Corn | 9G, 2C |
| Crabgrass | 0 |
| Rice | 7G |
| Nutsedge | 9G, 4C |
| Barnyardgrass | 8C |
| Wheat | 0 |
| Giant Foxtail | 7G, 3C |
| Wild Oats | 1G |
| Sorghum | 9G, 4C |
| | |
| | |
| | |
| | |
| | |
| | |
| | |
| | |
| | |

TABLE C (Continued)

| | 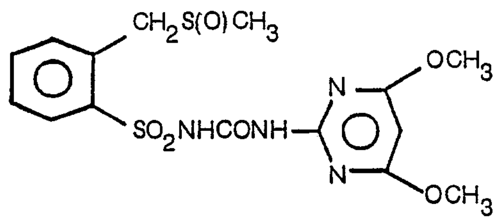 | |
|---|---|---|
| Rate, kg/ha | 0.12 | 0.03 |
| | | |
| Soybeans | 10G, 7C | 10G, 7C |
| Velvetleaf | 10G, 1C | 9G, 5C |
| Sesbania | 10G, 8C | 10G, 8C |
| Cassia | 10G, 7C | 9G, 5C |
| Cotton | 9G, 5C | 9G, 5C |
| Morningglory | 10G, 8C | 10G, 5C |
| Alfalfa | 9C | 7C |
| Jimsonweed | 9G, 7C | 5G |
| Cocklebur | 10G, 8C | 10G, 7C |
| Corn | 10C | 9C |
| Crabgrass | 7G, 3C | 5G |
| Rice | 9G, 3C | 9G, 3C |
| Nutsedge | 10C | 9G, 3C |
| Barnyardgrass | 10C | 9C |
| Wheat | 3G | 1C |
| Giant Foxtail | 10C | 8C |
| Wild Oats | 10C | – |
| Sorghum | 10C | 10C |

TABLE C (Continued)

| | OVER-THE-TOP SOIL/FOLIAGE TREATMENT | |
|---|---|---|
| | COMPOUND 8 | |
| Rate, kg/ha | 0.06 | 0.25 |
| | | |
| Soybeans | 3G, 1C | 6G, 3C |
| Velvetleaf | 9G, 4C | 10G, 8C |
| Sesbania | 9G, 3C | 10G, 6C |
| Cassia | 10G,3C | 10G, 8C |
| Cotton | 8G, 3C | 9G, 4C |
| Morningglory | 10G, 4C | 10C |
| Alfalfa | 1C | 1C |
| Jimsonweed | 1C | 3G, 1C |
| Cocklebur | 9G, 3C | 10G, 5C |
| Corn | 0 | 0 |
| Crabgrass | 0 | 0 |
| Rice | 5G | 5G |
| Nutsedge | 0 | 0 |
| Barnyardgrass | 0 | 0 |
| Wheat | 0 | 0 |
| Giant Foxtail | 0 | 1G |
| Wild Oats | 0 | 1C |
| Sorghum | 0 | 0 |

TABLE C (Continued)

| | | |
|---|---|---|
| | 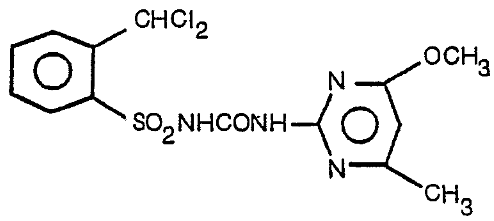 | |
| Rate, kg/ha | 0.06 | 0.25 |
| | | |
| Soybeans | 10G, 6C | 10G, 8C |
| Velvetleaf | 10G, 6C | 10G, 8C |
| Sesbania | 9C | 9C |
| Cassia | 10G, 5C | 10G, 7C |
| Cotton | 9C | 9C |
| Morningglory | 10G, 7C | 10G, 8C |
| Alfalfa | | 9C |
| Jimsonweed | | |
| Cocklebur | 10G, 9C | 10G, 8C |
| Corn | 9G, 3C | 9C |
| Crabgrass | 0 | 3G, 1C |
| Rice | 6G, 1C | 8G, 2C |
| Nutsedge | 6G | 9G |
| Barnyardgrass | 6G, 3C | 8G, 3C |
| Wheat | 3G, 2C | 7G, 2C |
| Giant Foxtail | 4G | 9G |
| Wild Oats | 5G | 7G, 2C |
| Sorghum | 9G, 3C | 10 ,7C |
| | | |
| | | |
| | | |
| | | |
| | | |
| | | |
| | | |
| | | |
| | | |

TABLE C (Continued)

| | | |
|---|---|---|
| | 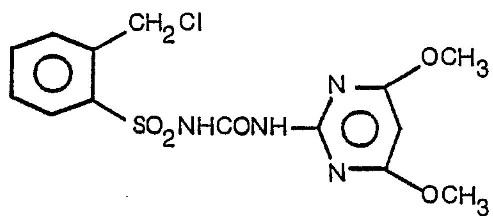 | |
| Rate, kg/ha | 0.03 | 0.125 |
| | | |
| Soybeans | 10G, 6C | 10G, 7C |
| Velvetleaf | 6G | 7G, 3C |
| Sesbania | 10G, 3C | 10G, 9C |
| Cassia | 3G, 1C | 8G, 1C |
| Cotton | 3G, 1C | 4G, 2C |
| Morningglory | 5G | 5G, 1C |
| Alfalfa | 5G, 2C | 7G, 3C |
| Jimsonweed | | |
| Cocklebur | 4G | 3G, 1C |
| Corn | 5G, 5H | 9G, 5C |
| Crabgrass | 2G | 3G, 1C |
| Rice | 5G | 8G, 3C |
| Nutsedge | 4G | 7G, 2C |
| Barnyardgrass | 5G, 1C | 8G, 2C |
| Wheat | 0 | 0 |
| Giant Foxtail | 6G, 1C | 8G, 2C |
| Wild Oats | 0 | 0 |
| Sorghum | 5G, 1C | 7G, 2C |
| | | |
| | | |
| | | |
| | | |
| | | |
| | | |
| | | |
| | | |
| | | |

Test D

Two ten-inch in diameter plastic pans lined with polyethylene liners were filled with prepared Fallsington silt loam soil. One pan was planted with seeds of wheat (*Triticum aestivum*), barley (*Hordeum vulgare), wild oats (Avena fatua*), downy brome (*Bromus tectorum*), cheatgrass (*Bromus secalinus*), blackgrass (*Alopecurus myosuroides*), annual bluegrass (*Poa annua*), green foxtail (*Setaria viridis*), quackgrass (*Agropyron repens*), Italian ryegrass (*Lolium multiflorum*) and ripgut brome (*Bromus rigidus*). The other pan was planted with seeds of Russian thistle (*Salsola kali*), tansy mustard (*Descuraina pinnata*), smartweed (*Polygonum pennsylvanicum*), tumble mustard (*Sisymbrioaltissium*), kochia (*Kochia scoparia*), shepherd's purse (*Capsella burnsa-pastoris*), *Matricaria inodora*, black nightshade (*Solanum nigrum*), yellow rocket (*Barbarea vulgaris*), wild mustard (*Brassica kaber*) and wild buckwheat (*Polygonum convolvulus*). The above two pans were treated pre-emergence. At the same time two pans in which the above plant species were growing were treated post-emergence. Plant height at the time of treatment ranged from 1—15 cm depending on plant species.

The compounds applied were diluted with a nonphytotoxic solvent and sprayed over-the-top of the pans. An untreated control and a solvent alone control were included for comparison. All treatments were maintained in the greenhouse for 20 days at which time the treatments were compared to the controls and the effects visually rated in accordance with the rating system described for Test A. The recorded data are presented in Table D. The data indicate that certain compounds from within the scope of this invention have utility for weed control in cereal crops, such as wheat and barley.

TABLE D

| | 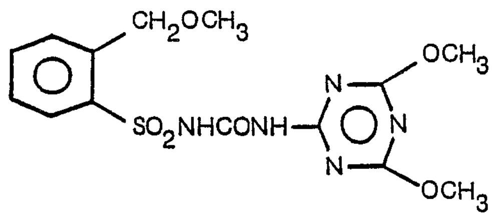 | |
|---|---|---|
| | Post-emergence | Pre-emergence |
| Rate, kg/ha | 0.06 | 0.06 |
| | | |
| Wheat | 0 | 0 |
| Barley | 0 | 1C,2G |
| Wild oats | 0 | 0 |
| Downy brome | 1G | 2C,2G |
| Cheatgrass | 2G | 2G |
| Blackgrass | 2G | 2C,3G. |
| Annual bluegrass | 5G | 5G |
| Green foxtail | 0 | 0 |
| Quackgrass | 0 | 1C,3G |
| Italian ryegrass | 2G | 3G |
| Ripgut brome | 0 | 2G |
| Russian thistle | 10C | 2C,3G |
| Tansy mustard | 10C | 10C |
| Smartweed | – | – |
| Jimhill mustard | 10C | 8C,8G |
| Kochia | 0 | 7G |
| Shepherd's purse | 10C | 9C,9G |
| False chamomile | 9C,9G | 7C,8G |
| Black nightshade | 7C,8G | 3C,7G |
| Yellow rocket | 9C,9G | 7C,8G |
| Wild mustard | 10C | 9C,8G |
| Wild buckwheat | 7C,7G | 4C,6G |
| | | |
| | | |
| | | |
| | | |
| | | |

TABLE D (Continued)

| | 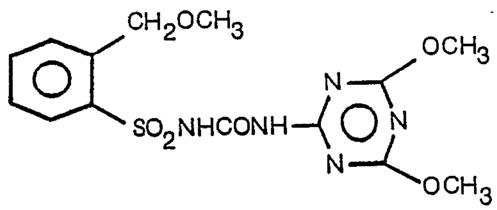 | |
|---|---|---|
| | Post-emergence | Pre-emergence |
| Rate, kg/ha | 0.25 | 0.25 |
| | | |
| Wheat | 0 | 0 |
| Barley | 0 | 2C,2G |
| Wild oats | 0 | 2G |
| Downy brome | 5C,4G | 7C,6G |
| Cheatgrass | 3C,7G | 7C,8G |
| Blackgrass | 6G | 3C,7G |
| Annual bluegrass | 3C,6G | 1C,7G |
| Green foxtail | 1C,1G | 3C,4G |
| Quackgrass | 0 | 4C,6G |
| Italian ryegrass | 1C,3G | 4C,7G |
| Ripgut brome | 0 | 3C,4G |
| Russian thistle | 10C | 3C,5G |
| Tansy mustard | 10C | 10C |
| Smartweed | – | – |
| Jimhill mustard | 10C | 9C,9G |
| Kochia | 2C,3G | 8G |
| Shepherd's purse | 10C | 9C,9G |
| False chamomile | 10C | 8C,9G |
| Black nightshade | 10C | 7C,8G |
| Yellow rocket | 10C | 9C,9G |
| Wild mustard | 10C | 9C,9G |
| Wild buckwheat | 10C | 8C,7G |
| | | |
| | | |
| | | |
| | | |
| | | |

TABLE D (Continued)

| | 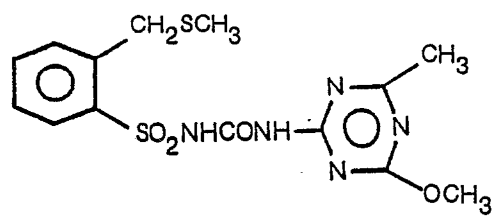 | |
|---|---|---|
| | Post-emergence | Pre-emergence |
| Rate, kg/ha | 0.25 | 0.25 |
| | | |
| Wheat | 0 | 0 |
| Barley | 0 | 0 |
| Wild oats | 0 | 0 |
| Downy brome | 0 | 2G |
| Cheatgrass | 0 | 1G |
| Blackgrass | 2C, 4G | 2G |
| Annual bluegrass | 2G | 3G |
| Green foxtail | 1C,2G | 0 |
| Quackgrass | 1C,1G | 0 |
| Italian ryegrass | 3G | 0 |
| Ripgut brome | 0 | 0 |
| Russian thistle | 2G | 2G |
| Tansy mustard | 7C,6G | 1C,2G |
| Smartweed | – | – |
| Jimhill mustard | 8C, 7G | 2C, 2G |
| Kochia | 5C,7G | 3G |
| Shepherd's purse | 3C,4G | 1C,2G |
| False chamomile | 0 | 0 |
| Black nightshade | 2C,4G | 1G |
| Yellow rocket | 3C,5G | 0 |
| Wild mustard | 10C | 3C,5G |
| Wild buckwheat | 1C,2G | 1C,2G |
| | | |
| | | |
| | | |
| | | |
| | | |

TABLE D (Continued)

| | 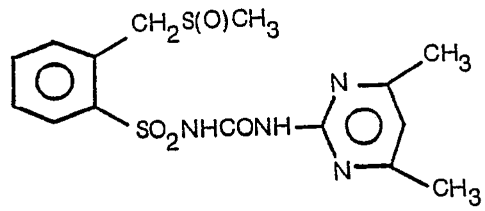 | |
|---|---|---|
| | Post-emergence | Pre-emergence |
| Rate, kg/ha | 0.06 | 0.06 |
| | | |
| Wheat | 2C,2G | 0 |
| Barley | 7C,6G | 0 |
| Wild oats | 7C,7G | 0 |
| Downy brome | 6C,7G | 2C,3G |
| Cheatgrass | 5C,6G | 1C,3G |
| Blackgrass | 2C,3G | 2C,3G |
| Annual bluegrass | 1C,2G | 1C,5G |
| Green foxtail | 1C,1G | 0 |
| Quackgrass | 5C,5G | 0 |
| Italian ryegrass | 10C | 0 |
| Ripgut brome | 7C,7G | 0 |
| Russian thistle | 0 | 2G |
| Tansy mustard | 2C,2G | 5C,7G |
| Smartweed | – | – |
| Jimhill mustard | 7C,6G | 3C,7G |
| Kochia | 2G | 3G |
| Shepherd's purse | 3C,2G | 3C,8G |
| False chamomile | 1C,2G | 3G |
| Black nightshade | 0 | 1C,2G |
| Yellow rocket | 1C,2G | 7C,8G |
| Wild mustard | 3C,4G | 1C,4G |
| Wild buckwheat | 0 | 2C,2G |
| | | |
| | | |
| | | |
| | | |
| | | |

TABLE D (Continued)

| | 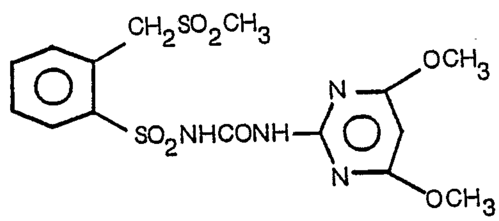 | |
|---|---|---|
| | Post-emergence | Pre-emergence |
| Rate, kg/ha | 0.015 | 0.015 |
| | | |
| Wheat | 0 | 1C |
| Barley | 0 | 0 |
| Wild oats | 0 | 2C |
| Downy brome | 0 | 2C,3G |
| Cheatgrass | 0 | 7C,7G |
| Blackgrass | 0 | 7C,8G |
| Annual bluegrass | 0 | 2C,3G |
| Green foxtail | 0 | 3G |
| Quackgrass | 0 | 1G |
| Italian ryegrass | 0 | 0 |
| Ripgut brome | 0 | 0 |
| Russian thistle | 3C,4C | 0 |
| Tansy mustard | 10C | 3C,5G |
| Smartweed | – | – |
| Jimhill mustard | 10C | 4C,8G |
| Kochia | 2C,2G | 2G |
| Shepherd's purse | 5C,4G | 7C,9G |
| False chamomile | 3C,4G | 2C,3G |
| Black nightshade | 0 | 2C,2G |
| Yellow rocket | 2C,3C | 8C,9G |
| Wild mustard | 10C | 7C,7G |
| Wild buckwheat | 2C,3G | 3G |
| | | |
| | | |
| | | |
| | | |
| | | |

TABLE D (Continued)

| | 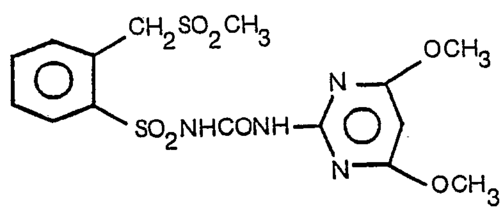 | |
|---|---|---|
| | Post-emergence | Pre-emergence |
| Rate, kg/ha | 0.06 | 0.06 |
| | | |
| Wheat | 0 | 1C,1G |
| Barley | 3G | 3G |
| Wild oats | 0 | 1C,2G |
| Downy brome | 2C,2G | 5C,4G |
| Cheatgrass | 5C,6G | 8C,8G |
| Blackgrass | 7C,8C | 7C,8G |
| Annual bluegrass | 2C,3G | 4C,7G |
| Green foxtail | 3C,5G | 6C,7G |
| Quackgrass | 1C,2G | 3C,4G |
| Italian ryegrass | 1C | 1C,6G |
| Ripgut brome | 2G | 1C,3G |
| Russian thistle | 10C | 10C |
| Tansy mustard | 10C | 10C |
| Smartweed | – | – |
| Jimhill mustard | 10C | 9C,9G |
| Kochia | 7C,6G | 3C,7G |
| Shepherd's purse | 10C | 9C,9G |
| False chamomile | 7C,6G | 7C,8G |
| Black nightshade | 2C,4G | 4C,7G |
| Yellow rocket | 10C | 9C,9G |
| Wild mustard | 10C | 9C,9G |
| Wild buckwheat | 8C,7G | 7C,7G |
| | | |
| | | |
| | | |
| | | |
| | | |

TABLE D (Continued)

| | | |
|---|---|---|
| | 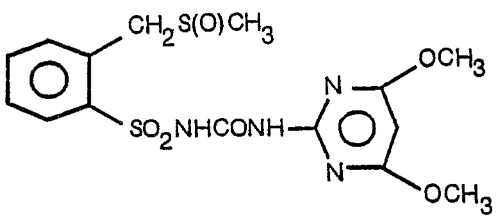 | |
| | Post-emergence | Pre-emergence |
| Rate, kg/ha | 0.015 | 0.015 |
| | | |
| Wheat | 1C,1G | 0 |
| Barley | 3C,2G | 3G |
| Wild oats | 6C,4G | 2C,2G |
| Downy brome | 10C | 3C,7G |
| Cheatgrass | 8C,6G | 7C,8G |
| Blackgrass | 10C | 7C,7G |
| Annual bluegrass | 3C,6G | 7C,8G |
| Green foxtail | 8C,7G | 3C,7G |
| Quackgrass | 8C,7G | 5G |
| Italian ryegrass | 2C,3C | 3G |
| Ripgut brome | 3C,4G | 2G |
| Russian thistle | 7C,8G | 5C,4G |
| Tansy mustard | 10C | 9C,9G |
| Smartweed | – | – |
| Jimhill mustard | 10C | 8C,8G |
| Kochia | 0 | 3G |
| Shepherd's purse | 8C,7G | 9C,9G |
| False chamomile | 7C,8C | 3C,5G |
| Black nightshade | 0 | 2C,5G |
| Yellow rocket | 10C | 4C,8G |
| Wild mustard | 10C | 7C,7G |
| Wild buckwheat | 7C,8G | 3C,4G |
| | | |
| | | |
| | | |
| | | |
| | | |

TABLE D (Continued)

| | 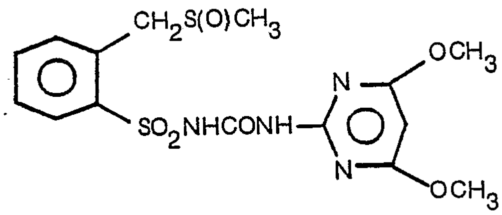 | |
|---|---|---|
| | Post-emergence | Pre-emergence |
| Rate, kg/ha | 0.06 | 0.06 |
| | | |
| Wheat | 6C,5G | 0 |
| Barley | 8C,7G | 3C,7G |
| Wild oats | 10C | 6C,6G |
| Downy brome | 10C | 10C |
| Cheatgrass | 10C | 10C |
| Blackgrass | 10C | 10C |
| Annual bluegrass | 10C | 10C |
| Green foxtail | 10C | 10C |
| Quackgrass | 10C | 7C,7G |
| Italian ryegrass | 10C | 6C,7G |
| Ripgut brome | 10C | 3C,7G |
| Russian thistle | 8C,7G | 6C,7G |
| Tansy mustard | 10C | 10C |
| Smartweed | – | – |
| Jimhill mustard | 10C | 10C |
| Kochia | 4C,5G | 3C,7G |
| Shepherd's purse | 10C | 9C,9G |
| False chamomile | 10C | 7C,8G |
| Black nightshade | 3C,5G | 3C,8G |
| Yellow rocket | 10C | 9C,8G |
| Wild mustard | 10C | 9C,9G |
| Wild buckwheat | 9C,8G | 4C,5G |
| | | |
| | | |
| | | |
| | | |
| | | |

TABLE D (Continued)

| | 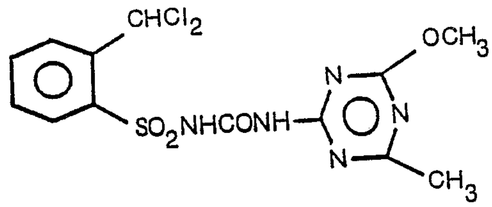 | |
|---|---|---|
| | Post-emergence | Pre-emergence |
| Rate, kg/ha | 0.015 | 0.015 |
| | | |
| Wheat | 0 | 0 |
| Barley | 0 | 0 |
| Wild oats | 0 | 0 |
| Downy brome | 0 | 0 |
| Cheatgrass | 0 | 0 |
| Blackgrass | 2C | 2G |
| Annual bluegrass | 0 | 1G |
| Green foxtail | 0 | 0 |
| Quackgrass | 0 | 0 |
| Italian ryegrass | 0 | 0 |
| Ripgut brome | 0 | 0 |
| Russian thistle | 10C | 0 |
| Tansy mustard | 10C | 7C,8G |
| Smartweed | 9C | – |
| Jimhill mustard | 10C | 9C,8G |
| Kochia | 7C,8G | 5C,5G |
| Shepherd's purse | 10C | 9C,8G |
| False chamomile | 10C | 5C,8G |
| Black nightshade | – | – |
| Yellow rocket | 10C | 10C |
| Wild mustard | 10C | 7C,7G |
| Wild buckwheat | 6C,5G | 1C,1G |
| | | |
| | | |
| | | |
| | | |
| | | |

TABLE D (Continued)

| | 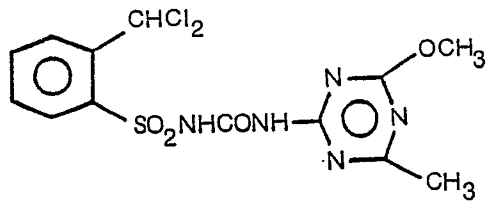 | |
|---|---|---|
| | Post-emergence | Pre-emergence |
| Rate, kg/ha | 0.06 | 0.06 |
| | | |
| Wheat | 0 | 0 |
| Barley | 0 | 0 |
| Wild oats | 0 | 1G |
| Downy brome | 1G | 0 |
| Cheatgrass | 0 | 1G |
| Blackgrass | 7C | 3G |
| Annual bluegrass | 2C,2G | 3C,7G |
| Green foxtail | 10C | 3G |
| Quackgrass | 0 | 3G |
| Italian ryegrass | 2G | 5G |
| Ripgut brome | 0 | 0 |
| Russian thistle | 10C | 3C,3G |
| Tansy mustard | 10C | 8C,8G |
| Smartweed | 10C | – |
| Jimhill mustard | 10C | 9C,9G |
| Kochia | 9C | 7C,8G |
| Shepherd's purse | 10C | 10C |
| False chamomile | 10C | 7C,8G |
| Black nightshade | – | – |
| Yellow rocket | 10C | 10C |
| Wild mustard | 10C | 9C,8G |
| Wild buckwheat | 8C,7G | 2C,3G |
| | | |
| | | |
| | | |
| | | |
| | | |

TABLE D (Continued)

| | 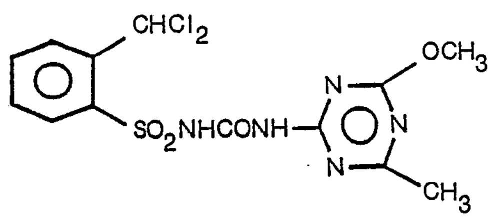 | |
|---|---|---|
| | Post-emergence | Pre-emergence |
| Rate, kg/ha | 0.125 | 0.125 |
| | | |
| Wheat | 0 | 0 |
| Barley | 0 | 0 |
| Wild oats | 0 | 0 |
| Downy brome | 0 | 2G |
| Cheatgrass | 0 | 3G |
| Blackgrass | – | 4C,8G |
| Annual bluegrass | 2G | 6G |
| Green foxtail | 4C,4G | 2C,6G |
| Quackgrass | 0 | 3G |
| Italian ryegrass | 2C,4G | 2G |
| Ripgut brome | 0 | 0 |
| Russian thistle | 10C | 4C,5G |
| Tansy mustard | 10C | 9C,9G |
| Smartweed | – | – |
| Jimhill mustard | 10C | 9C,9G |
| Kochia | 10C | 5G |
| Shepherd's purse | 10C | 8C,9G |
| False chamomile | 10C | 9C,9G |
| Black nightshade | – | 5G |
| Yellow rocket | 10C | 9C,9G |
| Wild mustard | 10C | 7C,8G |
| Wild buckwheat | 10C | 2C,2G |
| | | |
| | | |
| | | |
| | | |
| | | |

TABLE D (Continued)

| | 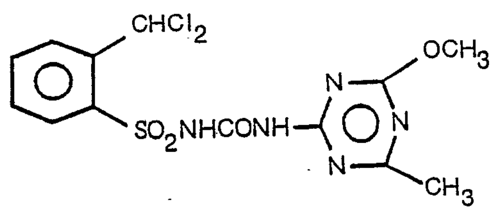 | |
|---|---|---|
| | Post-emergence | Pre-emergence |
| Rate, kg/ha | 0.5 | 0.5 |
| | | |
| Wheat | 0 | 0 |
| Barley | 0 | 1G |
| Wild oats | 0 | 0 |
| Downy brome | 2G | 1G |
| Cheatgrass | 2G | 3C,4G |
| Blackgrass | – | 2C,6G |
| Annual bluegrass | 6C,5G | 5C,7G |
| Green foxtail | 10C | 4C,7G |
| Quackgrass | 2G | 7C,7G |
| Italian ryegrass | 4C,6G | 5G |
| Ripgut brome | 0 | 2G |
| Russian thistle | 10C | 5C,5G |
| Tansy mustard | 10C | 9C,9G |
| Smartweed | – | – |
| Jimhill mustard | 10C | 9C,9G |
| Kochia | 10C | 6G |
| Shepherd's purse | 10C | 9C,9G |
| False chamomile | 10C | 9C,9G |
| Black nightshade | – | 4C,6G |
| Yellow rocket | 10C | 9C,9G |
| Wild mustard | 10C | 9C,9G |
| Wild buckwheat | 10C | 5C,6G |
| | | |
| | | |
| | | |
| | | |
| | | |

Test E

Test samples were formulated and applied directly to the water of paddies three days after transplanting of Japonica rice. The paddies were maintained in a greenhouse, and plant response ratings were taken one and three weeks after application.

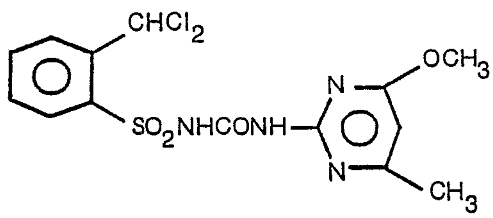

| Treatment | Rice 1 Week | Rice 3 Weeks | Barnyard-* grass 3 Weeks | Water* Chestnut 3 Weeks | Scirpus* 3 Weeks |
|---|---|---|---|---|---|
| 5 g/ha | 0 | 0 | 5G | 8G | 0 |
| 20 g/ha | 0 | 5G | 8G | 9G | 5G |

**Echinochloa* sp., *Eleocharis* sp., and *Scirpus* sp., respectively.

Test F

A test sample of compound 8 was formulated and applied directly to the water of simulated paddies, three days after transplanting of rice. The paddies were maintained in a greenhouse, and plant response ratings were taken one week and four weeks after application.

| Rate, kg ai/ha | Rice 1 wk. | Rice 4 wks. | Barnyard-grass* 4 weeks | Water Chestnut* 4 weeks | Arrowhead* 4 weeks |
|---|---|---|---|---|---|
| 0.125 | 0 | 0 | 0 | 8G | 0 |
| 0.5 | 0 | 0 | 0 | 10C | 6G |

**Echinochloa* sp., *Eleocharis* sp., and *Sagittaria* sp., respectively.

Reference to the table above indicates that rice tolerated the application of the test sample at 500 g ai/ha, whereas the important weed (in rice culture in some areas) water chestnut was completely controlled. Arrowhead, which also is an important weed in some rice culture areas, was partially controlled at the same application rate.

Test G

Purple nutsedge (*Cyperus rotundus*) tubers were planted about 2 cm deep in Fallsington loam soil contained in 10 cm diameter plastic pots. Five tubers were planted in each pot. Compounds of this invention were dissolved in a non-phytotoxic diluent and sprayed at 560 l/ha in four methods of application: soil surface, tuber/soil, soil incorporated and post-emergence. The soil surface spray consisted of spraying the compound on the surface of the firmed covering soil. The tuber/soil spray consisted of spraying the compound on exposed tubers and subtending soil before adding the untreated covering soil. Soil incorporated treatment consisted in mixing the compound with the covering soil before using it to cover the tubers. The post-emergence treatment was sprayed on nutsedge foliage and the surrounding soil surface when nutsedge had emerged and grown to a height of about 12 cm. Pots receiving the post-emergence treatments were placed directly in the greenhouse. Pots receiving the other treatments were misted with about 0.3 cm water before being transferred to the greenhouse. Response ratings assessed after four weeks are recorded in Table G based on the same rating system as described in procedure A.

TEST G

NUTSEDGE

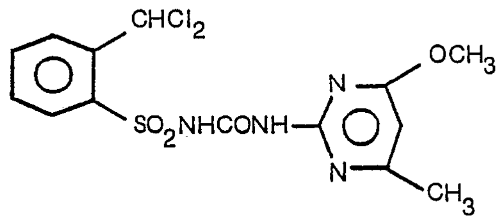

| Rate kg/ha | Response Rating (after 4 weeks) | | | |
|---|---|---|---|---|
| | Preemerg. Soil Surface | Tuber Spray | Soil Incorp. | Postemerg. |
| 0.01 | 3G | 6G | 7G | 0 |
| 0.03 | 7G | 7G | 8G | 0 |
| 0.125 | 9G | 9G | 9G | 4C, 7G |

**Claims for the Contracting States: BE CH DE FR GB IT LI LU NL SE**

1. A compound selected from:

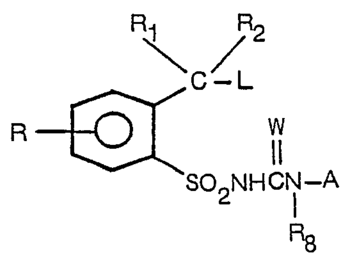

(I)

wherein

L is $CO_2R_{10}$, $CONR_3R_4$, CN, Cl, Br, $NR_3R_4$, $\overset{+}{N}R_3R_4'$, $N(R_4)C(O)R_5$, $N(R_4)C(O)NHR_6$, $N(R_4)C(O)OR_7$, $S(O)_nR_7$, $OR_9$, $SO_2NR_3R_4$, OH, $OC(O)R_{11}$, $OC(O)NHR_{12}$ or $OC(O)OR_{13}$;

R is H, F, Cl, Br, $NO_2$, $CF_3$, $C_1$—$C_3$ alkyl or $C_1$—$C_3$ alkoxy;

$R_1$ is H, Cl or $C_1$—$C_4$ alkyl;

$R_2$ is H or $CH_3$;

$R_3$ is H, $C_1$—$C_4$ alkyl or $OCH_3$;

$R_4$ is H or $C_1$—$C_4$ alkyl;

$R_3$ and $R_4$ can be taken together to form $—(CH_2)_4—$, $—(CH_2)_5—$ or $—(CH_2CH_2)_2O$;

$R_4'$ is H, $CH_3$ or $CH_2CH_3$;

$R_5$ is $C_1$—$C_4$ alkyl optionally substituted with 1—3 atoms of F, Cl or Br, or $C_3$—$C_4$ alkenyl;

$R_6$ is H, $C_1$—$C_4$ alkyl or $C_3$—$C_4$ alkenyl;

$R_7$ is $C_1$—$C_4$ alkyl;

$R_8$ is H, $CH_3$ or $OCH_3$;

$R_9$ is $C_1$—$C_6$ alkyl, $C_3$—$C_4$ alkenyl, $CH_2CF_3$, $CF_2CF_2H$ or $C_5$—$C_6$ cycloalkyl;

$R_{10}$ is H, $C_1$—$C_4$ alkyl, $C_3$—$C_4$ alkenyl, $CH_2CH_2Cl$ or $CH_2CH_2OCH_3$;

$R_{11}$ is H, $C_1$—$C_5$ alkyl, $C_2$—$C_3$ alkenyl, $C_2$—$C_3$ alkynyl, $C_3$—$C_4$ cycloalkyl,

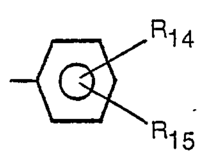,

$C_1$—$C_4$ alkyl substituted with 1—4 substituents selected from 0—3 F, 0—3 Cl or 0—3 Br, or $C_2$—$C_3$ alkenyl substituted with 1—3 Cl;

$R_{12}$ is H, $C_1$—$C_6$ alkyl, $C_3$—$C_4$ alkenyl, $C_5$—$C_6$ cycloalkyl,

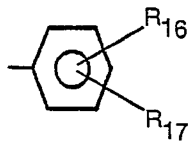 , 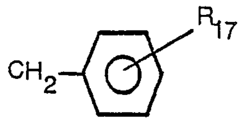

or $C_5$—$C_6$ cycloalkyl substituted with $CH_3$;

$R_{13}$ is $C_1$—$C_6$ alkyl or 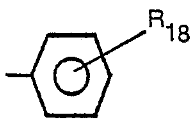 ;

$R_{14}$ and $R_{15}$ are independently H, $NO_2$, $CH_3$, Cl or $OCH_3$;
$R_{16}$ is H, F, Cl, Br, $C_1$—$C_3$ alkyl, $NO_2$, CN, $SO_2CH_3$, $OCH_3$, $SCH_3$ or $CF_3$;
$R_{17}$ is H, Cl or $C_1$—$C_3$ alkyl;
$R_{18}$ is H, $CH_3$ or Cl;
n is 0, 1 or 2;

A is 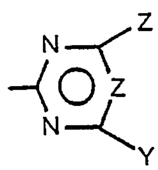 , 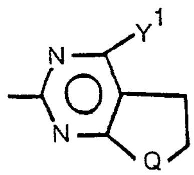 or 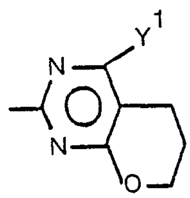

W is O or S;
X is H, Cl, Br, $CH_3$, $CH_2CH_3$, $C_1$—$C_3$ alkoxy, $CF_3$, $SCH_3$ or $CH_2OCH_3$;
Y is $CH_3$ or $OCH_3$;
Z is N, CH, CCl, CBr, CCN, $CCH_3$, $CCH_2CH_3$, $CCH_2CH_2Cl$ or $CCH_2CH{=}CH_2$;
$Y^1$ is H, $CH_3$, $OCH_3$ or $OCH_2CH_3$; and
Q is O or $CH_2$;

and their agriculturally suitable salts; provided that:
(1) when $R_1$ is Cl, then L is Cl or Br and $R_2$ is H;
(2) when n is 1, then W is O;
(3) when L is $CONR_3R_4$, then Z is CH or N;
(4) when $R_3$ is $OCH_3$, then $R_4$ is $CH_3$;
(5) the total number of carbon atoms of $R_3$ and $R_4$ is five or less;
(6) when W is S, then $R_8$ is H; and
(7) when L is $SO_2NR_3R_4$, then $R_3$ and $R_4$ are not H.

2. A compound of claim 1 wherein Z is N, CH, CCl, CBr or $CCH_3$; W is O; and $R_8$ is H or $CH_3$.

3. A compound of claim 1 wherein L is OH; R is H; $R_1$ and $R_2$ are both $CH_3$; $R_8$ is H or $CH_3$;

A is 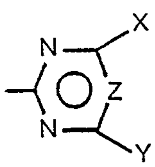 ;

Z is CH or N; X and Y are independently $CH_3$ or $OCH_3$; and W is O.

4. A compound of claim 2 wherein L is Cl, Br, OH, $NR_3R_4$, $+NR_3R_4'$, $CO_2R_{10}$, $S(O)_nR_7$, $OR_9$, $SO_2NR_3R_4$, $OC(O)R_{11}$, $OC(O)NHR_{12}$, $OC(O)OR_{13}$; Z is CH or N; X is $CH_3$ or $OCH_3$; R, $R_1$, $R_2$ are H; and when n is 1 or 2, Z is CH.

5. A compound of claim 4 wherein A is 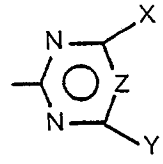 ;

$R_8$ is H; L is Cl, Br, $NR_3R_4$, $CO_2R_{10}$, $S(O)_nR_7$, $OR_9$, $SO_2NR_3R_4$, OH, $OC(O)R_{11}$, $OC(O)NHR_{12}$, $OC(O)OR_{13}$; and $R_{11}$, $R_{12}$ and $R_{13}$ are $C_1$—$C_3$ alkyl.

6. A compound of claim 5 wherein L is Cl, Br, $NR_3R_4$, $CO_2R_{10}$ $S(O)_nR_7$, $OR_9$, $SO_2NR_3R_4$ or OH.

7. A compound of claim 6 wherein $R_3$ is $C_1$—$C_3$ alkyl or $OCH_3$; $R_4$ is $CH_3$; $R_9$ is $C_1$—$C_4$ alkyl; and $R_{10}$ is $CH_3$ or $CH_2CH_3$;

8. A compound of claim 7 wherein $R_7$ and $R_9$ are $CH_3$.

9. A compound of claim 8 wherein L is $CO_2R_{10}$, $S(O)_nR_7$ or $OR_9$.

10. A compound of claim 1 selected from:

2-(Dichloromethyl)-N-[(4-methoxy-6-methylpyrimidin-2-yl)aminocarbonyl]benzenesulfonamide;

2-[[(4-methoxy-6-methylpyrimidin-2-yl)aminocarbonyl]aminosulfonyl]benzeneacetic acid, methyl ester;

2-[[(4-methoxy-6-methylpyrimidin-2-yl)aminocarbonyl]aminosulfonyl]benzeneacetic acid, ethyl ester;

N-[(4-Methoxy-6-methylpyrimidin-2-yl)aminocarbonyl-2-(methoxymethyl)benzenesulfonamide;

N-[(4-Methoxy-6-methylpyrimidin-2-yl)aminocarbonyl]-2-(methylthiomethyl)benzenesulfonamide;

N-[(4-Methoxy-6-methylpyrimidin-2-yl)aminocarbonyl]-2-(methylsulfonylmethyl)benzenesulfonamide;

N-[(4-Methoxy-6-methylpyrimidin-2-yl)aminocarbonyl]-2-(methylsulfinylmethyl)benzenesulfonamide;

2-[(Dimethylamino)sulfonylmethyl]-N-[(4-methoxy-6-methylpyrimidin-2-yl)aminocarbonyl]benzenesulfonamide;

N-[(4-methoxy-6-methylpyrimidin-2-yl)aminocarbonyl]-2-(hydroxymethyl)benzenesulfonamide;

and agriculturally suitable salts thereof.

11. A compound of claim 1 selected from:

2-(Dichloromethyl)-N-[(4,6-dimethylpyrimidin-2-yl)aminocarbonyl]benzenesulfonamide;

2-(Chloromethyl)-N-[(4,6-dimethylpyrimidin-2-yl)aminocarbonyl]benzenesulfonamide;

2-[[(4,6-dimethylpyrimidin-2-yl)aminocarbonyl]aminosulfonyl]benzeneacetic acid, methyl ester;

N-[(4,6-Dimethylpyrimidin-2-yl)aminocarbonyl]-2-methoxymethyl)benzenesulfonamide;

N-[(4,6-dimethylpyrimidin-2-yl)aminocarbonyl]-2-(methylthiomethyl)benzenesulfonamide;

N-[(4,6-Dimethylpyrimidin-2-yl)aminocarbonyl]-2-(methylsulfinylmethyl)benzenesulfonamide;

N-[(4,6-Dimethylpyrimidin-2-yl)aminocarbonyl]-2-(methylsulfonylmethyl)benzenesulfonamide;

2-[(Dimethylamino)sulfonylmethyl]-N-[(4,6-dimethylpyrimidin-2-yl)aminocarbonyl]benzenesulfonamide;

N-[(4,6-Dimethylpyrimidin-2-yl)aminocarbonyl-2-(hydroxymethyl)benzenesulfonamide;

and agriculturally suitable salts thereof.

12. A compound of claim 1 selected from:

N-[(4,6-Dimethoxypyrimidin-2-yl)aminocarbonyl]-2-(methylaminocarbonylmethyl)benzenesulfonamide;

2-(Chloromethyl)-N-[(4,6-dimethoxypyrimidin-2-yl)aminocarbonyl]benzenesulfonamide;

2-(1-Pyrrolidinylmethyl)-N-[(4,6-dimethoxypyrimidin-2-yl)aminocarbonyl]benzenesulfonamide, hydrochloride;

2-(1-Pyrrolidinylmethyl)-N-[(4,6-dimethoxypyrimidin-2-yl)aminocarbonyl]benzenesulfonamide;

2-[[(4,6-dimethoxypyrimidin-2-yl)aminocarbonyl]aminosulfonyl]benzeneacetic acid, methyl ester;

2-[[(4,6-dimethoxypyrimidin-2-yl-aminocarbonyl]aminosulfonyl]benzeneacetic acid, ethyl ester;

N-[(4,6-Dimethoxypyrimidin-2-yl)aminocarbonyl]-2-(methoxymethyl)benzenesulfonamide;

N-[(4,6-Dimethoxypyrimidin-2-yl)aminocarbonyl]-2-(methylthiomethyl)benzenesulfonamide;

N-[(4,6-Dimethoxypyrimidin-2-yl)aminocarbonyl]-2-(methylsulfonylmethyl)benzenesulfonamide;

N-[(4,6-Dimethoxypyrimidin-2-yl)aminocarbonyl]-2-(methylsulfinylmethyl)benzenesulfonamide;

N-[(4,6-dimethoxypyrimidin-2-yl)aminocarbonyl]-2-(butoxymethyl)benzenesulfonamide;

N-[(4,6-dimethoxypyrimidin-2-yl)aminocarbonyl]-2-(ethoxymethyl)benzenesulfonamide;

N-[(4,6-dimethoxypyrimidin-2-yl)aminocarbonyl]-2-(1-methylpropyloxymethyl)benzenesulfonamide;

2-[(Dimethylamino)sulfonylmethyl]-N-[(4,6-dimethoxypyrimidin-2-yl)aminocarbonyl]benzenesulfonamide;

N-[(4,6-Dimethoxypyrimidin-2-yl)aminocarbonyl]-2-(hydroxymethyl)benzenesulfonamide;

and agriculturally suitable salts thereof.

13. A compound of claim 1 selected from:

2-(Dichloromethyl)-N-[(4-methoxy-6-methyl-1,3,5-triazin-2-yl)aminocarbonyl]benzenesulfonamide;

2-[[(4-methoxy-6-methyl-1,3,5-triazin-2-yl)aminocarbonyl]aminosulfonyl]benzeneacetic acid, methyl ester;

N-[(4-Methoxy-6-methyl-1,3,5-triazin-2-yl)aminocarbonyl]-2-(methoxymethyl)benzenesulfonamide;

N-[(4-Methoxy-6-methyl-1,3,5-triazin-2-yl)aminocarbonyl]-2-(methylthiomethyl)benzenesulfonamide;

2-[(Dimethylamino)sulfonylmethyl]-N-[(4-methoxy-6-methyl-1,3,5-triazin-2-yl)aminocarbonyl]-benzenesulfonamide;

N-[(4-Methoxy-6-methyl-1,3,5-triazin-2-yl)aminocarbonyl]-2-(hydroxymethyl)benzenesulfonamide;

and agriculturally suitable salts thereof.

14. A compound of claim 1 selected from:

2-(Chloromethyl)-N-[(4,6-dimethoxy-1,3,5-triazin-2-yl)aminocarbonyl]benzenesulfonamide;

2-(1-Chloroethyl)-N-[(4,6-dimethoxy-1,3,5-triazin-2-yl)aminocarbonyl]benzenesulfonamide;

2-[[(4,6-dimethoxy-1,3,5-triazin-2-yl)aminocarbonyl]aminosulfonyl]benzeneacetic acid, methyl ester;

N-[(4,6-Dimethoxy-1,3,5-triazin-2-yl)aminocarbonyl]-2-(methoxymethyl)benzenesulfonamide;

N-[(4,6-Dimethoxy-1,3,5-triazin-2-yl)aminocarbonyl]-2-(methylthiomethyl)benzenesulfonamide;

2-[(Dimethylamino)sulfonylmethyl]-N-[(4,6-dimethoxy-1,3,5-triazin-2-yl)aminocarbonyl]benzenesulfonamide;

N-[(4,6-Dimethoxy-1,3,5-triazin-2-yl)aminocarbonyl]-2-(hydroxymethyl)benzenesulfonamide;

and agriculturally suitable salts thereof.

15. A compound of claim 1 selected from:

N-[(4,6-Dimethyl-1,3,5-triazin-2-yl)aminocarbonyl]-2-(methoxymethyl)benzenesulfonamide;

N-[(4,6-Dimethyl-1,3,5-triazin-2-yl)aminocarbonyl]-2-(methylthiomethyl)benzenesulfonamide;

2-[(Dimethylamino)sulfonylmethyl]-N-[(4,6-dimethyl-1,3,5-triazin-2-yl)aminocarbonyl]benzenesulfonamide;

N-[(4,6-Dimethyl-1,3,5-triazin-2-yl)aminocarbonyl]-2-(hydroxymethyl)benzenesulfonamide;

and agriculturally suitable salts thereof.

16. A compound of claim 1 wherein R, $R_1$ and $R_2$ are as defined in claim 1, W is O, $R_8$ is H, A is

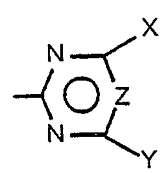 or 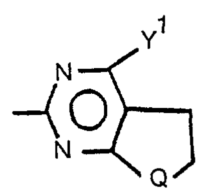

where Q, X, Y and Z are as defined in claim 1 and $Y^1$ is H, $CH_3$ or $OCH_3$; and L is $CO_2R_{10}$, $CONR_3R_4$ or CN, where $R_{10}$ is $C_1$—$C_4$ alkyl; and $R_3$ and $R_4$ are independently H or $C_1$—$C_4$ alkyl.

17. A compound of claim 1 wherein: R is as defined in claim 1; $R_1$ is H or $CH_3$; $R_2$ is H or $CH_3$; L is $SO_2NR_3R_4$ where $R_3$ and $R_4$ are as defined in claim 1; $R_8$ is H; W is O; and A is defined in claim 16.

18. A compound of claim 1 wherein: R is as defined in claim 1; $R_1$ is H or $C_1$—$C_4$ alkyl; $R_2$ is H or $CH_3$; $R_8$ is H; L is $S(O)_nR_7$ or $OR_9$; $R_7$ and n are as defined in claim 1; $R_9$ is $C_1$—$C_4$ alkyl, $C_3$—$C_4$ alkenyl; A is as defined in claim 16; and W is O.

19. A compound of claim 1 wherein R is as defined in claim 1; $R_1$ and $R_2$ are independently H or $CH_3$; $R_8$ is H; L is OH, $OC(O)R_{11}$, $OC(O)NHR_{12}$, or $OC(O)OR_{13}$; W is O; and A is as defined in claim 16.

20. A compound of claim 1 wherein: R, $R_1$ and $R_2$ are as defined in claim 1; L is Cl, Br, $NR_3R_4$, $\overset{+}{N}R_3R_4R_4'$, $N(R_4)C(O)R_5$ $N(R_4)C(O)NHR_6$ or $N(R_4)C(O)R_7$; $R_8$ is H; W is O; and A is as defined in claim 16.

21. A compound selected from

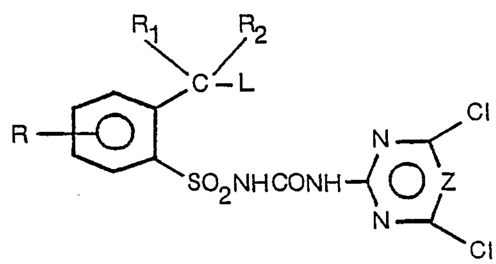

(II)

wherein

R, $R_1$, and $R_2$ are as previously defined in claim 1;

Z is CH or N;

n is 0 or 2; and

L is as previously defined in claim 1,

provided that:

(1) L is not OH;

(2) when $R_3$ is $OCH_3$, then $R_4$ is $CH_3$ and that $R_{10}$ cannot be H;

(3) when $R_1$ is Cl, then L is Cl or Br and $R_2$ is H;

(4) when L is $NR_3R_4$, $NR_3R_4R_4$; or $SO_2NR_3R_4$, then $R_3$ or $R_4$ cannot be H;

(5) the total number of carbon atoms of $R_3$ and $R_4$ is five or less.

22. A compound selected from

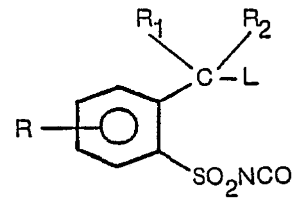

(III)

wherein

R, $R_1$, $R_2$, $R_7$ and $R_9$ are as previously defined in claim 1;
n is 0 or 2;
L is Cl, Br, $CO_2R_{10}$, $SO_2NR_3R_4$, $S(O)_nR_7$ or $OR_9$,

provided that:

(1) when $R_1$ is Cl, then L is Cl or Br and $R_2$ is H;
(2) when L is $SO_2NR_3R_4$, then $R_3$ and $R_4$ cannot be H;
(3) when $R_3$ is $OCH_3$, then $R_4$ is $CH_3$;
(4) $R_{10}$ cannot be H; and
(5) n is 0 or 2.

23. A composition suitable for controlling the growth of undesired vegetation which comprises an effective amount of a herbicidal composition and at least one of the following: surfactant, solid or liquid diluent, characterised in that said herbicidal compound comprises a compound of any of claims 1 to 20.

24. A method for controlling the growth of undesired vegetation by applying to the locus of such vegetation an effective amount of a herbicidal compound, characterised in that said herbicidal compound comprises a compound of any of claims 1 to 20.

25. A method for regulating the growth of plants by applying to the locus of said plants an effective but substantially non-phytotoxic amount of a plant growth regulant, characterised in that said plant growth regulant comprises a compound of any of claims 1 to 20.

26. A process for preparing a compound of claim 1 which comprises reacting together

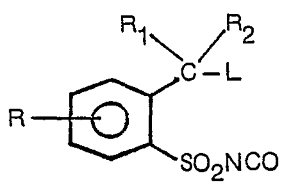

and

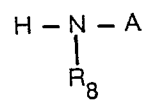

wherein

L is Cl, Br, $CO_2R_{10}$, $SO_2NR_3R_4$, $S(O)_nR_7$, $OR_9$;
n is 0 or 2;
R, $R_1$, $R_2$, $R_3$, $R_4$, $R_7$, $R_8$, $R_9$, $R_{10}$ and A are as defined in claim 1 except that $R_3$, $R_4$ and $R_{10}$ cannot be H.

**Claims for the Contracting State: AT**

1. A method for the preparation of a compound selected from:

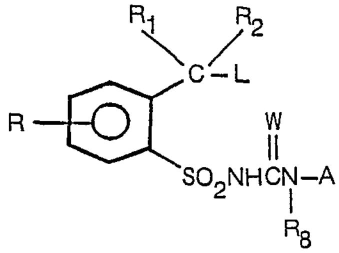

(I)

wherein

L is $CO_2R_{10}$, $CONR_3R_4$, CN, Cl, Br, $NR_3R_4$, $\overset{+}{N}R_3R_4R_4'$, $N(R_4)C(O)R_5$, $N(R_4)C(O)NHR_6$, $N(R_4)C(O)OR_7$, $S(O)_nR_7$, $OR_9$, $SO_2NR_3R_4$, OH, $OC(O)R_{11}$, $OC(O)NHR_{12}$ or $OC(O)OR_{13}$;
R is H, F, Cl, Br, $NO_2$, $CF_3$, $C_1$—$C_3$ alkyl or $C_1$—$C_3$ alkoxy;
$R_1$ is H, Cl or $C_1$—$C_4$ alkyl;
$R_2$ is H or $CH_3$;

$R_3$ is H, $C_1$—$C_4$ alkyl or $OCH_3$;
$R_4$ is H or $C_1$—$C_4$ alkyl;
$R_3$ and $R_4$ can be taken together to form —$(CH_2)_4$—, —$(CH_2)_5$— or —$(CH_2CH_2)_2O$;
$R_4'$ is H, $CH_3$ or $CH_2CH_3$;
$R_5$ is $C_1$—$C_4$ alkyl optionally substituted with 1—3 atoms of F, Cl or Br, or $C_3$—$C_4$ alkenyl;
$R_6$ is H, $C_1$—$C_4$ alkyl or $C_3$—$C_4$ alkenyl;
$R_7$ is $C_1$—$C_4$ alkyl;
$R_8$ is H, $CH_3$ or $OCH_3$;
$R_9$ is $C_1$—$C_6$ alkyl, $C_3$—$C_4$ alkenyl, $CH_2CF_3$, $CF_2CF_2H$ or $C_5$—$C_6$ cycloalkyl;
$R_{10}$ is H, $C_1$—$C_4$ alkyl, $C_3$—$C_4$ alkenyl, $CH_2CH_2Cl$ or $CH_2CH_2OCH_3$;
$R_{11}$ is H, $C_1$—$C_5$ alkyl, $C_2$—$C_3$ alkenyl, $C_2$—$C_3$ alkynyl, $C_3$—$C_4$ cycloalkyl,

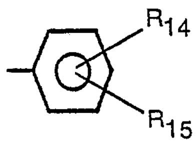 ,

$C_1$—$C_4$ alkyl substituted with 1—4 substituents selected from 0—3 F, 0—3 Cl or 0—3 Br, or $C_2$—$C_3$ alkenyl substituted with 1—3 Cl;
$R_{12}$ is H, $C_1$—$C_6$ alkyl, $C_3$—$C_4$ alkenyl, $C_5$—$C_6$ cycloalkyl,

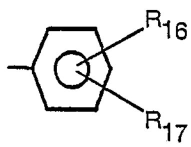 , 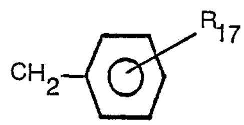

or $C_5$—$C_6$ cycloalkyl substituted with $CH_3$;

$R_{13}$ is $C_1$—$C_6$ alkyl or 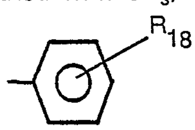 ;

$R_{14}$ and $R_{15}$ are independently H, $NO_2$, $CH_3$, Cl or $OCH_3$;
$R_{16}$ is H, F, Cl, Br, $C_1$—$C_3$ alkyl, $NO_2$, CN, $SO_2CH_3$, $OCH_3$, $SCH_3$ or $CF_3$;
$R_{17}$ is H, Cl or $C_1$—$C_3$ alkyl;
$R_{18}$ is H, $CH_3$ or Cl;
n is 0, 1 or 2;

A is 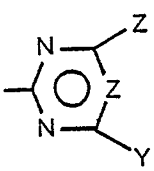 , 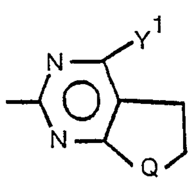 or 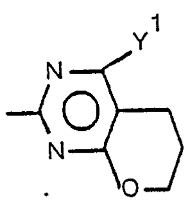 .

W is O or S;
X is H, Cl, Br, $CH_3$, $CH_2CH_3$, $C_1$—$C_3$ alkoxy, $CF_3$, $SCH_3$ or $CH_2OCH_3$;
Y is $CH_3$ or $OCH_3$;
Z is N, CH, CCl, CBr, CCN, $CCH_3$, $CCH_2CH_3$, $CCH_2CH_2Cl$ or $CCH_2CH=CH_2$;
$Y^1$ is H, $CH_3$, $OCH_3$ or $OCH_2CH_3$; and
Q is O or $CH_2$;

and their agriculturally suitable salts; provided that:
(1) when $R_1$ is Cl, then L is Cl or Br and $R_2$ is H;
(2) when n is 1, then W is O;
(3) when L is $CONR_3R_4$, then Z is CH or N;
(4) when $R_3$ is $OCH_3$, then $R_4$ is $CH_3$;
(5) the total number of carbon atoms of $R_3$ and $R_4$ is five or less;
(6) when W is S, then $R_8$ is H; and

(7) when L is $SO_2NR_3R_4$, then $R_3$ and $R_4$ are not H;
which comprises:

(a) reacting together

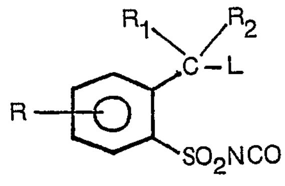

and

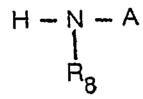

wherein

L is Cl, Br, $CO_2R_{10}$, $SO_2NR_3R_4$, $S(O)_nR_7$, $OR_9$;

n is 0 or 2;

R, $R_1$, $R_2$, $R_3$, $R_4$, $R_7$, $R_8$, $R_9$, $R_{10}$ and A are as defined above except that $R_3$, $R_4$ and $R_{10}$ cannot be H;

(b) oxidising a compound of formula (I) wherein n is 0 or 1 to a compound wherein n is 1 or 2;

(c) hydrolyzing a compound of formula (I) wherein $R_{10}$ is $C_1$—$C_4$ alkyl to a compound wherein $R_{10}$ is H;

(d) reacting a compound of formula (I) wherein L is $CO_2R_{10}$ (where $R_{10}$ is $C_1$—$C_4$ alkyl) with a dialkylaluminum-N-alkylamide derivative of general formula

$$(Alk)_2Al—NR_3R_4$$

wherein $R_3$ and $R_4$ are as defined above and Alk is an alkyl group, to obtain a compound of formula (I) wherein L is $C(O)NR_3R_4$;

(e) reacting an appropriately substituted sulfonamide of general formula

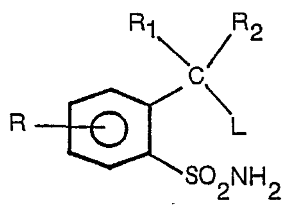

with the methylcarbamate of the appropriate aminoheterocycle, inert under the reaction conditions of general formula

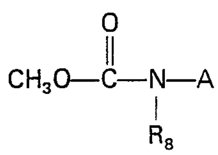

wherein R, $R_1$, $R_2$, $R_8$, L and A are groups as defined above;

(f) displacing one or both chlorine atoms in the heterocyclic moiety of a compound of general formula

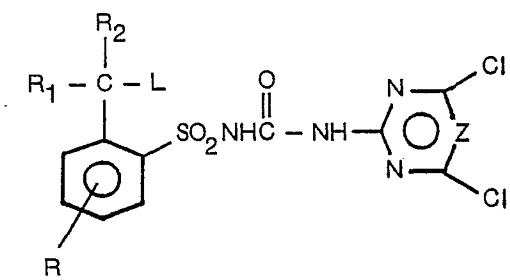

(wherein R, $R_1$, $R_2$ and L are as defined above and Z is N or CH), by alkoxy groups within the definitions of X and Y above;

(g) reacting together compounds of general formulae

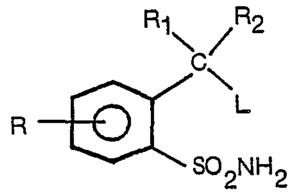

and SCN—A

wherein R, $R_1$, $R_2$, L and A are as defined above, to obtain a compound of formula (I) wherein W is S;

(h) reacting a compound of formula (I) wherein L is OH with

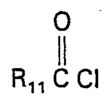

wherein R is as defined above to obtain a compound of formula (I) wherein L is $OC(O)R_{11}$;

(i) reacting a comopund of formula (I) wherein L is OH with an isocyanate of general formula

$R_{12}NCO$

wherein $R_{12}$ is as defined above, to obtain a compound of formula (I) wherein L is

$OC(O)NHR_{12}$;

(j) reacting a compound of formula (I) wherein L is OH with a chloroformate of general formula

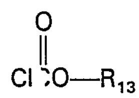

wherein R is as defined above, to obtain a compound of formula (I) wherein L is $OC(O)OR_{13}$;

(k) reducing a compound of general formula

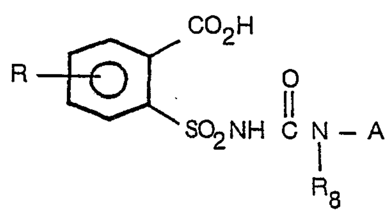

or an ester thereof to obtain a compound of formula (I) wherein $R_1$ and $R_2$ are H and R, $R_8$ and A are as defined above;

(l) reducing a ketone of general formula

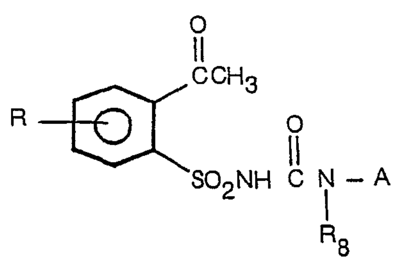

wherein R, $R_8$ and A are as defined above, to obtain a compound of formula I, wherein $R_1$ is H, $R_2$ is $CH_3$ and L is OH; or reacting said ketone with methyl lithium to obtain a compound of formula (I) wherein $R_1$ and $R_2$ are $CH_3$ and L is OH, the substituent groups containing no displaceable halogen, $NO_2$ or CN; or

(m) reacting a compound of general formula

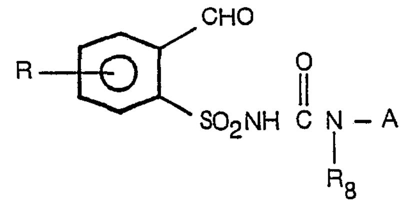

wherein R, $R_8$ and A are as defined above, with the proviso that they contain no displaceable halogens, $NO_2$ or CN, with methyl lithium to obtain a compound of formula (I) wherein $R_1$ is H, $R_2$ is $CH_3$ and L is OH.

2. A process of claim 1 wherein R, $R_1$ and $R_2$ are as defined in claim 1, W is O, $R_8$ is H, A is

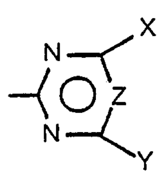

or

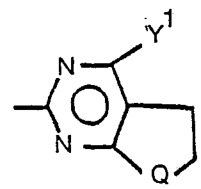

where Q, X, Y and Z are as defined in claim 1 and $Y^1$ is H, $CH_3$ or $OCH_3$; and
L is $CO_2R_{10}$, $CONR_3R_4$ or CN,
where $R_{10}$ is $C_1$—$C_4$ alkyl; and
$R_3$ and $R_4$ are independently H or $C_1$—$C_4$ alkyl.

3. A process of claim 1 wherein: R is as defined in claim 1; $R_1$ is H or $CH_3$; $R_2$ is H or $CH_3$; L is $SO_2NR_3R_4$ where $R_3$ and $R_4$ are as defined in claim 1; $R_8$ is H; W is O; and A is defined in claim 2.

4. A process of claim 1 wherein: R is as defined in claim 1; $R_1$ is H or $C_1$—$C_4$ alkyl; $R_2$ is H or $CH_3$; $R_8$ is H; L is $S(O)_nR_7$ or $OR_9$; $R_7$ and n are as defined in claim 1; $R_9$ is $C_1$—$C_4$ alkyl, $C_3$—$C_4$ alkenyl; A is as defined in claim 2; and W is O.

5. A process of claim 1 wherein R is as defined in claim 1; $R_1$ and $R_2$ are independently H or $CH_3$; $R_8$ is H; L is OH, $OC(O)R_{11}$, $OC(O)NHR_{12}$ or $OC(O)OR_{13}$; W is O; and A is as defined in claim 2.

6. A process of claim 1 wherein: R, $R_1$ and $R_2$ are as defined in claim 1; L is Cl, Br, $NR_3R_4$, $\overset{+}{N}R_3R_4R_4'$, $N(R_4)C(O)R_5$ $N(R_4)C(O)NHR_6$ or $N(R^4)C(O)R_7$; $R_8$ is H; W is O; and A is as defined in claim 2.

7. A process of any of the preceding claims wherein Z is N, CH, CCl, CBr or $CCH_3$; W is O; and $R_8$ is H or $CH_3$.

8. A process of any of claims 1 to 6 wherein L is OH; R is H; $R_1$ and $R_2$ are both $CH_3$; $R_8$ is H or $CH_3$;

A is 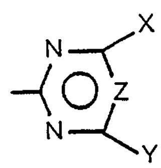 ;

Z is CH or N; X and Y are independently $CH_3$ or $OCH_3$; and W is O.

9. A process of claim 7 wherein L is Cl, Br, OH, $NR_3R_4$, $+NR_3R_4R_4'$, $CO_2R_{10}$, $S(O)_nR_7$, $OR_9$, $SO_2NR_3R_4$, $OC(O)R_{11}$, $OC(O)NHR_{12}$, $OC(O)OR_{13}$; Z is CH or N; X is $CH_3$ or $OCH_3$; R, $R_1$, $R_2$ are H; and when n is 1 or 2, Z is CH.

10. A process of claim 9 wherein

A is 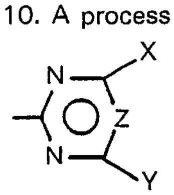 ;

$R_8$ is H; L is Cl, Br, $NR_3R_4$, $CO_2R_{10}$, $S(O)_nR_7$, $OR_9$, $SO_2NR_3R_4$, OH, $OC(O)R_{11}$, $OC(O)NHR_{12}$, $OC(O)OR_{13}$; and $R_{11}$, $R_{12}$ and $R_{13}$ are $C_1$—$C_3$ alkyl.

11. A process of claim 10 wherein L is Cl, Br, $NR_3R_4$, $CO_2R_{10}$, $S(O)_nR_7$, $OR_9$, $SO_2NR_3R_4$ or OH.

12. A process of claim 11 wherein $R_3$ is $C_1$—$C_3$ alkyl or $OCH_3$; $R_4$ is $CH_3$; $R_9$ is $C_1$—$C_4$ alkyl; and $R_{10}$ is $CH_3$ or $CH_2CH_3$;

13. A process of claim 12 wherein $R_7$ and $R_9$ are $CH_3$.

14. A process of claim 13 wherein L is $CO_2R_{10}$, $S(O)_nR_7$ or $OR_9$.

15. A process of claim 1 wherein the product is a compound selected from:
2-(Dichloromethyl)-N-[(4-methoxy-6-methylpyrimidin-2-yl)aminocarbonyl]benzenesulfonamide;
2-[[(4-methoxy-6-methylpyrimidin-2-yl)aminocarbonyl]aminosulfonyl]benzeneacetic acid, methyl ester;
2-[[(4-methoxy-6-methylpyrimidin-2-yl)aminocarbonyl]aminosulfonyl]benzeneacetic acid, ethyl ester;
N-[(4-Methoxy-6-methylpyrimidin-2-yl)aminocarbonyl-2-(methoxymethyl)benzenesulfonamide;
N-[(4-Methoxy-6-methylpyrimidin-2-yl)aminocarbonyl]-2-(methylthiomethyl)benzenesulfonamide;
N-[(4-Methoxy-6-methylpyrimidin-2-yl)aminocarbonyl]-2-(methylsulfonylmethyl)benzenesulfonamide;
N-[(4-Methoxy-6-methylpyrimidin-2-yl)aminocarbonyl]-2-(methylsulfinylmethyl)benzenesulfonamide;
2-[(Dimethylamino)sulfonylmethyl]-N-[(4-methoxy-6-methylpyrimidin-2-yl)aminocarbonyl]benzenesulfonamide;
N-[(4-methoxy-6-methylpyrimidin-2-yl)aminocarbonyl]-2-(hydroxymethyl)benzenesulfonamide;
and agriculturally suitable salts thereof.

16. A process of claim 1 wherein the product is a compound selected from:
2-(Dichloromethyl)-N-[(4,6-dimethylpyrimidin-2-yl)aminocarbonyl]benzenesulfonamide;
2-(Chloromethyl)-N-[(4,6-dimethylpyrimidin-2-yl)aminocarbonyl]benzenesulfonamide;
2-[[(4,6-dimethylpyrimidin-2-yl)aminocarbonyl]aminosulfonyl]benzeneacetic acid, methyl ester;
N-[(4,6-Dimethylpyrimidin-2-yl)aminocarbonyl]-2-methoxymethyl)benzenesulfonamide;
N-[(4,6-dimethylpyrimidin-2-yl)aminocarbonyl]-2-(methylthiomethyl)benzenesulfonamide;
N-[(4,6-Dimethylpyrimidin-2-yl)aminocarbonyl]-2-(methylsulfinylmethyl)benzenesulfonamide;
N-[(4,6-Dimethylpyrimidin-2-yl)aminocarbonyl]-2-(methylsulfonylmethyl)benzenesulfonamide;
2-[(Dimethylamino)sulfonylmethyl]-N-[(4,6-dimethylpyrimidin-2-yl)aminocarbonyl]benzenesulfonamide;
N-[(4,6-Dimethylpyrimidin-2-yl)aminocarbonyl-2-(hydroxymethyl)benzenesulfonamide;
and agriculturally suitable salts thereof.

17. A process of claim 1 wherein the product is a compound selected from:
2-(Chloromethyl)-N-[(4,6-dimethoxypyrimidin-2-yl)aminocarbonyl]benzenesulfonamide;
2-(1-Pyrrolidinylmethyl)-N-[(4,6-dimethoxypyrimidin-2-yl)aminocarbonyl]benzenesulfonamide, hydrochloride;
2-(1-Pyrrolidinylmethyl)-N-[(4,6-dimethoxypyrimidin-2-yl)aminocarbonyl]benzenesulfonamide;
2-[[(4,6-dimethoxypyrimidin-2-yl)aminocarbonyl]aminosulfonyl]benzeneacetic acid, methyl ester;
2-[[(4,6-dimethoxypyrimidin-2-yl-aminocarbonyl]aminosulfonyl]benzeneacetic acid, ethyl ester;
N-[(4,6-Dimethoxypyrimidin-2-yl)aminocarbonyl]-2-(methoxymethyl)benzenesulfonamide;
N-[(4,6-Dimethoxypyrimidin-2-yl)aminocarbonyl]-2-(methylaminocarbonylmethyl)benzenesulfonamide;
N-[(4,6-Dimethoxypyrimidin-2-yl)aminocarbonyl]-2-(methylthiomethyl)benzenesulfonamide;
N-[(4,6-Dimethoxypyrimidin-2-yl)aminocarbonyl]-2-(methylsulfonylmethyl)benzenesulfonamide;
N-[(4,6-Dimethoxypyrimidin-2-yl)aminocarbonyl]-2-(methylsulfinylmethyl)benzenesulfonamide;
N-[(4,6-dimethoxypyrimidin-2-yl)aminocarbonyl]-2-(butoxymethyl)benzenesulfonamide;
N-[(4,6-dimethoxypyrimidin-2-yl)aminocarbonyl]-2-(ethoxymethyl)benzenesulfonamide;
N-[(4,6-dimethoxypyrimidin-2-yl)aminocarbonyl]-2-(1-methylpropyloxymethyl)benzenesulfonamide;
2-[(Dimethylamino)sulfonylmethyl]-N-[(4,6-dimethoxypyrimidin-2-yl)aminocarbonyl]benzenesulfonamide;
N-[(4,6-Dimethoxypyrimidin-2-yl)aminocarbonyl]-2-(hydroxymethyl)benzenesulfonamide;
and agriculturally suitable salts thereof.

18. A process of claim 1 wherein the product is a compound selected from:
2-(Dichloromethyl)-N-[(4-methoxy-6-methyl-1,3,5-triazin-2-yl)aminocarbonyl]benzenesulfonamide;
2-[[(4-methoxy-6-methyl-1,3,5-triazin-2-yl)aminocarbonyl]aminosulfonyl]benzeneacetic acid, methyl ester;
N-[(4-Methoxy-6-methyl-1,3,5-triazin-2-yl)aminocarbonyl]-2-(methoxymethyl)benzenesulfonamide;
N-[(4-Methoxy-6-methyl-1,3,5-triazin-2-yl)aminocarbonyl]-2-(methylthiomethyl)benzenesulfonamide;
2-[(Dimethylamino)sulfonylmethyl]-N-[(4-methoxy-6-methyl-1,3,5-triazin-2-yl)aminocarbonyl]-benzenesulfonamide;
N-[(4-Methoxy-6-methyl-1,3,5-triazin-2-yl)aminocarbonyl]-2-(hydroxymethyl)benzenesulfonamide;
and agriculturally suitable salts thereof.

19. A process of claim 1 wherein the product is a compound selected from:

2-(Chloromethyl)-N-[(4,6-dimethoxy-1,3,5-triazin-2-yl)aminocarbonyl]benzenesulfonamide;
2-(1-Chloroethyl)-N-[(4,6-dimethoxy-1,3,5-triazin-2-yl)aminocarbonyl]benzenesulfonamide;
2-[[(4,6-dimethoxy-1,3,5-triazin-2-yl)aminocarbonyl]aminosulfonyl]benzeneacetic acid, methyl ester;
N-[(4,6-Dimethoxy-1,3,5-triazin-2-yl)aminocarbonyl]-2-(methoxymethyl)benzenesulfonamide;
N-[(4,6-Dimethoxy-1,3,5-triazin-2-yl)aminocarbonyl]-2-(methylthiomethyl)benzenesulfonamide;
2-[(Dimethylamino)sulfonylmethyl]-N-[(4,6-dimethoxy-1,3,5-triazin-2-yl)aminocarbonyl]benzenesulfonamide;
N-[(4,6-Dimethoxy-1,3,5-triazin-2-yl)aminocarbonyl]-2-(hydroxymethyl)benzenesulfonamide;
and agriculturally suitable salts thereof.

20. A process of claim 1 wherein the product is a compound selected from:
N-[(4,6-Dimethyl-1,3,5-triazin-2-yl)aminocarbonyl]-2-(methoxymethyl)benzenesulfonamide;
N-[(4,6-Dimethyl-1,3,5-triazin-2-yl)aminocarbonyl]-2-(methylthiomethyl)benzenesulfonamide;
2-[(Dimethylamino)sulfonylmethyl]-N-[(4,6-dimethyl-1,3,5-triazin-2-yl)aminocarbonyl]benzenesulfonamide;
N-[(4,6-Dimethyl-1,3,5-triazin-2-yl)aminocarbonyl]-2-(hydroxymethyl)benzenesulfonamide;
and agriculturally suitable salts thereof.

21. A method of controlling the growth of undesired vegtation by applying to the locus of such vegetation an effective amount of a herbicidal compound, characterised in that said herbicidal compound comprises a compound of formula (I) as defined in any of claims 1 to 20.

22. A method for regulating the growth of plants by applying to the locus of said plants an effective but substantially non-phytotoxic amount of a plant growth regulant, characterised in that said plant growth regulant comprises a compound of formula (I) as defined in any of claims 1 to 20.

23. A process for the production of a compound selected from

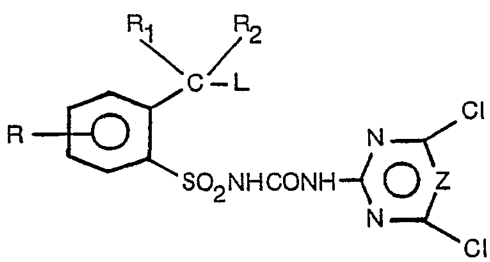

(II)

wherein
R, $R_1$, and $R_2$ are as previously defined in claim 1;
Z is CH or N;
n is 0 or 2; and
L is as previously defined in claim 1,
provided that:
(1) L is not OH;
(2) when $R_3$ is $OCH_3$, then $R_4$ is $CH_3$ and that $R_{10}$ cannot be H;
(3) when $R_1$ is Cl, then L is Cl or Br and $R_2$ is H;
(4) when L is $NR_3R_4$, $NR_3R_4R_4$, or $SO_2NR_3R_4$, then $R_3$ or $R_4$ cannot be H;
(5) the total number of carbon atoms of $R_3$ and $R_4$ is five or less;
which comprises reacting an aromatic sulfonamide of formula

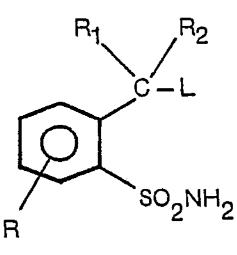

(V)

with a heterocyclic isocyanate of formula

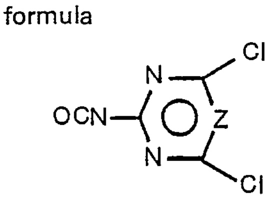

wherein R, $R_1$, $R_2$, Z and L are as defined above.

24. A process for the production of a compound selected from

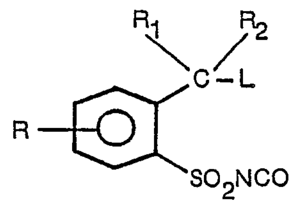

(III)

wherein

R, $R_1$, $R_2$, $R_7$ and $R_9$ are as previously defined in claim 1;

n is 0 or 2;

L is Cl, Br, $CO_2R_{10}$, $SO_2NR_3R_4$, $S(O)_nR_7$ or $OR_9$,

provided that:

(1) when $R_1$ is Cl, then L is Cl or Br and $R_2$ is H;

(2) when L is $SO_2NR_3R_4$, then $R_3$ and $R_4$ cannot be H;

(3) when $R_3$ is $OCH_3$, then $R_4$ is $CH_3$;

(4) $R_{10}$ cannot be H; and

(5) n is 0 or 2.

which comprises

(a) reacting a sulfonamide of formula

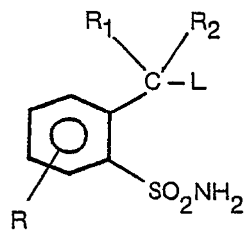

(V)

or a urea of formula

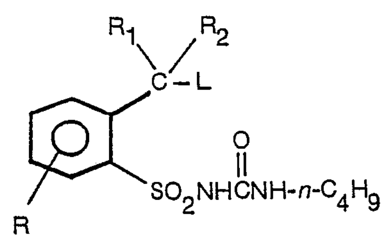

(VI)

with phosgene; or

(b) reacting a compound of formula

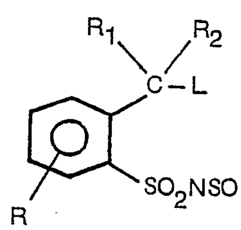

with phosgene;

R, $R_1$, $R_2$ and L being as defined above.

**Patentansprüche für die Vertragsstaaten: BE CH DE FR GB IT LI LU NL SE**

1. Verbindung ausgewählt aus

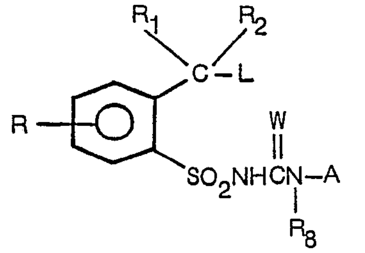

(I)

worin

L $CO_2R_{10}$, $CONR_3R_4$, CN, Cl, Br, $NR_3R_4$, $\overset{+}{N}R_3R_4R_4'$, $N(R_4)C(O)R_5$, $N(R_4)C(O)NHR_6$, $N(R_4)C(O)OR_7$, $S(O)_nR_7$, $OR_9$, $SO_2NR_3R_4$, OH, $OC(O)R_{11}$, $OC(O)NHR_{12}$ oder $OC(O)OR_{13}$ ist;

R H, F, Cl, Br, $NO_2$, $CF_3$, $C_1$—$C_3$-Alkyl oder $C_1$—$C_3$-Alkoxy ist;

$R_1$ H, Cl oder $C_1$—$C_4$-Alkyl ist;

$R_2$ H oder $CH_3$ ist;

$R_3$ H, $C_1$—$C_4$-Alkyl oder $OCH_3$ ist;

$R_4$ H oder $C_1$—$C_4$-Alkyl ist;

$R_3$ und $R_4$ zusammengenommen werden können zur Bildung von $-(CH_2)_4-$, $-(CH_2)_5-$ oder $-(CH_2CH_2)_2O$;

$R_4'$ H, $CH_3$ oder $CH_2CH_3$ ist;

$R_5$ $C_1$—$C_4$-Alkyl, gegebenenfalls substituiert mit 1—3 Atomen von F, Cl oder Br, oder $C_3$—$C_4$-Alkyenyl ist;

$R_6$ H, $C_1$—$C_4$-Alkyl oder $C_3$—$C_4$-Alkenyl ist;

$R_7$ $C_1$—$C_4$-Alkyl ist;

$R_8$ H, $CH_3$ oder $OCH_3$ ist;

$R_9$ $C_1$—$C_6$-Alkyl, $C_3$—$C_4$-Alkenyl, $CH_2CF_3$, $CF_2CF_2H$ oder $C_5$—$C_6$-Cycloalkyl ist;

$R_{10}$ H, $C_1$—$C_4$-Alkyl, $C_3$—$C_4$-Alkenyl, $CH_2CH_2Cl$ oder $CH_2CH_2OCH_3$ ist;

$R_{11}$ H, $C_1$—$C_5$-Alkyl, $C_2$—$C_3$-Alkenyl, $C_2$—$C_3$-Alkinyl, $C_3$—$C_4$-Cycloalkyl,

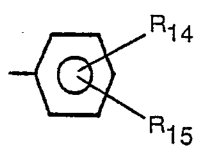

,

$C_1$—$C_4$-Alkyl substituiert mit 1—4 Substituenten, ausgewählt aus 0—3 F, 0—3 Cl oder 0—3 Br, oder $C_2$—$C_3$-Alkenyl, substituiert mit 1—3 Cl, ist;

$R_{12}$ H, $C_1$—$C_6$-Alkyl, $C_3$—$C_4$-Alkenyl, $C_5$—$C_6$-Cycloalkyl,

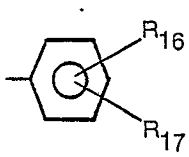

,

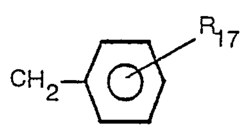

oder $C_5$—$C_6$-Cycloalkyl, substituiert mit $CH_3$, ist;

$R_{13}$ $C_1$—$C_6$-Alkyl oder 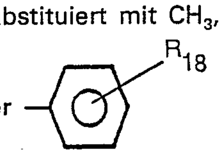 ;

ist;

$R_{14}$ und $R_{15}$ unabhängig H, $NO_2$, $CH_3$, Cl oder $OCH_3$ sind;

$R_{16}$ H, F, Cl, Br, $C_1$—$C_3$-Alkyl, $NO_2$, CN, $SO_2CH_3$, $OCH_3$, $SCH_3$ oder $CF_3$ ist;

$R_{17}$ H, Cl oder $C_1$—$C_3$-Alkyl ist;

$R_{18}$ H, $CH_3$ oder Cl ist;

n 0, 1 oder 2 ist;

A 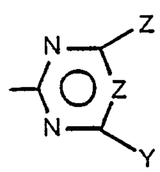 , 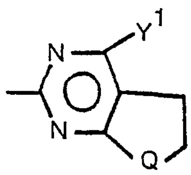 oder 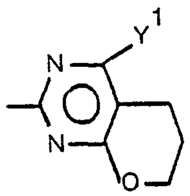

ist;

W O oder S ist;

X H, Cl, Br, $CH_3$, $CH_2CH_3$, $C_1$—$C_3$-Alkoxy, $CF_3$, $SCH_3$ oder $CH_2OCH_3$ ist;

Y $CH_3$ oder $OCH_3$ ist;

Z N, CH, CCl, CBr, CCN, $CCH_3$, $CCH_2CH_3$, $CCH_2CH_2Cl$ oder $CCH_2CH{=}CH_2$ ist;

$Y^1$ H, $CH_3$, $OCH_3$ oder $OCH_2CH_3$ ist; und

Q O oder $CH_2$ ist;

und ihre landwirtschaftlich brauchbaren Salze; vorausgesetzt daß:

(1) wenn $R_1$ Cl ist, dann L Cl oder Br ist und $R_2$ H ist;

(2) wenn n 1 ist, dann W O ist;

(3) wenn L $CONR_3R_4$ ist, dann Z CH oder N ist;

(4) wenn $R_3$ $OCH_3$ ist, dann $R_4$ $CH_3$ ist;

(5) die Gesamtanzahl der Kohlenstoffatome von $R_3$ und $R_4$ 5 oder weniger ist;

(6) wenn W S ist, dann $R_8$ H ist; und

(7) wenn L $SO_2NR_3R_4$ ist, dann $R_3$ und $R_4$ nicht H sind.

2. Verbindung nach Anspruch 1, worin Z, N, CH, CCl, CBr oder $CCH_3$ ist; W O ist; und $R_8$ H oder $CH_3$ ist.

3. Verbindung nach Anspruch 1, worin L OH ist; R H ist; $R_1$ und $R_2$ beide $CH_3$ sind; $R_8$ H oder $CH_3$ ist;

A 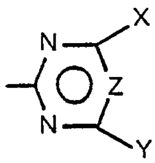 ;

ist; Z CH oder N ist; X und Y unabhängig $CH_3$ oder $OCH_3$ sind; und W O ist.

4. Verbindung nach Anspruch 2, worin L Cl, Br, OH, $NR_3R_4$, $+NR_3R_4R_4'$, $CO_2R_{10}$, $S(O)_nR_7$, $OR_9$, $SO_2NR_3R_4$, $OC(O)R_{11}$, $OC(O)NHR_{12}$, $OC(O)OR_{13}$ ist; Z CH oder N ist; X $CH_3$ oder $OCH_3$ ist; R, $R_1$, $R_2$ H sind; und wenn n 1 oder 2 ist, Z CH ist.

5. Verbindung nach Anspruch 4,

worin A 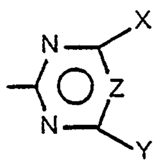 ;

ist; $R_8$ H ist; L Cl, Br, $NR_3R_4$, $CO_2R_{10}$, $S(O)_nR_7$, $OR_9$, $SO_2NR_3R_4$, OH, $OC(O)R_{11}$, $OC(O)NHR_{12}$, $OC(O)OR_{13}$ ist; und $R_{11}$, $R_{12}$ und $R_{13}$ $C_1$—$C_3$-Alkyl sind.

6. Verbindung nach Anspruch 5, worin L, Cl, Br, $NR_3R_4$, $CO_2R_{10}$, $S(O)_nR_7$, $OR_9$, $SO_2NR_3R_4$ oder OH ist.

7. Verbindung nach Anspruch 6, worin $R_3$ $C_1$—$C_3$-Alkyl oder $OCH_3$ ist; $R_4$ $CH_3$ ist; $R_9$ $C_1$—$C_4$-Alkyl ist; und $R_{10}$ $CH_3$ oder $CH_2CH_3$ ist.

8. Verbindung nach Anspruch 7, worin $R_7$ und $R_9$ $CH_3$ sind.

9. Verbindung nach Anspruch 8, worin L $CO_2R_{10}$, $S(O)_nR_7$ oder $OR_9$ ist.

10. Verbindung nach Anspruch 1, ausgewählt aus:

2-(Dichlormethyl)-N-[(4-methoxy-6-methylpyrimidin-2-yl)-aminocarbonyl]-benzolsulfonamid;

2-[[(4-Methoxy-6-methylpyrimidin-2-yl)-aminocarbonyl] - aminosulfonyl] - benzol - essigsäure-methylester;

2-[[(4-Methoxy-6-methylpyrimidin-2-yl)-aminocarbonyl]-aminosulfonyl]-benzol-essigsäure-ethylester;

N-[(4-Methoxy-6-methylpyrimidin-2-yl)-aminocarbonyl]-2-(methoxymethyl)-benzolsulfonamid;

N-[(4-Methoxy-6-methylpyrimidin-2-yl)-aminocarbonyl]-2-(methylthiomethyl)-benzolsulfonamid;

N-[(4-Methoxy-6-methylpyrimidin-2-yl)-aminocarbonyl]-2-(methylsulfonylmethyl)-benzolsulfonamid;

N-[(4-Methoxy-6-methylpyrimidin-2-yl)-aminocarbonyl]-(methylsulfinylmethyl)-benzolsulfonamid;

2-[(Dimethylamino)-sulfonylmethyl]-N-[(4-methoxy-6-methylpyrimidin-2-yl)-aminocarbonyl]-benzolsulfonamid;
N-[(4-Methoxy-6-methylpyrimidin-2-yl)-aminocarbonyl]-2-(hydroxymethyl)-benzolsulfonamid;
und die landwirtschaftlich brauchbaren Salze davon.

11. Verbindung nach Anspruch 1, ausgewählt aus:
2-(Dichlormethyl)-N-[4,6-dimethylpyrimidin-2-yl)-aminocarbonyl]-benzolsulfonamid;
2-(Chlormethyl)-N-[(4,6-dimethylpyrimidin-2-yl)-aminocarbonyl]-benzolsulfonamid;
2-[[(4,6-Dimethylpyrimidin-2-yl)-aminocarbonyl]-aminosulfonyl]-benzol-essigsäure-methylester;
N-[(4,6-Dimethylpyrimidin-2-yl)-aminocarbonyl]-2-(methoxymethyl)-benzolsulfonamid;
N-[(4,6-Dimethylpyrimidin-2-yl)-aminocarbonyl]-2-(methylthiomethyl)-benzolsulfonamid;
N-[(4,6-Dimethylpyrimidin-2-yl)-aminocarbonyl]-2-(methylsulfinylmethyl)-benzolsulfonamid;
N-[(4,6-Dimethylpyrimidin-2-yl)-aminocarbonyl]-2-(methylsulfonylmethyl)-benzolsulfonamid;
2-[(Dimethylamino)-sulfonylmethyl]-N-[(4,6-dimethylpyrimidin-2-yl)-aminocarbonyl]-benzolsulfonamid;
N-[(4,6-Dimethylpyrimidin-2-yl)-aminocarbonyl]-2-(hydroxymethyl)-benzolsulfonamid;
und die landwirtschaftlich brauchbaren Salze davon.

12. Verbindung nach Anspruch 1, ausgewählt aus:
N-[(4,6-Dimethoxypyrimidin-2-yl)-aminocarbonyl]-2-(methylamino-carbonylmethyl)-benzolsulfonamid;
2-(Chlormethyl)-N-[(4,6-dimethoxypyrimidin-2-yl)-aminocarbonyl]-benzolsulfonamid;
2-(1-Pyrrolidinylmethyl)-N-[4,6-dimethoxypyrimidin-2-yl)-aminocarbonyl]-benzolsulfonamid-hydrochlorid;
2-(1-Pyrrolidinylmethyl)-N-[(4,6-dimethoxypyrimidin-2-yl)-aminocarbonyl]-benzolsulfonamid;
2-[[(4,6-Dimethoxypyrimidin-2-yl)-aminocarbonyl]-aminosulfonyl]-benzol-essigsäure-methylester;
2-[[(4,6-Dimethoxypyrimidin-2-yl)-aminocarbonyl]-aminosulfonyl]-benzol-essigsäure-ethylester;
N-[(4,6-Dimethoxypyrimidin-2-yl)-aminocarbonyl]-2-(methoxymethyl)-benzolsulfonamid;
N-[(4,6-Dimethoxypyrimidin-2-yl)-aminocarbonyl]-2-(methylthiomethyl)-benzolsulfonamid;
N-[(4,6-Dimethoxypyrimidin-2-yl)-aminocarbonyl]-2-(methylsulfonylmethyl)-benzolsulfonamid;
N-[(4,6-Dimethoxypyrimidin-2-yl)-aminocarbonyl]-2-(methylsulfinylmethyl)-benzolsulfonamid;
N-[(4,6-Dimethoxypyrimidin-2-yl)-aminocarbonyl]-2-(butoxymethyl)-benzolsulfonamid;
N-[(4,6-Dimethoxypyrimidin-2-yl)-aminocarbonyl]-2-(ethoxymethyl)-benzolsulfonamid;
N-[(4,6-Dimethoxypyrimidin-2-yl)-aminocarbonyl]-2-(1-methylpropyloxymethyl)-benzolsulfonamid;
2-[(Dimethylamino)-sulfonylmethyl]-N-[(4,6-dimethoxypyrimidin-2-yl)-aminocarbonyl]-benzolsulfonamid;
N-[(4,6-Dimethoxypyrimidin-2-yl)-aminocarbonyl]-2-(hydroxymethyl)-benzolsulfonamid;
und die landwirtschaftlich brauchbaren Salze davon.

13. Verbindung nach Anspruch 1, ausgewählt aus:
2-(Dichlormethyl)-N-[(4-methoxy-6-methyl-1,3,5-triazin-2-yl)-aminocarbonyl]-benzolsulfonamid;
2-[[(4-Methoxy-6-methyl-1,3,5-triazin-2-yl)-aminocarbonyl]-aminosulfonyl]-benzol-essigsäure-methylester;
N-[(4-Methoxy-6-methyl-1,3,5-triazin-2-yl)-aminocarbonyl]-2-(methoxymethyl)-benzolsulfonamid;
N-[(4-Methoxy-6-methyl-1,3,5-triazin-2-yl)-aminocarbonyl]-2-(methylthiomethyl)-benzolsulfonamid;
2-[(Dimethylamino)-sulfonylmethyl]-N-[(4-methoxy-6-methyl-1,3,5-triazin-2-yl)-aminocarbonyl]-benzolsulfonamid;
N-[(4-Methoxy-6-methyl-1,3,5-triazin-2-yl)-aminocarbonyl]-2-(hydroxymethyl)-benzolsulfonamid;
und die landwirtschaftlich brauchbaren Salze davon.

14. Verbindung nach Anspruch 1, ausgewählt aus:
2-(Chlormethyl)-N-[(4,6-dimethoxy-1,3,5-triazin-2-yl)-aminocarbonyl]-benzolsulfonamid;
2-(1-Chlorethyl)-N-[(4,6-dimethoxy-1,3,5-triazin-2-yl)-aminocarbonyl]-benzolsulfonamid;
2-[[(4,6-Dimethoxy-1,3,5-triazin-2-yl)-aminocarbonyl]-aminosulfonyl]-benzol-essigsäure-methylester;
N-[(4,6-Dimethoxy-1,3,5-triazin-2-yl)-aminocarbonyl]-2-(methoxymethyl)-benzosulfonamid;
N-[(4,6-Dimethoxy-1,3,5-triazin-2-yl)-aminocarbonyl]-2-(methylthiomethyl)-benzolsulfonamid;
2-[(Dimethylamino)-sulfonylmethyl]-N-[(4,6-dimethoxy-1,3,5-triazin-2-yl)-aminocarbonyl]-benzolsulfonamid;
N-[(4,6-Dimethoxy-1,3,5-triazin-2-yl)-aminocarbonyl]-2-(hydroxymethyl)-benzolsulfonamid;
und die landwirtschaftlich brauchbaren Salze davon.

15. Verbindung nach Anspruch 1, ausgewählt aus:
N-[(4,6-Dimethyl-1,3,5-triazin-2-yl)-aminocarbonyl]-2-(methoxymethyl)-benzolsulfonamid;
N-[(4,6-Dimethyl-1,3,5-triazin-2-yl)-aminocarbonyl]-2-(methylthiomethyl)-benzolsulfonamid;
2-[(Dimethylamino)-sulfonylmethyl]-N-[(4,6-dimethyl-1,3,5-triazin-2-yl)-aminocarbonyl]-benzolsulfonamid;
N-[(4,6-Dimethyl-1,3,5-triazin-2-yl)-aminocarbonyl]-2-(hydroxymethyl)-benzolsulfonamid;
und die landwirtschaftlich brauchbaren Salze davon.

16. Verbindung nach Anspruch 1, worin R, $R_1$ und $R_2$ wie in Anspruch 1 definiert sind, W O ist, $R_8$ H ist, A

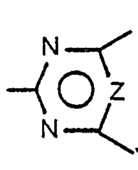

oder

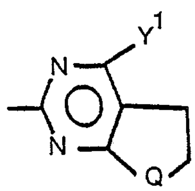

worin Q, X, Y und Z wie in Anspruch 1 definiert sind und
$Y^1$ H, $CH_3$ oder $OCH_3$ ist; und
L $CO_2R_{10}$, $CONR_3R_4$ oder CN ist, worin $R_{10}$ $C_1$—$C_4$-Alkyl ist; und
$R_3$ und $R_4$ unabhängig H oder $C_1$—$C_4$-Alkyl sind.

17. Verbindung nach Anspruch 1, worin
R wie in Anspruch 1 definiert ist; $R_1$ H oder $CH_3$ ist; $R_2$ H oder $CH_3$ ist; L $SO_2NR_3R_4$ ist, worin $R_3$ und $R_4$ wie in Anspruch 1 definiert sind; $R_8$ H ist; W O ist; und A wie in Anspruch 16 definiert ist.

18. Verbindung nach Anspruch 1, worin R wie in Anspruch 1 definiert ist; $R_1$ H oder $C_1$—$C_4$-Alkyl ist; $R_2$ H oder $CH_3$ ist; $R_8$ H ist; L $S(O)_nR_7$ oder $OR_9$ ist; $R_7$ und n wie in Anspruch 1 definiert sind; $R_9$ $C_1$—$C_4$-Alkyl, $C_3$—$C_4$-Alkenyl ist; A wie in Anspruch 16 definiert ist; und W O ist.

19. Verbindung nach Anspruch1, worin R wie in Anspruch 1 definiert ist; $R_1$ und $R_2$ unabhängig H oder $CH_3$ sind; $R_8$ H ist; L OH, $OC(O)R_{11}$, $OC(O)NHR_{12}$ oder $OC(O)OR_{13}$ ist; W O ist; und A wie in Anspruch 16 definiert ist.

20. Verbindung nach Anspruch 1, worin R, $R_1$ und $R_2$ wie in Anspruch 1 definiert sind; L Cl, Br, $NR_3R_4$, $\overset{+}{N}R_3R_4R_4'$, $N(R_4)C(O)R_5$, $N(R_4)C(O)NHR_6$ oder $N(R_4)C(O)R_7$, ist; $R_8$ H ist; W O ist; und A wie in Anspruch 16 definiert ist.

21. Verbindung ausgewählt aus

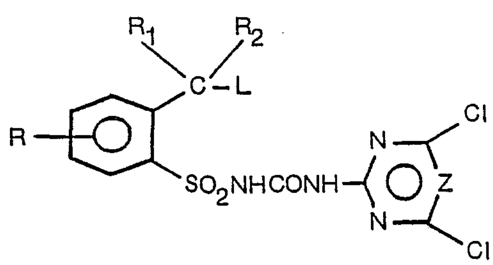

(II)

worin R,
$R_1$ und $R_2$ wie vorstehend in Anspruch 1 definiert sind;
Z CH oder N ist;
n O oder 2 ist; und
L wie vorstehend in Anspruch 1 definiert ist,
vorausgesetzt daß:
(1) L nicht OH ist;
(2) wenn $R_3$ $OCH_3$ ist, dann $R_4$ $CH_3$ ist und daß $R_{10}$ nicht H sein kann;
(3) wenn $R_1$ Cl ist, dann L Cl oder Br ist und $R_2$ H ist;
(4) wenn L $NR_3R_4$, $NR_3R_4R_4'$ oder $SO_2NR_3R_4$ ist, dann $R_3$ oder $R_4$ nicht H sein können;
(5) die Gesamtanzahl der Kohlenstoffatome von $R_3$ und $R_4$ 5 oder weniger ist.

22. Verbindung ausgewählt aus

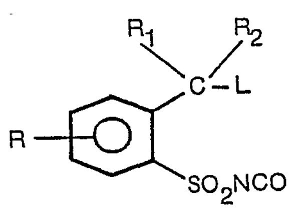

(III)

worin

$R, R_1, R_2, R_7$ und $R_9$ wie vorstehend in Anspruch 1 definiert sind;
n 0 oder 2 ist;
L Cl, Br, $CO_2R_{10}$, $SO_2NR_3R_4$, $S(O)_nR_7$ oder $OR_9$ ist,
vorausgesetzt daß:
(1) wenn $R_1$ Cl ist, dann L Cl oder Br ist und $R_2$ H ist;
(2) wenn L $SO_2NR_3R_4$ ist, dann $R_3$ und $R_4$ nicht H sein können;
(3) wenn $R_3$ $OCH_3$ ist, dann $R_4$ $CH_3$ ist;
(4) $R_{10}$ nicht H sein kann; und
(5) n 0 oder 2 ist.

23. Zusammensetzung geeignet zur Bekämpfung des Wachstums unerwünschter Vegetation, die ein wirksame Menge einer herbiziden Zusammensetzung und mindestens eines der folgenden enthält: oberflächenaktives Mittel, festes oder flüssiges Verdünnungsmittel, dadurch gekennzeichnet, daß die herbizide Verbindung eine Verbindung gemäß einem der Ansprüche 1 bis 20 umfaßt.

24. Verfahren zur Bekämpfung des Wachstums unerwünschter Vegetation durch Auftrag auf den Ort einer solchen Vegetation von einer wirksamen Menge einer herbiziden Verbindung, dadurch gekennzeichnet, daß die herbizide Verbindung eine Verbindung gemäß einem der Ansprüche 1 bis 20 umfaßt.

25. Verfahren zur Regulierung des Pflanzenwachstums durch Auftrag auf den Ort solcher Pflanzen von einer wirksamen, jedoch im wesentlichen nicht-phytotoxischen, Menge eines das Pflanzenwachstum regulierenden Mittels, dadurch gekennzeichnet, daß das das Pflanzenwachstum regulierende Mittel, eine Verbindung gemäß einem der Ansprüche 1 bis 20 umfaßt.

26. Verfahren zur Herstellung einer Verbindung nach Anspruch 1, durch Reaktion von

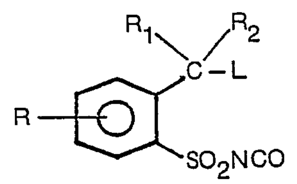

und

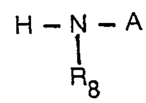

miteinander,
worin
L Cl, Br, $CO_2R_{10}$, $SO_2NR_3R_4$, $S(O)_nR_7$, $OR_9$ ist;
n 0 oder 2 ist;
$R, R_1, R_2, R_3, R_4, R_7, R_8, R_9, R_{10}$ und A wie in Anspruch 1 definiert sind, mit der Ausnahme, daß $R_3$, $R_4$ und $R_{10}$ nicht H sein können.

## Patentansprüche für den Vertragsstaat: AT

1. Verfahren zur Herstellung einer Verbindung ausgewählt aus

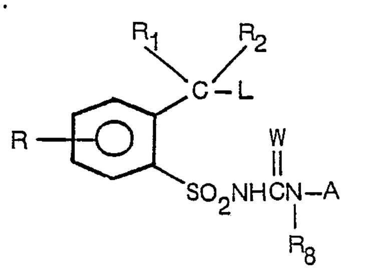

(I)

worin
L $CO_2R_{10}$, $CONR_3R_4$, CN, Cl, Br, $NR_3R_4$, $\overset{+}{N}R_3R_4R_4'$, $N(R_4)C(O)R_5$, $N(R_4)C(O)NHR_6$, $N(R_4)C(O)OR_7$, $S(O)_nR_7$, $OR_9$, $SO_2NR_3R_4$, OH, $OC(O)R_{11}$, $OC(O)NHR_{12}$ oder $OC(O)OR_{13}$ ist;
R H, F, Cl, Br, $NO_2$, $CF_3$, $C_1$—$C_3$-Alkyl oder $C_1$—$C_3$-Alkoxy ist;
$R_1$ H, Cl oder $C_1$—$C_4$-Alkyl ist;
$R_2$ H oder $CH_3$ ist;
$R_3$ H, $C_1$—$C_4$-Alkyl oder $OCH_3$ ist;
$R_4$ H oder $C_1$—$C_4$-Alkyl ist;
$R_3$ und $R_4$ zusammengenommen werden können zur Bildung von $-(CH_2)_4-$, $-(CH_2)_5-$ oder $-(CH_2CH_2)_2O$;
$R_4'$ H, $CH_3$ oder $CH_2CH_3$ ist;

$R_5$ $C_1$—$C_4$-Alkyl, gegebenenfalls substituiert mit 1—3 Atomen von F, Cl oder Br, oder $C_3$—$C_4$-Alkyenyl ist;

$R_6$ H, $C_1$—$C_4$-Alkyl oder $C_3$—$C_4$-Alkenyl ist;

$R_7$ $C_1$—$C_4$-Alkyl ist;

$R_8$ H, $CH_3$ oder $OCH_3$ ist;

$R_9$ $C_1$—$C_6$-Alkyl, $C_3$—$C_4$-Alkenyl, $CH_2CF_3$, $CF_2CF_2H$ oder $C_5$—$C_6$-Cycloalkyl ist;

$R_{10}$ H, $C_1$—$C_4$-Alkyl, $C_3$—$C_4$-Alkenyl, $CH_2CH_2Cl$ oder $CH_2CH_2OCH_3$ ist;

$R_{11}$ H, $C_1$—$C_5$-Alkyl, $C_2$—$C_3$-Alkenyl, $C_2$—$C_3$-Alkinyl, $C_3$—$C_4$-Cycloalkyl,

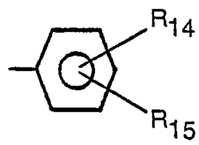,

$C_1$—$C_4$-Alkyl substituiert mit 1—4 Substituenten, ausgewählt aus 0—3 F, 0—3 Cl oder 0—3 Br, oder $C_2$—$C_3$-Alkenyl, substituiert mit 1—3 Cl, ist;

$R_{12}$ H, $C_1$—$C_6$-Alkyl, $C_3$—$C_4$-Alkenyl, $C_5$—$C_6$-Cycloalkyl,

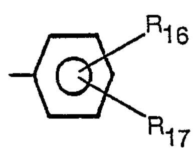, 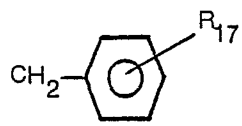

oder $C_5$—$C_6$-Cycloalkyl, substituiert mit $CH_3$, ist;

$R_{13}$ $C_1$—$C_6$-Alkyl oder 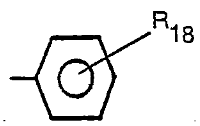;

ist;

$R_{14}$ und $R_{15}$ unabhängig H, $NO_2$, $CH_3$, Cl oder $OCH_3$ sind;

$R_{16}$ H, F, Cl, Br, $C_1$—$C_3$-Alkyl, $NO_2$, CN, $SO_2CH_3$, $OCH_3$, $SCH_3$ oder $CF_3$ ist;

$R_{17}$ H, Cl oder $C_1$—$C_3$-Alkyl ist;

$R_{18}$ H, $CH_3$ oder Cl ist;

n 0, 1 oder 2 ist;

A 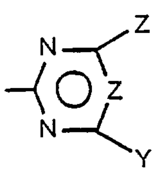, 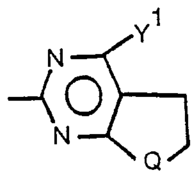 oder 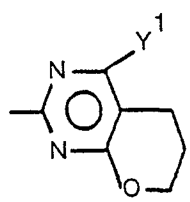

ist;

W O oder S ist;

X H, Cl, Br, $CH_3$, $CH_2CH_3$, $C_1$—$C_3$-Alkoxy, $CF_3$, $SCH_3$ oder $CH_2OCH_3$ ist;

Y $CH_3$ oder $OCH_3$ ist;

Z N, CH, CCl, CBr, CCN, $CCH_3$, $CCH_2CH_3$, $CCH_2CH_2Cl$ oder $CCH_2CH{=}CH_2$ ist;

$Y^1$ H, $CH_3$, $OCH_3$ oder $OCH_2CH_3$ ist; und

Q O oder $CH_2$ ist;

und ihrer landwirtschaftlich brauchbaren Salze; vorausgesetzt daß:

(1) wenn $R_1$ Cl ist, dann L Cl oder Br ist und $R_2$ H ist;

(2) wenn n 1 ist, dann W O ist;

(3) wenn L $CONR_3R_4$ ist, dann Z CH oder N ist;

(4) wenn $R_3$ $OCH_3$ ist, dann $R_4$ $CH_3$ ist;

(5) die Gesamtanzahl der Kohlenstoffatome von $R_3$ und $R_4$ 5 oder weniger ist;

(6) wenn W S ist, dann $R_8$ H ist; und

(7) wenn L $SO_2NR_3R_4$ ist, dann $R_3$ und $R_4$ nicht H sind;
durch

(a) Reaktion von

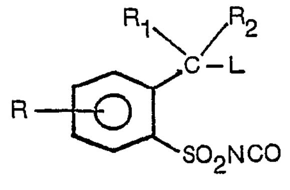

und

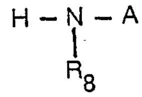

miteinander,
worin

L Cl, Br, $CO_2R_{10}$, $SO_2NR_3R_4$, $S(O)_nR_7$, $OR_9$ ist;

n 0 oder 2 ist;

R, $R_1$, $R_2$, $R_3$, $R_4$, $R_7$, $R_8$, $R_9$, $R_{10}$ und A wie vorstehend definiert sind, mit der Ausnahme, daß $R_3$, $R_4$ und $R_{10}$ nicht H sein können;

(b) Oxidation einer Verbindung der Formel I, worin n 0 oder 1 ist, zu einer Verbindung, worin n 1 oder 2 ist;

(c) Hydrolyse einer Verbindung der Formel I, worin $R_{10}$ $C_1$—$C_4$-Alkyl ist, zu einer Verbindung, worin $R_{10}$ H ist;

(d) Reaktion einer Verbindung der Formel I, worin L $CO_2R_{10}$ ist (worin $R_{10}$ $C_1$—$C_4$-Alkyl ist) mit einem Dialkylaluminium-N-alkylamidderivat der allgemeinen Formel

$$(Alk)_2Al—NR_3R_4$$

worin $R_3$ und $R_4$ wie vorstehend definiert sind und Alk eine Alkylgruppe ist, zur Erzielung einer Verbindung der Formel I, worin L $(C(O)NR_3R_4$ ist;

(e) Reaktion eines geeignet substituierten Sulfonamids der allgemeinen Formel

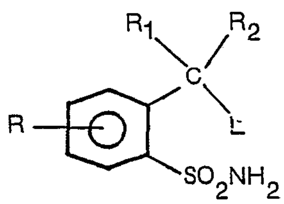

mit dem Methylcarbamat des entsprechenden Aminoheterocyclus, der unter den Reaktionsbedingungen inder ist, mit der allgemeinen Formel

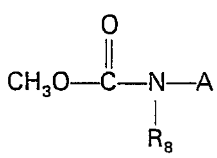

worin R, $R_1$, $R_2$, $R_8$, L und A Gruppen, wie vorstehend definiert sind;

(f) Ersatz eines oder beider Chloratome in dem heterocyclischen Rest einer Verbindung der allgemeinen Formel

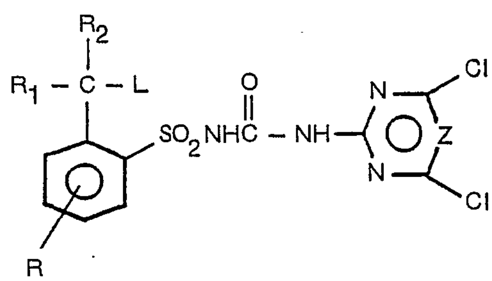

(worin R, $R_1$, $R_2$ und L wie vorstehend definiert sind und Z N oder CH ist), durch Alkoxygruppen, innerhalb der vorstehenden Definitionen von X und Y;

(g) Reaktion von Verbindungen der allgemeinen Formeln

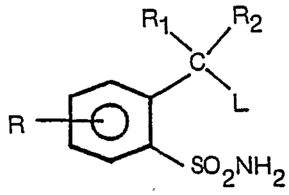 und SCN—A

miteinander, worin R, $R_1$, $R_2$, L und A wie vorstehend definiert sind, unter Erzeilung einer Verbindung der Formel I, worin W S ist;

(h) Reaktion einer Verbindung der Formel I, worin L OH ist mit

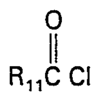

worin R wie vorstehend definiert ist, unter Erzielung einer Verbindung der Formel I, worin L $OC(O)R_{11}$ ist;

(i) Reaktion einer Verbindung der Formel I, worin L OH ist, mit einem Isocyanat der allgemeinen Formel

$$R_{12}NCO$$

worin $R_{12}$ wie vorstehend definiert ist, unter Erzielung einer Verbindung der Formel I, worin L

$$OC(O)NHR_{12} \text{ ist;}$$

(j) Reaktion einer Verbindung der Formel I, worin L OH ist, mit einem Chlorformiat der allgemeinen Formel

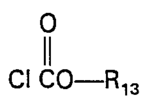

worin R wie vorstehend definiert ist, unter Erzielung einer Verbindung der Formel I, worin L $OC(O)OR_{13}$ ist;

(k) Reduktion einer Verbindung der allgemeinen Formel

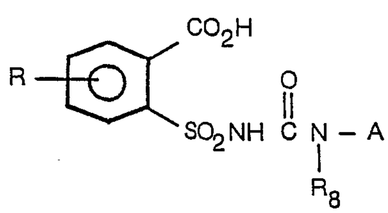

oder eines Esters davon, unter Erzielung einer Verbindung der Formel I, worin $R_1$ und $R_2$ sind und R, $R_8$ und A wie vorstehend definiert sind;

(1) Reduktion eines Ketons der allgemeinen Formel

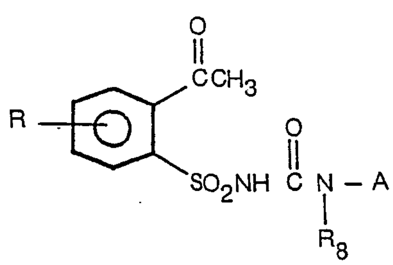

worin R, $R_8$ und A wie vorstehend definiert sind, unter Erzielung einer Verbindung der Formel I, worin $R_1$ H ist, $R_2$ $CH_3$ ist und L OH ist; oder Reaktion dieses Ketons mit Methyllithium, unter Erzielung einer Verbindung der Formel I, worin $R_1$ und $R_2$ $CH_3$ sind und L OH ist, wobei die Substituentengruppen kein ersetzbares Halogen, $NO_2$ oder CN enthalten; odert

(m) Reaktion einer Verbindung der allgemeinen Formel

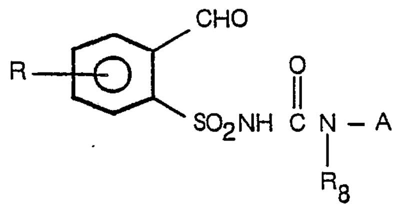

worin R, $R_8$ und A wie vorstehend definiert sind, mit der Maßgabe, daß sie keine ersetzbaren Halogene, $NO_2$ oder CN enthalten, mit Methyllithium, unter Erzielung einer Verbindung der Formel I, worin $R_1$ H ist, $R_2$ $CH_3$ ist und L OH ist.

2. Verfahren nach Anspruch 1, worin R, $R_1$ und $R_2$ wie in Anspruch 1 definiert sind, W O ist, $R_8$ H ist, A

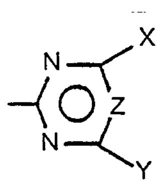
oder
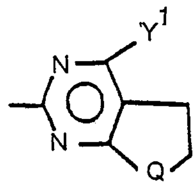

ist
worin Q, X, Y und Z wie in Anspruch 1 definiert sind und
$Y^1$ H, $CH_3$ oder $OCH_3$ ist; und
L $CO_2R_{10}$, $CONR_3R_4$ oder CN ist, worin $R_{10}$ $C_1$—$C_4$-Alkyl ist; und
$R_3$ und $R_4$ unabhängig H oder $C_1$—$C_4$-Alkyl sind.

3. Verfahren nach Anspruch 1, R wie in Anspruch 1 definiert ist; $R_1$ H oder $CH_3$ ist; $R_2$ H oder $CH_3$ ist; L $SO_2NR_3R_4$ ist, worin $R_3$ und $R_4$ wie in Anspruch 1 definiert sind; $R_8$ H ist; W O ist; und A wie in Anspruch 2 definiert ist.

4. Verfahren nach Anspruch 1, worin R wie in Anspruch 1 definiert ist; $R_1$ H oder $C_1$—$C_4$-Alkyl ist; $R_2$ H oder $CH_3$ ist; $R_8$ H ist; L $S(O)_nR_7$ oder $OR_9$ ist; $R_7$ und n wie in Anspruch 1 definiert sind; $R_9$ $C_1$—$C_4$-Alkyl, $C_3$—$C_4$-Alkenyl ist; A wie in Anspruch 2 definiert ist; und W O ist.

5. Verfahren nach Anspruch 1, worin R wie in Anspruch 1 definiert ist; $R_1$ und $R_2$ unabhängig H oder $CH_3$ sind; $R_8$ H ist; L OH, $OC(O)R_{11}$, $OC(O)NHR_{12}$ oder $OC(O)OR_{13}$ ist; W O ist; und A wie in Anspruch 2 definiert ist.

6. Verfahren nach Anspruch 1, worin R, $R_1$ und $R_2$ wie in Anspruch 1 definiert sind; L Cl, Br, $NR_3R_4$, $\overset{+}{N}R_3R_4R_4'$, $N(R_4)C(O)R_5$, $N(R_4)C(O)NHR_6$ oder $N(R_4)C(O)R_7$, ist; $R_8$ H ist; W O ist; und A wie in Anspruch 2 definiert ist.

7. Verfahren nach einem der vorhergehenden Ansprüche, worin Z N, CH, CCl, CBr oder $CCH_3$ ist; W O ist; und $R_8$ H oder $CH_3$ ist.

8. Verfahren nach einem der Ansprüche 1 bis 6, worin L OH ist; R H ist; $R_1$ und $R_2$ beide $CH_3$ sind; $R_8$ H oder $CH_3$ ist;

A 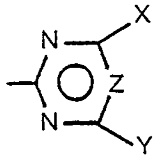 ;

ist; Z CH oder N ist; X und Y unabhängig $CH_3$ oder $OCH_3$ sind; und W O ist.

9. Verfahren nach Anspruch 7, L, Cl, Br, OH, $NR_3R_4$, $+NR_3R_4R_4'$, $CO_2R_{10}$, $S(O)_nR_7$, $OR_9$, $SO_2NR_3R_4$, $OC(O)R_{11}$, $OC(O)NHR_{12}$, $OC(O)(OR_{13}$ ist; Z CH oder N ist; X $CH_3$ oder $OCH_3$ ist; R, $R_1$, $R_2$ H sind; und wenn n 1 oder 2 ist, Z CH ist.

10. Verfahren nach Anspruch 9,

worin A 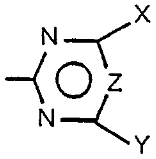 ;

ist; $R_8$ H ist; L Cl, Br, $NR_3R_4$, $CO_2R_{10}$, $S(O)_nR_7$, $OR_9$, $SO_2NR_3R_4$, OH, $OC(O)R_{11}$, $OC(O)NHR_{12}$, $OC(O)OR_{13}$ ist; und $R_{11}$, $R_{12}$ und

$R_{13}$ $C_1$—$C_3$-Alkyl sind.

11. Verfahren nach Anspruch 10, worin L, Cl, Br, $NR_3R_4$, $CO_2R_{10}$, $S(O)_nR_7$, $OR_9$, $SO_2NR_3R_4$ oder OH ist.

12. Verfahren nach Anspruch 11, worin $R_3$ $C_1$—$C_3$-Alkyl oder $OCH_3$ ist; $R_4$ $CH_3$ ist; $R_9$ $C_1$—$C_4$-Alkyl ist; und $R_{10}$ $CH_3$ oder $CH_2CH_3$ ist.

13. Verfahren nach Anspruch 12, worin $R_7$ und $R_9$ $CH_3$ sind.

14. Verfahren nach Anspruch 13, worin L $CO_2R_{10}$, $S(O)_nR_7$ oder $OR_9$ ist.

15. Verfahren nach Anspruch 1, worin das Produkt eine Verbindung ist, ausgewählt aus:
2-(Dichlormethyl)-N-[(4-methoxy-6-methylpyrimidin-2-yl)-aminocarbonyl]-benzolsulfonamid;
2-[[(4-Methoxy-6-methylpyrimidin-2-yl)-aminocarbonyl]-aminosulfonyl]-benzol-essigsäure-methylester;
2-[[(4-Methoxy-6-methylpyrimidin-2-yl)-aminocarbonyl]-aminosulfonyl]-benzol-essigsäure-ethylester;
N-[(4-Methoxy-6-methylpyrimidin-2-yl)-aminocarbonyl]-2-(methoxymethyl)-benzolsulfonamid;
N-[(4-Methoxy-6-methylpyrimidin-2-yl)-aminocarbonyl]-2-(methylthiomethyl)-benzolsulfonamid;
N-[(4-Methoxy-6-methylpyrimidin-2-yl)-aminocarbonyl]-2-(methylsulfonylmethyl)-benzolsulfonamid;
N-[(4-Methoxy-6-methylpyrimidin-2-yl)-aminocarbonyl]-(methylsulfinylmethyl)-benzolsulfonamid;
2-[(Dimethylamino)-sulfonylmethyl]-N-[(4-methoxy-6-methylpyrimidin-2-yl)-aminocarbonyl]-benzolsulfonamid;
N-[(4-Methoxy-6-methylpyrimidin-2-yl)-aminocarbonyl]-2-(hydroxymethyl)-benzolsulfonamid;
und die landwirtschaftlich brauchbaren Salze davon.

16. Verfahren nach Anspruch 1, worin das Produkt eine Verbindung ist, ausgewählt aus:
2-(Dichlormethyl)-N-[4,6-dimethylpyrimidin-2-yl)-aminocarbonyl]-benzolsulfonamid;
2-(Chlormethyl)-N-[(4,6-dimethylpyrimidin-2-yl)-aminocarbonyl]-benzolsulfonamid;
2-[[(4,6-Dimethylpyrimidin-2-yl)-aminocarbonyl]-aminosulfonyl]-benzol-essigsäure-methylester;
N-[(4,6-Dimethylpyrimidin-2-yl)-aminocarbonyl]-2-(methoxymethyl)-benzolsulfonamid;
N-[(4,6-Dimethylpyrimidin-2-yl)-aminocarbonyl]-2-(methylthiomethyl)-benzolsulfonamid;
N-[(4,6-Dimethylpyrimidin-2-yl)-aminocarbonyl]-2-(methylsulfinylmethyl)-benzolsulfonamid;
N-[(4,6-Dimethylpyrimidin-2-yl)-aminocarbonyl]-2-(methylsulfonylmethyl)-benzolsulfonamid;
2-[(Dimethylamino)-sulfonylmethyl]-N-[(4,6-dimethylpyrimidin-2-yl)-aminocarbonyl]-benzolsulfonamid;
N-[(4,6-Dimethylpyrimidin-2-yl)-aminocarbonyl]-2-(hydroxymethyl)-benzolsulfonamid;
und den landwirtschaftlich brauchbaren Salzen davon.

17. Verfahren nach Anspruch 1, worin das Produkt eine Verbindung ist, ausgewählt aus:
2-(Chlormethyl)-N-[(4,6-dimethoxypyrimidin-2-yl)-aminocarbonyl]-benzolsulfonamid;
2-(1-Pyrrolidinylmethyl)-N-[4,6-dimethoxypyrimidin-2-yl)-aminocarbonyl]-benzolsulfonamid-hydrochlorid;
2-(1-Pyrrolidinylmethyl)-N-[(4,6-dimethoxypyrimidin-2-yl)-aminocarbonyl]-benzolsulfonamid;
2-[[(4,6-Dimethoxypyrimidin-2-yl)-aminocarbonyl]-aminosulfonyl]-benzol-essigsäure-methylester;
2-[[(4,6-Dimethoxypyrimidin-2-yl)-aminocarbonyl]-aminosulfonyl]-benzol-essigsäure-ethylester;
N-[(4,6-Dimethoxypyrimidin-2-yl)-aminocarbonyl]-2-(methoxymethyl)-benzolsulfonamid;
N-[(4,6-Dimethoxypyrimidin-2-yl)-aminocarbonyl]-2-(methylamino-carbonylmethyl)benzolsulfonamid;
N-[(4,6-Dimethoxypyrimidin-2-yl)-aminocarbonyl]-2-(methylthiomethyl)-benzolsulfonamid;
N-[(4,6-Dimethoxypyrimidin-2-yl)-aminocarbonyl]-2-(methylsulfonylmethyl)-benzolsulfonamid;
N-[(4,6-Dimethoxypyrimidin-2-yl)-aminocarbonyl]-2-(methylsulfinylmethyl)-benzolsulfonamid;
N-[(4,6-Dimethoxypyrimidin-2-yl)-aminocarbonyl]-2-(butoxymethyl)-benzolsulfonamid;
N-[(4,6-Dimethoxypyrimidin-2-yl)-aminocarbonyl]-2-(ethoxymethyl)-benzolsulfonamid;
N-[(4,6-Dimethoxypyrimidin-2-yl)-aminocarbonyl]-2-(1-methylpropyloxymethyl)-benzolsulfonamid;
2-[(Dimethylamino)-sulfonylmethyl]-N-[(4,6-dimethoxypyrimidin-2-yl)-aminocarbonyl]-benzolsulfonamid;
N-[(4,6-Dimethoxypyrimidin-2-yl)-aminocarbonyl]-2-(hydroxymethyl)-benzolsulfonamid;
und den landwirtschaftlich brauchbaren Salzen davon.

18. Verfahren nach Anspruch 1, worin das Produkt eine Verbindung ist, ausgewählt aus:
2-(Dichlormethyl)-N-[(4-methoxy-6-methyl-1,3,5-triazin-2-yl)-aminocarbonyl]-benzolsulfonamid;
2-[[(4-Methoxy-6-methyl-1,3,5-triazin-2-yl)-aminocarbonyl]-aminosulfonyl]-benzol-essigsäure-methylester;
N-[(4-Methoxy-6-methyl-1,3,5-triazin-2-yl)-aminocarbonyl]-2-(methoxymethyl)-benzolsulfonamid;
N-[(4-Methoxy-6-methyl-1,3,5-triazin-2-yl)-aminocarbonyl]-2-(methylthiomethyl)-benzolsulfonamid;

2-[(Dimethylamino)-sulfonylmethyl]-N-[(4-methoxy-6-methyl-1,3,5-triazin-2-yl)-aminocarbonyl]-benzolsulfonamid;

N-[(4-Methoxy-6-methyl-1,3,5-triazin-2-yl)-aminocarbonyl]-2-(hydroxymethyl)-benzolsulfonamid;

und den landwirtschaftlich brauchbaren Salzen davon.

19. Verfahren nach Anspruch 1, worin das Produkt eine Verbindung ist, ausgewählt aus:

2-(Chlormethyl)-N-[(4,6-dimethoxy-1,3,5-triazin-2-yl)-aminocarbonyl]-benzolsulfonamid;

2-(1-Chlorethyl)-N-[(4,6-dimethoxy-1,3,5-triazin-2-yl)-aminocarbonyl]-benzolsulfonamid;

2-[[(4,6-Dimethoxy-1,3,5-triazin-2-yl)-aminocarbonyl]-aminosulfonyl]-benzol-essigsäure-methylester;

N-[(4,6-Dimethoxy-1,3,5-triazin-2-yl)aminocarbonyl]-2-(methoxymethyl)-benzolsulfonamid;

N-[(4,6-Dimethoxy-1,3,5-triazin-2-yl)-aminocarbonyl]-2-(methylthiomethyl)-benzolsulfonamid;

2-[(Dimethylamino)-sulfonylmethyl]-N-[(4,6-dimethoxy-1,3,5-triazin-2-yl)-aminocarbonyl]-benzolsulfonamid;

N-[(4,6-Dimethoxy-1,3,5-triazin-2-yl)-aminocarbonyl]-2-(hydroxymethyl)-benzolsulfonamid;

und den landwirtschaftlich brauchbaren Salzen davon.

20. Verfahren nach Anspruch 1, worin das Produkt eine Verbindung ist, ausgewält aus:

N-[(4,6-Dimethyl-1,3,5-triazin-2-yl)-aminocarbonyl]-2-(methoxymethyl)-benzolsulfonamid;

N-[(4,6-Dimethyl-1,3,5-triazin-2-yl)-aminocarbonyl]-2-(methylthiomethyl)-benzolsulfonamid;

2-[(Dimethylamino)-sulfonylmethyl]-N-[(4,6-dimethyl-1,3,5-triazin-2-yl)-aminocarbonyl]-benzolsulfonamid;

N-[(4,6-Dimethyl-1,3,5-triazin-2-yl)-aminocarbonyl]-2-(hydroxymethyl)-benzolsulfonamid;

und den landwirtschaftlich brauchbaren Salzen davon.

21. Verfahren zur Bekämpfung des Wachstums unerwünschter Vegetation durch Auftrag auf den Ort einer solchen Vegetation von einer wirksamen Menge einer herbiziden Verbindung, dadurch gekennzeichnet, daß die herbizide Verbindung eine Verbindung der Formel I, wie in einem der Ansprüche 1 bis 20 definiert, umfaßt.

22. Verfahren zur Regulierung des Pflanzenwachstums durch Auftrag auf den Ort dieser Pflanzen von einer wirksamen, jedoch im wesentlichen nicht-phytotoxischen, Menge eines das Pflanzenwachstum regulierenden Mittels, dadurch gekennzeichnet, daß das das Pflanzenwachstum regulierende Mittel eine Verbindung der Formel I, wie in einem der Ansprüche 1 bis 20 definiert, umfaßt.

23. Verfahren zur Herstellung einer Verbindung ausgewählt aus

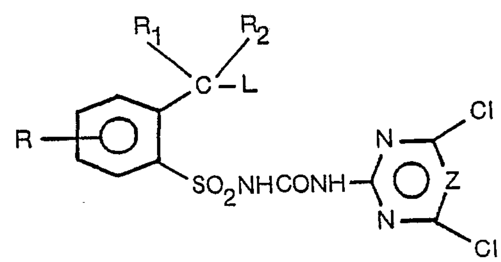

(II)

worin R,

$R_1$ und $R_2$ wie vorstehend in Anspruch 1 definiert sind;

Z CH oder N ist;

n O oder 2 ist; und

L wie vorstehend in Anspruch 1 definiert ist,

vorausgesetzt daß:

(1) L nicht OH ist;

(2) wenn $R_3$ $OCH_3$ ist, dann $R_4$ $CH_3$ ist und daß $R_{10}$ nicht H sein kann;

(3) wenn $R_1$ Cl ist, dann L Cl oder Br ist und $R_2$ H ist;

(4) wenn L $NR_3R_4$, $NR_3R_4R_4'$ oder $SO_2NR_3R_4$ ist, dann $R_3$ oder $R_4$ nicht H sein können;

(5) die Gesamtanzahl der Kohlenstoffatome von $R_3$ und $R_4$ 5 oder weniger ist.

durch Reaktion eines aromatischen Sulfonamids der Formel

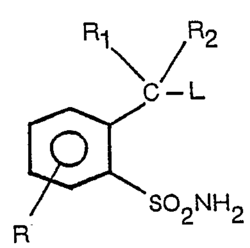

(V)

mit einem heterocyclischen Isocyanat der Formel

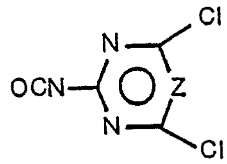

worin

$R$, $R_1$, $Z$ und $L$ wie vorstehend definiert sind.

24. Verfahren zur Herstellung einer Verbindung ausgewählt aus

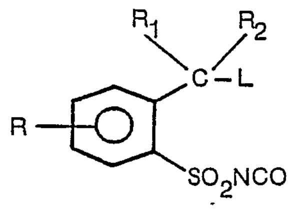

(III)

worin

$R$, $R_1$, $R_2$, $R_7$ und $R_9$ wie vorstehend in Anspruch 1 definiert sind;

n 0 oder 2 ist;

L Cl, Br, $CO_2R_{10}$, $SO_2NR_3R_4$, $S(O)_nR_7$ oder $OR_9$ ist,

vorausgesetzt daß:

(1) wenn $R_1$ Cl ist, dann L Cl oder Br ist und $R_2$ H ist;

(2) wenn L $SO_2NR_3R_4$ ist, dann $R_3$ und $R_4$ nicht H sein können;

(3) wenn $R_3$ $OCH_3$ ist, dann $R_4$ $CH_3$ ist;

(4) $R_{10}$ nicht H sein kann; und

(5) n 0 oder 2 ist.

durch

(a) Reaktion eines Sulfonamids der Formel

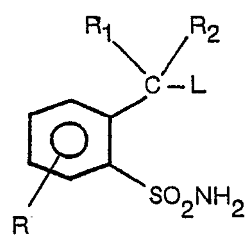

(V)

oder eines Harnstoffs der Formel

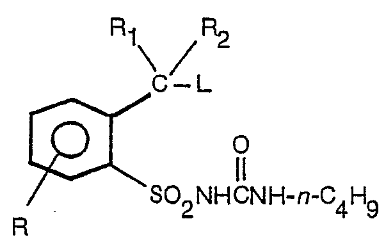

(VI)

mit Phosgen; oder

(b) Reaktion einer Verbindung der Formel

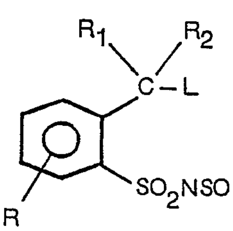

mit Phosgen;

wobei $R$, $R_1$, $R_2$ und L wie vorstehend definiert sind.

**Revendications pour les Etats contractants: BE CH DE FR GB IT LI LU NL SE**

1. Un composé choisi parmi:

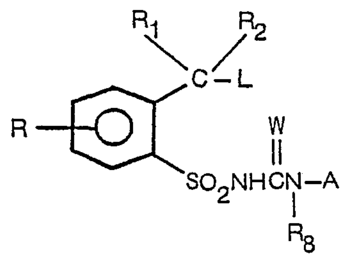

où:

L est $CO_2R_{10}$, $CONR_3R_4$, CN, Cl, Br, $NR_3R_4$, $\overset{+}{N}R_3R_4R_4'$, $N(R_4)C(O)R_5$, $N(R_4)C(O)NHR_6$, $N(R_4)C(O)OR_7$, $S(O)_nR_7$, $OR_9$, $SO_2NR_3R_4$, OH,$OC(O)R_{11}$, $OC(O)NHR_{12}$ ou $OC(O)OR_{13}$;

R est H, F, Cl, Br, $NO_2$, $CF_3$, un radical alcoyle en $C_1$—$C_3$ ou alcoxy en $C_1$—$C_3$;

$R_1$ est H, Cl ou un radical alcoyle en $C_1$—$C_4$;

$R_2$ est H ou $CH_3$;

$R_3$ est H, un radical alcoyle en $C_1$—$C_4$ ou $OCH_3$;

$R_4$ est H ou un radical alcoyle en $C_1$—$C_4$;

$R_3$ et $R_4$ puevent être pris ensemble pour former $-(CH_2)_4-$, $-(CH_2)_5-$ ou $-(CH_2CH_2)_2O$;

$R_4'$ est H, $CH_3$ ou $CH_2CH_3$;

$R_5$ est un radical alcoyle en $C_1$—$C_4$ éventuellement substitué par 1—3 atomes de F, Cl ou Br, ou un radical alcényle en $C_3$—$C_4$;

$R_6$ est H, un radical alcoyle en $C_1$—$C_4$ ou alcényle en $C_3$—$C_4$;

$R_7$ est un radical alcoyle en $C_1$—$C_4$;

$R_8$ est H, $CH_3$ ou $OCH_3$;

$R_9$ est un radical alcoyle en $C_1$—$C_6$, alcényle en $C_3$—$C_4$, $CH_2CF_3$, $CF_2CF_2H$ ou cycloalcoyle en $C_5$—$C_6$;

$R_{10}$ est H, un radical alcoyle en $C_1$—$C_4$, alcényle en $C_3$—$C_4$, $CH_2CH_2Cl$ ou $CH_2CH_2OCH_3$;

$R_{11}$ est H, un radical alcoyle en $C_1$—$C_5$, alcényle en $C_2$—$C_3$, alcynyle en $C_2$—$C_3$, cycloalcoyle en $C_3$—$C_4$,

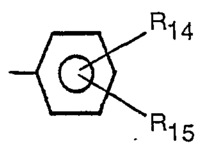
,

un radical alcoyle en $C_1$—$C_4$ substitué par 1—4 substituants choisis parmi 0—3 F, 0—3 Cl ou 0—3 Br,
ou un radical alcényle en $C_2$—$C_3$ substitué par 1—3 Cl;

$R_{12}$ est H, un radical alcoyle en $C_1$—$C_6$, alcényle en $C_3$—$C_4$, cycloalcoyle en $C_5$—$C_6$,

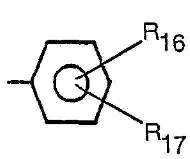
,
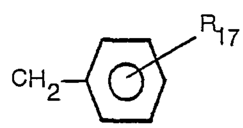

ou cycloalcoyle en $C_5$—$C_6$ substitué par $CH_3$;

$R_{13}$ est un radical acoyle en $C_1$—$C_6$ ou 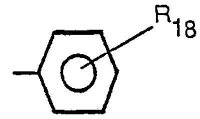 ;

$R_{14}$ et $R_{15}$ sont indépendamment H, $NO_2$, $CH_3$, Cl ou $OCH_3$;

$R_{16}$ est H, F, Cl, Br, un radical alcoyle en $C_1$—$C_3$, $NO_2$, CN, $SO_2CH_3$, $OCH_3$, $SCH_3$ ou $CF_3$;

$R_{17}$ est H, Cl ou un radical alcoyle en $C_1$—$C_3$;

$R_{18}$ est H, $CH_3$ ou Cl;

n est 0, 1 ou 2;

A est 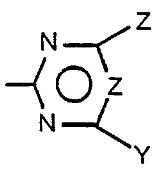 , 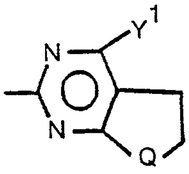 ou 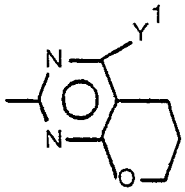

W est O ou S;
X est H, Cl, Br, $CH_3$, $CH_2CH_3$, un radical alcoxy en $C_1$—$C_3$, $CF_3$, $SCH_3$ ou $CH_2OCH_3$;
Y est $CH_3$ ou $OCH_3$;
Z est N, CH, CCl, CBr, CCN, $CCH_3$, $CCH_2CH_3$, $CCH_2CH_2Cl$ ou $CCH_2CH{=}CH_2$;
$Y^1$ est H, $CH_3$, $OCH_3$ ou $OCH_2CH_3$; et
Q est O ou $CH_2$;

et leurs sels acceptables en agriculture; avec les conditions que:
(1) quand $R_1$ est Cl, alors L est Cl ou Br et $R_2$ est H;
(2) quand n est 1, alors W est O;
(3) quand L est $CONR_3R_4$, alors Z est CH ou N;
(4) quand $R_3$ est $OCH_3$, alors $R_4$ est $CH_3$;
(5) le nombre total d'atomes de carbone de $R_3$ et de $R_4$ est de cinq ou moins;
(6) quand W est S, alors $R_8$ est H; et
(7) quand L est $SO_2NR_3R_4$, alors $R_3$ et $R_4$ ne sont pas H.

2. Un composé selon la revendication 1, dans lequel Z est N, CH, CCl, CBr ou $CCH_3$; W est O; et $R_8$ est H ou $CH_3$

3. Un composé selon la revendication 1, dans lequel L est OH; R est H; $R_1$ et $R_2$ sont tous deux $CH_3$; $R_8$ est H ou $CH_3$;

A est 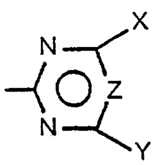 ;

Z est CH ou N; X et Y sont chacun indépendamment $CH_3$ ou $OCH_3$; et W est O.

4. Un composé selon la revendication 2, dans lequel L est Cl, Br, OH, $NR_3R_4$, $+NR_3R_4R_4'$, $CO_2R_{10}$, $S(O)_nR_7$, $OR_9$, $SO_2NR_3R_4$, $OC(O)R_{11}$, $OC(O)NHR_{12}$, $OC(O)OR_{13}$; Z est CH ou N; X est $CH_3$ ou $OCH_3$; R, $R_1$, $R_2$ sont H; et quand n est 1 ou 2, Z est CH.

5. Un composé selon la revendication 4, dans lequel A est

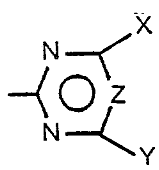 ;

$R_8$ est H; L est Cl, Br, $NR_3R_4$, $CO_2R_{10}$, $S(O)_nR_7$, $OR_9$, $SO_2NR_3R_4$, OH,$OC(O)R_{11}$, $OC(O)NHR_{12}$, $OC(O)OR_{13}$; et $R_{11}$, $R_{12}$ et $R_{13}$ sont des radicaux alcoyle en $C_1$—$C_3$.

6. Un compose selon la revendication 5, dans lequel L est Cl, Br, $NR_3R_4$, $CO_2R_{10}$, $S(O)_nR_7$, $OR_9$, $SO_2NR_3R_4$ ou OH.

7. Un composé selon la revendication 6, dans lequel $R_3$ est un radical alcoyle en $C_1$—$C_3$ ou $OCH_3$; $R_4$ est $CH_3$; $R_9$ est un radical alcoyle en $C_1$—$C_4$; et $R_{10}$ est $CH_3$ ou $CH_2CH_3$.

8. Un composé selon la revendication 7, dans lequel $R_7$ et $R_9$ sont $CH_3$.

9. Un composé selon la revendication 8, dans lequel L est $CO_2R_{10}$, $S(O)_nR_7$ ou $OR_9$.

10. Un composé selon la revendication 1, choisi parmi les suivants:
2-(dichlorométhyl)-N-[(4-méthoxy-6-méthylpyrimidin-2-yl)aminocarbonyl]benzènesulfonamide;
acide 2-[[(4-méthoxy-6-méthylpyrimidin-2-yl)aminocarbonyl]aminosulfonyl]benzèneacétique, ester méthylique;
acide 2-[[(4-méthoxy-6-méthylpyrimidin-2-yl)aminocarbonyl]aminosulfonyl]benzèneacétique, ester éthylique;
N-[(4-méthoxy-6-méthylpyrimidin-2-yl)aminocarbonyl]-2-(méthoxyméthyl)benzènesulfonamide;
N-[(4-méthoxy-6-méthylpyrimidin-2-yl)aminocarbonyl]-2-(méthylthiométhyl)benzènesulfonamide;

N-[(4-méthoxy-6-méthylpyrimidin-2-yl)aminocarbonyl]-2-(méthylsulfonylméthyl)benzènesulfonamide;

N-[(4-méthoxy-6-méthylpyrimidin-2-yl)aminocarbonyl]-2-(méthylsulfinylméthyl)benzènesulfonamide;

2-[(diméthylamino)sulfonylméthyl]-N-[(4-méthoxy-6-méthylpyrimidin-2-yl)aminocarbonyl]-benzènesulfonamide;

N-[(4-méthoxy-6-méthylpyrimidin-2-yl)aminocarbonyl]-2-(hydroxyméthyl)benzènesulfonamide;

et leurs sels acceptables en agriculture.

11. Un composé selon la revendication 1, choisi parmi les suivants:

2-(dichlorométhyl)-N-[(4,6-diméthylpyrimidin-2-yl)aminocarbonyl]benzènesulfonamide;

2-(chlorométhyl)-N-[(4,6-diméthylpyrimidin-2-yl)aminocarbonyl]benzènesulfonamide;

acide 2-[[(4,6-diméthylpyrimidin-2-yl)aminocarbonyl]aminosulfonyl]benzèneacétique, ester méthylique;

N-[(4,6-diméthylpyrimidin-2-yl)aminocarbonyl]-2-(méthoxyméthyl)benzènesulfonamide;

N-[(4,6-diméthylpyrimidin-2-yl)aminocarbonyl]-2-(méthylthiométhyl)benzènesulfonamide;

N-[(4,6-diméthylpyrimidin-2-yl)aminocarbonyl]-2-(méthylsulfinylméthyl)benzènesulfonamide;

N-[(4,6-diméthylpyrimidin-2-yl)aminocarbonyl]-2-(méthylsulfonylméthyl)benzènesulfonamide;

2-[(diméthylamino)sulfonylméthyl]-N-[(4,6-diméthylpyrimidin-2-yl)aminocarbonyl]benzènesulfonamide;

N-[(4,6-diméthylpyrimidin-2-yl)aminocarbonyl]-2-(hydroxyméthyl)benzènesulfonamide;

et leurs sels acceptables en agriculture.

12. Un composé selon la revendication 1, choisi parmi les suivants:

N-[(4,6-diméthoxypyrimidin-2-yl)aminocarbonyl]-2-(méthylaminocarbonylméthyl)benzènesulfonamide;

2-(chlorométhyl)-N-[(4,6-diméthoxypyrimidin-2-yl)aminocarbonyl]benzènesulfonamide;

2-(1-pyrrolidinylméthyl)-N-[(4,6-diméthoxypyrimidin-2-yl)aminocarbonyl]benzènesulfonamide, chlorhydrate;

2-(1-pyrrolidinylméthyl)-N-[(4,6-diméthoxypyrimidin-2-yl)aminocarbonyl]benzènesulfonamide;

acide 2-[[(4,6-diméthoxypyrimidin-2-yl)aminocarbonyl]aminosulfonyl]benzèneacétique, ester méthylique;

acide 2-[[(4,6-diméthoxypyrimidin-2-yl)-aminocarbonyl]aminosulfonyl]benzèneacétique, ester éthylique;

N-[(4,6-diméthoxypyrimidin-2-yl)aminocarbonyl]-2-(méthoxyméthyl)benzènesulfonamide;

N-[(4,6-diméthoxypyrimidin-2-yl)aminocarbonyl]-2-(méthylthiométhyl)benzènesulfonamide;

N-[(4,6-diméthoxypyrimidin-2-yl)aminocarbonyl]-2-(méthylsulfonylméthyl)benzènesulfonamide;

N-[(4,6-diméthoxypyrimidin-2-yl)aminocarbonyl]-2-(méthylsulfinylméthyl)benzènesulfonamide;

N-[(4,6-diméthoxypyrimidin-2-yl)aminocarbonyl]-2-(butoxyméthyl)benzènesulfonamide;

N-[(4,6-diméthoxypyrimidin-2-yl)aminocarbonyl]-2-(éthoxyméthyl)benzènesulfonamide;

N-[(4,6-diméthoxypyrimidin-2-yl)aminocarbonyl]-2-(1-méthylpropyloxyméthyl)benzènesulfonamide;

2-[(diméthylamino)sulfonylméthyl]-N-[(4,6-diméthoxypyrimidin-2-yl)aminocarbonyl]benzènesulfonamide;

N-[(4,6-diméthoxypyrimidin-2-yl)aminocarbonyl]-2-(hydroxyméthyl)benzènesulfonamide;

et leurs sels acceptables en agriculture.

13. Un composé selon la revendication 1, choisi parmi les suivants:

2-(dichlorométhyl)-N-[(4-méthoxy-6-méthyl-1,3,5-triazin-2-yl)aminocarbonyl]benzènesulfonamide;

acide 2-[[(4-méthoxy-6-méthyl-1,3,5-triazin-2-yl)aminocarbonyl]aminosulfonyl]benzèneacétique, ester méthylique;

N-[(4-méthoxy-6-méthyl-1,3,5-triazin-2-yl)aminocarbonyl]-2-(méthylthiométhyl)benzènesulfonamide;

N-[(4-méthoxy-6-méthyl-1,3,5-triazin-2-yl)aminocarbonyl]-2-(méthoxyméthyl)benzènesulfonamide;

2-[(diméthylamine)sulfonylméthyl]-N-[(4-méthoxy-6-méthyl-1,3,5-triazin-2-yl)aminocarbonyl]-benzènesulfonamide;

N-[(4-méthoxy-6-méthyl-1,3,5-triazin-2-yl)aminocarbonyl]-2-(hydroxyméthyl)benzènesulfonamide;

et leurs sels acceptables en agriculture.

14. Un composé selon la revendication 1, choisi parmi les suivants:

2-(chlorométhyl)-N-[(4,6-diméthoxy-1,3,5-triazin-2-yl)aminocarbonyl]benzènesulfonamide;

2-(1-chloroéthyl)-N-[(4,6-diméthoxy-1,3,5-triazin-2-yl)aminocarbonyl]benzènesulfonamide;

acide 2-[[(4,6-diméthoxy-1,3,5-triazin-2-yl)aminocarbonyl]aminosulfonyl]benzèneacétique, ester méthylique;

N-[(4,6-diméthoxy-1,3,5-triazin-2-yl)aminocarbonyl]-2-(méthoxyméthyl)benzènesulfonamide;

N-[(4,6-diméthoxy-1,3,5-triazin-2-yl)aminocarbonyl]-2-(méthylthiométhyl)benzènesulfonamide;

2-[(diméthylamino)sulfonylméthyl]-N-[(4,6-diméthoxy-1,3,5-triazin-2-yl)aminocarbonyl]-benzènesulfonamide;

N-[(4,6-diméthoxy-1,3,5-triazin-2-yl)aminocarbonyl]-2-(hydroxyméthyl)benzènesulfonamide; et leurs sels acceptables en agriculture.

15. Un composé selon la revendication 1, choisi parmi les suivants:

N-[(4,6-diméthyl-1,3,5-triazin-2-yl)aminocarbonyl]-2-(méthoxyméthyl)benzènesulfonamide;

N-[(4,6-diméthyl-1,3,5-triazin-2-yl)aminocarbonyl]-2-(méthylthiométhyl)benzènesulfonamide;

2-[(diméthylamino)sulfonylméthyl]-N-[(4,6-diméthyl-1,3,5-triazin-2-yl)aminocarbonyl]-benzènesulfonamide;

N-[(4,6-diméthyl-1,3,5-triazin-2-yl)aminocarbonyl]-2-(hydroxyméthyl)benzènesulfonamide; et leurs sels acceptables en agriculture.

16. Un composé selon la revendication 1, dans lequel R, $R_1$ et $R_2$ sont tels que définis dans la revendication 1, W est O, $R_8$ est H, A est

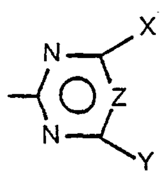 ou 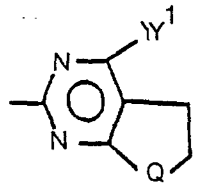

où Q, X, Y et Z sont tels que définis dans la revendication 1 et $Y^1$ est H, $CH_3$ ou $OCH_3$; et

L est $CO_2R_{10}$, $CONR_3R_4$ ou CN,

où $R_{10}$ est un radical alcoyle en $C_1$—$C_4$; et

$R_3$ et $R_4$ sont chacun indépendamment H ou un radical alcoyle en $C_1$—$C_4$.

17. Un composé selon la revendication 1, dans lequel: R est tel que défini dans la revendication 1, $R_1$ est H ou $CH_3$; $R_2$ est H ou $CH_3$; L est $SO_2NR_3R_4$ où $R_3$ et $R_4$ sont tels que définis dans la revendication 1; $R_8$ est H; W est O; et A est tel que défini dans la revendication 16.

18. Un composé selon la revendication 1, dans lequel: R est tel que défini dans la revendication 1, $R_1$ est H ou un radical alcoyle en $C_1$—$C_4$; $R_2$ est H ou $CH_3$; $R_8$ est H; L est $S(O)_nR_7$ ou $OR_9$; $R_7$ et n sont tels que définis dans la revendication 1; $R_9$ est un radical alcoyle en $C_1$—$C_4$, alcényle en $C_3$—$C_4$; A est tel que défini dans la revendication 16; et W est O.

19. Un composé selon la revendication 1, dans lequel: R est tel que défini dans la revendication 1; $R_1$ et $R_2$ sont chacun indépendamment H ou $CH_3$; $R_8$ est H; L est OH, $OC(O)R_{11}$, $OC(O)NHR_{12}$ ou $OC(O)OR_{13}$; W est O; et A est tel que défini dans la revendication 16.

20. Un composé selon la revendication 1, dans lequel: R, $R_1$ et $R_2$ sont tels que définis dans la revendication 1; L est Cl, Br, $NR_3R_4$, $\overset{+}{N}R_3R_4R_4'$, $N(R_4)C(O)R_5$, $N(R_4)C(O)NHR_6$ ou $N(R_4)C(O)R_7$; $R_8$ est H; W est O; et A est tel que défini dans la revendication 16.

21. Un composé choisi parmi:

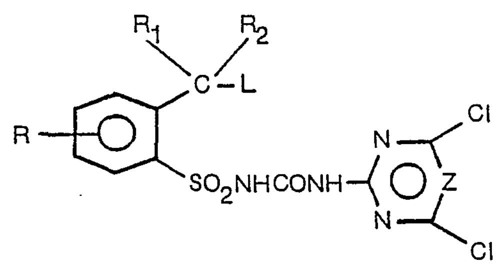

(II)

où

R, $R_1$ et $R_2$ sont tels que définis précédemment dans la revendication 1;

Z est CH ou N;

n est 0 ou 2; et

L est tel que défini précédemment dans la revendication 1, avec les conditions que:

(1) L n'est pas OH;

(2) quand $R_3$ est $OCH_3$, alors $R_4$ est $CH_3$ et $R_{10}$ ne peut pas être H;

(3) quand $R_1$ est Cl, alors L est Cl ou Br et $R_2$ est H;

(4) quand L est $NR_3R_4$, $NR_3R_4R_4'$, ou $SO_2NR_3R_4$, alors $R_3$ ou $R_4$ ne peut pas être H;

(5) le nombre total d'atomes de carbone de $R_3$ et $R_4$ est cinq ou moins.

22. Un composé choisi parmi:

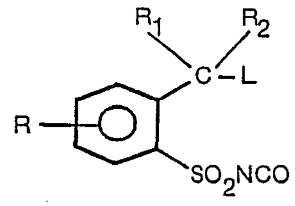

(III)

où:

$R$, $R_1$, $R_2$, $R_7$ et $R_9$ sont tels que définis précédemment dans la revendication 1;
n est 0 ou 2;
L est Cl, Br, $CO_2R_{10}$, $SO_2NR_3R_4$, $S(O)_nR_7$ ou $OR_9$, avec les conditions que:
(1) quand $R_1$ est Cl, alors L est Cl ou Br et $R_2$ est H;
(2) quand L est $SO_2NR_3R_4$, alors $R_3$ et $R_4$ ne peuvent pas être H;
(3) quand $R_3$ est $OCH_3$, alors $R_4$ est $CH_3$;
(4) $R_{10}$ ne peut pas être H; et
(5) n est 0 ou 2.

23. Une composition utilisable pour lutter contre la croissance d'une végétation indésirable, qui comprend une quantité efficace d'un composé herbicide et au moins un des constituants suivants: agent tensio-actif, diluant solide ou liquide, caractérisée en ce que le composé herbicide comprend un composé selon l'une quelconque des revendications 1 à 20.

24. Un procédé pour lutter contre la croissance d'une végétation indésirable en appliquant au site de cette végétation une quantité efficace d'un composé herbicide, caractérisé en ce que le composé herbicide comprend un composé selon l'une quelconque des revendications 1 à 20.

25. Un procédé pour régler la croissance de plantes en appliquant au site de ces plantes une quantité efficace, mais substantiellement non toxique, d'un agent de réglage de la croissance de plantes, caractérisé en ce que l'agent de réglage de la croissance de plantes comprend un composé selon l'une quelconque des revendications 1 à 20.

26. Un procédé pour la préparation d'un composé de la revendication 1, selon lequel on fait réagir ensemble

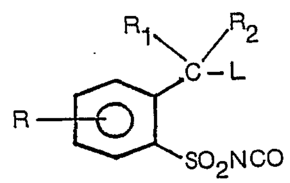

; et

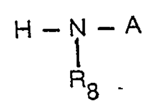

où
L est Cl, Br, $CO_2R_{10}$, $SO_2NR_3R_4$, $S(O)_nR_7$, $OR_9$;
n est 0 ou 2;
$R$, $R_1$, $R_2$, $R_3$, $R_4$, $R_7$, $R_8$, $R_9$, $R_{10}$ et A sont tels que définis dans la revendication 1, à ceci près que $R_3$, $R_4$ et $R_{10}$ ne peuvent pas être H.

**Revendications pour l'Etat contractant: AT**

1. Un procédé pour la préparation d'un composé choisi parmi:

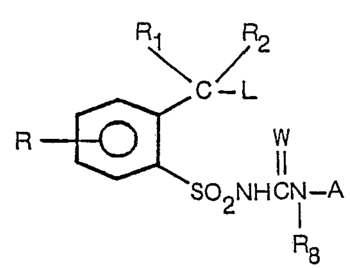

(I)

où:

L est $CO_2R_{10}$, $CONR_3R_4$, CN, Cl, Br, $NR_3R_4$, $\overset{+}{N}R_3R_4R_4'$, $N(R_4)C(O)R_5$, $N(R_4)C(O)NHR_6$, $N(R_4)C(O)OR_7$, $S(O)_nR_7$, $OR_9$, $SO_2NR_3R_4$, OH,$OC(O)R_{11}$, $OC(O)NHR_{12}$ ou $OC(O)OR_{13}$;

R est H, F, Cl, Br, $NO_2$, $CF_3$, un radical alcoyle en $C_1$—$C_3$ ou alcoxy en $C_1$—$C_3$;

$R_1$ est H, Cl ou un radical alcoyle en $C_1$—$C_4$;
$R_2$ est H ou $CH_3$;
$R_3$ est H, un radical alcoyle en $C_1$—$C_4$ ou $OCH_3$;
$R_3$ et $R_4$ peuvent être pris ensemble pour former —$(CH_2)_4$—, —$(CH_2)_5$— ou —$(CH_2CH_2)_2O$;
$R_4'$ est H, $CH_3$ ou $CH_2CH_3$;
$R_5$ est un radical alcoyle en $C_1$—$C_4$ éventuellement substitué par 1—3 atomes de F, Cl ou Br, ou un radical alcényle en $C_3$—$C_4$;
$R_6$ est H, un radical alcoyle en $C_1$—$C_4$ ou alcényle en $C_3$—$C_4$;
$R_7$ est un radical alcoyle en $C_1$—$C_4$;
$R_8$ est H, $CH_3$ ou $OCH_3$;
$R_9$ est un radical alcoyle en $C_1$—$C_6$, alcényle en $C_3$—$C_4$, $CH_2CF_3$, $CF_2CF_2H$ ou cycloalcoyle en $C_5$—$C_6$;
$R_{10}$ est H, un radical alcoyle en $C_1$—$C_4$, alcényle en $C_3$—$C_4$, $CH_2CH_2Cl$ ou $CH_2CH_2OCH_3$;
$R_{11}$ est H, un radical alcoyle en $C_1$—$C_5$, alcényle en $C_2$—$C_3$, alcynyle en $C_2$—$C_3$, cycloalcoyle en $C_3$—$C_4$,

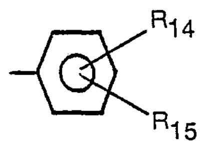,

un radical alcoyle en $C_1$—$C_4$ substitué par 1—4 substituants choises parmi 0—3 F, 0—3 Cl ou 0—3 Br,
ou un radical alcényle en $C_2$—$C_3$ substitué par 1—3 Cl;
$R_{12}$ est H, un radical alcoyle en $C_1$—$C_6$, alcényle en $C_3$—$C_4$, cycloalcoyle en $C_5$—$C_6$,

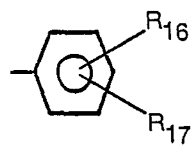, 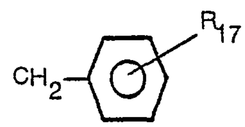

ou cycloalcoyle en $C_5$—$C_6$ substitué par $CH_3$;

$R_{13}$ est un radical alcoyle en $C_1$—$C_6$ ou 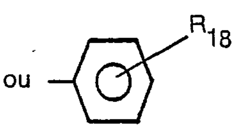 ;

$R_{14}$ et $R_{15}$ sont indépendamment H, $NO_2$, $CH_3$, Cl ou $OCH_3$;
$R_{16}$ est H, F, Cl, Br, un radical alcoyle en $C_1$—$C_3$, $NO_2$, CN, $SO_2CH_3$, $OCH_3$, $SCH_3$ ou $CF_3$;
$R_{17}$ est H, Cl ou un radical alcoyle en $C_1$—$C_3$;
$R_{18}$ est H, $CH_3$ ou Cl;
n est 0, 1 ou 2;

A est 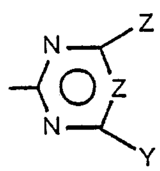, 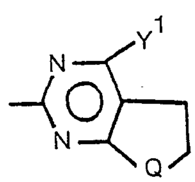 ou 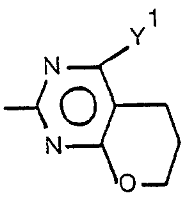

W est O ou S;
X est H, Cl, Br, $CH_3$, $CH_2CH_3$, un radical alcoxy en $C_1$—$C_3$, $CF_3$, $SCH_3$ ou $CH_2OCH_3$;
Y est $CH_3$ ou $OCH_3$;
Z est N, CH, CCl, CBr, CCN, $CCH_3$, $CCH_2CH_3$, $CCH_2CH_2Cl$ ou $CCH_2CH{=}CH_2$;
$Y^1$ est H, $CH_3$, $OCH_3$ ou $OCH_2CH_3$; et
Q est O ou $CH_2$;
et leurs sels acceptables en agriculture; avec les conditions que:
(1) quand $R_1$ est Cl, alors L est Cl ou Br et $R_2$ est H;
(2) quand n est 1, alors W est O;
(3) quand L est $CONR_3R_4$, alors Z est CH ou N;
(4) quand $R_3$ est $OCH_3$, alors $R_4$ est $CH_3$;
(5) le nombre total d'atomes de carbone de $R_3$ et de $R_4$ est de cinq ou moins;

(6) quand W est S, alors $R_8$ est H; et

(7) quand L est $SO_2NR_3R_4$, alors $R_3$ et $R_4$ ne sont pas H, selon lequel

(a) on fait réagir ensemble

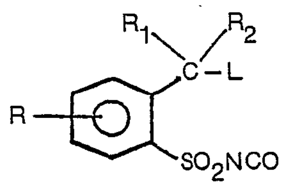

et

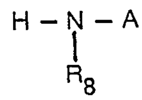

où

L est Cl, Br, $CO_2R_{10}$, $SO_2$, $NR_3R_4$, $S(O)_nR_7$, $OR_9$;

n est 0 ou 2;

R, $R_1$, $R_2$, $R_3$, $R_4$, $R_7$, $R_8$, $R_9$, $R_{10}$ et A sont tels que définis ci-dessus, à ceci près que $R_3$, $R_4$ et $R_{10}$ ne peuvent pas être H;

(b) on oxyde un composé de formule (I) dans lequel n est 0 ou 1 de façon à obtenir un composé dans lequel n est 1 ou 2;

(c) on hydrolyse un composé de formule (I) dans lequel $R_{10}$ est un radical alcoyle en $C_1$—$C_4$ de façon à obtenir un composé dans lequel $R_{10}$ est H;

(d) on fait réagir un composé de formule (I) dans lequel L est $CO_2R_{10}$ (où $R_{10}$ est un radical alcoyle en $C_1$—$C_4$) avec un dérivé de dialcoylaluminium-N-alcoylamide de formule générale:

$$(Alk)_2Al—NR_3R_4$$

où $R_3$ et $R_4$ sont tels que définis ci-dessus et Alk est un groupe alcoyle, de façon à obtenir un composé de formule (I) dans lequel L est $C(O)NR_3R_4$;

(e) on fait réagir un sulfonamide substitué de manière appropriée de formule générale:

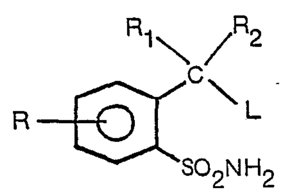

avec le méthylcarbamate de l'aminohétérocycle approprié, inerte dans les conditions de réaction, de formule générale

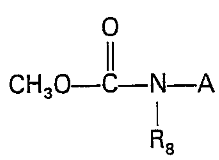

où R, $R_1$, $R_2$, $R_8$, L et A sont des groupes tels que définis ci-dessus;

(f) on déplace un atome de chlore ou les deux dans la portion hétérocyclique d'un composé de formule générale:

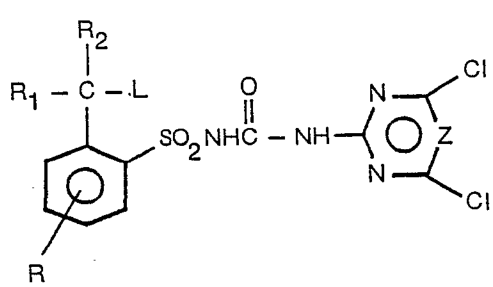

(où R, $R_1$, $R_2$ et L sont tels que définis ci-dessus, et Z est N ou CH) par des groupes alcoxy compris dans les définitions de X et Y ci-dessus;

(g) on fait réagir ensemble des composés des formules générales:

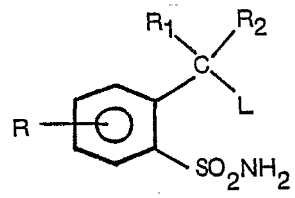 et SCN—A

où R, $R_1$, $R_2$, L et A sont tels que définis ci-dessus, de fa — on à obtenir un composé de formule (I) dans lequel W est S;

(h) on fait réagir un composé de formule (I) dans lequel L est OH avec

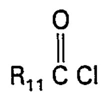

où R est tel que défini ci-dessus de façon à obtenir un composé de formule (I) dans lequel L est $OC(O)R_{11}$;

(i) on fait réagir un composé de formule (I) dans lequel L est OH avec un isocyanate de formule générale:

$$R_{12}NCO$$

où $R_{12}$ est tel que défini ci-dessus, de façon à obtenir un composé de formule (I) dans lequel L est:

$$OC(O)NHR_{12};$$

(j) on fait réagir un composé de formule (I) dans lequel L est OH avec un chloroformiate de formule générale:

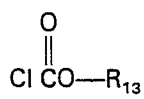

où R est tel que défini ci-dessus, de façon à obtenir un composé de formule (I) dans lequel L est $OC(O)OR_{13}$;

(k) on réduit un composé de formule générale:

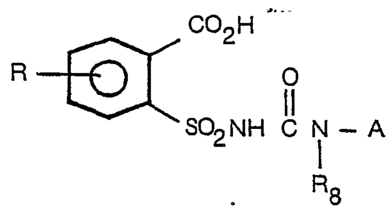

ou un ester d'un tel composé de façon à obtenir un composé de formule (I) dans lequel $R_1$ et $R_2$ sont H et R, $R_8$ et A sont tels que définis ci-dessus;

(l) on réduit une cétone de formule générale:

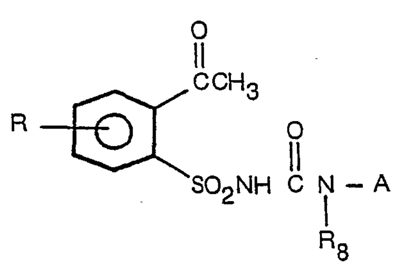

où R, $R_8$ et A sont tels que définis ci-dessus, de façon à obtenir un composé de formule I dans lequel $R_1$ est H,

$R_2$ est $CH_3$ et L est OH; ou on fait réagir cette cétone avec du méthyllithium de façon à obtenir un composé de formule (I) dans lequel

$R_1$ et $R_2$ sont $CH_3$ et L est OH, les groupes substituants ne contenant pas d'halogène déplaçable, $NO_2$ ou CN; ou

(m) on fait réagir un composé de formule générale:

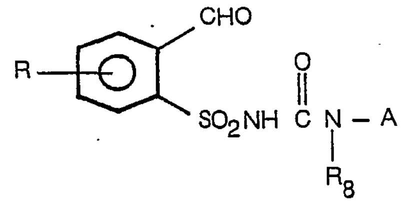

où R, $R_8$ et A sont tels que définis ci-dessus, avec la condition qu'ils ne contiennent pas d'halogènes déplaçables, $NO_2$ ou CN, avec du méthyllithium de façon à obtenir un composé de formule (I) dans lequel $R_1$ est H, $R_2$ est $CH_3$ et L est OH.

2. Un procédé selon la revendication 1, dans lequel R, $R_1$ et $R_2$ sont tels que définis dans la revendication 1, W est O, $R_8$ est H, A est

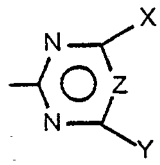

ou

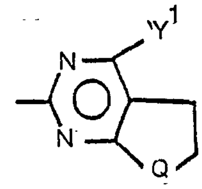

où Q, X, Y et Z sont tels que définis dans la revendication 1 et $Y^1$ est H, $CH_3$ ou $OCH_3$; et

L est $CO_2R_{10}$, $CONR_3R_4$ ou CN,

où $R_{10}$ est un radical alcoyle en $C_1$—$C_4$; et

$R_3$ et $R_4$ sont chacun indépendamment H ou un radical alcoyle en $C_1$—$C_4$.

3. Un procédé selon la revendication 1, dans lequel: R est tel que défini dans la revendication 1, $R_1$ est H ou $CH_3$; $R_2$ est H ou $CH_3$; L est $SO_2NR_3R_4$ où $R_3$ et $R_4$ sont tels que définis dans la revendication 1; $R_8$ est H; W est O; et A est tel que défini dans la revendication 2.

4. Un procédé selon la revendication 1, dans lequel: R est tel que défini dans la revendication 1, $R_1$ est H ou un radical alcoyle en $C_1$—$C_4$; $R_2$ est H ou $CH_3$; $R_8$ est H; L est $S(O)_nR_7$ ou $OR_9$; $R_7$ et n sont tels que définis dans la revendication 1; $R_9$ est un radical alcoyle en $C_1$—$C_4$, alcényle en $C_3$—$C_4$; A est tel que défini dans la revendication 2; et W est O.

5. Un procédé selon la revendication 1, dans lequel: R est tel que défini dans la revendication 1; $R_1$ et $R_2$ sont, indépendamment H ou $CH_3$; $R_8$ est H; L est OH, $OC(O)R_{11}$, $OC(O)NHR_{12}$ ou $OC(O)OR_{13}$; W est O; et A est tel que défini dans la revendication 2.

6. Un procédé selon la revendication 1, dans lequel: R, $R_1$ et $R_2$ sont tels que définis dans la revendication 1; L est Cl, Br, $NR_3R_4$, $\overset{+}{N}R_3R_4R_4'$, $N(R_4)C(O)R_5$, $N(R_4)C(O)NHR_6$ ou $N(R_4)C(O)R_7$; $R_8$ est H; W est O; et A est tel que défini dans la revendication 2.

7. Un procédé selon l'une quelconque des revendications précédentes, dans lequel Z est N, CH, CCl, CBr ou $CCH_3$; W est O; et $R_8$ est H ou $CH_3$

8. Un procédé selon l'une quelconque des revendications 1 à 6, dans lequel L est OH; R est H; $R_1$ et $R_2$ sont tous deux $CH_3$; $R_8$ est H ou $CH_3$;

A est 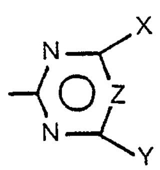 ;

Z est CH ou N; X et Y sont, indépendamment $CH_3$ ou $OCH_3$; et W est O.

9. Un procédé selon la revendication 7, dans lequel L est Cl, Br, OH, $NR_3R_4$, $+NR_3R_4R_4'$, $CO_2R_{10}$, $S(O)_nR_7$, $OR_9$, $SO_2NR_3R_4$, $OC(O)R_{11}$, $OC(O)NHR_{12}$, $OC(O)OR_{13}$; Z est CH ou N; X est $CH_3$ ou $OCH_3$; R, $R_1$, $R_2$ sont H; et quand n est 1 ou 2, Z est CH.

10. Un procédé selon la revendication 9, dans lequel

A est 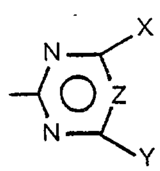 ;

$R_8$ est H; L est Cl, Br, $NR_3R_4$, $CO_2R_{10}$, $S(O)_nR_7$, $OR_9$, $SO_2NR_3R_4$, OH,OC(O)$R_{11}$, OC(O)NH$R_{12}$, OC(O)O$R_{13}$; et $R_{11}$, $R_{12}$ et $R_{13}$ sont des radicaux alcoyle en $C_1$—$C_3$.

11. Un procédé selon la revendication 10, dans lequel L est Cl, Br, $NR_3R_4$, $CO_2R_{10}$, $S(O)_nR_7$, $OR_9$, $SO_2NR_3R_4$ ou OH.

12. Un procédé selon la revendication 11, dans lequel $R_3$ est un radical alcoyle en $C_1$—$C_3$ ou $OCH_3$; $R_4$ est $CH_3$; $R_9$ est un radical alcoyle en $C_1$—$C_4$; et $R_{10}$ est $CH_3$ ou $CH_2CH_3$.

13. Un procédé selon la revendication 12, dans lequel $R_7$ et $R_9$ sont $CH_3$.

14. Un procédé selon la revendication 13, dans lequel L est $CO_2R_{10}$, $S(O)_nR_7$ ou $OR_9$.

15. Un procédé selon la revendication 1, dans lequel le produit est un composé choisi parmi les suivants:

2-(dichlorométhyl)-N-[(4-méthoxy-6-méthylpyrimidin-2-yl)aminocarbonyl]benzènesulfonamide;

acide 2-[[(4-méthoxy-6-méthylpyrimidin-2-yl)aminocarbonyl]aminosulfonyl]benzèneacétique, ester méthylique;

acide 2-[[4-méthoxy-6-méthoxy-6-méthylpyrimidin-2-yl)aminocarbonyl]aminosulfonyl]benzène-acétique, ester éthylique;

N-[(4-méthoxy-6-méthylpyrimidin-2-yl)aminocarbonyl]-2-(méthoxyméthyl)benzènesulfonamide;

N-[(4-méthoxy-6-méthylpyrimidin-2-yl)aminocarbonyl]-2-(méthylthiométhyl)benzènesulfon-amide;

N-[(4-méthoxy-6-méthylpyrimidin-2-yl)aminocarbonyl]-2-(méthylsulfonylméthyl)benzènesulfon-amide;

N-[(4-méthoxy-6-méthylpyrimidin-2-yl)aminocarbonyl]-2-(méthylsulfinylméthyl)benzènesulfon-amide;

2-[(diméthylamino)sulfonylméthyl]-N-[(4-méthoxy-6-méthylpyrimidin-2-yl)aminocarbonyl]-benzènesulfonamide;

N-[(4-méthoxy-6-méthylpyrimidin-2-yl)aminocarbonyl]-2-(hydroxyméthyl)benzènesulfonamide;

et leurs sels acceptables en agriculture.

16. Un procédé selon la revendication 1, dans lequel le produit est un composé choisi parmi les suivants:

2-(dichlorométhyl)-N-[(4,6-diméthylpyrimidin-2-yl)aminocarbonyl]benzènesulfonamide;

2-(chlorométhyl)-N-[(4,6-diméthylpyrimidin-2-yl)aminocarbonyl]benzènesulfonamide;

acide 2-[[(4,6-diméthylpyrimidin-2-yl)aminocarbonyl]aminosulfonyl]benzèneacétique, ester méthylique;

N-[(4,6-diméthylpyrimidin-2-yl)aminocarbonyl]-2-(méthoxyméthyl)benzènesulfonamide;

N-[(4,6-diméthylpyrimidin-2-yl)aminocarbonyl]-2-(méthylthiométhyl)benzènesulfonamide;

N-[(4,6-diméthylpyrimidin-2-yl)aminocarbonyl]-2-(méthylsulfinylméthyl)benzènesulfonamide;

N-[(4,6-diméthylpyrimidin-2-yl)aminocarbonyl]-2-(méthylsulfonylméthyl)benzènesulfonamide;

2-[diméthylamino)sulfonylméthyl]-N-[(4,6-diméthylpyrimidin-2-yl)aminocarbonyl]benzène-sulfonamide;

N-[(4,6-diméthylpyrimidin-2-yl)aminocarbonyl]-2-(hydroxyméthyl)benzènesulfonamide;

et leurs sels acceptables en agriculture.

17. Un procédé selon la revendication 1, dans lequel le produit est un composé choisi parmi les suivants:

2-(chlorométhyl)-N-[(4,6-diméthoxypyrimidin-2-yl)aminocarbonyl]benzènesulfonamide;

2-(1-pyrrolidinylméthyl)-N-[(4,6-diméthoxypyrimidin-2-yl)aminocarbonyl]benzènesulfonamide, chlorhydrate;

2-(1-pyrrolidinylméthyl)-N-[(4,6-diméthoxypyrimidin-2-yl)aminocarbonyl]benzènesulfonamide;

acide 2-[[(4,6-diméthoxypyrimidin-2-yl)aminocarbonyl]aminosulfonyl]benzèneacétique, ester méthylique;

acide 2-[[(4,6-diméthoxypyrimidin-2-yl-aminocarbonyl]aminosulfonyl]benzèneacétique, ester éthylique;

N-[(4,6-diméthoxypyrimidin-2-yl)aminocarbonyl]-2-(méthoxyméthyl)benzènesulfonamide;

N-[(4,6-diméthoxypyrimidin-2-yl)aminocarbonyl]-2-(méthylaminocarbonylméthyl)benzène-sulfonamide;

N-[(4,6-diméthoxypyrimidin-2-yl)aminocarbonyl]-2-(méthylthiométhyl)benzènesulfonamide;

N-[(4,6-diméthoxypyrimidin-2-yl)aminocarbonyl]-2-(méthylsulfonylméthyl)benzènesulfonamide;

N-[(4,6-diméthoxypyrimidin-2-yl)aminocarbonyl]-2-(méthylsulfinylméthyl)benzènesulfonamide;

N-[(4,6-diméthoxypyrimidin-2-yl)aminocarbonyl]-2-(butoxyméthyl)benzènesulfonamide;

N-[(4,6-diméthoxypyrimidin-2-yl)aminocarbonyl]-2-(éthoxyméthyl)benzènesulfonamide;

N-[(4,6-diméthoxypyrimidin-2-yl)aminocarbonyl]-2-(1-méthylpropyloxyméthyl)benzènesulfon-amide;

2-[(diméthylamino)sulfonylméthyl]-N-[(4,6-diméthoxypyrimidin-2-yl)aminocarbonyl))benzêne-sulfonamide;

N-[(4,6-diméthoxypyrimidin-2-yl)aminocarbonyl]-2-(hydroxyméthyl)benzènesulfonamide;

et leurs sels acceptables en agriculture.

18. Un procédé selon la revendication 1, dans lequel le produit est un composé choisi parmi les suivants:

2-(dichlorométhyl)-N-[(4-méthoxy-6-méthyl-1,3,5-triazin-2-yl)aminocarbonyl]benzènesulfonamide;

acide 2-[[(4-méthoxy-6-méthyl-1,3,5-triazin-2-yl)aminocarbonyl]aminosulfonyl]benzèneacétique, ester méthylique;

N-[(4-méthoxy-6-méthyl-1,3,5-triazin-2-yl)aminocarbonyl]-2-(méthoxyméthyl)benzènesulfonamide;

N-[(4-méthoxy-6-méthyl-1,3,5-triazin-2-yl)aminocarbonyl]-2-(méthylthiométhyl)benzènesulfonamide;

2-[(diméthylamino)sulfonylméthyl]-N-[(4-méthoxy-6-méthyl-1,3,5-triazin-2-yl)aminocarbonyl]-benzènesulfonamide;

N-[(4-méthoxy-6-méthyl-1,3,5-triazin-2-yl)aminocarbonyl]-2-(hydroxyméthyl)benzènesulfonamide;

et leurs sels acceptables en agriculture.

19. Un procédé selon la revendication 1, dans lequel le produit est un composé choisi parmi les suivants:

2-(chlorométhyl)-N-[(4,6-diméthoxy-1,3,5-triazin-2-yl)aminocarbonyl]benzènesulfonamide;

2-(1-chloroéthyl)-N-[(4,6-diméthoxy-1,3,5-triazin-2-yl)aminocarbonyl]benzènesulfonamide;

acide 2-[[(4,6-diméthoxy-1,3,5-triazin-2-yl)aminocarbonyl]aminosulfonyl]benzèneacétique, ester méthylique;

N-[(4,6-diméthoxy-1,3,5-triazin-2-yl)aminocarbonyl]-2-(méthoxyméthyl)benzènesulfonamide;

N-[(4,6-diméthoxy-1,3,5-triazin-2-yl)aminocarbonyl]-2-(méthylthiométhyl)benzènesulfonamide;

2-[(diméthylamine)sulfonylméthyl]-N-[(4,6-diméthoxy-1,3,5-triazin-2-yl)aminocarbonyl]benzènesulfonamide;

N-[(4,6-diméthoxy-1,3,5-triazin-2-yl)aminocarbonyl]-2-(hydroxyméthyl)benzènesulfonamide;

et leurs sels acceptables en agriculture.

20. Un procédé selon la revendication 1, dans lequel le produit est un composé choisi parmi les suivants:

N-[(4,6-diméthyl-1,3,5-triazin-2-yl)aminocarbonyl]-2-(méthoxyméthyl)benzènesulfonamide;

N-[(4,6-diméthyl-1,3,5-triazin-2-yl)aminocarbonyl]-2-(méthylthiométhyl)benzènesulfonamide;

2-[(diméthylamino)sulfonylméthyl]-N-[(4,6-diméthyl-1,3,5-triazin-2-yl)aminocarbonyl]benzènesulfonamide;

N-[(4,6-diméthyl-1,3,5-triazin-2-yl)aminocarbonyl]-2-(hydroxyméthyl)benzènesulfonamide;

et leurs sels acceptables en agriculture.

21. Un procédé pour lutter contre le développement d'une végétation indésirable en appliquant au site de cette végétation une quantité efficace d'un composé herbicide, caractérisé en ce que ce composé herbicide comprend un composé de formule (I) tel que défini dans l'une quelconque des revendications 1 à 20.

22. Un procédé pour régler la croissance de plantes en appliquant au site de ces plantes une quantité efficace, mais substantiellement non-phytotoxique, d'un agent de réglage de la croissance de plantes, caractérisé en ce que l'agent de réglage de la croissance des plantes comprend un composé de formule (I) tel que défini dans l'une quelconque des revendications 1 à 20.

23. Un procédé pour la production d'un composé choisi parmi:

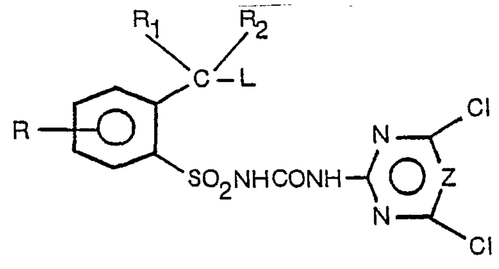

(II)

où

R, $R_1$ et $R_2$ sont tels que définis précédemment dans la revendication 1;

Z est CH ou N;

n est 0 ou 2; et

L est tel que défini précédemment dans la revendication 1, avec les conditions que:

(1) L n'est pas OH;

(2) quand $R_3$ est $OCH_3$, alors $R_4$ est $CH_3$ et $R_{10}$ ne peut pas être H;

(3) quand $R_1$ est Cl, alors L est Cl ou Br et $R_2$ est H;

(4) quand L est $NR_3R_4$, $NR_3R_4R_4'$, ou $SO_2NR_3R_4$, alors $R_3$ ou $R_4$ ne peut pas être H;

(5) le nombre total d'atomes de carbone de $R_3$ et $R_4$ est cinq ou moins; selon lequel on fait réagir un sulfonamide aromatique de formule:

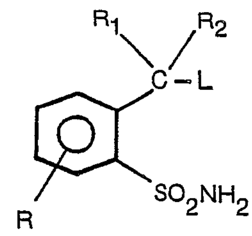

(V)

avec un isocyanate hétérocyclique de formule:

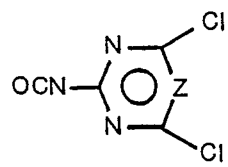

où R, $R_1$, $R_2$, Z et L sont tels que définis ci-dessus.

24. Un procédé pour la production d'un composé choisi parmi:

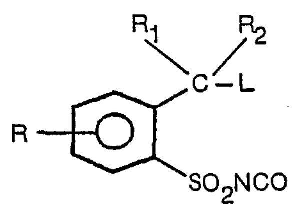

(III)

où:

R, $R_1$, $R_2$, $R_7$ et $R_9$ sont tels que définis précédemment dans la revendication 1;
n est 0 ou 2;
L est Cl, Br, $CO_2R_{10}$, $SO_2NR_3R_4$, $S(O)_nR_7$ ou $OR_9$, avec les conditions que:
(1) quand $R_1$ est Cl, alors L est Cl ou Br et $R_2$ est H;
(2) quand L est $SO_2NR_3R_4$, alors $R_3$ et $R_4$ ne peuvent pas être H;
(3) quand $R_3$ est $OCH_3$, alors $R_4$ est $CH_3$;
(4) $R_{10}$ ne peut pas être H; et
(5) n est 0 ou 2.

selon lequel:

(a) on fait réagir un sulfonamide de formule:

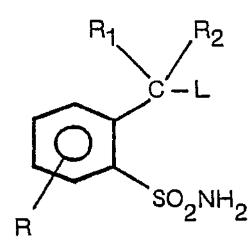

(V)

ou une urée de formule:

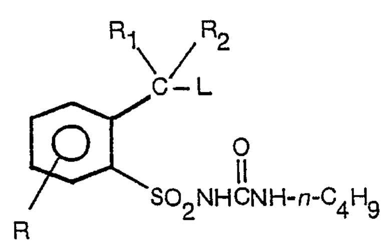

(VI)

avec du phosgène; ou

(b) on fait réagir un composé de formule:

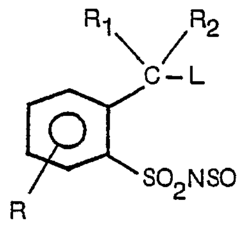

avec du phosgène;

R, $R_1$, $R_2$ et L étant tels que définis ci-dessus.